# EUROPÄISCHE PATENTSCHRIFT

(11) **EP 4 330 257 B1**
(45) Veröffentlichungstag und Bekanntmachung des Hinweises auf die Patenterteilung: **19.03.2025**
(21) Anmeldenummer: 22725835.7
(22) Anmeldetag: 27.04.2022
(51) Int. Cl.: C07D 471/14, C07D 471/22, C07D 487/04, C07D 487/06, C07D 487/08, C07D 487/10, C07D 487/14, C07D 487/16, C07D 487/22, C07D 491/147, C07D 491/16, C07D 491/22, C07D 495/14, C07D 495/22, C07D 519/00, C07F 5/02, H10K 85/60, C07F 7/08

(54) **STICKSTOFFHALTIGE, HETEROCYCLISCHE VERBINDUNGEN FÜR ORGANISCHE ELEKTROLUMINESZENZVORRICHTUNGEN**
NITROGEN-CONTAINING HETEROCYCLIC COMPOUNDS FOR ELECTROLUMINESCENT DEVICES
COMPOSÉS HÉTÉROCYCLIQUES CONTENANT DE L'AZOTE POUR DES DISPOSITIFS ÉLECTROLUMINESCENTS ORGANIQUES

(30) Priorität: 30.04.2021 EP 21171569
(43) Veröffentlichungstag der Anmeldung: 06.03.2024
(73) Patentinhaber: Merck Patent GmbH, 64293 Darmstadt (DE)
(72) Erfinder: STOESSEL, Philipp, 64293 DARMSTADT (DE); PARHAM, Amir Hossain, 64293 DARMSTADT (DE); LINGE, Rouven, 64293 DARMSTADT (DE)
(74) Vertreter: Merck Patent Association
(86) Internationale Anmeldenummer: PCT/EP2022/061126
(87) Internationale Veröffentlichungsnummer: WO 2022/229234

(56) Entgegenhaltungen:
- US-A1- 2019 074 445
- PAVLOPOULOS THEODORE G ET AL: "Triplet-triplet absorption and polarization spectra of some syn-dioxabimanes", SPETROCHIMICA ACTA, vol. 45, no. 10, 1 January 1989 (1989-01-01), pages 1057 - 1065, XP055937037, Retrieved from the Internet <URL:https://reader.elsevier.com/reader/sd/pii/0584853989800698?token=2B7647243BAA614032B54B130A75B9BDB22107DFC453FD817123D208800265609CD352E32DD71AB442ADB3E319DD2B89&originRegion=eu-west-1&originCreation=20220630080955>
- TOMIOKA HIDEO ET AL: "Synthesis and Photochemistry of 5,7-Bis(diazo)-1,2,3,4-dibenzocyclohepta-1,3-dien-6-one. Generation and Reactions of Phenanthrenodiazacyclopentadiene, Phenanthrenocyclopropenone, and 9,10-Phenanthryne", THE JOURNAL OF ORGANIC CHEMISTRY, vol. 60, no. 8, 1 April 1995 (1995-04-01), pages 2344 - 2352, XP055937040, ISSN: 0022-3263, DOI: 10.1021/jo00113a011
- LAU C M ET AL: "BIMANES (1,5-DIAZABICYCLOÄ3.3.0ÜOCTADIENEDIONES): LASER ACTIVITY IN SYN-BIMANES", HETEROATOM CHEMISTRY, JOHN WILEY & SONS, INC, US, vol. 1, no. 3, 1 January 1990 (1990-01-01), pages 195 - 209, XP009035236, ISSN: 1042-7163, DOI: 10.1002/HC.520010302

## Beschreibung

Die vorliegende Erfindung betrifft stickstoffhaltige, heterocyclische heterocyclische Verbindungen für die Verwendung in elektronischen Vorrichtungen, insbesondere in organischen Elektrolumineszenzvorrichtungen, sowie elektronische Vorrichtungen, insbesondere organische Elektrolumineszenzvorrichtungen, enthaltend diese heterocyclischen Verbindungen.

In organischen Elektrolumineszenzvorrichtungen werden als emittierende Materialien häufig phosphoreszierende metallorganische Komplexe oder fluoreszierende Verbindungen eingesetzt. Generell gibt es bei Elektrolumineszenzvorrichtungen immer noch Verbesserungsbedarf.

Aus US 6,322,908, WO 03/001569 A2 und US 2019/0074445 A1 sind polycyclische Verbindungen bekannt, die in organischen Elektrolumineszenzvorrichtungen eingesetzt werden können. Verbindungen gemäß der vorliegenden Erfindung sind nicht offenbart. Weiterhin werden in der Druckschrift J. Org. Chem. 1995, 60, 2344-2352 photochemische Eigenschaften von Verbindungen beschrieben, wobei unter anderem stickstoffhaltige, heterocyclische Verbindungen erhalten werden. Die Synthese von stickstoffhaltigen, heterocyclischen Verbindungen wird in Chin. J. Chem. 2011, 29, 2769 dargelegt. Allerdings wird die Verwendung dieser Verbindungen in organischen Elektrolumineszenzvorrichtungen nicht in den genannten Druckschriften beschrieben.

Generell besteht bei diesen stickstoffhaltigen, heterocyclischen Verbindungen, beispielsweise für die Verwendung als Emitter, insbesondere als fluoreszierender Emitter, noch Verbesserungsbedarf, insbesondere in Bezug auf die Lebensdauer, die Farbreinheit, aber auch in Bezug auf die Effizienz und die Betriebsspannung der Vorrichtung.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung eignen, und welche bei Verwendung in dieser Vorrichtung zu guten Device-Eigenschaften führen, sowie die Bereitstellung der entsprechenden elektronischen Vorrichtung.

Insbesondere ist es die Aufgabe der vorliegenden Erfindung Verbindungen zur Verfügung zu stellen, die zu hoher Lebensdauer, guter Effizienz und geringer Betriebsspannung führen.

Weiterhin sollten die Verbindungen eine ausgezeichnete Verarbeitbarkeit aufweisen, wobei die Verbindungen insbesondere eine gute Löslichkeit zeigen sollten.

Eine weitere Aufgabe der vorliegenden Erfindung kann darin gesehen werden, Verbindungen bereitzustellen, welche sich für den Einsatz in einer phosphoreszierenden oder fluoreszierenden Elektrolumineszenzvorrichtungen eignen, insbesondere als Emitter. Insbesondere ist es eine Aufgabe der vorliegenden Erfindung, Emitter bereitzustellen, welche sich für rote, grüne oder blaue Elektrolumineszenzvorrichtungen eignen.

Weiterhin sollten die Verbindungen, insbesondere bei ihrem Einsatz als Emitter in organischen Elektrolumineszenzvorrichtungen, zu Vorrichtungen führen, die eine ausgezeichnete Farbreinheit aufweisen.

Eine weitere Aufgabe der vorliegenden Erfindung kann darin gesehen werden, Verbindungen bereitzustellen, welche sich für den Einsatz in einer phosphoreszierenden oder fluoreszierenden Elektrolumineszenzvorrichtungen eignen, insbesondere als Matrixmaterial. Insbesondere ist es eine Aufgabe der vorliegenden Erfindung, Matrixmaterialien bereitzustellen, welche sich für rot, gelb und blau phosphoreszierende Elektrolumineszenzvorrichtungen eignen.

Weiterhin sollten die Verbindungen, insbesondere bei ihrem Einsatz als Matrixmaterialien oder als Elektronentransportmaterialien in organischen Elektrolumineszenzvorrichtung zu Vorrichtungen führen, die eine ausgezeichnete Farbreinheit aufweisen.

Eine weitere Aufgabe kann darin gesehen werden, elektronische Vorrichtungen mit einer ausgezeichneten Leistungsfähigkeit möglichst kostengünstig und in konstanter Qualität bereitzustellen

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass bestimmte, unten näher beschriebene Verbindungen diese Aufgabe lösen, sich sehr gut für die Verwendung in Elektrolumineszenzvorrichtungen eignen und zu organischen Elektrolumineszenzvorrichtungen führen, die insbesondere in Bezug auf die Lebensdauer, der Farbreinheit, der Effizienz und der Betriebsspannung sehr gute Eigenschaften vorweisen. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung ist eine Verbindung umfassend mindestens eine Struktur der Formel (I), vorzugsweise eine Verbindung gemäß der Formel (I), 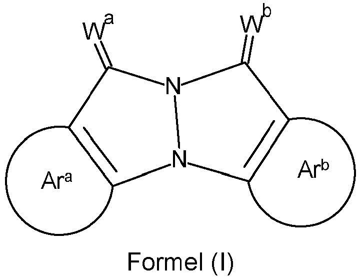
wobei der Ring Ar^{a} bei jedem Auftreten gleich oder verschieden für ein aromatisches oder heteroaromatisches Ringsystem mit 5 bis 60 aromatischen Ringatomen steht, das mit einem oder mehreren Resten Ar oder R^{a} substituiert sein kann, der Ring Ar^{b} bei jedem Auftreten gleich oder verschieden für ein aromatisches oder heteroaromatisches Ringsystem mit 5 bis 60 aromatischen Ringatomen steht, das mit einem oder mehreren Resten Ar oder R^{b} substituiert sein kann;
und für die weiteren verwendeten Symbole und Indizes gilt:
   - W^{a}. W^{b}: ist bei jedem Auftreten gleich oder verschieden O oder S, vorzugsweise O;
   - Ar: ist bei jedem Auftreten gleich oder verschieden ein aromatisches oder heteroaromatisches Ringsystem mit 5 bis 60 aromatischen Ringatomen, das mit einem oder mehreren Resten R substituiert sein kann, hierbei kann die Gruppe Ar mit mindestens einer Gruppe Ar, R, R^{a}, R^{b} oder einer weiteren Gruppe ein Ringsystem bilden;
   - R, R^{a}, R^{b}: ist bei jedem Auftreten gleich oder verschieden H, D, OH, F, Cl, Br, I, CN, NO₂, N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann; oder Heteroarylthiogruppe mit 5 bis 60 aromatischen Ringatomen, die durch einen oder mehrere Reste R¹ substituiert sein kann, oder eine Diarylamino-, Arylheteroarylamino-, Diheteroarylaminogruppe mit 5 bis 60 aromatischen Ringatomen, die durch einen oder mehrere Reste R¹ substituiert sein kann, oder eine Arylalkyl- oder Heteroarylalkylgruppe mit 5 bis 60 aromatischen Ringatomen und 1 bis 10 C-Atomen im Alkylrest, die durch einen oder mehrere Reste R¹ substituiert sein kann; dabei können zwei Reste R, R^{a}, R^{b} auch miteinander oder einer weiteren Gruppe ein Ringsystem bilden;
   - Ar': ist bei jedem Auftreten gleich oder verschieden ein aromatisches oder heteroaromatisches Ringsystem mit 5 bis 60 aromatischen Ringatomen, das mit einem oder mehreren Resten R¹ substituiert sein kann, dabei können zwei Reste Ar', welche an dasselbe C-Atom, Si-Atom, N-Atom, P-Atom oder B-Atom binden, auch durch eine Einfachbindung oder eine Brücke, ausgewählt aus B(R¹), C(R¹)2, Si(R¹)₂, C=O, C=NR¹, C=C(R¹)2, O, S, S=O, SO₂, N(R¹), P(R¹) und P(=O)R¹, miteinander verbrückt sein;
   - R¹: ist bei jedem Auftreten gleich oder verschieden H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch -R²C=CR²-, -C≡C-, Si(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei oder mehrere, vorzugsweise benachbarte Reste R¹ miteinander ein Ringsystem bilden, dabei können einer oder mehrere Reste R¹ mit einem weiteren Teil der Verbindung ein Ringsystem bilden;
   - Ar": ist bei jedem Auftreten gleich oder verschieden ein aromatisches oder heteroaromatisches Ringsystem mit 5 bis 30 aromatischen Ringatomen, das mit einem oder mehreren Resten R² substituiert sein kann, dabei können zwei Reste Ar", welche an dasselbe C-Atom, Si-Atom, N-Atom, P-Atom oder B-Atom binden, auch durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verbrückt sein;
   - R²: ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann, dabei können zwei oder mehrere, vorzugsweise benachbarte Substituenten R² miteinander ein Ringsystem bilden;
wobei Verbindungen der Formeln (A), (B) und (C) 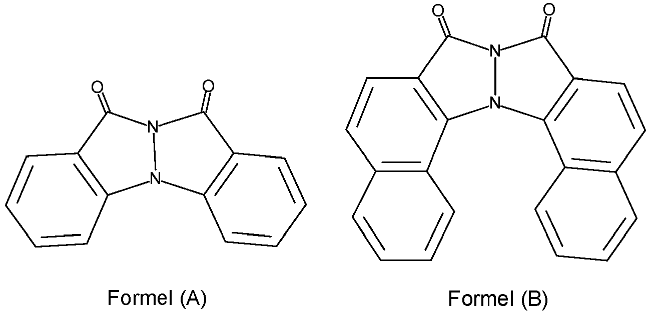 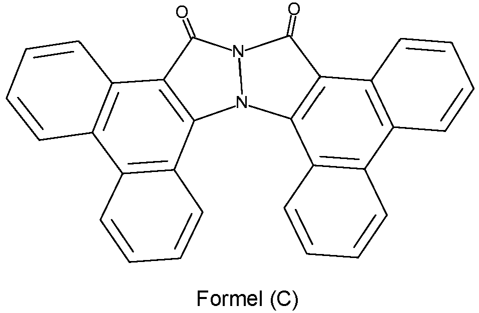
vom Schutz ausgenommen sind.

Verbindungen der Formeln (A), (B) und (C) sind vom Schutz ausgenommen. Die Verbindung (A), welche die CAS-Nr. 125213-40-3 aufweist, die Verbindung (B), welche die CAS-Nr. 131847-09-1 aufweist, und die Verbindung (C), welche die CAS-Nr. 166042-85-9 aufweist, sind aus dem Stand der Technik bekannt, nicht jedoch deren Verwendung in einer elektronischen Vorrichtung.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Eine elektronenarme Heteroarylgruppe im Sinne der vorliegenden Erfindung ist eine Heteroarylgruppe, die mindestens einen heteroaromatischen Sechsring mit mindestens einem Stickstoffatom aufweist. An diesen Sechsring können noch weitere aromatische oder heteroaromatische Fünfringe oder Sechsringe ankondensiert sein. Beispiele für elektronenarme Heteroarylgruppen sind Pyridin, Pyrimidin, Pyrazin, Pyridazin, Triazin, Chinolin, Chinazolin oder Chinoxalin.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Bevorzugt ist das aromatische Ringsystem gewählt aus Fluoren, 9,9'-Spirobifluoren, 9,9-Diarylamin oder Gruppen, in denen zwei oder mehr Aryl- und/oder Heteroarylgruppen durch Einfachbindungen miteinander verknüpft sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 bzw. 5 bis 40 aromatischen Ringatomen, welches noch jeweils mit den oben genannten Resten substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombinationen dieser Systeme.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

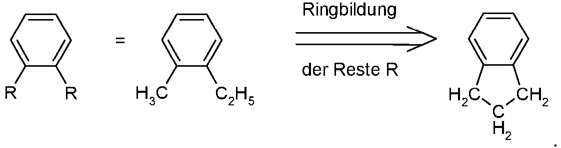

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

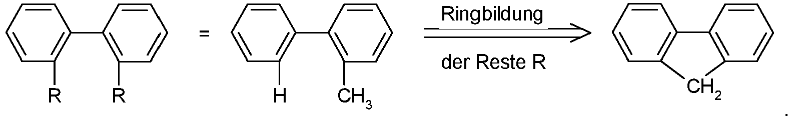

In einer bevorzugten Ausgestaltung können die erfindungsgemäßen Verbindungen eine Struktur der Formeln (II-1) bis (II-42) umfassen, besonders bevorzugt können die erfindungsgemäßen Verbindungen ausgewählt sein aus den Verbindungen der Formeln (II-1) bis (II-42), 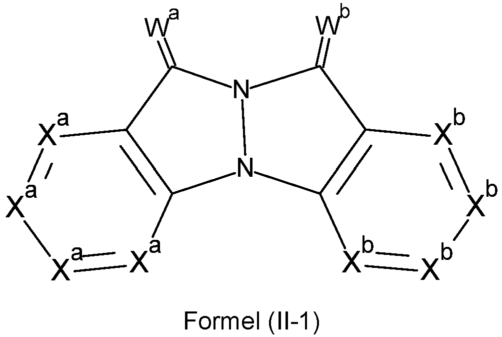 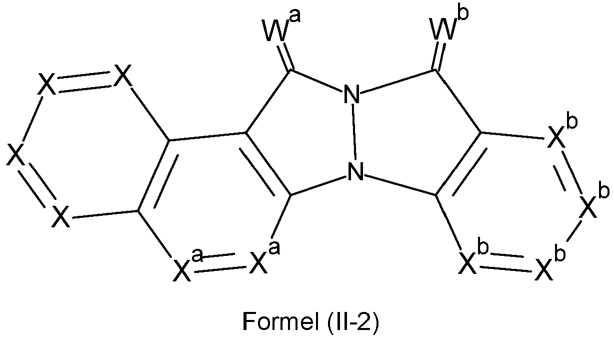 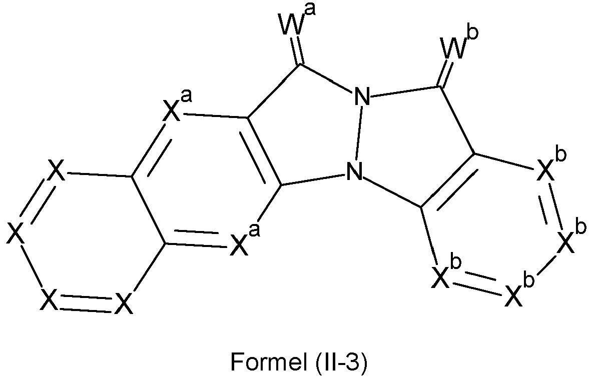 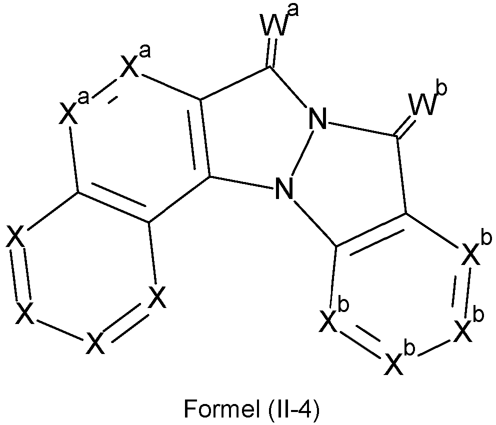 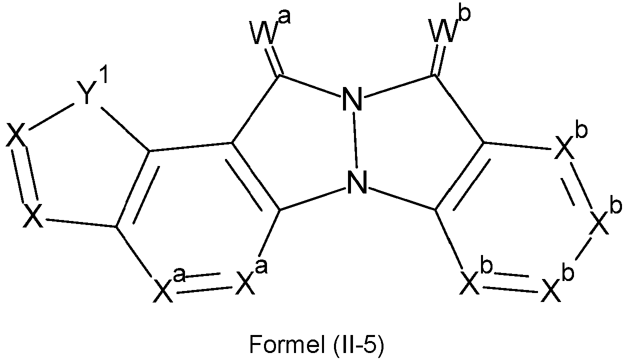 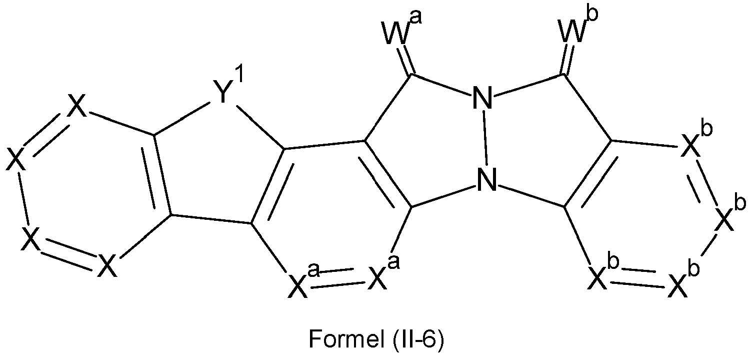 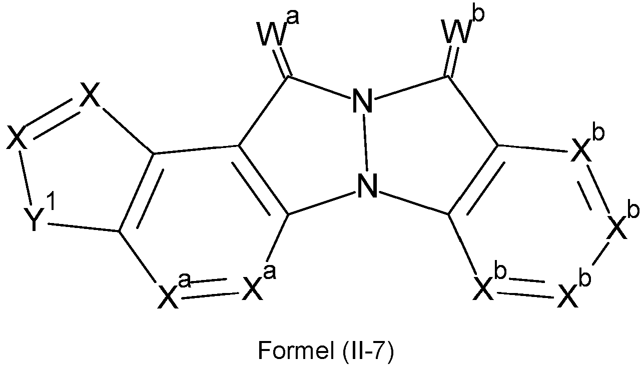 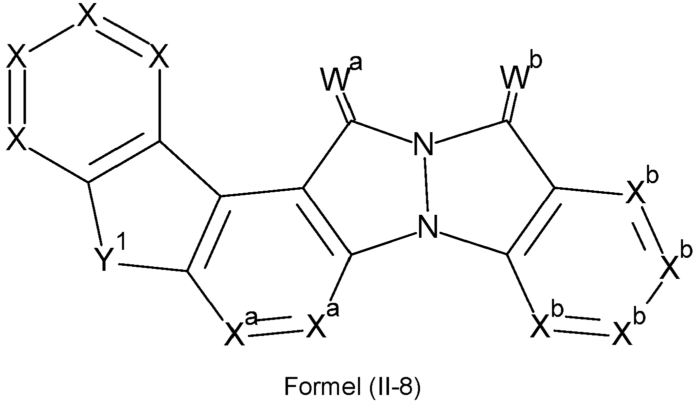 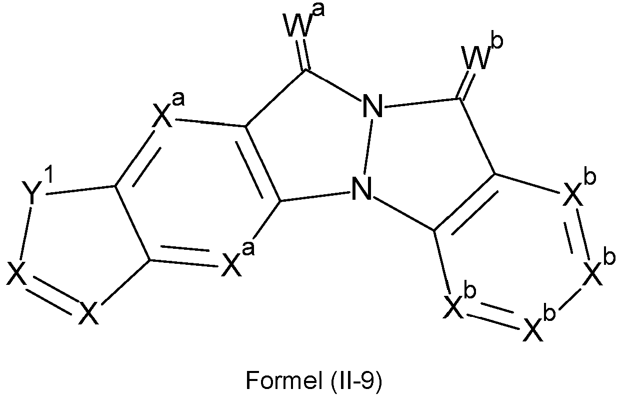 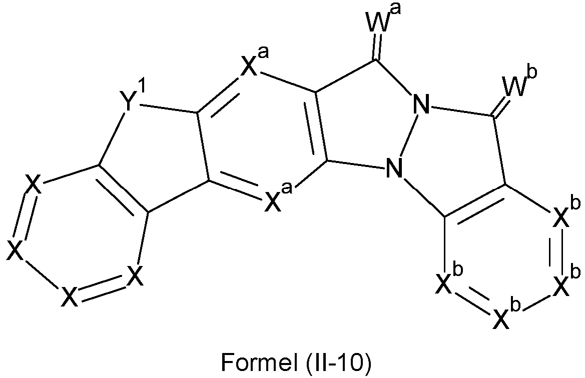 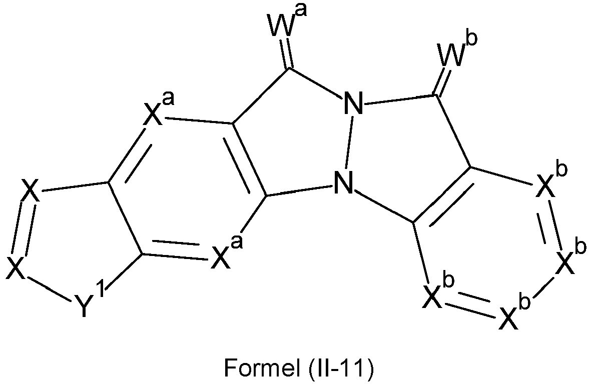 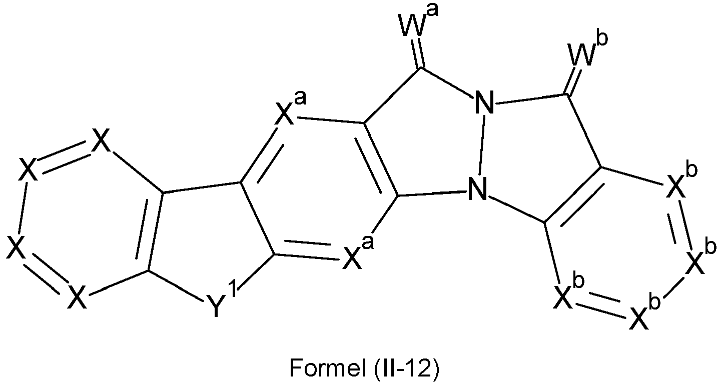 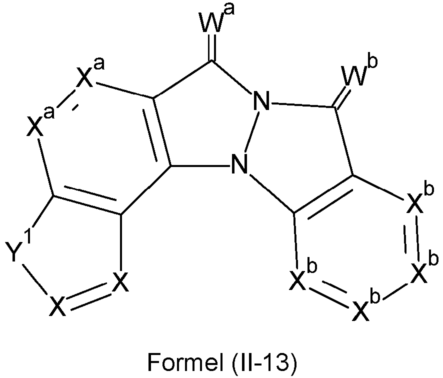 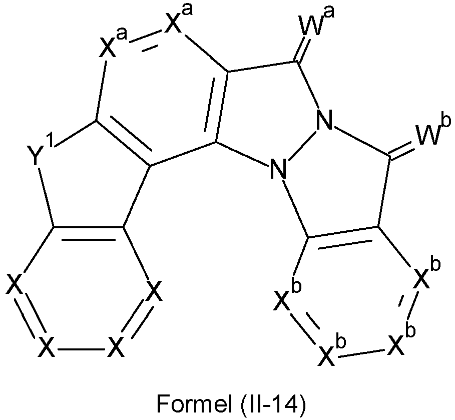 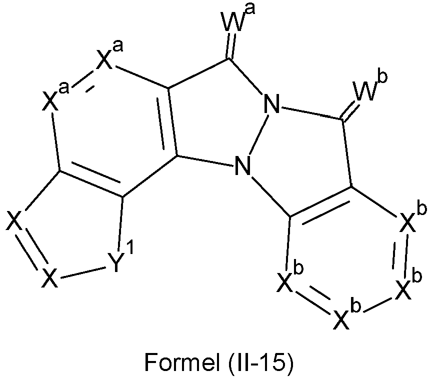 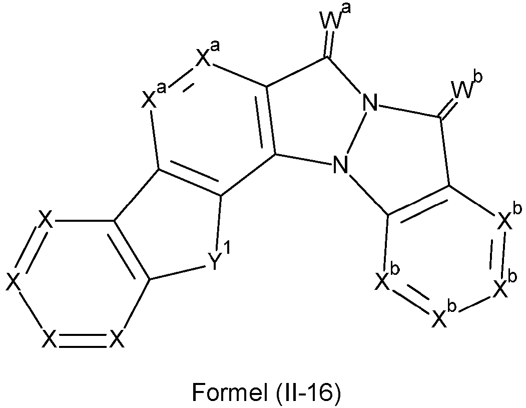 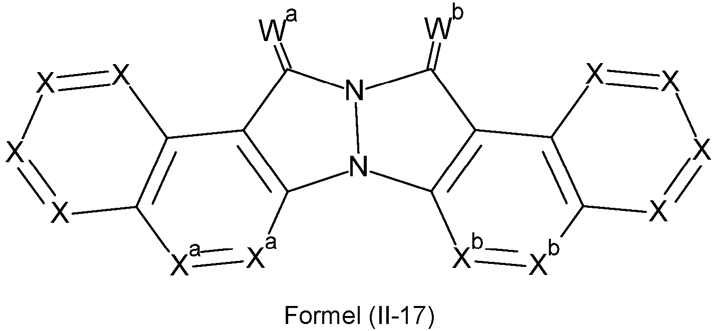 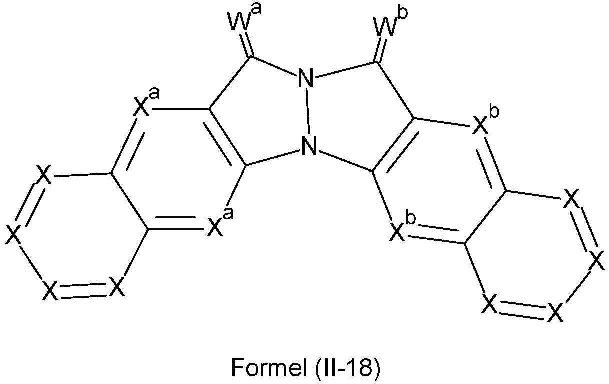 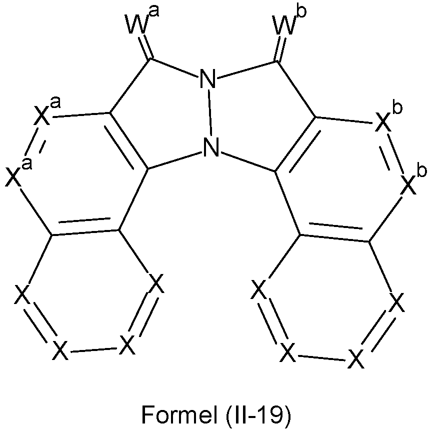 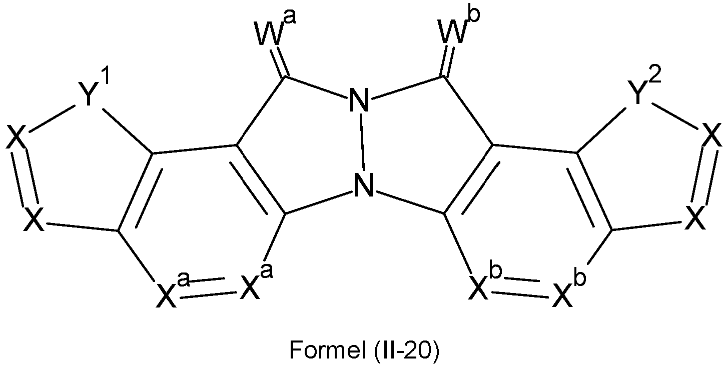 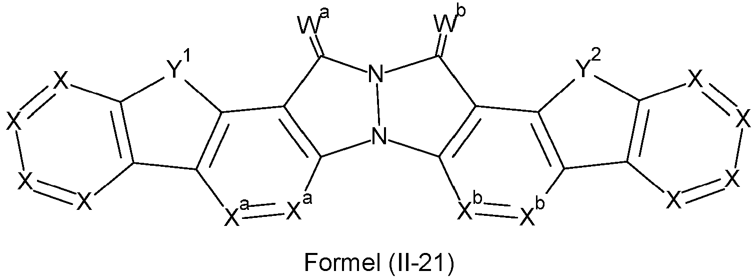 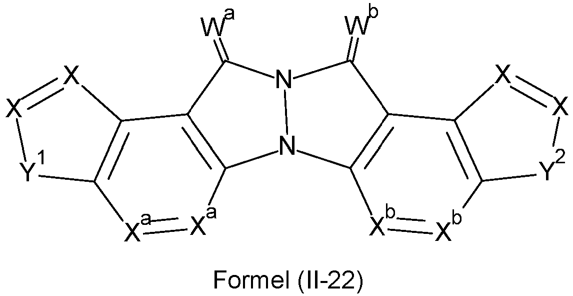 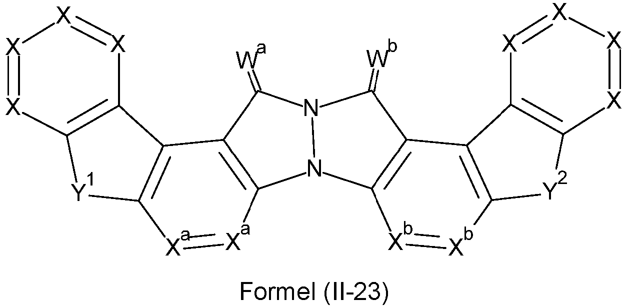 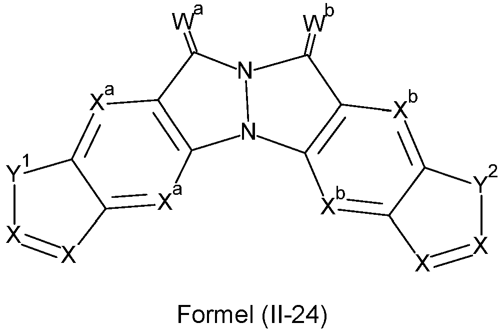 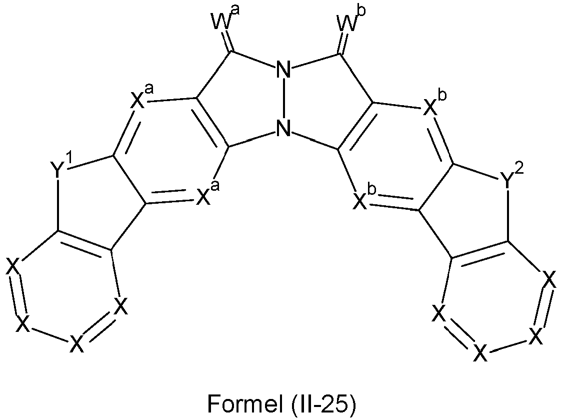 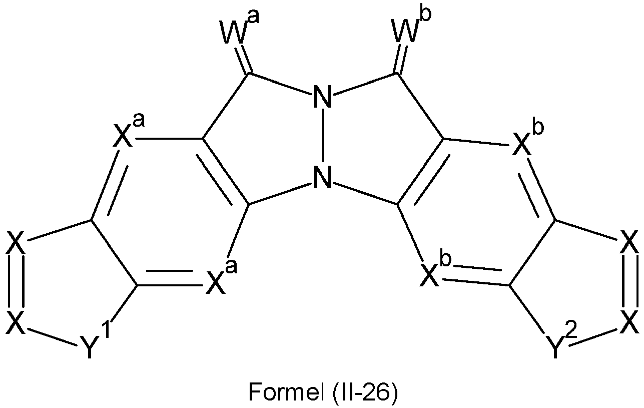 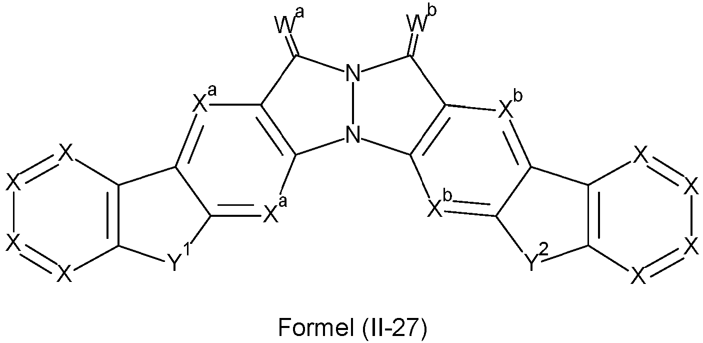 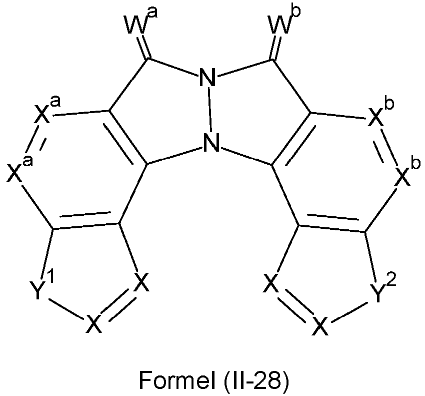 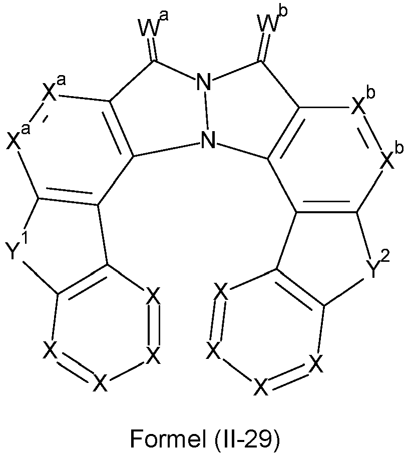 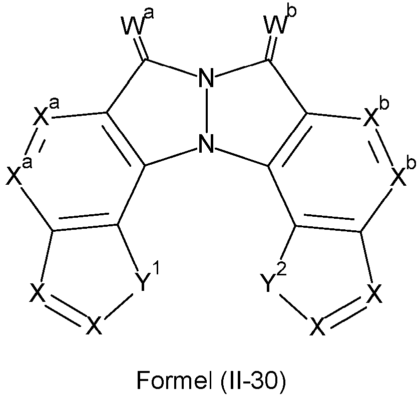 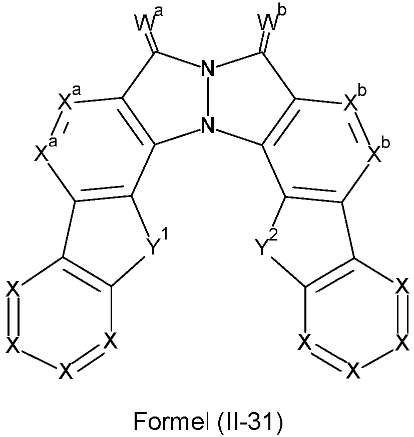 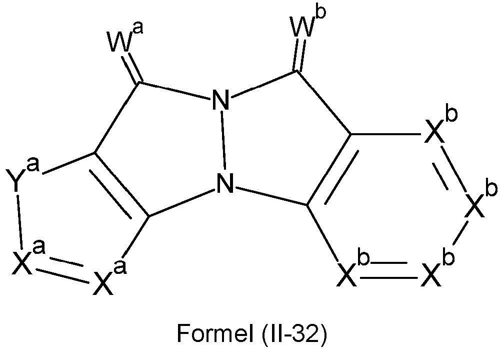 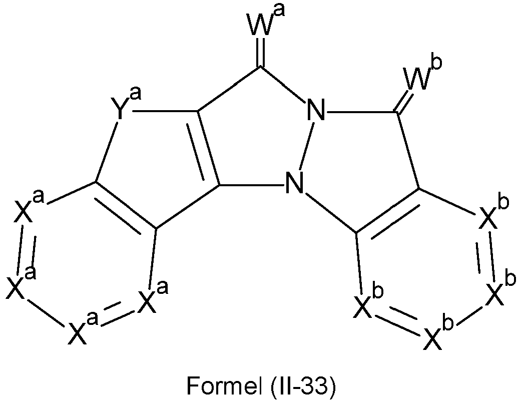 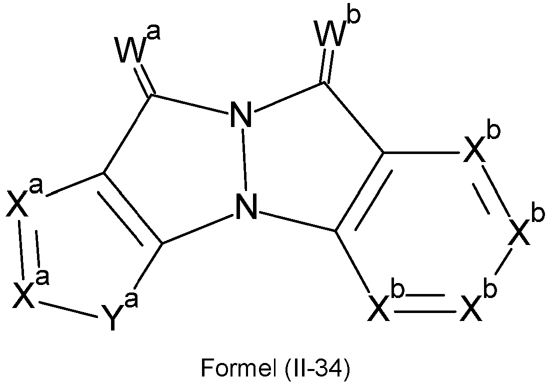 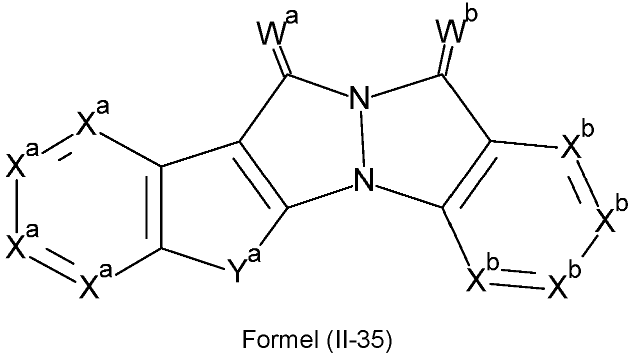 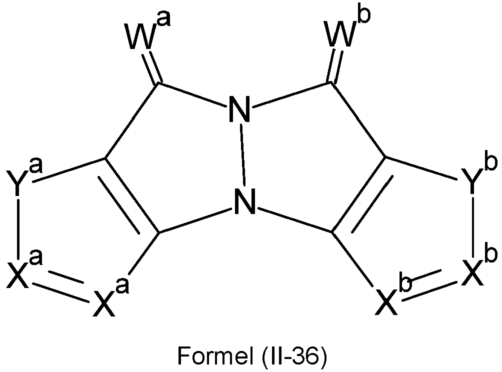 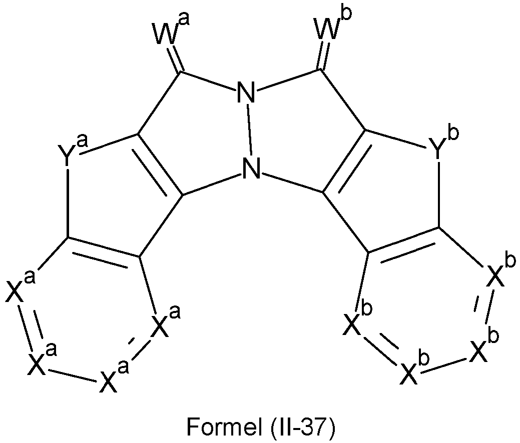 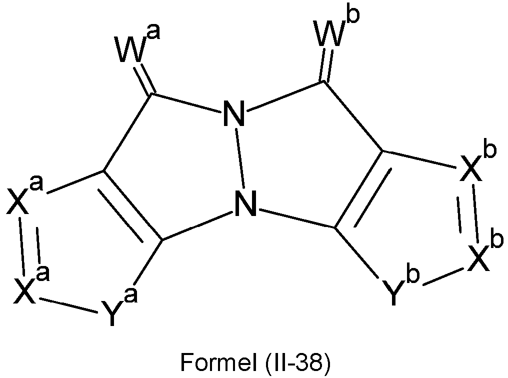 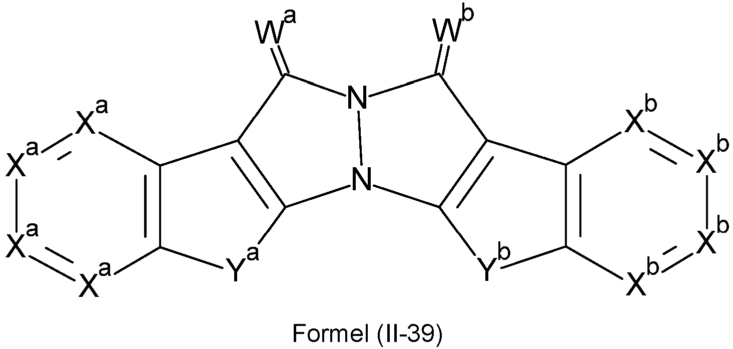 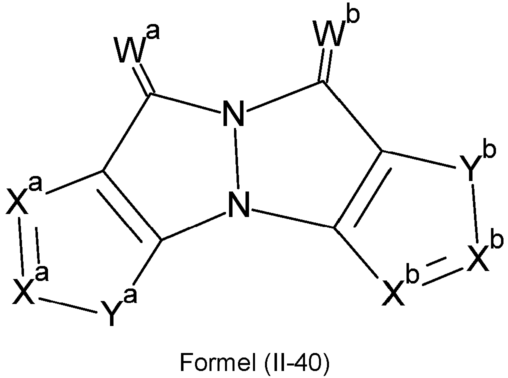 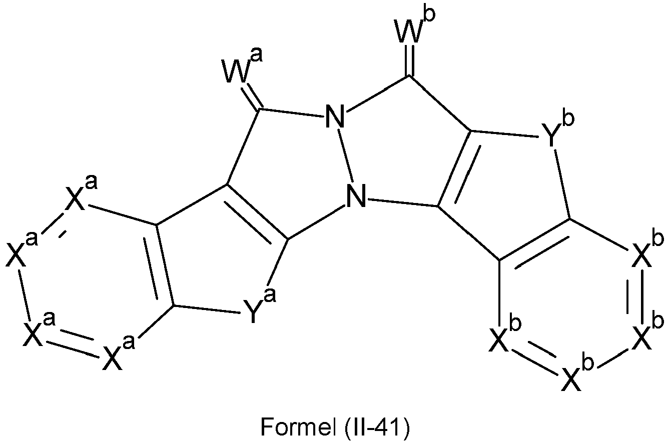 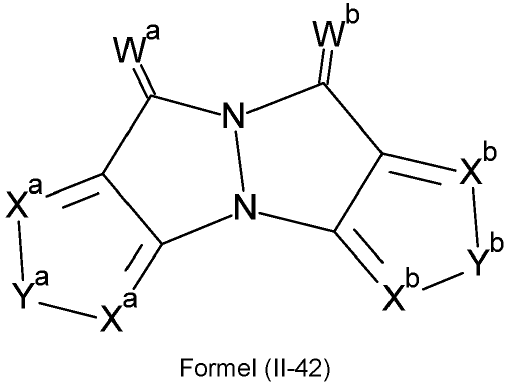 wobei die Symbole W^{a} und W^{b} die zuvor, insbesondere für Formel (I) genannten Bedeutungen aufweisen und für die weiteren Symbole gilt:
- Y^{a}: ist bei jedem Auftreten gleich oder verschieden N(Ar), N(R^{a}), P(Ar), P(R^{a}), P(=O)Ar, P(=O)R^{a}, P(=S)Ar, P(=S)R^{a}, B(Ar), B(R^{a}), Al(Ar), AI(R^{a}), Ga(Ar), Ga(R^{a}), C=O, C(R^{a})₂, Si(R^{a})₂, Ge(R^{a})₂, C=NR^{a}, C=NAr, C=C(R^{a})₂, C=C(R^{a})(Ar), O, S, Se, S=O, oder SO₂, vorzugsweise N(Ar), N(R^{a}), B(Ar), B(R^{a}), P(=O)R^{a}, P(=O)Ar, C=O, C(R^{a})₂, C=C(R^{a})₂, C=C(R^{a})(Ar), Si(R^{a})₂, O, S, Se, S=O oder SO₂, besonders bevorzugt N(Ar), C(R^{a})₂, O oder S, wobei R^{a} die zuvor, insbesondere für Formel (I) dargelegte Bedeutung aufweist;
- Y^{b}: ist bei jedem Auftreten gleich oder verschieden N(Ar), N(R^{b}), P(Ar), P(R^{b}), P(=O)Ar, P(=O)R^{b}, P(=S)Ar, P(=S)R^{b}, B(Ar), B(R^{b}), Al(Ar), Al(R^{a}), Ga(Ar), Ga(R^{b}), C=O, C(R^{b})₂, Si(R^{b})₂, Ge(R^{b})₂, C=NR^{b}, C=NAr, C=C(R^{b})₂, C=C(R^{b})(Ar), O, S, Se, S=O, oder SO₂, vorzugsweise N(Ar), N(R^{b}), B(Ar), B(R^{b}), P(=O)R^{b}, P(=O)Ar, C=O, C(R^{b})₂, C=C(R^{b})₂, C=C(R^{b})(Ar), Si(R^{b})₂, O, S, Se, S=O oder SO₂, besonders bevorzugt N(Ar), C(R^{b})₂, O oder S, wobei R^{b} die zuvor, insbesondere für Formel (I) dargelegte Bedeutung aufweist;
- Y¹, Y²: ist bei jedem Auftreten gleich oder verschieden N(Ar), N(R), P(Ar), P(R), P(=O)Ar, P(=O)R, P(=S)Ar, P(=S)R, B(Ar), B(R), Al(Ar), Al(R), Ga(Ar), Ga(R), C=O, C(R)₂, Si(R)₂, Ge(R)₂, C=NR, C=NAr, C=C(R)₂, C=C(R)(Ar), O, S, Se, S=O, oder SO₂, vorzugsweise N(Ar), N(R), B(Ar), B(R), P(=O)R, P(=O)Ar, C=O, C(R)₂, C=C(R)₂, C=C(R)(Ar), Si(R)₂, O, S, Se, S=O oder SO₂, besonders bevorzugt N(Ar), C(R)₂, O oder S, wobei R die zuvor, insbesondere für Formel (I) dargelegte Bedeutung aufweist;
- X: steht bei jedem Auftreten gleich oder verschieden für N oder CR, vorzugsweise für CR, mit der Maßgabe, dass nicht mehr als zwei der Gruppen X in einem Cyclus für N stehen, wobei R die zuvor, insbesondere für Formel (I) dargelegte Bedeutung aufweist;
- X^{a}: steht bei jedem Auftreten gleich oder verschieden für N oder CR^{a}, vorzugsweise für CR^{a}, mit der Maßgabe, dass nicht mehr als zwei der Gruppen X^{a} in einem Cyclus für N stehen, wobei R^{a} die zuvor, insbesondere für Formel (I) dargelegte Bedeutung aufweist;
- X^{b}: steht bei jedem Auftreten gleich oder verschieden für N oder CR^{b}, vorzugsweise für CR^{b}, mit der Maßgabe, dass nicht mehr als zwei der Gruppen X^{b} in einem Cyclus für N stehen, wobei R^{b} die zuvor, insbesondere für Formel (I) dargelegte Bedeutung aufweist.

Hierbei sind Strukturen der Formeln (II-1) bis (II-31) bevorzugt und Strukturen der Formeln (II-1) bis (II-4), (II-6), (II-8), (II-10), (II-12), (II-14), (II-16) bis (II-19), (II-21), (II-23), (II-25), (II-27), (II-29), (II-31) besonders bevorzugt.

Ferner kann insbesondere für Verbindungen umfassend Strukturen der Formeln (I) und (II-1) bis (II-42) vorgesehen sein, dass mindestens eine der Gruppen W^{a}, W^{b} für O steht, vorzugsweise beide Gruppen W^{a}, W^{b} für O stehen.

Falls in einer Verbindung zwei oder mehr Stickstoffatome enthalten sind, so sind diese Stickstoffatome bevorzugt nicht benachbart, so dass keine N-N-Bindungen enthalten sind.

In einer weiterhin bevorzugten Ausgestaltung kann vorgesehen sein, dass die erfindungsgemäßen Verbindungen eine Struktur der Formeln (III-1) bis (III-41) umfassen, wobei die erfindungsgemäßen Verbindungen besonders bevorzugt ausgewählt sein können aus den Verbindungen der Formeln (III-1) bis (III-41), 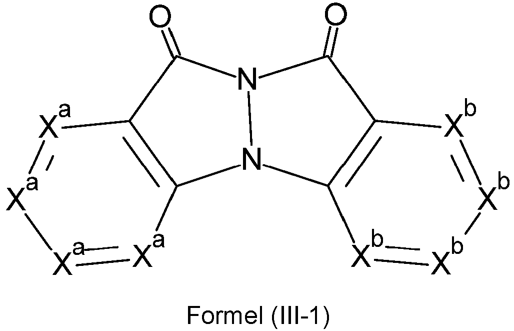 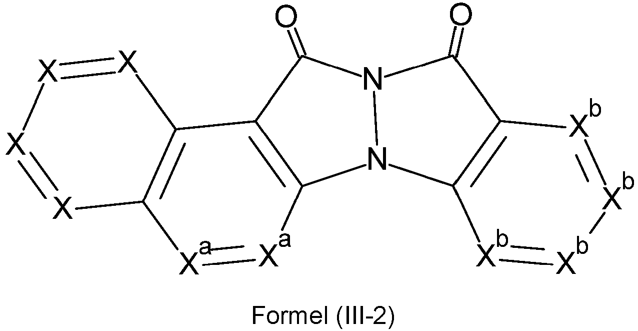 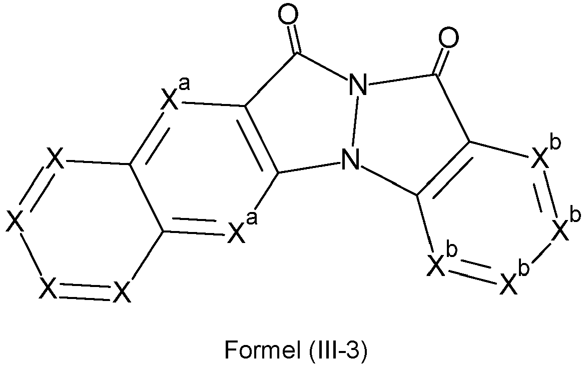 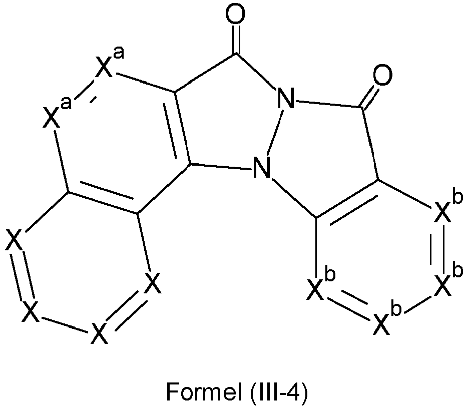 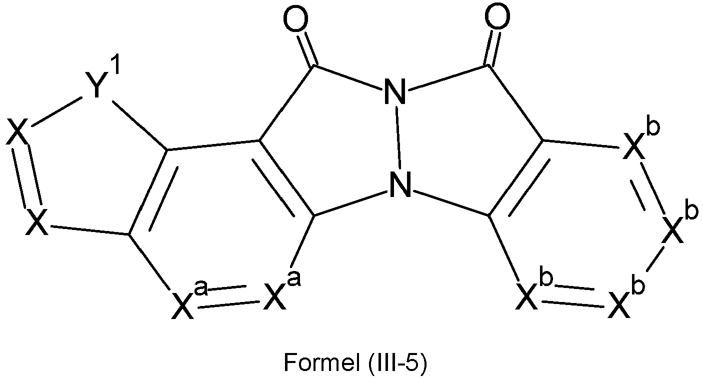 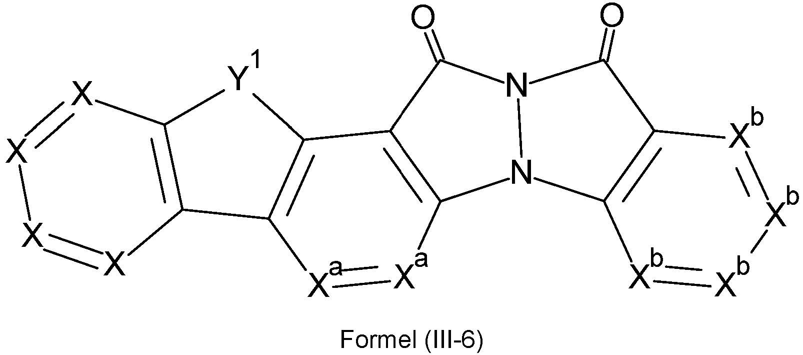 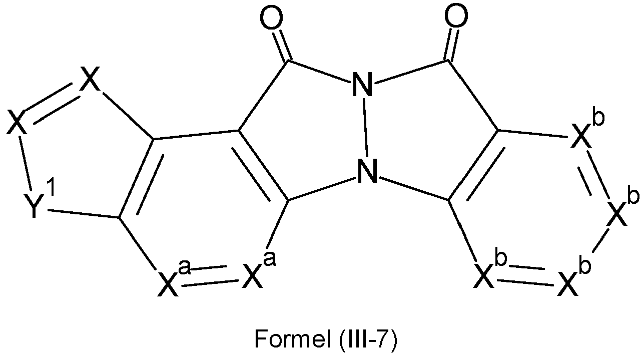 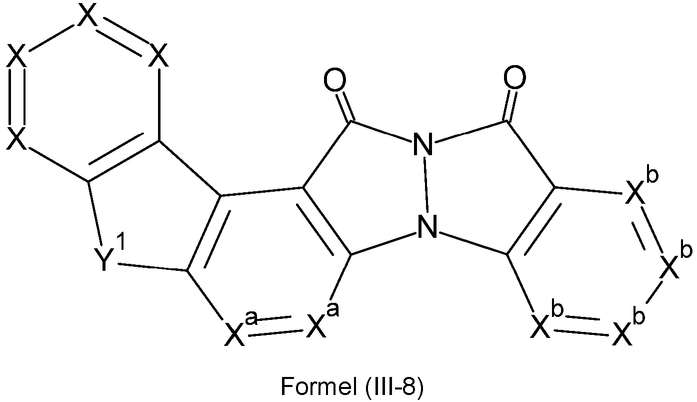 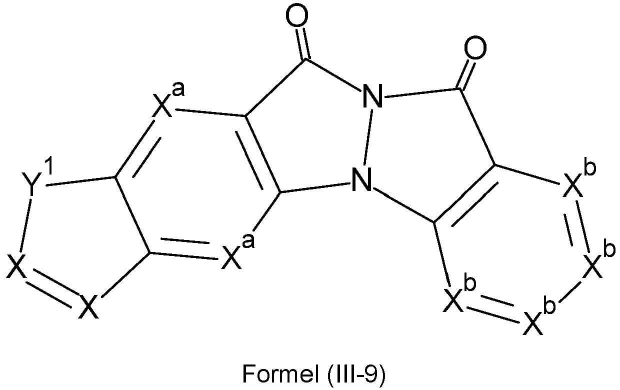 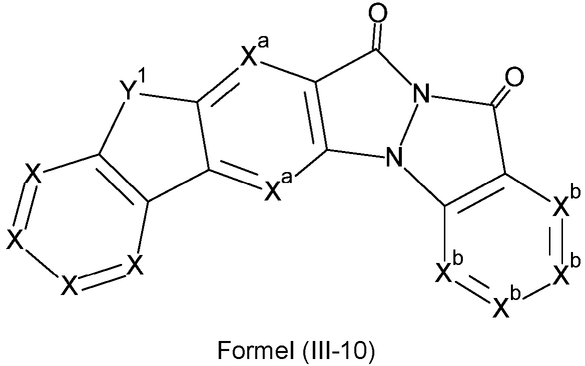 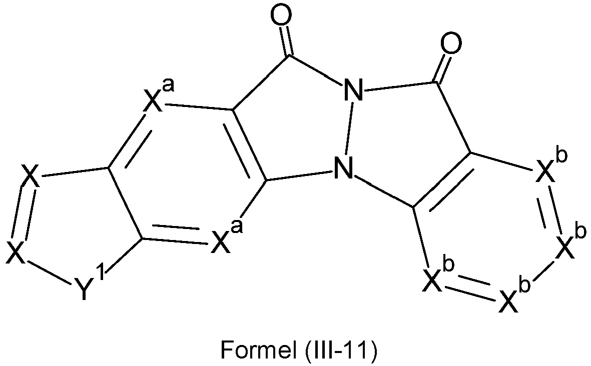 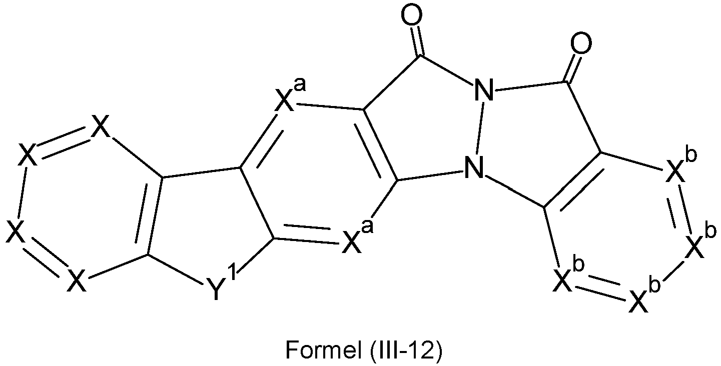 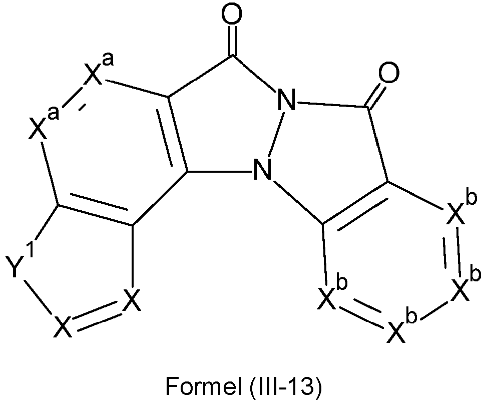 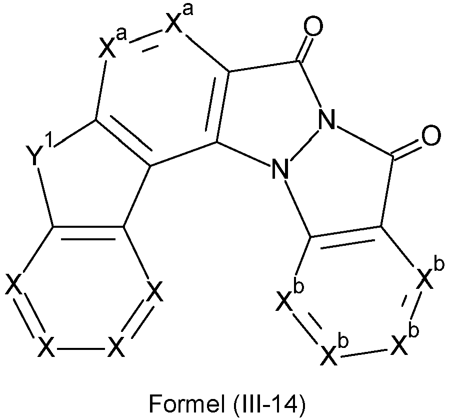 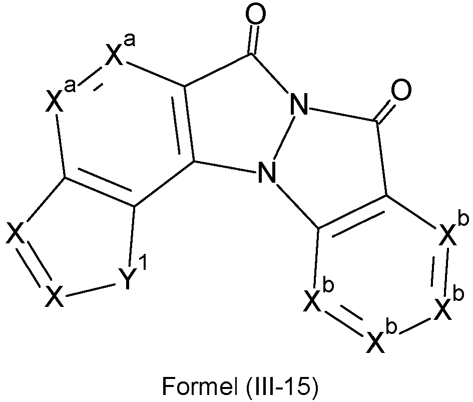 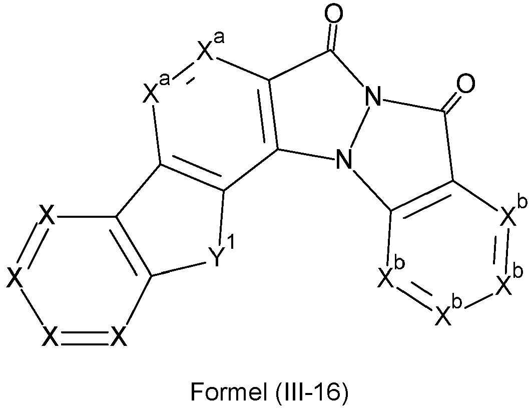 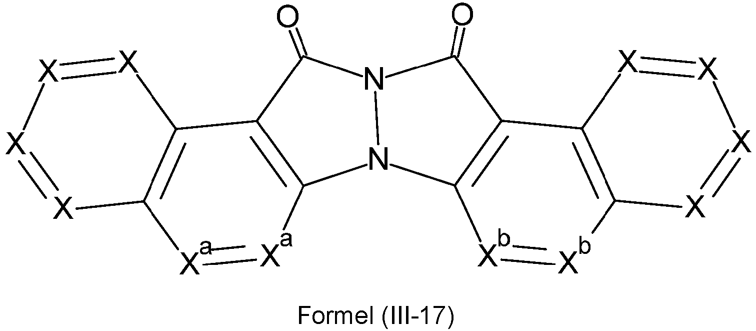 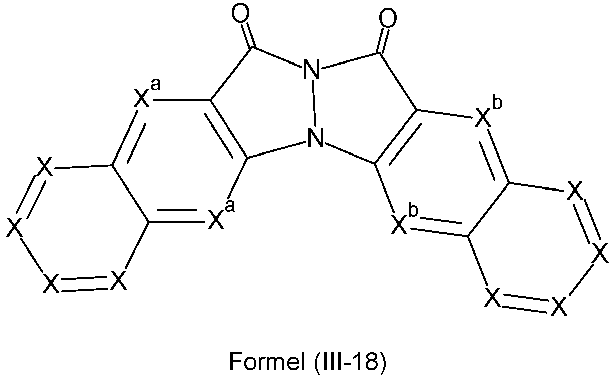 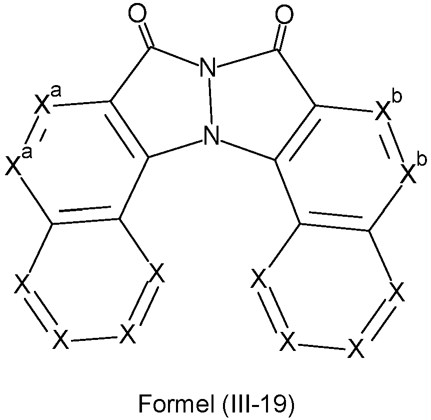 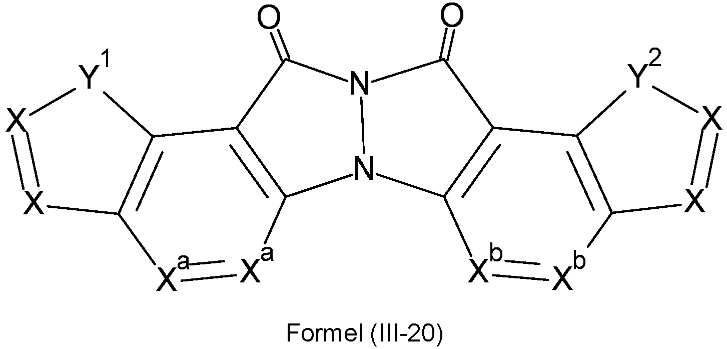 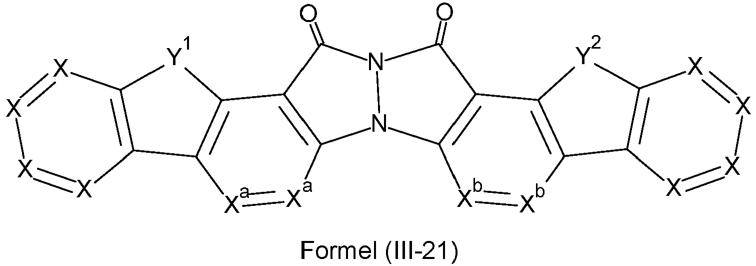 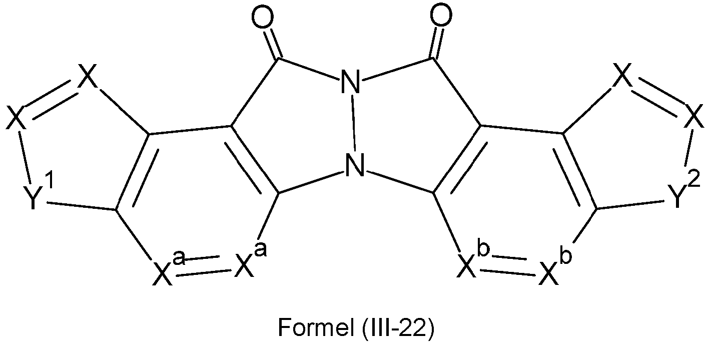 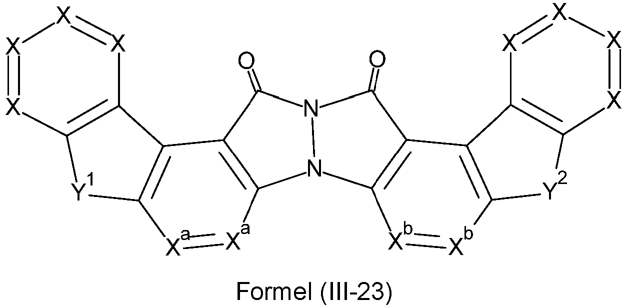 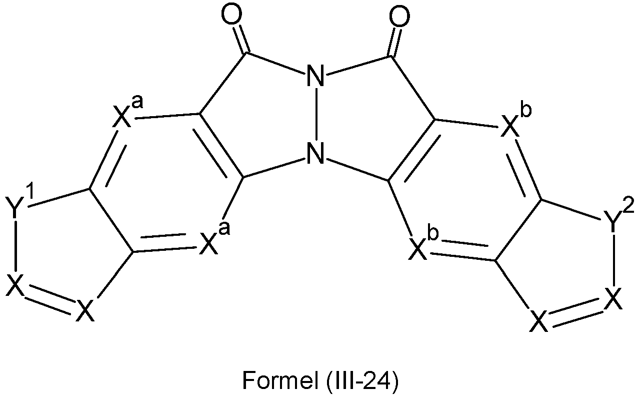 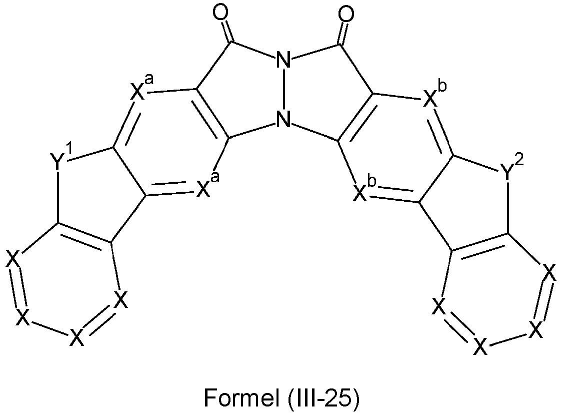 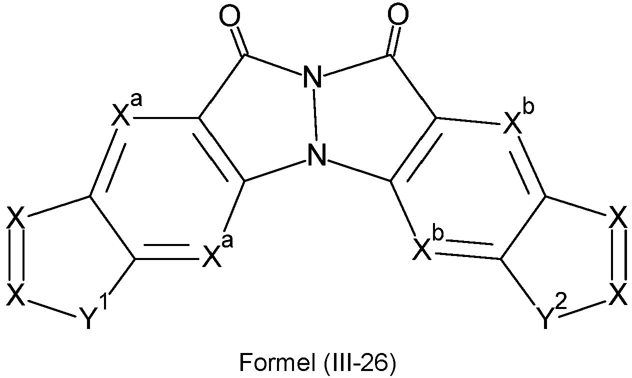 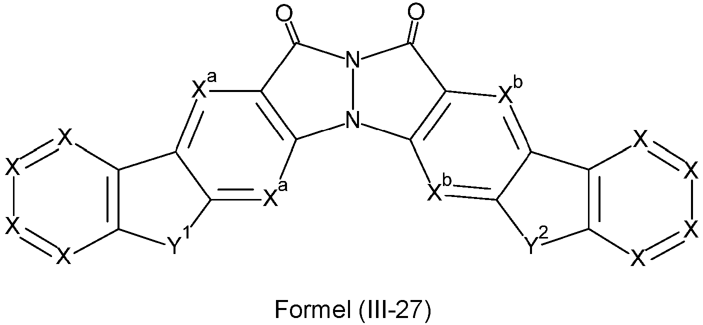 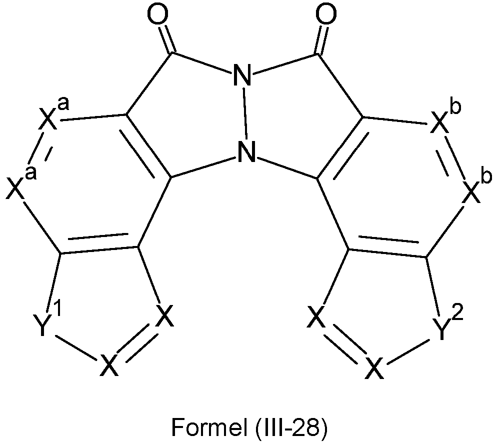 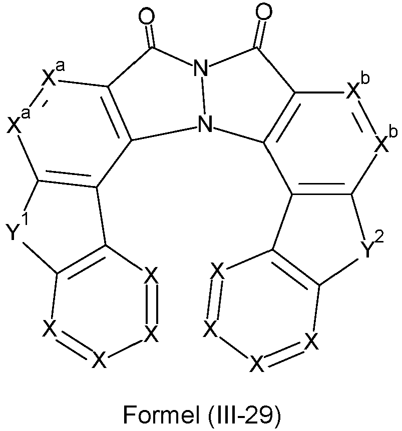 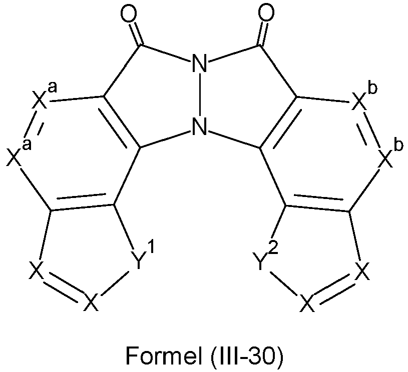 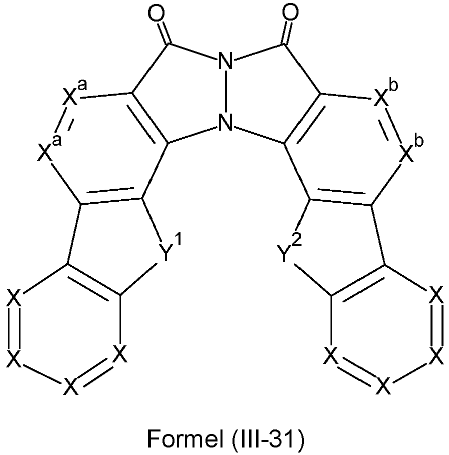 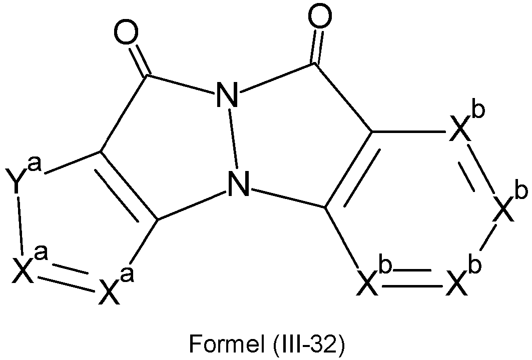 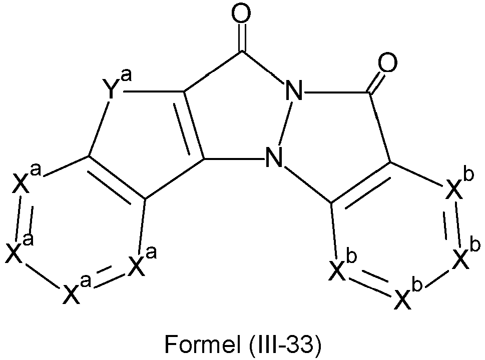 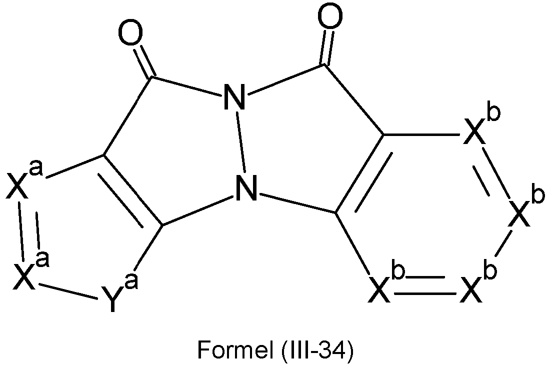 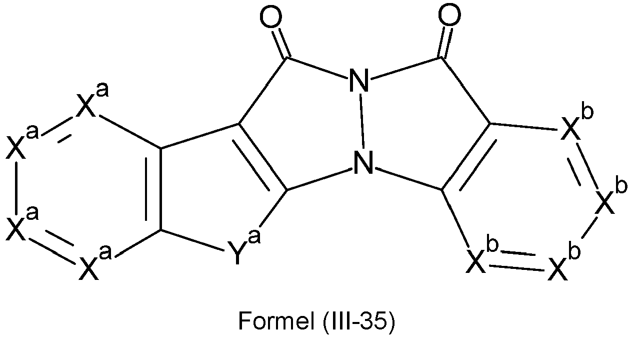 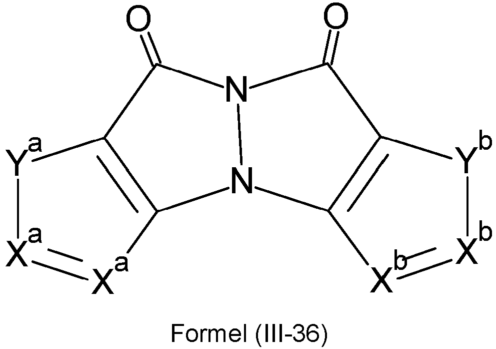 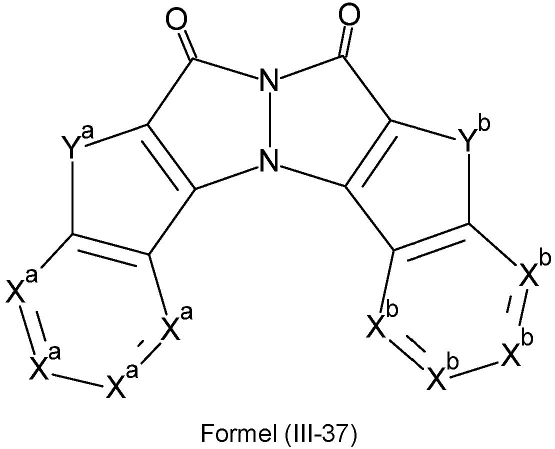 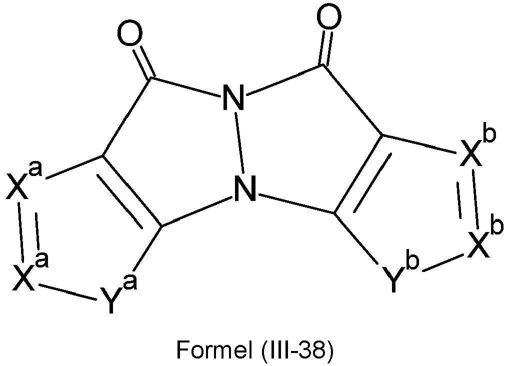 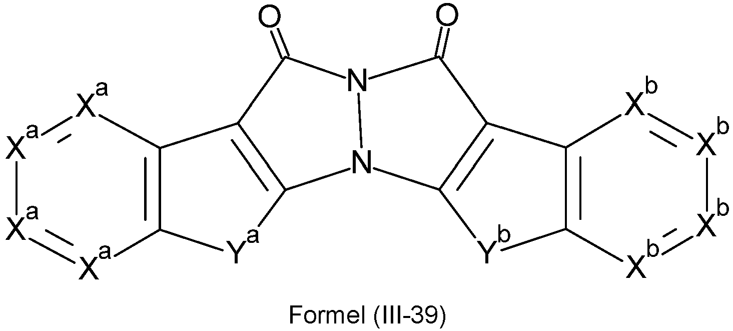 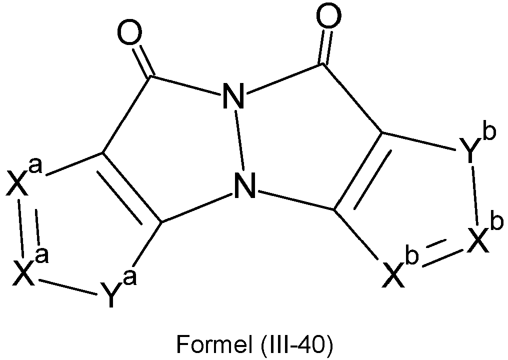 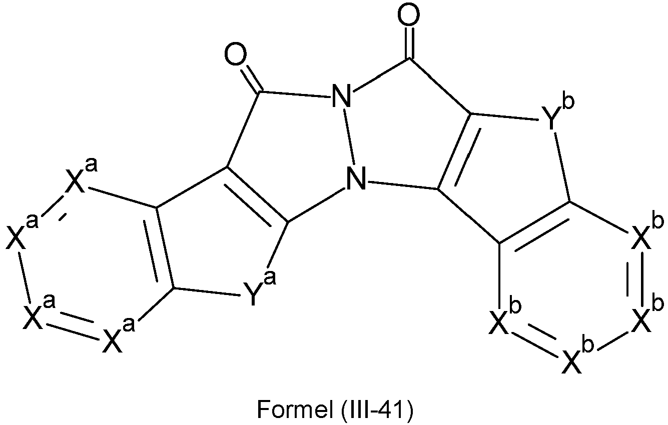 wobei die Symbole Y¹, Y², Y^{a}, Y^{b}, X, X^{a} und X^{b} die zuvor, insbesondere für Formeln (II-1) bis (II-42) genannten Bedeutungen aufweisen.

Hierbei sind Strukturen der Formeln (III-1) bis (III-31) bevorzugt und Strukturen der Formeln (III-1) bis (III-4), (III-6), (III-8), (III-10), (III-12), (III-14), (III-16) bis (III-19), (III-21), (III-23), (III-25), (III-27), (III-29), (III-31) besonders bevorzugt.

Vorzugsweise kann vorgesehen sein, dass in Formeln (II-1) bis (II-42) und/oder (III-1) bis (III-41) nicht mehr als vier, vorzugsweise nicht mehr als zwei Gruppen X, X^{a} und X^{b} für N stehen, besonders bevorzugt alle Gruppen X, X^{a} und X^{b} für CR, CR^{a} oder CR^{b} stehen.

In einer weiterhin bevorzugten Ausgestaltung kann vorgesehen sein, dass die erfindungsgemäßen Verbindungen eine Struktur der Formeln (IV-1) bis (IV-41) umfassen, wobei die erfindungsgemäßen Verbindungen besonders bevorzugt ausgewählt sein können aus den Verbindungen der Formeln (IV-1) bis (IV-41), 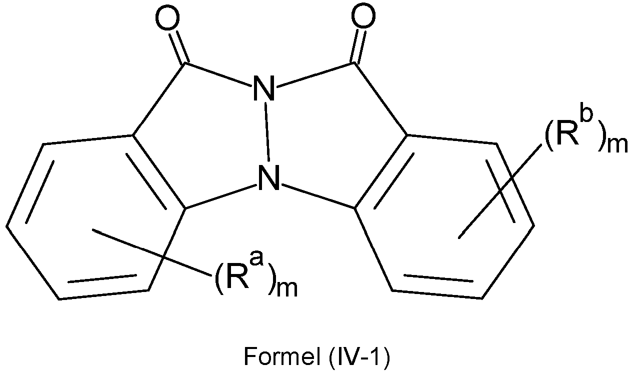 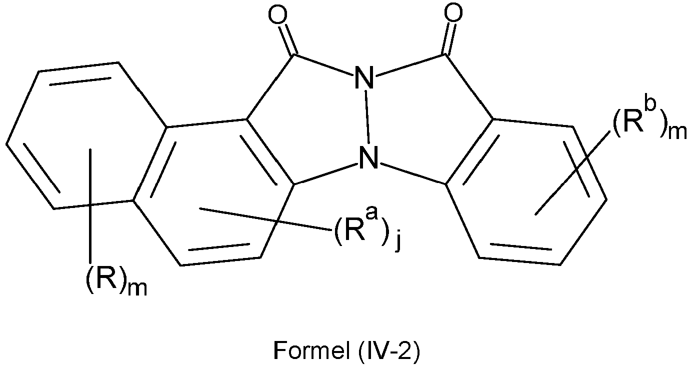 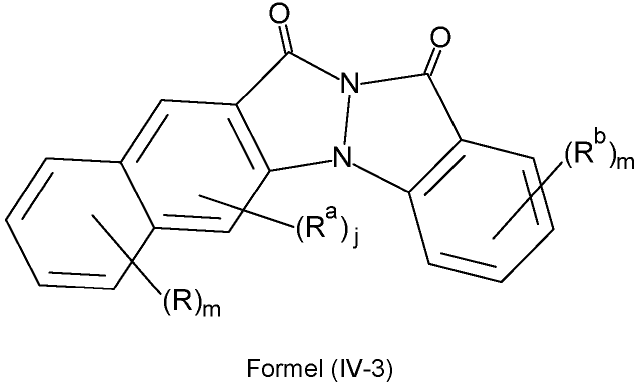 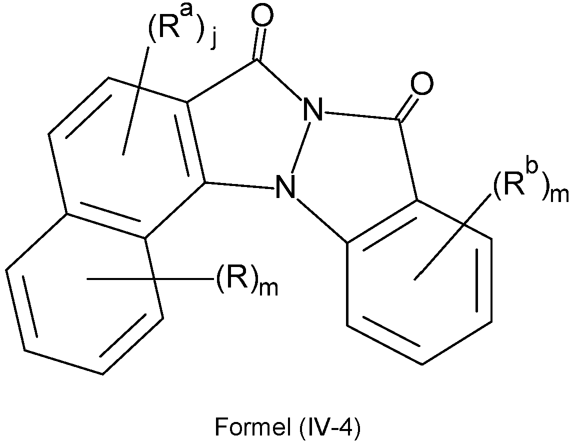 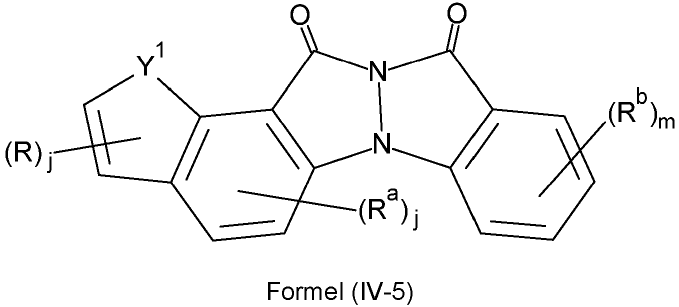 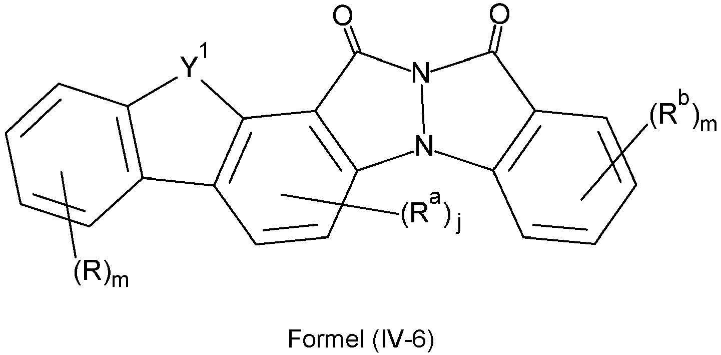 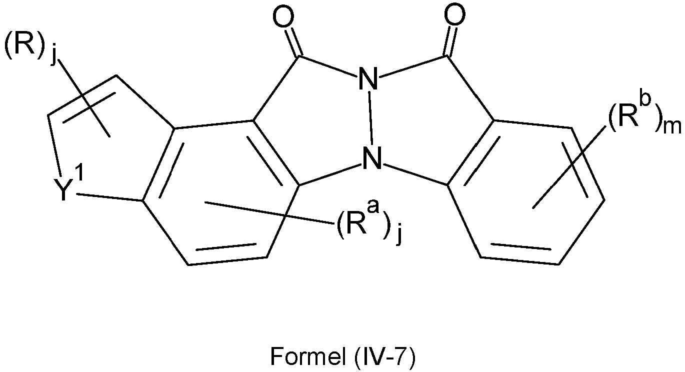 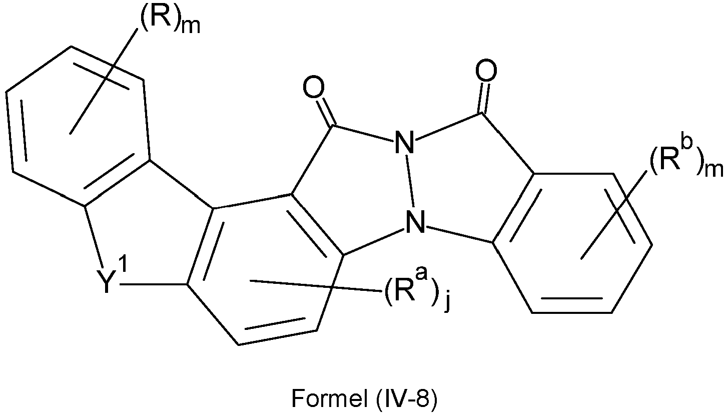 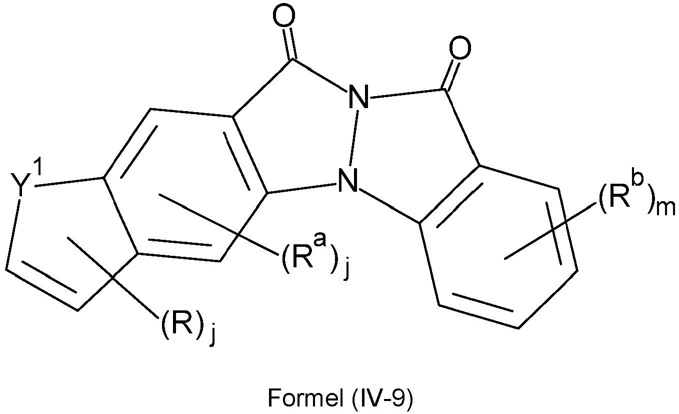 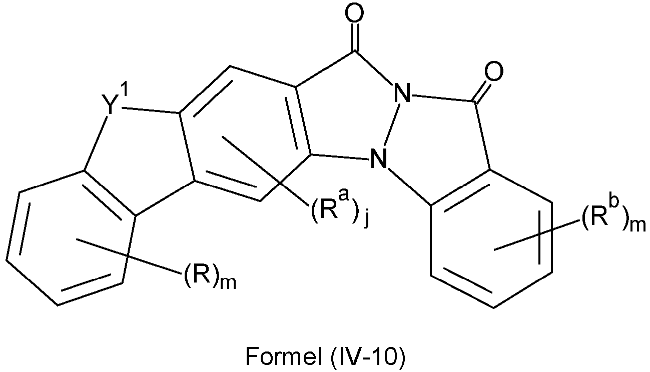 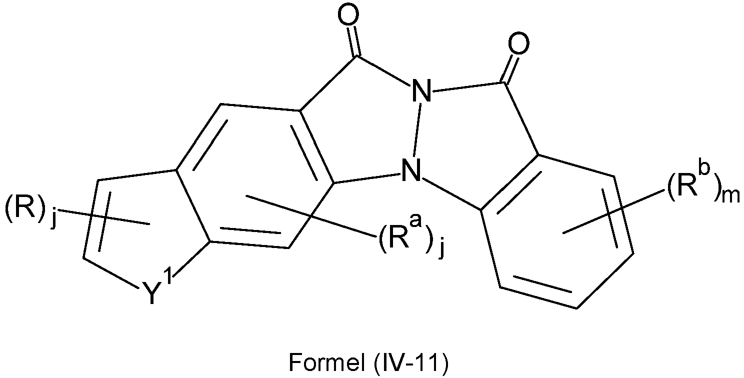 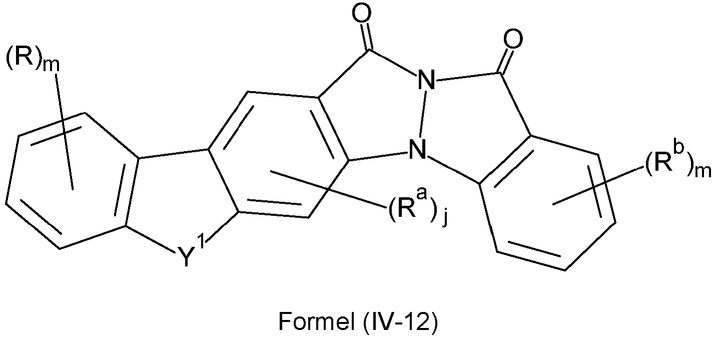 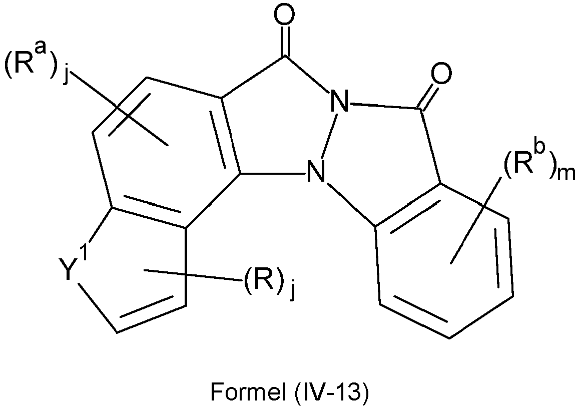 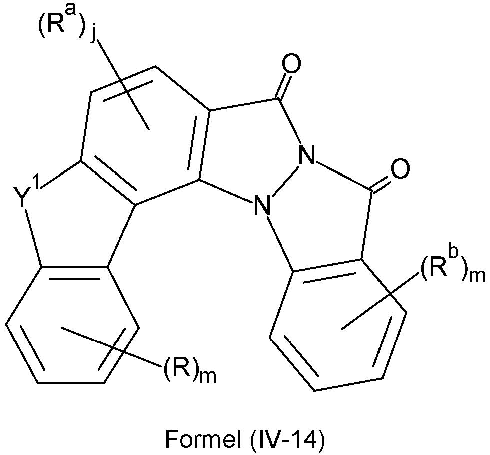 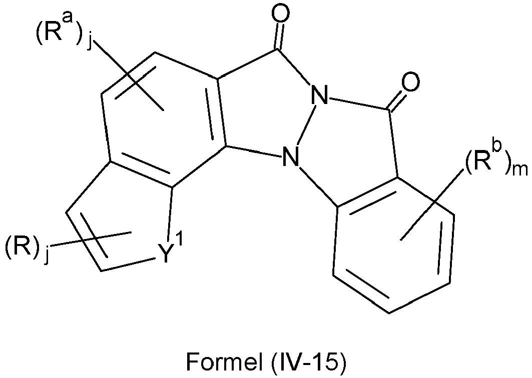 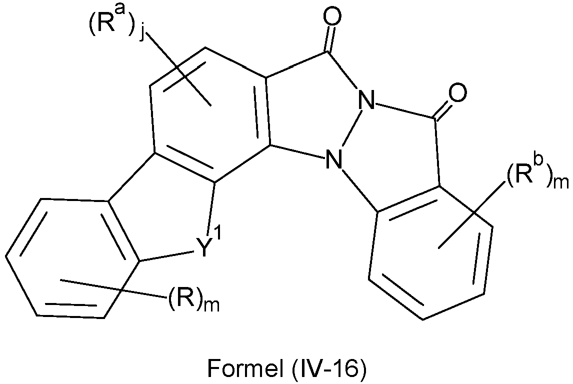 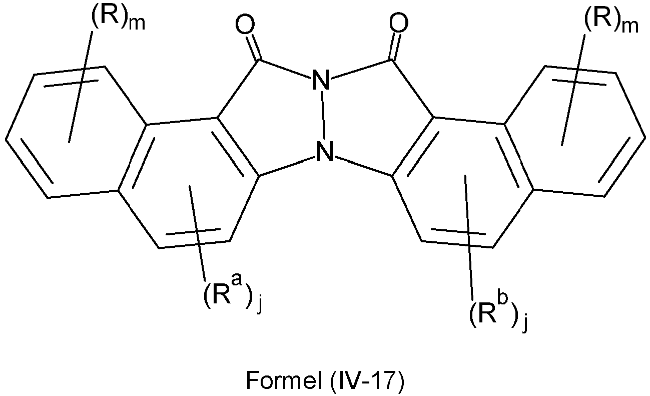 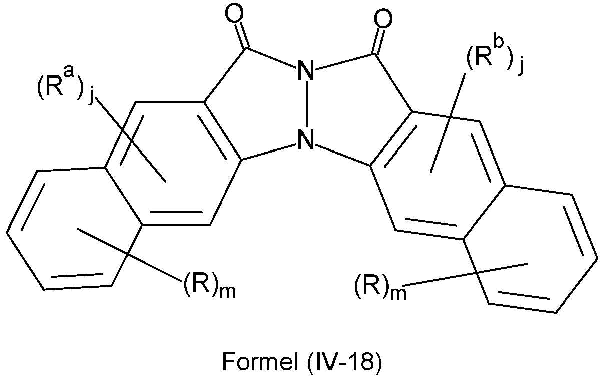 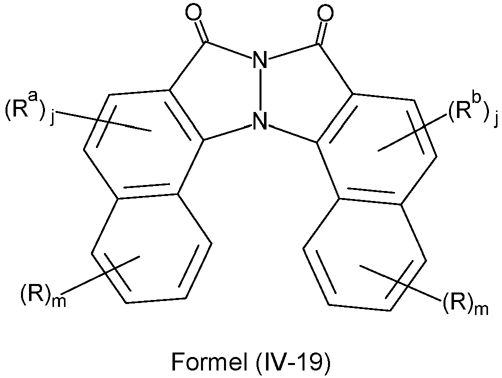 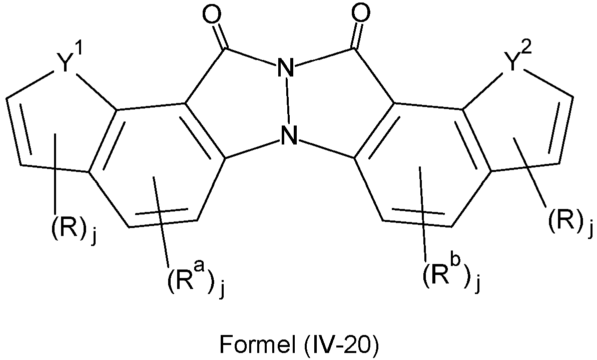 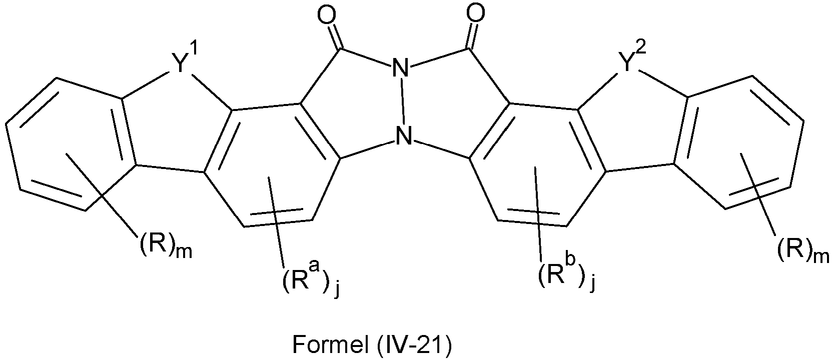 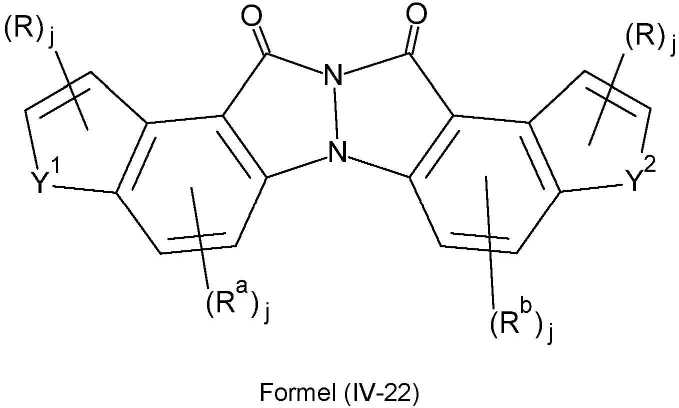 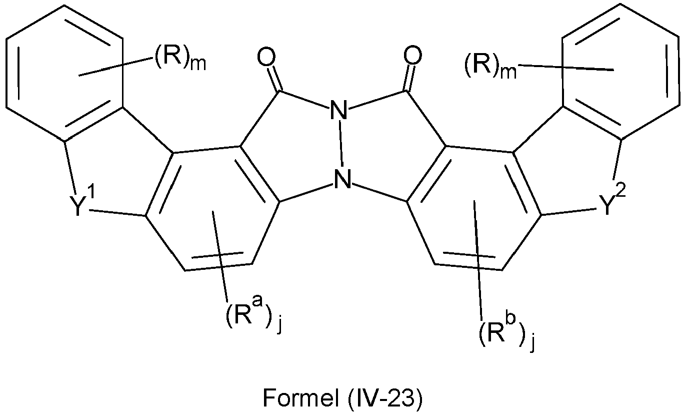 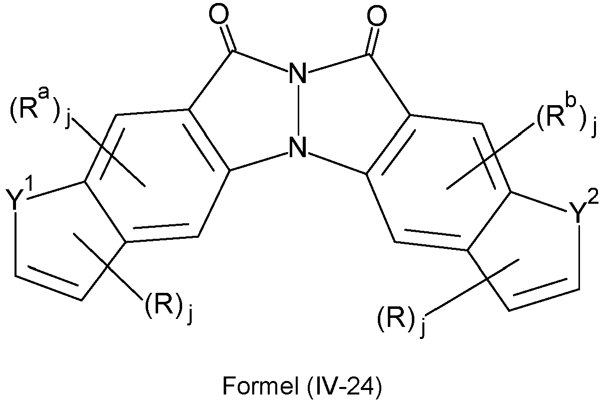 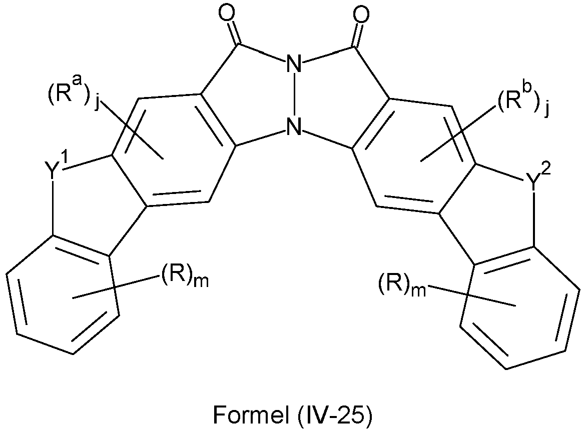 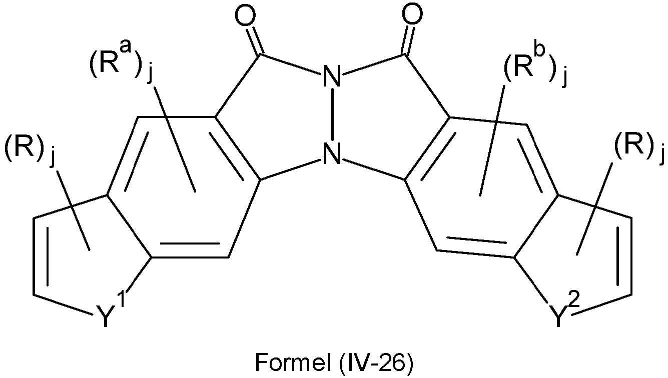 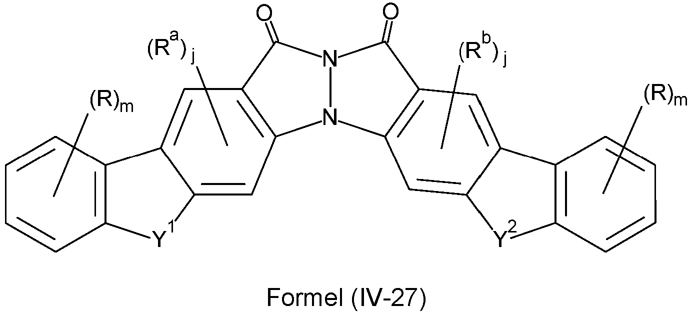 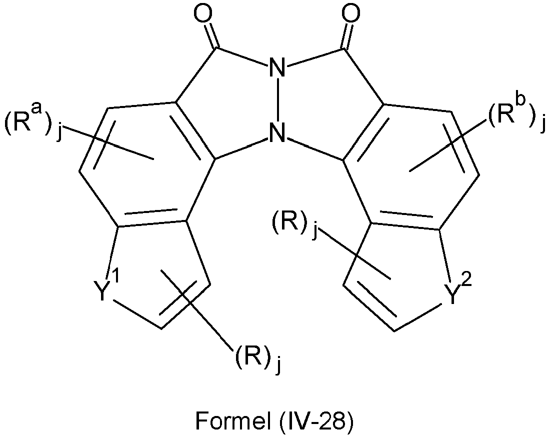 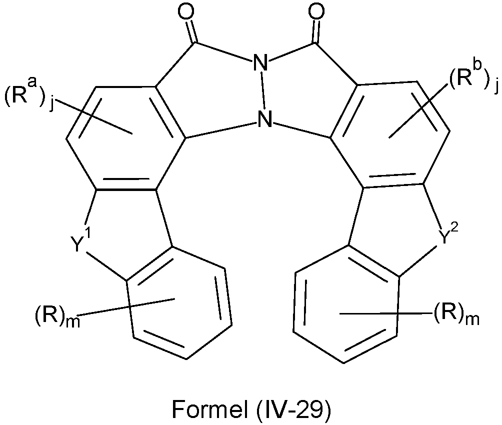 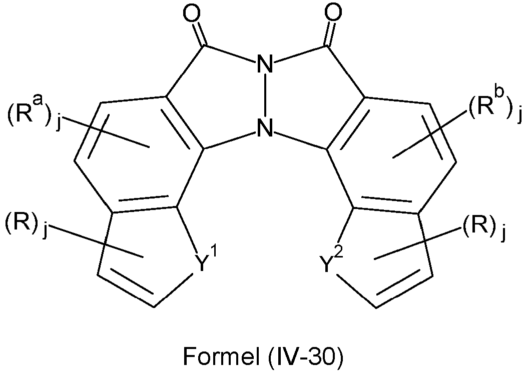 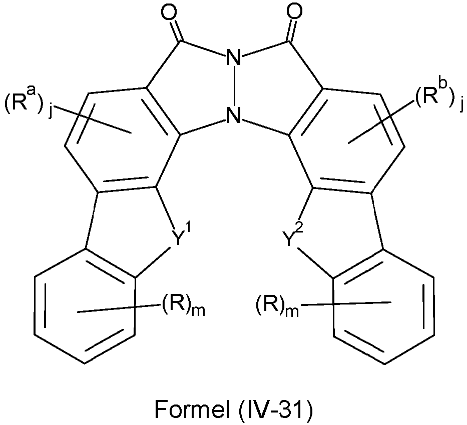 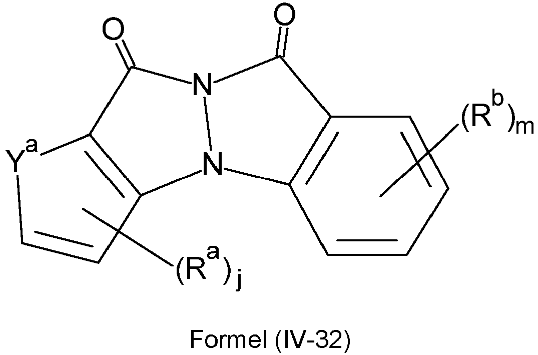 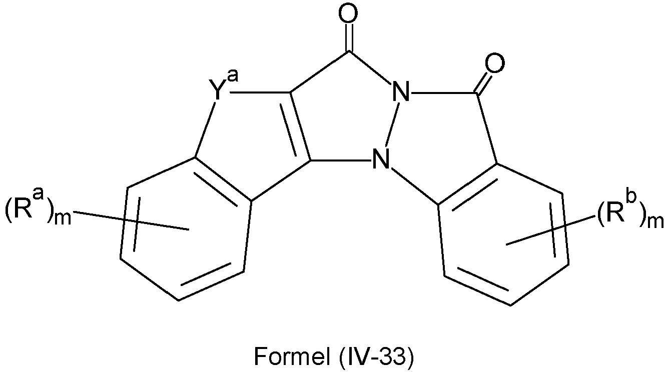 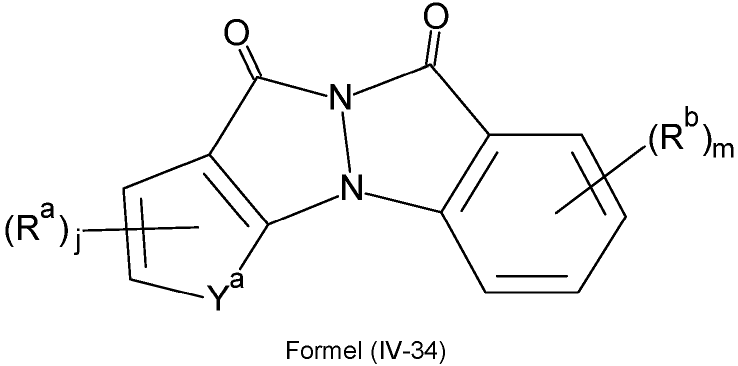 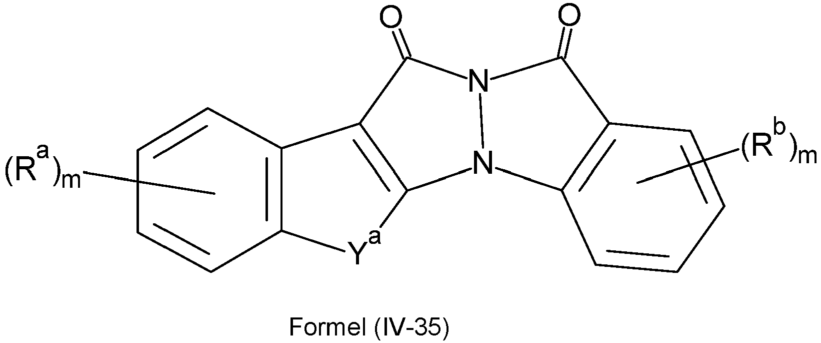 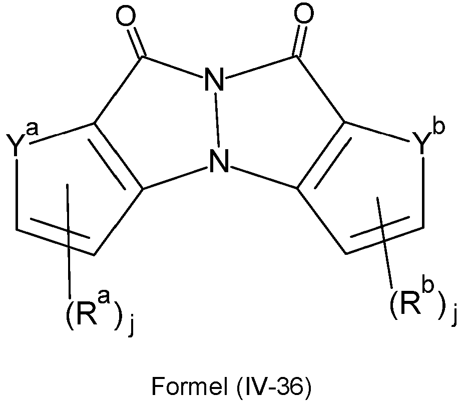 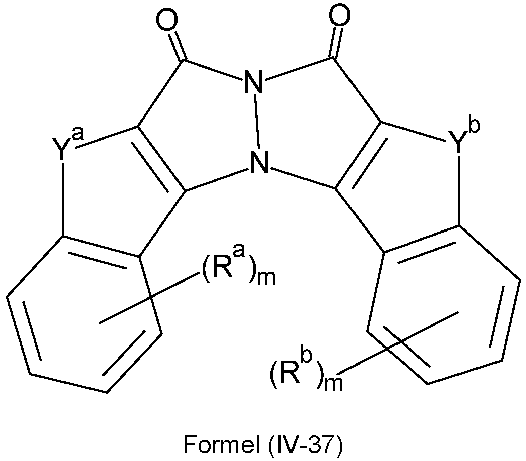 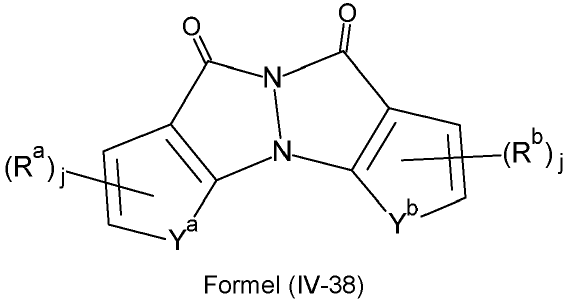 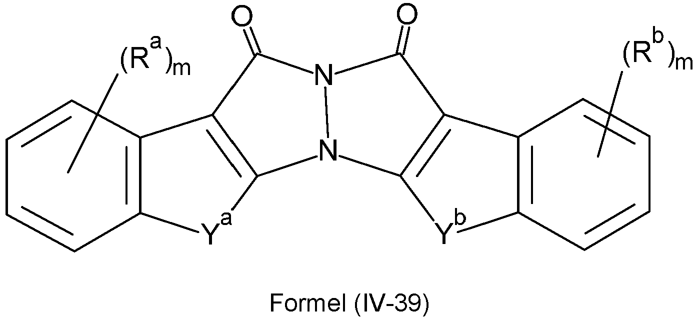 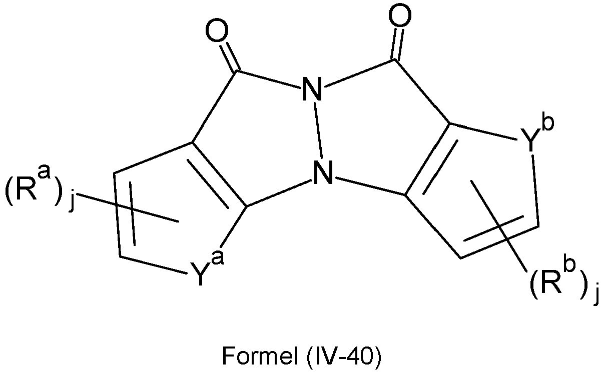 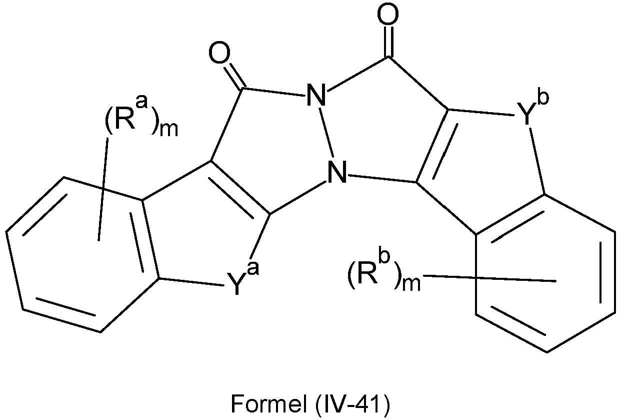 wobei die Symbole R, R^{a} und R^{b} die zuvor, insbesondere für Formel (I) genannten Bedeutungen aufweisen, die Symbole Y¹, Y², Y^{a} und Y^{b} die zuvor, insbesondere für Formeln (II-1) bis (II-42) genannten Bedeutungen aufweisen und die weiteren Symbole die folgende Bedeutung aufweisen:
- m: ist 0, 1, 2, 3 oder 4, vorzugsweise 0, 1 oder 2;
- j: ist 0, 1 oder 2, vorzugsweise 0 oder 1.

Hierbei sind Strukturen/Verbindungen der Formeln (IV-1) bis (IV-31) bevorzugt, Strukturen/Verbindungen der Formeln (IV-1) bis (IV-4), (IV-6), (IV-8), (IV-10), (IV-12), (IV-14), (IV-16) bis (IV-19), (IV-21), (IV-23), (IV-25), (IV-27), (IV-29), (IV-31) besonders bevorzugt.

Ferner kann vorgesehen sein, dass die Reste Y^{a} und Y^{b} gleich sind. Weiterhin können die Reste Y^{a} und Y^{b} verschieden sein.

In einer weiteren Ausgestaltung kann vorgesehen sein, dass einer Reste Y^{a} und Y^{b} für O steht und einer Reste Y^{a} und Y^{b} für C(R^{a})₂ beziehungsweise C(R^{b})₂ steht.

In einer weiteren Ausführungsform kann vorgesehen sein, dass einer Reste Y^{a} und Y^{b} für NAr steht und einer Reste Y^{a} und Y^{b} für C(R^{a})₂ beziehungsweise C(R^{b})₂ steht.

Darüber hinaus kann vorgesehen sein, dass die Reste Y¹ und Y² gleich sind. Weiterhin können die Reste Y¹ und Y² verschieden sein.

In einer weiteren Ausgestaltung kann vorgesehen sein, dass einer Reste Y¹ und Y² für O steht und einer Reste Y¹ und Y² für C(R^{a})₂ beziehungsweise C(R^{b})₂ steht.

In einer weiteren Ausführungsform kann vorgesehen sein, dass einer Reste Y¹ und Y² für NAr steht und einer Reste Y¹ und Y² für C(R^{a})₂ beziehungsweise C(R^{b})₂ steht.

In einer weiterhin bevorzugten Ausgestaltung kann vorgesehen sein, dass die erfindungsgemäßen Verbindungen eine Struktur der Formeln (V-1) bis (V-8) umfassen, wobei die erfindungsgemäßen Verbindungen besonders bevorzugt ausgewählt sein können aus den Verbindungen der Formeln (V-1) bis (V-8), 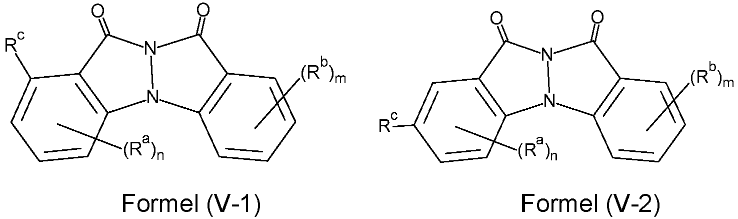 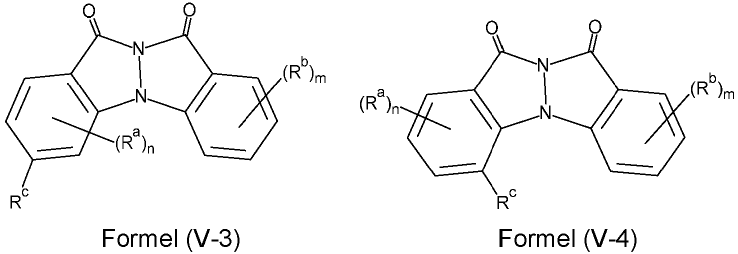 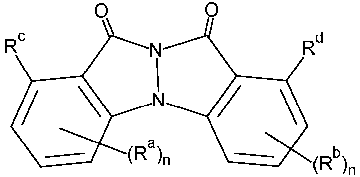 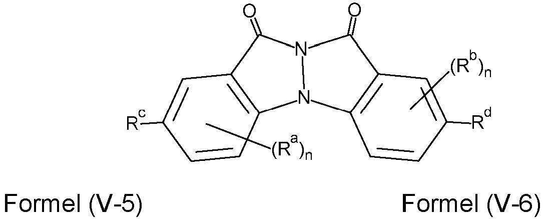 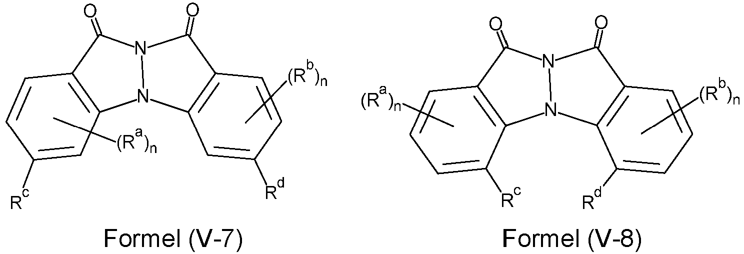 wobei die Symbole R^{a} und R^{b} die zuvor, insbesondere für Formel (I) genannten Bedeutungen aufweisen und die weiteren Symbole die folgende Bedeutung haben:
- R^{c}, R^{d}: ist bei jedem Auftreten gleich oder verschieden N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann; oder Heteroarylthiogruppe mit 5 bis 60 aromatischen Ringatomen, die durch einen oder mehrere Reste R¹ substituiert sein kann, oder eine Diarylamino-, Arylheteroarylamino-, Diheteroarylaminogruppe mit 5 bis 60 aromatischen Ringatomen, die durch einen oder mehrere Reste R¹ substituiert sein kann, oder eine Arylalkyl- oder Heteroarylalkylgruppe mit 5 bis 60 aromatischen Ringatomen und 1 bis 10 C-Atomen im Alkylrest, die durch einen oder mehrere Reste R¹ substituiert sein kann; dabei ein Rest R^{c} mit einem Rest R, R^{a}, R^{b}, R^{d} oder einer weiteren Gruppe ein Ringsystem bilden; dabei ein Rest R^{d} mit einem Rest R, R^{a}, R^{b}, R^{c} oder einer weiteren Gruppe ein Ringsystem bilden;
- m: ist 0, 1, 2, 3 oder 4, vorzugsweise 0, 1 oder 2;
- n: ist 0, 1, 2 oder 3, vorzugsweise 0, 1 oder 2.

Ferner kann insbesondere für Strukturen/Verbindungen der Formeln (V-1) bis (V-8) vorgesehen sein, dass die Reste R^{c} und R^{d} gleich sind. Weiterhin können die Reste R^{c} und R^{d} verschieden sein.

Die Summe der Indices j, m und n, insbesondere in Strukturen/Verbindungen der Formeln (IV-1) bis (IV-41) und/oder (V-1) bis (V-8), beträgt vorzugsweise höchstens 10, vorzugsweise höchstens 8, insbesondere bevorzugt höchstens 6 und besonders bevorzugt höchstens 4.

Weiterhin kann unter anderem in Formeln (I), (II-1) bis (II-42), (III-1) bis (III-42), (IV-1) bis (IV-41), (V-1) bis (V-8) und/oder den nachfolgend dargelegten bevorzugten Ausführungsformen dieser Formeln, vorgesehen sein, dass mindestens ein Rest R, R^{a}, R^{b}, R^{c}, R^{d} für eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, oder für ein aromatisches oder heteroaromatisches Ringsystem mit 5 bis 60 aromatischen Ringatomen, das jeweils durch einen oder mehrere Reste R¹ substituiert sein kann, oder für eine Aryloxy- oder Heteroaryloxygruppe mit 5 bis 60 aromatischen Ringatomen, die durch einen oder mehrere Reste R¹ substituiert sein kann; oder für eine Heteroarylthiogruppe mit 5 bis 60 aromatischen Ringatomen, die durch einen oder mehrere Reste R¹ substituiert sein kann, oder für eine Diarylamino-, Arylheteroarylamino-, Diheteroarylaminogruppe mit 5 bis 60 aromatischen Ringatomen, die durch einen oder mehrere Reste R¹ substituiert sein kann, oder für eine Arylalkyl- oder Heteroarylalkylgruppe mit 5 bis 60 aromatischen Ringatomen und 1 bis 10 C-Atomen im Alkylrest, die durch einen oder mehrere Reste R¹ substituiert sein kann, steht.

Vorzugsweise kann unter anderem in Formeln (I), (II-1) bis (II-42), (III-1) bis (III-42), (IV-1) bis (IV-41), (V-1) bis (V-8) und/oder den nachfolgend dargelegten bevorzugten Ausführungsformen dieser Formeln, vorgesehen sein, dass mindestens ein Rest R, R^{a}, R^{b}, R^{c}, R^{d} für ein aromatisches oder heteroaromatisches Ringsystem mit 5 bis 60 aromatischen Ringatomen, das jeweils durch einen oder mehrere Reste R¹ substituiert sein kann, oder für eine Aryloxy- oder Heteroaryloxygruppe mit 5 bis 60 aromatischen Ringatomen, die durch einen oder mehrere Reste R¹ substituiert sein kann; oder für eine Heteroarylthiogruppe mit 5 bis 60 aromatischen Ringatomen, die durch einen oder mehrere Reste R¹ substituiert sein kann, oder für eine Diarylamino-, Arylheteroarylamino-, Diheteroarylaminogruppe mit 5 bis 60 aromatischen Ringatomen, die durch einen oder mehrere Reste R¹ substituiert sein kann, oder für eine Arylalkyl- oder Heteroarylalkylgruppe mit 5 bis 60 aromatischen Ringatomen und 1 bis 10 C-Atomen im Alkylrest, die durch einen oder mehrere Reste R¹ substituiert sein kann, steht, besonders bevorzugt für ein aromatisches oder heteroaromatisches Ringsystem mit 5 bis 60 aromatischen Ringatomen, das jeweils durch einen oder mehrere Reste R¹ substituiert sein kann.

In einer bevorzugten Weiterbildung der vorliegenden Erfindung kann vorgesehen sein, dass mindestens zwei Reste R, R^{a}, R^{b}, R^{c}, R^{d} mit den weiteren Gruppen, an die die zwei Reste R, R^{a}, R^{b}, R^{c}, R^{d} binden, einen kondensierten Ring bilden, wobei die zwei Reste R, R^{a}, R^{b}, R^{c}, R^{d} mindestens eine Struktur der Formeln (RA-1) bis (RA-12) formen 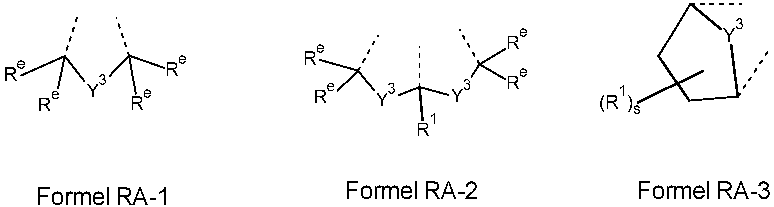 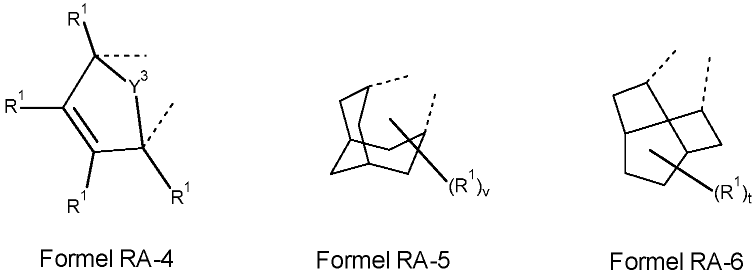 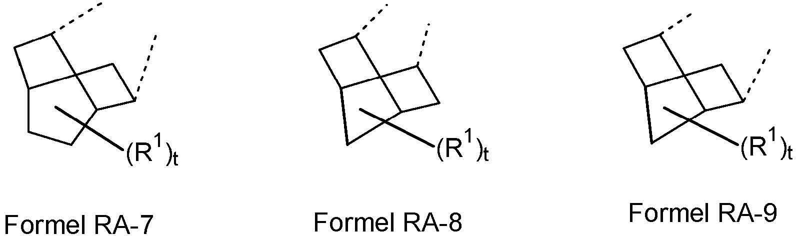 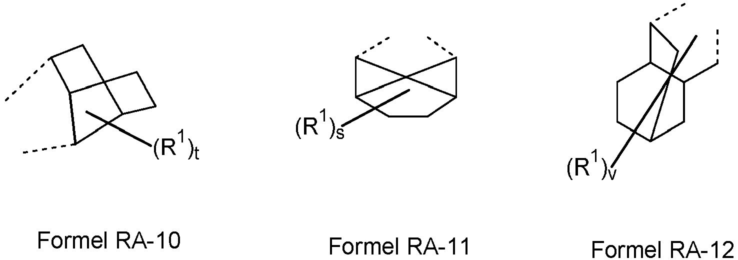 wobei R¹ die zuvor dargelegte Bedeutung hat, die gestrichelten Bindungen die Anbindungsstellen darstellen, über die die zwei Reste R, R^{a}, R^{b}, R^{c}, R^{d} an die weiteren Gruppen binden, und die weiteren Symbole die folgende Bedeutung aufweisen:
- Y³: ist bei jedem Auftreten gleich oder verschieden C(R¹)2, (R¹)₂C-C(R¹)₂, (R¹)C=C(R¹), NR¹, NAr', O oder S, vorzugsweise C(R¹)2, (R¹)₂C-C(R¹)₂, (R¹)C=C(R¹), O oder S;
- R^{e}: ist bei jedem Auftreten gleich oder verschieden F, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R² substituiert sein kann, wobei eine oder mehrere nicht benachbarte CH₂-Gruppen durch R²C=CR², C≡C, Si(R²)₂, C=O, C=S, C=Se, C=NR², -C(=O)O-, -C(=O)NR²-, NR², P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann; dabei können zwei Reste R^{e} auch miteinander oder ein Rest R^{e} mit einem Rest R¹ oder mit einer weiteren Gruppe ein Ringsystem bilden;
- s: ist 0, 1, 2, 3, 4, 5 oder 6, vorzugsweise 0, 1, 2, 3, oder 4, besonders bevorzugt 0, 1 oder 2;
- t: ist 0, 1, 2, 3, 4, 5, 6, 7 oder 8, vorzugsweise 0, 1, 2, 3, oder 4, besonders bevorzugt 0, 1 oder 2;
- v: ist 0, 1, 2, 3, 4, 5, 6, 7, 8 oder 9, vorzugsweise 0, 1, 2, 3, oder 4, besonders bevorzugt 0, 1 oder 2.

In einer bevorzugten Ausführungsform der Erfindung bilden die mindestens zwei Reste R, R^{a}, R^{b}, R^{c}, R^{d} mit den weiteren Gruppen, an die die zwei Reste R, R^{a}, R^{b}, R^{c}, R^{d} binden, einen kondensierten Ring, wobei die zwei Reste R, R^{a}, R^{b}, R^{c}, R^{d} vorzugsweise mindestens eine der Strukturen der Formeln (RA-1a) bis (RA-4f) formen 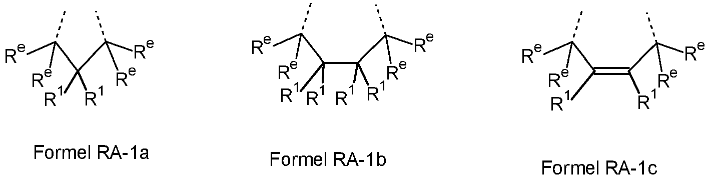 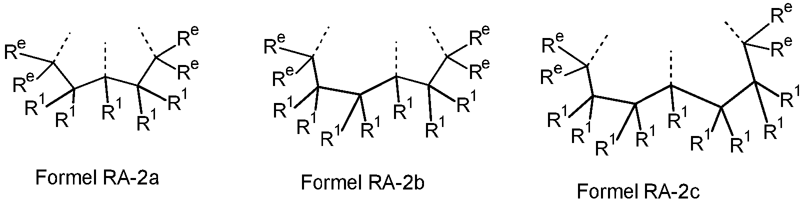 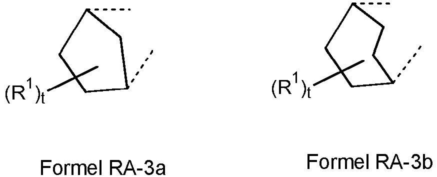 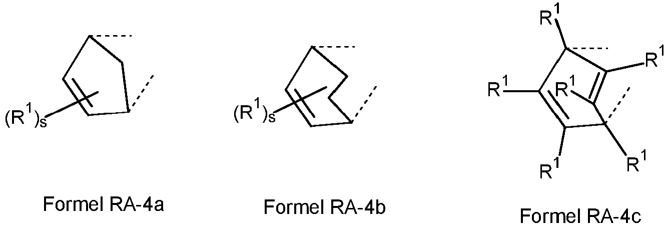 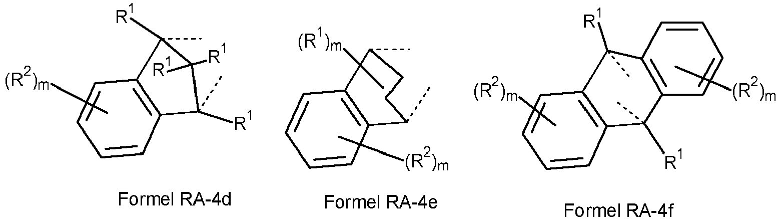 wobei die gestrichelten Bindungen die die Anbindungsstellen darstellen, über die die zwei Reste R, R^{a}, R^{b}, R^{c}, R^{d} an die weiteren Gruppen binden, der Index m 0, 1, 2, 3 oder 4, vorzugsweise 0, 1 oder 2 ist und die Symbole R¹, R², R^{e} und die Indices s, und t die zuvor, insbesondere für Formel (I) und/oder Formeln (RA-1) bis (RA-12) dargelegte Bedeutung haben.

Ferner kann vorgesehen sein, dass die mindestens zwei Reste R, R^{a}, R^{b}, R^{c}, R^{d} die Strukturen der Formeln (RA-1) bis (RA-12) und/oder (RA-1a) bis (RA-4f) formen und einen kondensierten Ring bilden, Reste R, R^{a}, R^{b}, R^{c}, R^{d} aus benachbarten Gruppen X, X^{a}, X^{b} darstellen oder Reste R, R^{a}, R^{b}, R^{c}, R^{d} darstellen, die jeweils an benachbarte C-Atome binden, wobei diese C-Atome vorzugsweise über eine Bindung verbunden sind.

In einer weiterhin bevorzugten Ausgestaltung bilden mindestens zwei Reste R, R^{a}, R^{b}, R^{c}, R^{d} mit den weiteren Gruppen, an die die zwei Reste R, R^{a}, R^{b}, R^{c}, R^{d} binden, einen kondensierten Ring, wobei die zwei Reste R, R^{a}, R^{b}, R^{c}, R^{d} Strukturen der Formel (RB), formen 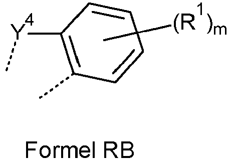 wobei R¹ die zuvor, insbesondere für Formel (I) genannte Bedeutung aufweist, die gestrichelten Bindungen die Anbindungsstellen darstellen, über die die zwei Reste R, R^{a}, R^{b}, R^{c}, R^{d} an die weiteren Gruppen binden, der Index m 0, 1, 2, 3 oder 4, vorzugsweise 0, 1 oder 2 ist, und Y⁴ C(R¹)2, NR¹, NAr', BR¹, BAr', O oder S ist, vorzugsweise C(R¹)2, NAr' oder O.

Hierbei kann vorgesehen sein, dass die mindestens zwei Reste R, R^{a}, R^{b}, R^{c}, R^{d}, die Strukturen der Formel (RB) formen und einen kondensierten Ring bilden, Reste R, R^{a}, R^{b}, R^{c}, R^{d} aus benachbarten Gruppen X, X^{a}, X^{b}, darstellen oder Reste R, R^{a}, R^{b}, R^{c}, R^{d} darstellen, die jeweils an benachbarte C-Atome binden, wobei diese C-Atome vorzugsweise über eine Bindung miteinander verbunden sind.

Besonders bevorzugt umfassen die Verbindungen mindestens eine Struktur der Formeln (VI-1) bis (VI-22), besonders bevorzugt sind die Verbindungen ausgewählt aus Verbindungen der Formeln (VI-1) bis (VI-22), wobei die Verbindungen mindestens einen kondensierten Ring aufweisen 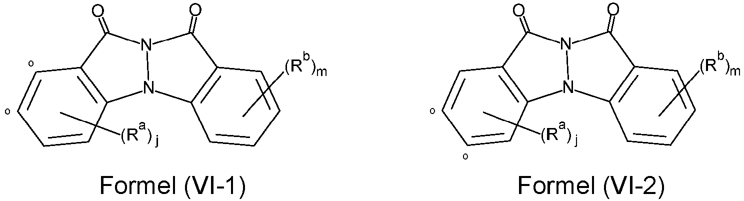 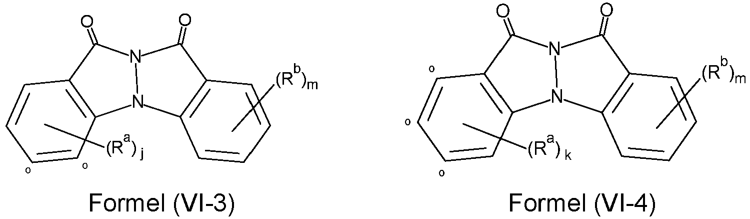 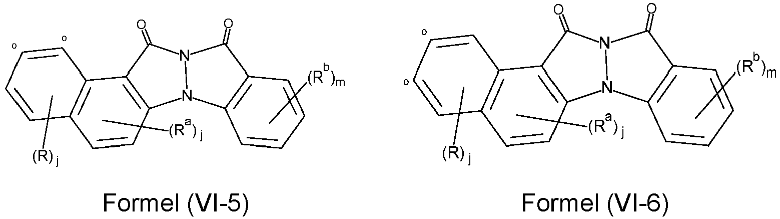 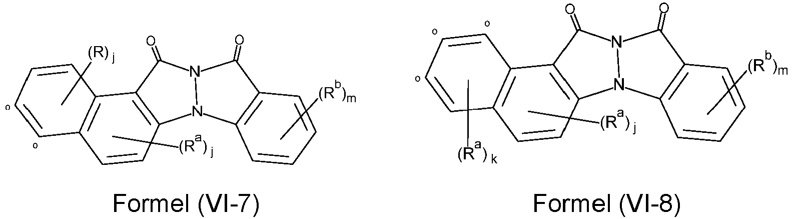 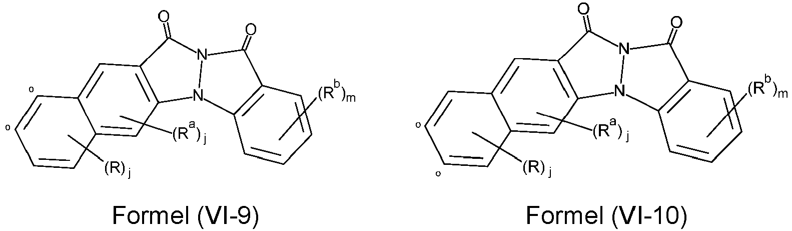 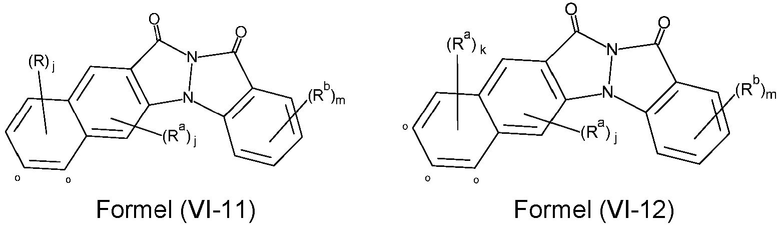 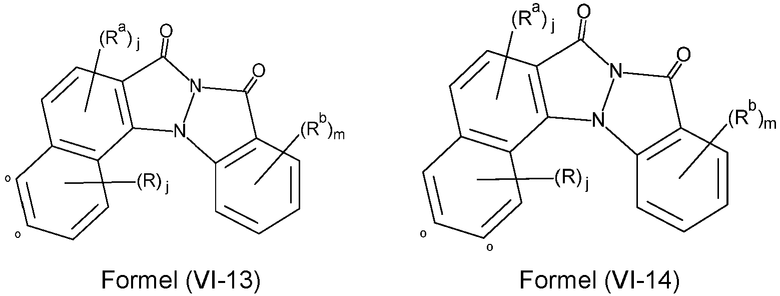 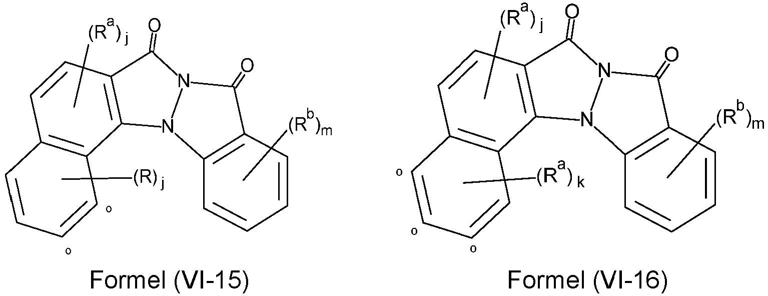 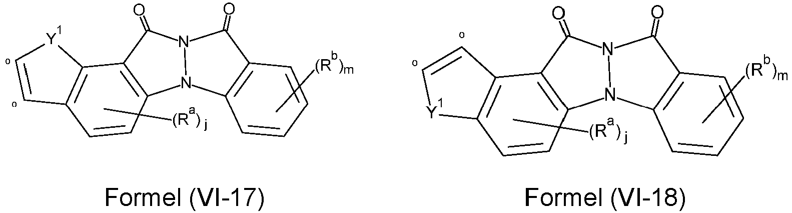 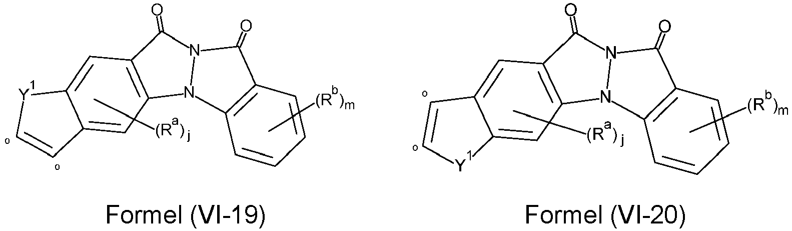 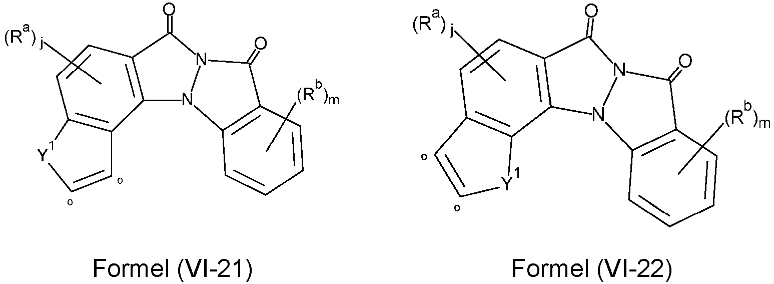 wobei die Symbole R, R^{a}, R^{b}, Y¹ und Y² die zuvor, insbesondere für Formel (I) und/oder Formeln (II-1) bis (II-42) genannten Bedeutungen aufweisen, das Symbol o für die Anbindungsstellen steht und die weiteren Symbole die folgende Bedeutung haben:
- m: ist 0, 1, 2, 3 oder 4, vorzugsweise 0, 1 oder 2;
- j: ist 0, 1 oder 2, vorzugsweise 0 oder 1;
- k: ist 0 oder 1.

Hierbei sind Strukturen/Verbindungen der Formel (VI-1) bis (VI-4) bevorzugt.

Besonders bevorzugt umfassen die Verbindungen mindestens eine Struktur der Formeln (VII-1) bis (VII-4), besonders bevorzugt sind die Verbindungen ausgewählt aus Verbindungen der Formeln (VII-1) bis (VII-4), wobei die Verbindungen mindestens einen kondensierten Ring aufweisen 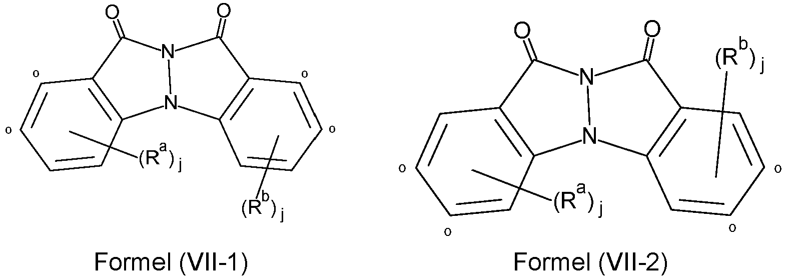 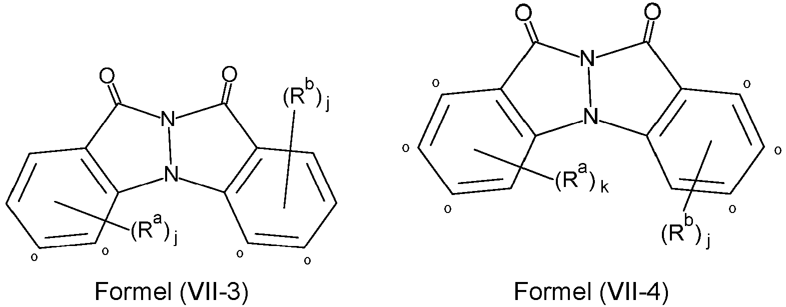 wobei die Symbole R, R^{a} und R^{b} die zuvor, insbesondere für Formel (I) genannten Bedeutungen aufweisen, das Symbol o für die Anbindungsstellen des kondensierten Rings steht und die weiteren Symbole die folgende Bedeutung haben:
- j: ist 0, 1 oder 2, vorzugsweise 0 oder 1;
- k: ist 0 oder 1.

Bevorzugt wird der kondensierte Ring, insbesondere in Formeln (VI-1) bis (VI-22) und/oder (VII-1) bis (VII-4), durch mindestens zwei Reste R, R^{a}, R^{b}, R^{c}, R^{d} und den weiteren Gruppen, an die die zwei Reste R, R^{a}, R^{b}, R^{c}, R^{d} binden, gebildet, wobei die mindestens zwei Reste R, R^{a}, R^{b}, R^{c}, R^{d} Strukturen der Formeln (RA-1) bis (RA-12) und/oder der Formel (RB) formen, vorzugsweise Strukturen der Formeln (RA-1) bis (RA-12).

Bevorzugt kann vorgesehen sein, dass die Verbindungen mindestens zwei kondensierte Ringe aufweisen, wobei mindestens ein kondensierter Ring durch Strukturen der Formeln (RA-1) bis (RA-12) und/oder (RA-1a) bis (RA-4f) gebildet ist und ein weiterer Ring durch Strukturen der Formeln (RA-1) bis (RA-12), (RA-1a) bis (RA-4f) oder (RB) gebildet ist.

Insbesondere in den Formeln (VI-1) bis (VI-22) und/oder (VII-1) bis (VII-4) ist die Summe der Indices k, j und m vorzugsweise 0, 1, 2 oder 3, besonders bevorzugt 1 oder 2.

Weiterhin kann vorgesehen sein, dass die Substituenten R, R^{a}, R^{b}, R^{c}, R^{d}, R^{e}, R¹ und R² gemäß obigen Formeln mit den Ringatomen des Ringssystems, an das die Substituenten R, R^{a}, R^{b}, R^{c}, R^{d}, R^{e}, R¹ und R² binden, kein kondensiertes aromatisches oder heteroaromatisches Ringsystem bilden. Dies schließt die Bildung eines kondensierten aromatischen oder heteroaromatischen Ringsystems mit möglichen Substituenten R¹ und R² ein, die an die Reste R, R^{a}, R^{b}, R^{c}, R^{d}, R^{e} und R¹ gebunden sein können.

Wenn zwei Reste, die insbesondere ausgewählt sein können aus R, R^{a}, R^{b}, R^{c}, R^{d}, R^{e}, R¹ und/oder R², miteinander ein Ringsystem bilden, so kann dieses mono- oder polycyclisch, aliphatisch, heteroaliphatisch, aromatisch oder heteroaromatisch sein. Dabei können die Reste, die miteinander ein Ringsystem bilden, benachbart sein, d.h. dass diese Reste an dasselbe Kohlenstoffatom oder an Kohlenstoffatome, die direkt aneinander gebunden sind, gebunden sind, oder sie können weiter voneinander entfernt sein. Weiterhin können die mit den Substituenten R, R^{a}, R^{b}, R^{c}, R^{d}, R^{e}, R¹ und/oder R² versehenen Ringsysteme auch über eine Bindung miteinander verbunden sein, so dass hierdurch ein Ringschluss bewirkt werden kann. In diesem Fall ist jede der entsprechenden Bindungsstellen vorzugsweise mit einem Substituenten R, R^{a}, R^{b}, R^{c}, R^{d}, R^{e}, R¹ und/oder R² versehen.

Gemäß einer bevorzugten Ausgestaltung ist eine erfindungsgemäße Verbindung durch mindestens eine der Strukturen gemäß Formeln (I), (II-1) bis (II-42), (III-1) bis (III-42), (IV-1) bis (IV-41), (V-1) bis (V-8), (VI-1) bis (VI-22) und/oder (VII-1) bis (VII-4) darstellbar. Vorzugsweise weisen erfindungsgemäße Verbindungen, bevorzugt umfassend Strukturen gemäß Formeln (I), (II-1) bis (II-42), (III-1) bis (III-42), (IV-1) bis (IV-41), (V-1) bis (V-8), (VI-1) bis (VI-22) und/oder (VII-1) bis (VII-4) ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1200 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Bevorzugte aromatische bzw. heteroaromatische Ringsysteme R, R^{a}, R^{b}, R^{c}, R^{d}, R^{e}, Ar' und/oder Ar sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3-, 4- oder 9-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R¹ oder R substituiert sein können.

Vorzugsweise kann vorgesehen sein, dass mindestens ein Substituent R, R^{a}, R^{b} gleich oder verschieden bei jedem Auftreten ausgewählt ist aus der Gruppe bestehend aus H, D, einer verzweigte oder cyclische Alkyl-, Alkoxy- oder Thioalkoxygruppe mit 3 bis 20 C-Atomen oder einem aromatischen oder heteroaromatischen Ringsystem ausgewählt aus den Gruppen der folgenden Formeln Ar-1 bis Ar-78, vorzugsweise die Substituenten R, R^{a}, R^{b} entweder einen kondensierten Ring, vorzugsweise gemäß den Strukturen der Formeln (RA-1) bis (RA-12) oder (RB) bilden oder der Substituent R, R^{a}, R^{b} gleich oder verschieden bei jedem Auftreten ausgewählt ist aus der Gruppe bestehend aus H, D oder einem aromatischen oder heteroaromatischen Ringsystem ausgewählt aus den Gruppen der folgenden Formeln Ar-1 bis Ar-78, und/oder die Gruppe Ar' gleich oder verschieden bei jedem Auftreten ausgewählt ist aus den Gruppen der folgenden Formeln Ar-1 bis Ar-78, 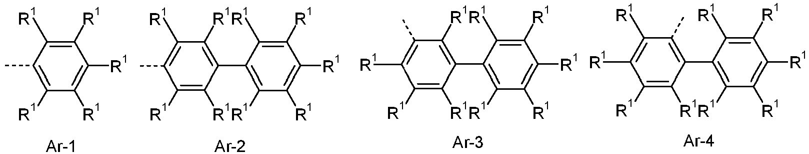 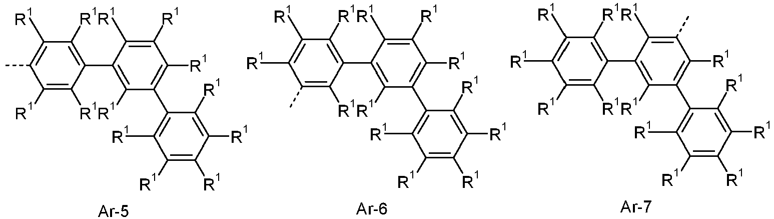 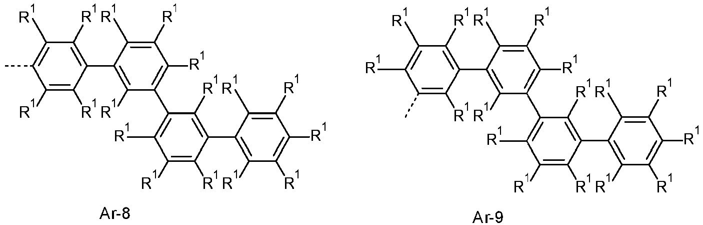 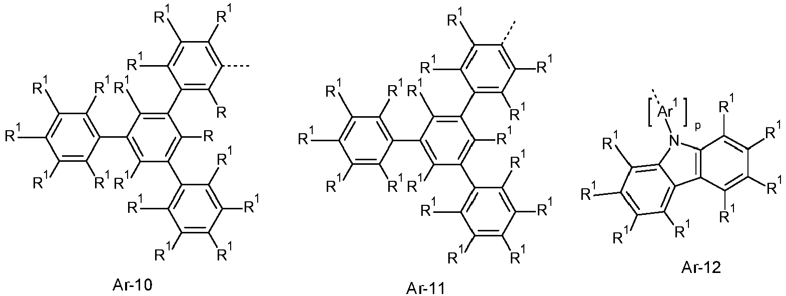 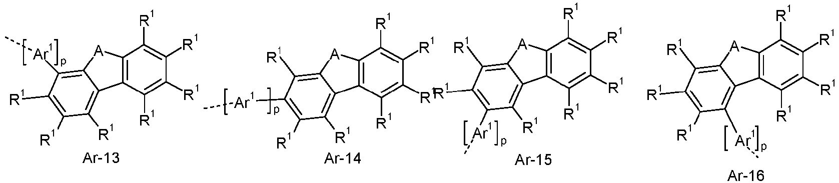 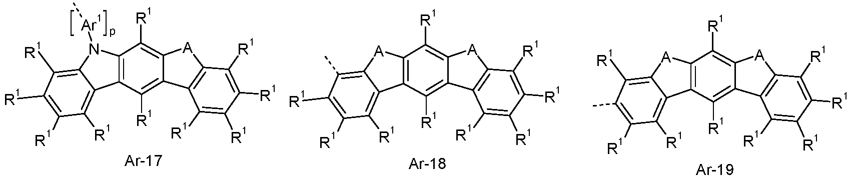 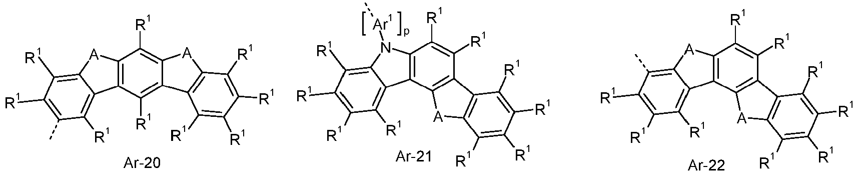 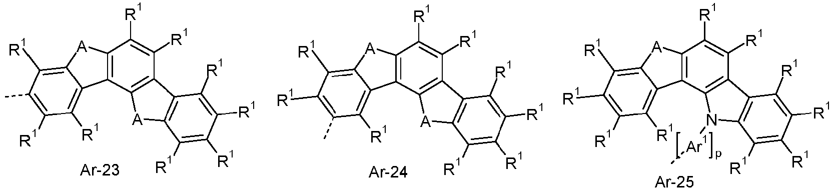 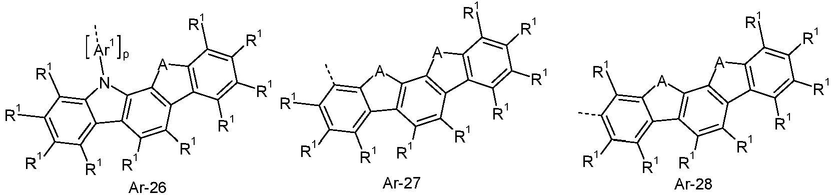 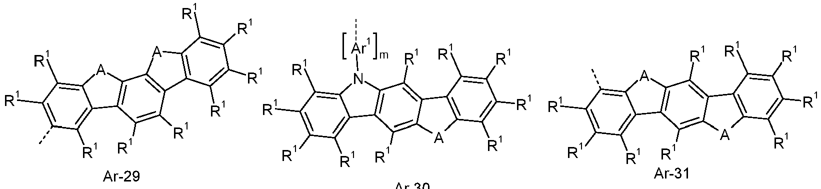 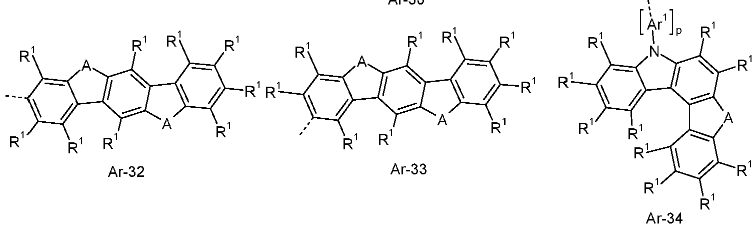 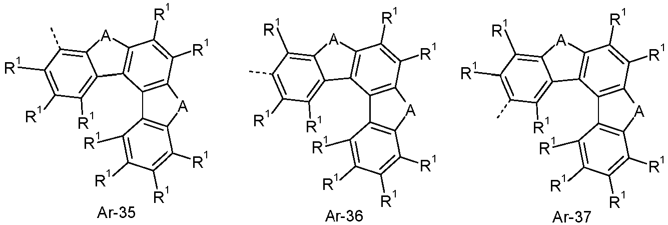 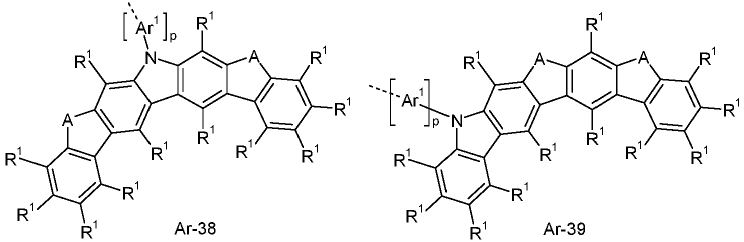 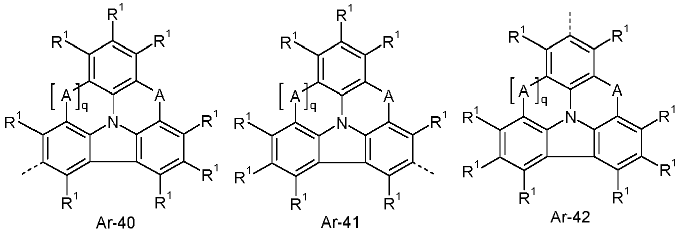 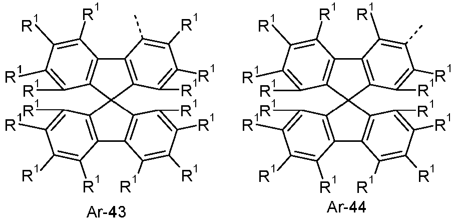 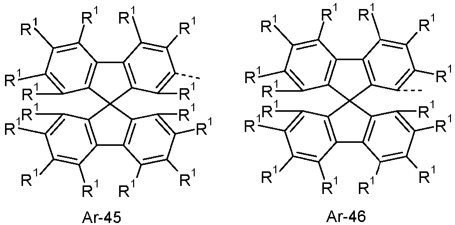 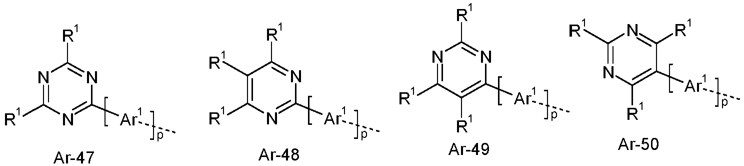 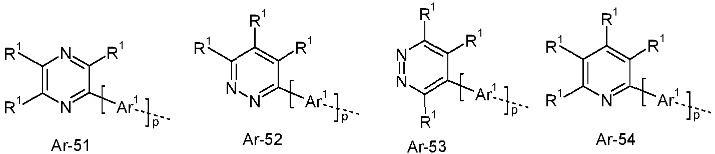 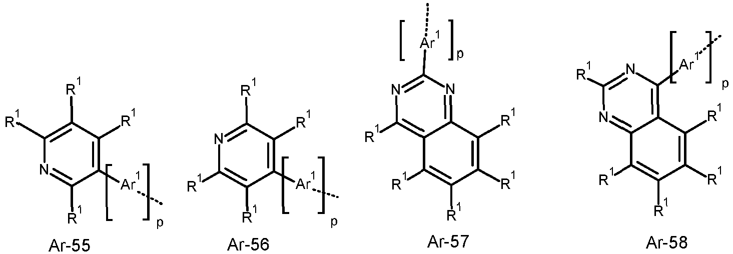 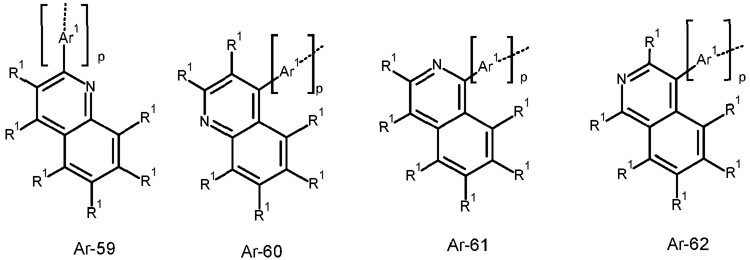 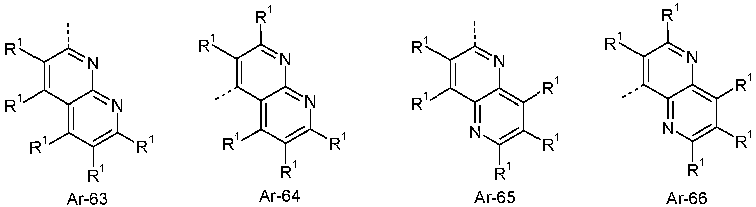 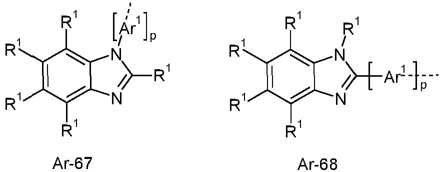 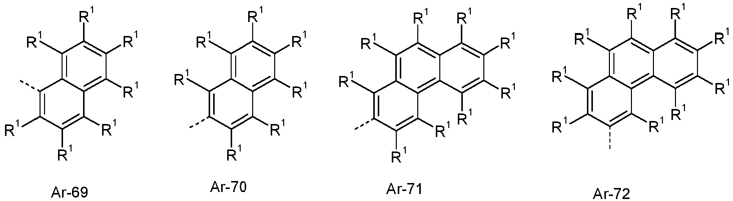 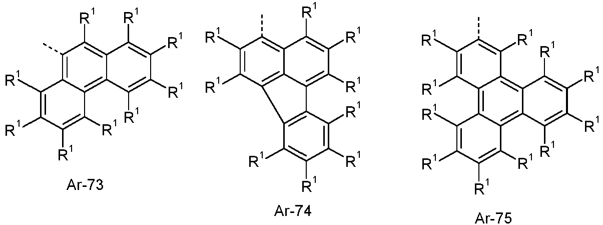 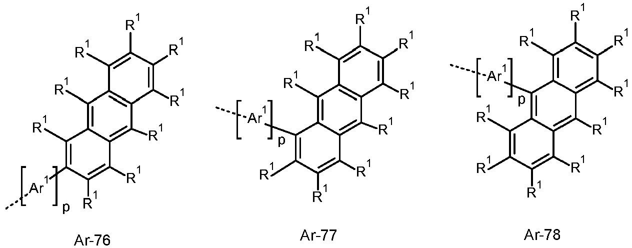 wobei R¹ die oben genannten Bedeutungen aufweist, die gestrichelte Bindung die Anbindungstelle darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
- A: ist bei jedem Auftreten gleich oder verschieden C(R¹)2, NR¹, O oder S;
- p: ist 0 oder 1, wobei p = 0 bedeutet, dass die Gruppe Ar¹ nicht vorhanden ist und dass die entsprechende aromatische bzw. heteroaromatische Gruppe direkt an den entsprechenden Rest gebunden ist;
- q: ist 0 oder 1, wobei q = 0 bedeutet, dass an dieser Position keine Gruppe A gebunden ist und an die entsprechenden Kohlenstoffatome statt dessen Reste R¹ gebunden sind.

Hierbei sind Strukturen der Formeln (Ar-1), (Ar-2), (Ar-3), (Ar-12), (Ar-13), (Ar-14), (Ar-15), (Ar-16), (Ar-40), (Ar-41), (Ar-42), (Ar-43), (Ar-45), (Ar-47), (Ar-57), (Ar-69), (Ar-70), (Ar-75), (Ar-78) bevorzugt und Strukturen der Formeln (Ar-1), (Ar-2), (Ar-3), (Ar-12), (Ar-13), (Ar-14), (Ar-15), (Ar-16) besonders bevorzugt.

Wenn die oben genannten Gruppen für Strukturen der Formeln (Ar-1) bis (Ar-78) mehrere Gruppen A aufweisen, so kommen hierfür alle Kombinationen aus der Definition von A in Frage. Bevorzugte Ausführungsformen sind dann solche, in denen eine Gruppe A für NR¹ und die andere Gruppe A für C(R¹)₂ steht oder in denen beide Gruppen A für NR¹ stehen oder in denen beide Gruppen A für O stehen.

Wenn A für NR¹ steht, steht der Substituent R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R² substituiert sein kann. In einer besonders bevorzugten Ausführungsform steht dieser Substituent R¹ gleich oder verschieden bei jedem Auftreten für ein aromatisches oder heteroaromatisches Ringsystem mit 6 bis 24 aromatischen Ringatomen, insbesondere mit 6 bis 18 aromatischen Ringatomen, welches keine kondensierten Arylgruppen aufweist und welches keine kondensierten Heteroarylgruppen, in denen zwei oder mehr aromatische bzw. heteroaromatische 6-RingGruppen direkt aneinander ankondensiert sind, aufweist, und welches jeweils auch durch einen oder mehrere Reste R² substituiert sein kann. Bevorzugt sind Phenyl, Biphenyl, Terphenyl und Quaterphenyl mit Verknüpfungsmustern, wie vorne für Ar-1 bis Ar-11 aufgeführt, wobei diese Strukturen statt durch R¹ durch einen oder mehrere Reste R² substituiert sein können, bevorzugt aber unsubstituiert sind. Bevorzugt sind weiterhin Triazin, Pyrimidin und Chinazolin, wie vorne für Ar-47 bis Ar-50, Ar-57 und Ar-58 aufgeführt, wobei diese Strukturen statt durch R¹ durch einen oder mehrere Reste R² substituiert sein können.

Im Folgenden werden bevorzugte Substituenten R, R^{a}, R^{b}, R^{c}, R^{d} und R^{e} beschrieben.

In einer bevorzugten Ausführungsform der Erfindung ist R, R^{a}, R^{b} gleich oder verschieden bei jedem Auftreten ausgewählt aus der Gruppe bestehend aus H, D, F, CN, NO₂, Si(R¹)₃, B(OR¹)₂, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R¹ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann.

In einer weiterhin bevorzugten Ausführungsform der Erfindung ist Substituent R, R^{a}, R^{b} gleich oder verschieden bei jedem Auftreten ausgewählt aus der Gruppe bestehend aus H, D, F, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R¹ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann.

Ferner kann vorgesehen sein, dass mindestens ein Substituent Substituent R, R^{a}, R^{b} gleich oder verschieden bei jedem Auftreten ausgewählt ist aus der Gruppe bestehend aus H, D, einem aromatischen oder heteroaromatischen Ringsystem mit 6 bis 30 aromatischen Ringatomen, welches mit einem oder mehreren Resten R¹ substituiert sein kann, oder einer Gruppe N(Ar')₂. In einer weiterhin bevorzugten Ausführungsform der Erfindung bilden die Substituenten R, R^{a}, R^{b}, R^{c}, R^{d} entweder einen Ring gemäß den Strukturen der Formeln (RA-1) bis (RA-12), (RA-1a) bis (RA-4f) oder (RB) oder der Substituent R, R^{a}, R^{b} ist gleich oder verschieden bei jedem Auftreten ausgewählt aus der Gruppe bestehend aus H, D, einem aromatischen oder heteroaromatischen Ringsystem mit 6 bis 30 aromatischen Ringatomen, welches mit einem oder mehreren Resten R¹ substituiert sein kann, oder einer Gruppe N(Ar')₂. Besonders bevorzugt ist Substituent R, R^{a}, R^{b} gleich oder verschieden bei jedem Auftreten ausgewählt aus der Gruppe bestehend aus H oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R¹ substituiert sein kann.

Bevorzugt kann vorgesehen sein, dass mindestens ein Substituent R, R^{a}, R^{b}, R^{c}, R^{d} ausgewählt ist aus Phenyl, Biphenyl, Terphenyl, Quaterphenyl, Fluoren, Spirobifluoren, Naphthalin, Indol, Benzofuran, Benzothiophen, Carbazol, Dibenzofuran, Dibenzothiophen, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R¹ substituiert sein können. Hierbei bedeutet der Ausdruck Substituent insbesondere, dass R, R^{a}, R^{b}, R^{c}, R^{d} ungleich H sind. Ferner können die Substituenten R, R^{a}, R^{b}, R^{c}, R^{d} gleich oder verschieden sein, falls zwei oder mehr Substituenten vorhanden sind, die aus den genannten aromatischen oder heteroaromatischen Gruppe ausgewählt sind.

In einer weiteren Ausführungsform kann vorgesehen sein, dass mindestens ein Substituent R, R^{a}, R^{b}, R^{c}, R^{d} ausgewählt ist aus o-Biphenyl, o,o'-Terphenyl, o.,o',p-Quaterphenyl, 4,6-Diphenyl-pyrimidin-2-yl, 4,6-Diphenyl-trianin-2yl, Naphthalin, Phenanthren, Chrysen, Spiro-Bifluoren, Triphenylen, Anthracen, Benzanthracen, Fluoren und/oder Pyren, welche jeweils mit einem oder mehreren Resten R¹ substituiert sein können. Hierbei sind Spiro-Bifluoren, o-Biphenyl, o,o'-Terphenyl, o,o',p-Quaterphenyl, 4,6-Diphenyl-pyrimidin-2-yl, 4,6-Diphenyl-trianin-2yl-Reste bevorzugt. Die Substituenten R, R^{a}, R^{b}, R^{c}, R^{d} können gleich oder verschieden sein, falls zwei oder mehr Substituenten vorhanden sind, die aus den genannten aromatischen Gruppe ausgewählt sind. Strukturen/Verbindungen mit einer Gruppe ausgewählt aus o-Biphenyl, o,o'-Terphenyl, o.,o',p-Quaterphenyl, 4,6-Diphenyl-pyrimidin-2-yl, 4,6-Diphenyl-trianin-2yl, Naphthalin, Phenanthren, Chrysen, Spiro-Bifluoren, Triphenylen, Anthracen, Benzanthracen, Fluoren und/oder Pyren sind insbesondere zur Verwendung als Elektronentransportmaterial und/oder als Matrixmaterial geeignet.

Für die Substituenten R^{c}, R^{d} gilt das für die zuvor in Bezug auf die Substituenten R, R^{a}, R^{b} dargelegten Bevorzugungen mit den zuvor genannten Einschränkungen entsprechend. Insbesondere kann vorgesehen sein, dass mindestens ein Substituent R^{c}, R^{d} gleich oder verschieden bei jedem Auftreten ausgewählt ist aus der Gruppe bestehend aus einem aromatischen oder heteroaromatischen Ringsystem mit 6 bis 30 aromatischen Ringatomen, welches mit einem oder mehreren Resten R¹ substituiert sein kann, oder einer Gruppe N(Ar')₂. Darüber hinaus kann vorgesehen sein, dass die Substituenten R^{c}, R^{d} entweder einen kondensierten Ring, vorzugsweise gemäß den Strukturen der Formeln (RA-1) bis (RA-12) oder (RB) bilden oder der Substituent R^{c}, R^{d} gleich oder verschieden bei jedem Auftreten ausgewählt ist aus der Gruppe bestehend aus einem aromatischen oder heteroaromatischen Ringsystem mit 6 bis 30 aromatischen Ringatomen, welches mit einem oder mehreren Resten R¹ substituiert sein kann, oder einer Gruppe N(Ar')₂.

Besonders bevorzugt kann vorgesehen sein, dass der Substituent R^{c}, R^{d} gleich oder verschieden bei jedem Auftreten ein aromatisches oder heteroaromatisches Ringsystem mit 6 bis 30 aromatischen Ringatomen darstellt, welches mit einem oder mehreren Resten R¹ substituiert sein kann, welches vorzugsweise ausgewählt ist aus den Gruppen der zuvor dargelegten Formeln (Ar-1) bis (Ar-78).

In einer bevorzugten Ausführungsform der Erfindung ist R^{e} gleich oder verschieden bei jedem Auftreten ausgewählt aus der Gruppe bestehend aus einer geradkettigen Alkylgruppe mit 1 bis 20 C-Atomen oder einer verzweigten oder cyclischen Alkylgruppe mit 3 bis 20 C-Atomen, wobei die Alkylgruppe jeweils mit einem oder mehreren Resten R¹ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R² substituiert sein kann.

In einer weiterhin bevorzugten Ausführungsform der Erfindung ist R^{e} gleich oder verschieden bei jedem Auftreten ausgewählt aus der Gruppe bestehend aus einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R² substituiert sein kann, einem aromatischen oder heteroaromatischen Ringsystem mit 6 bis 30 aromatischen Ringatomen, welches mit einem oder mehreren Resten R² substituiert sein kann. Besonders bevorzugt ist R^{e} gleich oder verschieden bei jedem Auftreten ausgewählt aus der Gruppe bestehend aus einer geradkettigen Alkylgruppe mit 1 bis 5 C-Atomen oder einer verzweigten oder cyclischen Alkylgruppe mit 3 bis 5 C-Atomen, wobei die Alkylgruppe jeweils mit einem oder mehreren Resten R² substituiert sein kann oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R² substituiert sein kann.

In einer bevorzugten Ausführungsform der Erfindung ist R^{e} bei jedem Auftreten gleich oder verschieden ausgewählt aus der Gruppe bestehend aus einer geradkettigen Alkylgruppe mit 1 bis 6 C-Atomen oder einer cyclischen Alkylgruppe mit 3 bis 6 C-Atomen, wobei die Alkylgruppe jeweils mit einem oder mehreren Resten R² substituiert sein kann, oder einem aromatischen oder heteroaromatischen Ringsystem mit 6 bis 24 aromatischen Ringatomen, das jeweils durch einen oder mehrere Reste R² substituiert sein kann; dabei können zwei Reste R^{e} auch miteinander ein Ringsystem bilden. Besonders bevorzugt ist R^{e} bei jedem Auftreten gleich oder verschieden ausgewählt aus der Gruppe bestehend aus einer geradkettigen Alkylgruppe mit 1, 2, 3 oder 4 C-Atomen oder einer verzweigten oder cyclischen Alkylgruppe mit 3 bis 6 C-Atomen, wobei die Alkylgruppe jeweils mit einem oder mehreren Resten R² substituiert sein kann, bevorzugt aber unsubstituiert ist, oder einem aromatischen Ringsystem mit 6 bis 12 aromatischen Ringatomen, insbesondere mit 6 aromatischen Ringatomen, das jeweils durch einen oder mehrere, bevorzugt nicht-aromatische Reste R² substituiert sein kann, bevorzugt aber unsubstituiert ist; dabei können zwei Reste R^{e} miteinander ein Ringsystem bilden. Ganz besonders bevorzugt ist R^{e} bei jedem Auftreten gleich oder verschieden ausgewählt aus der Gruppe bestehend aus einer geradkettigen Alkylgruppe mit 1, 2, 3 oder 4 C-Atomen, oder einer verzweigten Alkylgruppe mit 3 bis 6 C-Atomen. Ganz besonders bevorzugt steht R^{e} für eine Methylgruppe oder für eine Phenylgruppe, wobei zwei Phenylgruppen zusammen ein Ringsystem bilden können, wobei eine Methylgruppe gegenüber einer Phenylgruppe bevorzugt ist.

Bevorzugte aromatische bzw. heteroaromatische Ringsysteme, für die Substituent R, R^{a}, R^{b}, R^{c}, R^{d}, R^{e} beziehungsweise Ar oder Ar' stehen, sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R, R¹ beziehungsweise R² substituiert sein können. Besonders bevorzugt sind die oben aufgeführten Strukturen Ar-1 bis Ar-78, wobei Strukturen der Formeln (Ar-1), (Ar-2), (Ar-3), (Ar-12), (Ar-13), (Ar-14), (Ar-15), (Ar-16), (Ar-69), (Ar-70), (Ar-75) bevorzugt und Strukturen der Formeln (Ar-1), (Ar-2), (Ar-3), (Ar-12), (Ar-13), (Ar-14), (Ar-15), (Ar-16) besonders bevorzugt sind. Hinsichtlich der Strukturen Ar-1 bis Ar-78 ist festzuhalten, dass diese mit einem Substituenten R¹ dargestellt sind. Im Falle der Ringsysteme Ar sind diese Substituenten R¹ durch R und im Falle R^{e} sind diese Substituenten R¹ durch R² zu ersetzen.

Weitere geeignete Gruppen R, R^{a}, R^{b}, R^{c}, R^{d} sind Gruppen der Formel -Ar⁴-N(Ar²)(Ar³), wobei Ar², Ar³ und Ar⁴ gleich oder verschieden bei jedem Auftreten für ein aromatisches oder heteroaromatisches Ringsystem mit 5 bis 24 aromatischen Ringatomen stehen, welches jeweils mit einem oder mehreren Resten R¹ substituiert sein kann. Dabei beträgt die Gesamtzahl der aromatischen Ringatome von Ar², Ar³ und Ar⁴ maximal 60 und bevorzugt maximal 40.

Dabei können Ar⁴ und Ar² miteinander und/oder Ar² und Ar³ miteinander auch durch eine Gruppe ausgewählt aus C(R¹)2, NR¹, O oder S verbunden sein. Bevorzugt erfolgt die Verknüpfung von Ar⁴ und Ar² miteinander bzw. von Ar² und Ar³ miteinander jeweils ortho zur Position der Verknüpfung mit dem Stickstoffatom. In einer weiteren Ausführungsform der Erfindung sind keine der Gruppen Ar², Ar³ bzw. Ar⁴ miteinander verbunden.

Bevorzugt ist Ar⁴ ein aromatisches oder heteroaromatisches Ringsystem mit 6 bis 24 aromatischen Ringatomen, bevorzugt mit 6 bis 12 aromatischen Ringatomen, das jeweils mit einem oder mehreren Resten R¹ substituiert sein kann. Besonders bevorzugt ist Ar⁴ ausgewählt aus der Gruppe bestehend aus ortho-, meta- oder para-Phenylen oder ortho-, meta- oder para-Biphenyl, welche jeweils durch einen oder mehrere Reste R¹ substituiert sein können, bevorzugt aber unsubstituiert sind. Ganz besonders bevorzugt ist Ar⁴ eine unsubstituierte Phenylengruppe.

Bevorzugt sind Ar² und Ar³ gleich oder verschieden bei jedem Auftreten ein aromatisches oder heteroaromatisches Ringsystem mit 6 bis 24 aromatischen Ringatomen, das jeweils mit einem oder mehreren Resten R¹ substituiert sein kann. Besonders bevorzugte Gruppen Ar² bzw. Ar³ sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren oder Triphenylen, welche jeweils mit einem oder mehreren Resten R¹ substituiert sein können. Ganz besonders bevorzugt sind Ar² und Ar³ gleich oder verschieden bei jedem Auftreten ausgewählt aus der Gruppe bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 1-, 2-, 3- oder 4-Fluoren, oder Spirobifluoren, insbesondere 1-, 2-, 3- oder 4-Spirobifluoren.

In einer weiteren bevorzugten Ausführungsform der Erfindung ist R¹ gleich oder verschieden bei jedem Auftreten ausgewählt aus der Gruppe bestehend aus H, D, F, CN, einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R² substituiert sein kann, oder einem aromatischen oder heteroaromatischen Ringsystem mit 6 bis 24 aromatischen Ringatomen, das jeweils durch einen oder mehrere Reste R² substituiert sein kann. In einer besonders bevorzugten Ausführungsform der Erfindung ist R¹ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten R⁵ substituiert sein kann, bevorzugt aber unsubstituiert ist, oder einem aromatischen oder heteroaromatischen Ringsystem mit 6 bis 13 aromatischen Ringatomen, das jeweils durch einen oder mehrere Reste R⁵ substituiert sein kann, bevorzugt aber unsubstituiert ist.

In einer weiteren bevorzugten Ausführungsform der Erfindung ist R² gleich oder verschieden bei jedem Auftreten H, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Dabei haben in erfindungsgemäßen Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Ferner kann vorgesehen sein, dass die Verbindung genau zwei oder genau drei Strukturen gemäß Formel (I), (II-1) bis (II-42), (III-1) bis (III-41), (IV-1) bis (IV-41), (V-1) bis (V-8), (VI-1) bis (VI-22) und/oder (VII-1) bis (VII-4) umfasst.

In einer bevorzugten Ausgestaltung sind die Verbindungen ausgewählt aus Verbindungen der Formel (D-1) oder (D-2), 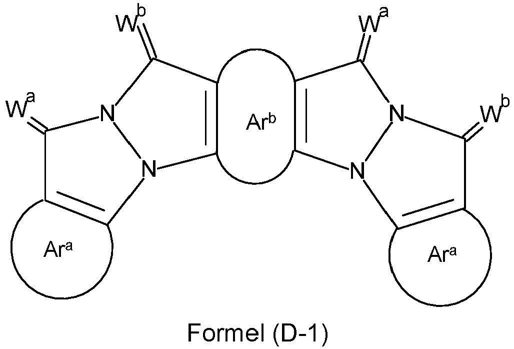 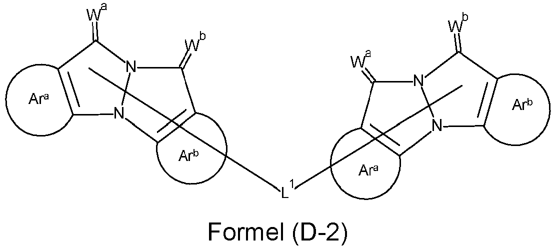 wobei die Gruppe L' eine Verbindungsgruppe, vorzugsweise eine Bindung oder ein aromatisches oder heteroaromatisches Ringsystem mit 5 bis 40, bevorzugt 5 bis 30 aromatischen Ringatomen darstellt, welches durch einen oder mehrere Reste R¹ substituiert sein kann, und die weiteren verwendeten Symbole die zuvor, insbesondere für Formel (I) genannten Bedeutungen aufweisen.

In einer weiteren bevorzugten Ausführungsform der Erfindung steht L¹ für eine Bindung oder für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen, welches durch einen oder mehrere Reste R¹ substituiert sein kann, bevorzugt aber unsubstituiert ist, wobei R¹ die zuvor, insbesondere für Formel (I) genannte Bedeutung aufweisen kann. Besonders bevorzugt steht L' für ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste R² substituiert sein kann, bevorzugt aber unsubstituiert ist, wobei R² die zuvor, insbesondere für Formel (I) genannte Bedeutung aufweisen kann.

Weiterhin bevorzugt steht das unter anderem in Formel (D2) dargelegte Symbol L' gleich oder verschieden bei jedem Auftreten für eine Bindung oder einen Aryl- oder Heteroarylrest mit 5 bis 24 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die in Formel (D2) dargelegte Gruppe L¹ ein aromatisches Ringsystem mit höchstens vier, bevorzugt höchstens drei, besonders bevorzugt höchstens zwei kondensierten aromatischen und/oder heteroaromatischen 6-Ringen, vorzugsweise kein kondensiertes aromatisches oder heteroaromatisches Ringsystem umfasst. Demgemäß sind Naphthylstrukturen gegenüber Anthracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt.

Besonders bevorzugt sind Strukturen, die keine Kondensation aufweisen, wie beispielsweise Phenyl-, Biphenyl-, Terphenyl- und/oder Quaterphenyl-Strukturen.

Beispiele für geeignete aromatische oder heteroaromatische Ringsysteme L¹ sind ausgewählt aus der Gruppe bestehend aus ortho-, meta- oder para-Phenylen, ortho-, meta- oder para-Biphenylen, Terphenylen, insbesondere verzweigtes Terphenylen, Quaterphenylen, insbesondere verzweigtes Quaterphenylen, Fluorenylen, Spirobifluorenylen, Dibenzofuranylen, Dibenzothienylen und Carbazolylen, die jeweils durch einen oder mehrere Reste R¹ substituiert sein können, bevorzugt aber unsubstituiert sind.

Die oben genannten bevorzugten Ausführungsformen können beliebig innerhalb der in Anspruch 1 definierten Einschränkungen miteinander kombiniert werden. In einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die in der folgenden Tabelle aufgeführten Verbindungen:

| | |
|---|---|
| 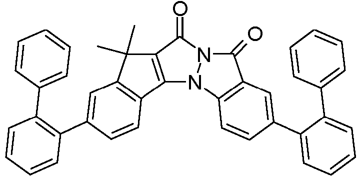 | 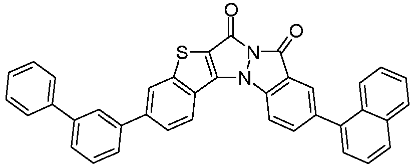 |
| 1 | 2 |
| 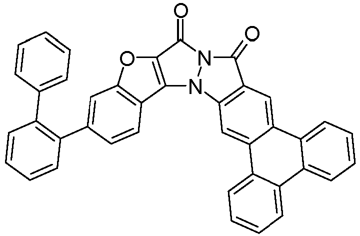 | 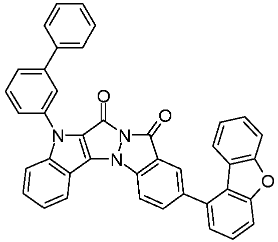 |
| 3 | 4 |
| 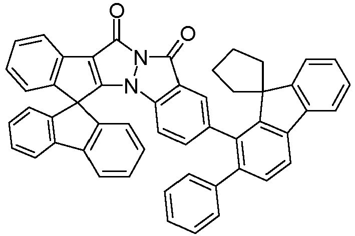 | 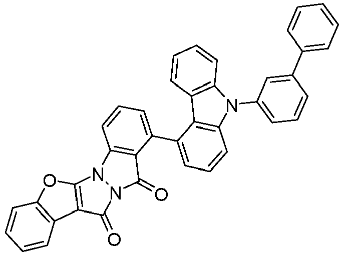 |
| 5 | 6 |
| 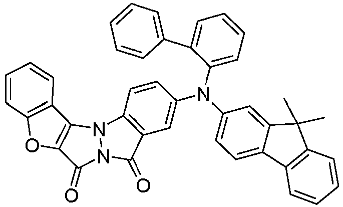 | 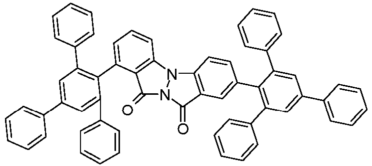 |
| 7 | 8 |
| 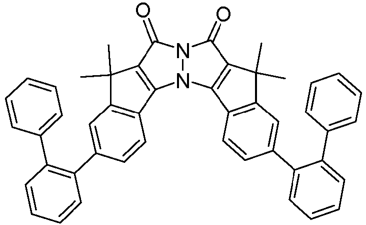 | 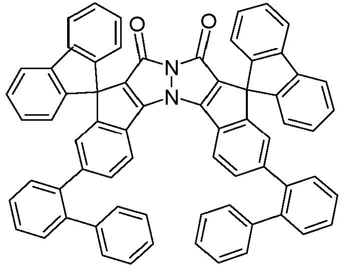 |
| 9 | 10 |
| 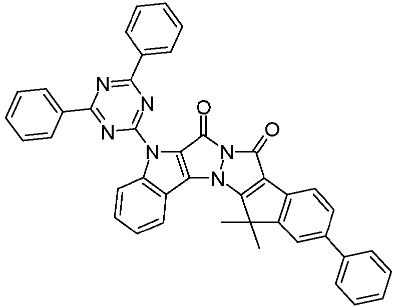 | 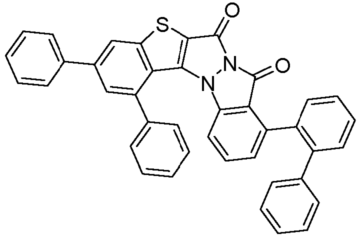 |
| 11 | 12 |
| 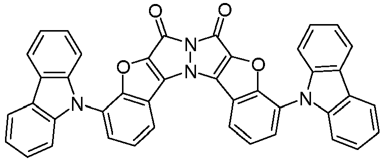 | 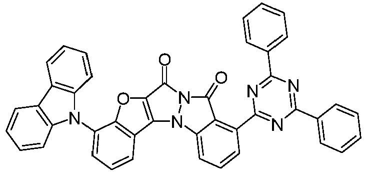 |
| 13 | 14 |
| 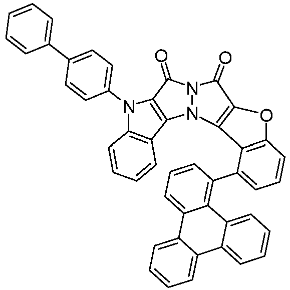 | 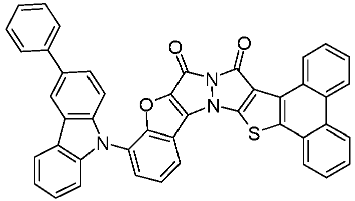 |
| 15 | 16 |
| 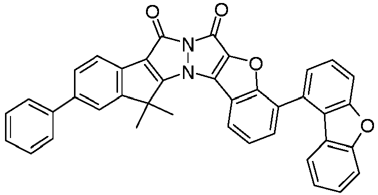 | 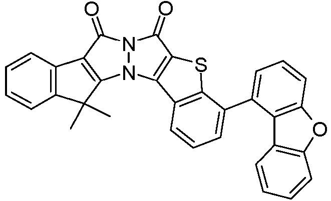 |
| 17 | 18 |
| 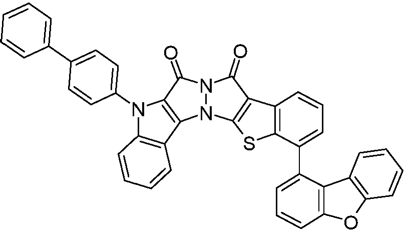 | 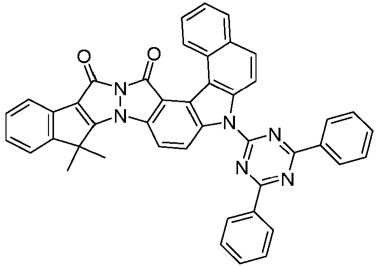 |
| 19 | 20 |
| 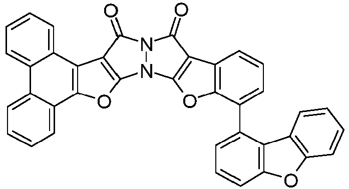 | 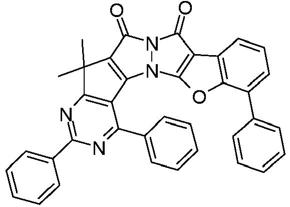 |
| 21 | 22 |
| 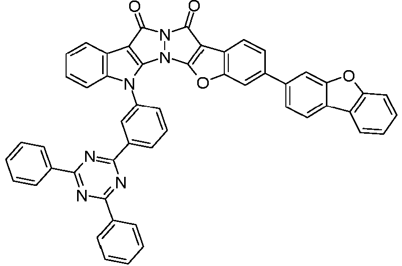 | 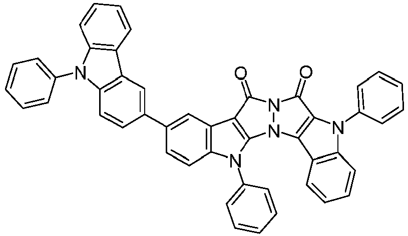 |
| 23 | 24 |
| 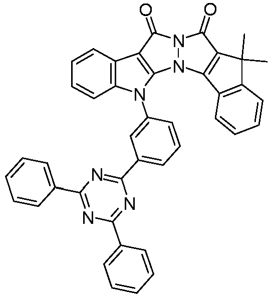 | 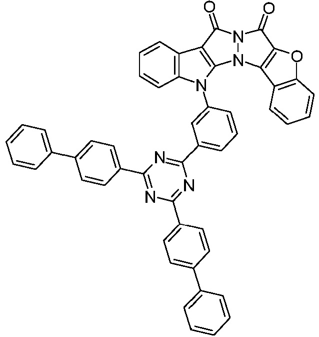 |
| 25 | 26 |
| 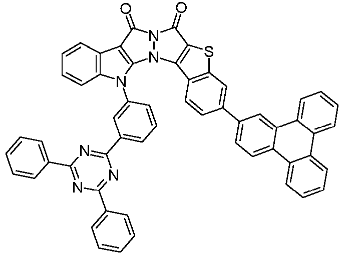 | 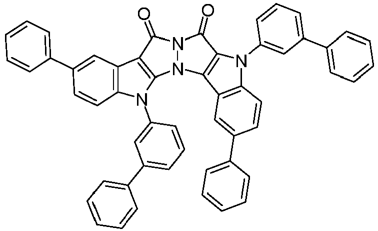 |
| 27 | 28 |
| 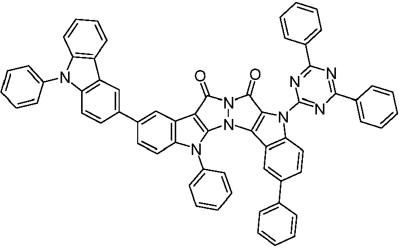 | 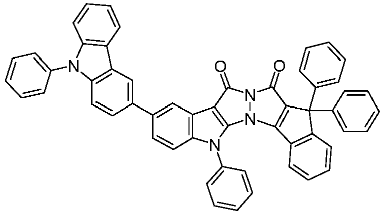 |
| 29 | 30 |
| 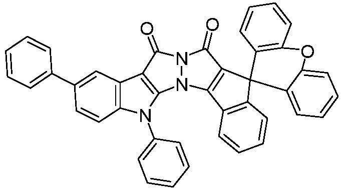 | 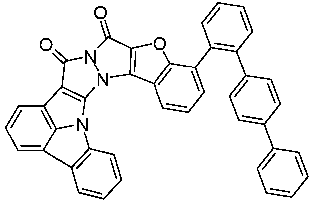 |
| 31 | 32 |
| 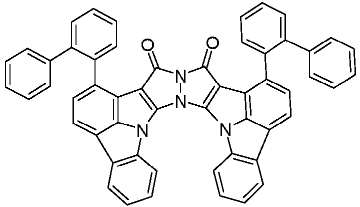 | 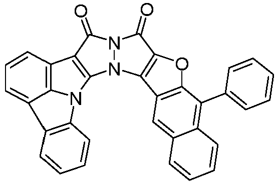 |
| 33 | 34 |
| 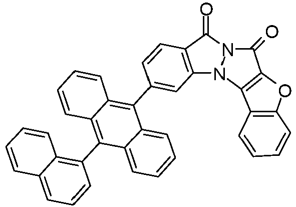 | 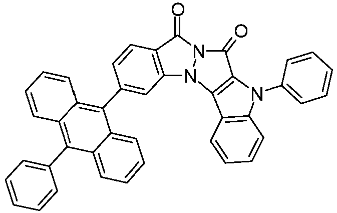 |
| 35 | 36 |
| 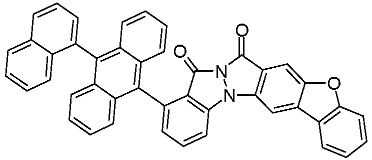 | 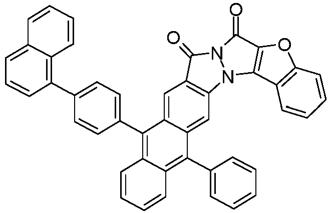 |
| 37 | 38 |
| 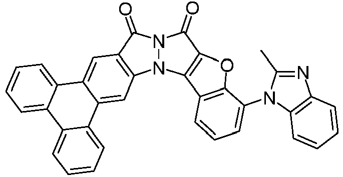 | 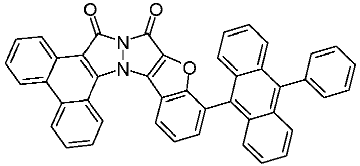 |
| 39 | 40 |
| 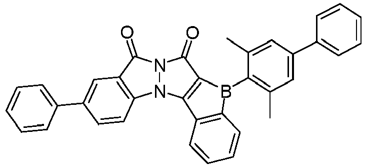 | 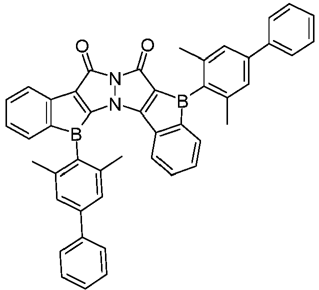 |
| 41 | 42 |
| 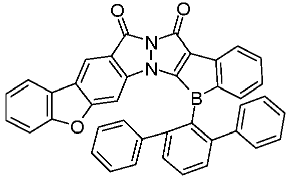 | 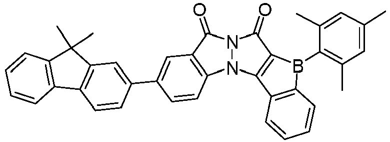 |
| 43 | 44 |
| 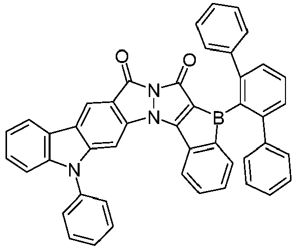 | 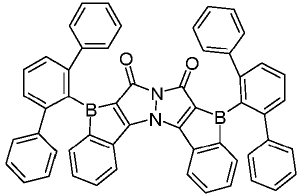 |
| 45 | 46 |
| 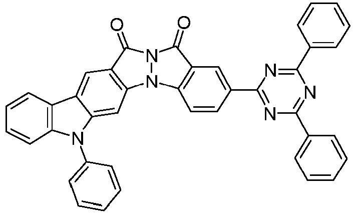 | 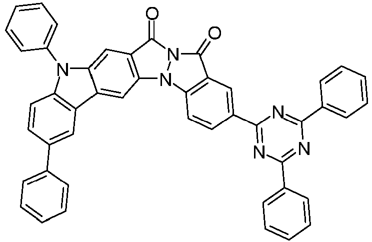 |
| 47 | 48 |
| 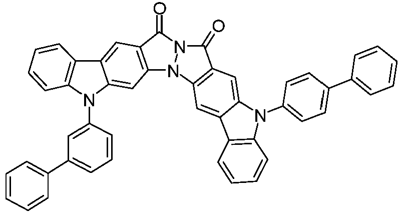 | 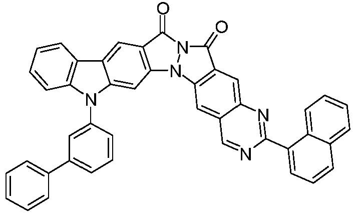 |
| 49 | 50 |
| 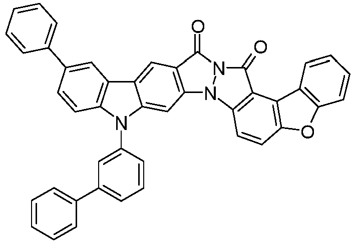 | 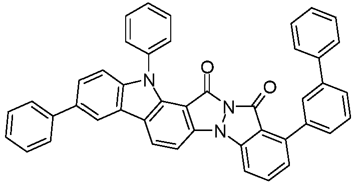 |
| 51 | 52 |
| 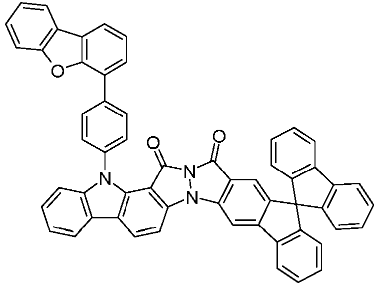 | 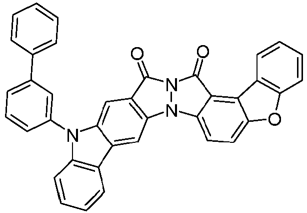 |
| 53 | 54 |
| 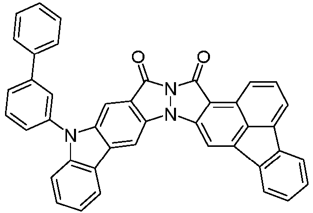 | 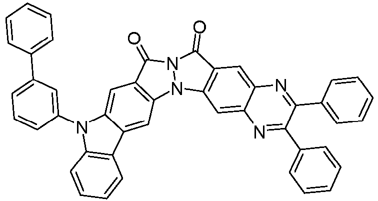 |
| 55 | 56 |
| 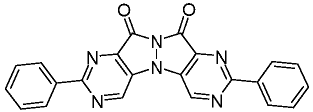 | 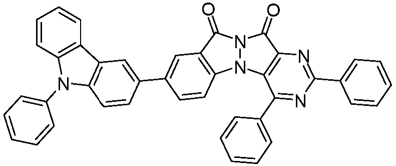 |
| 57 | 58 |
| 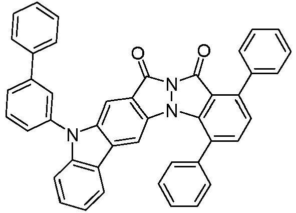 | 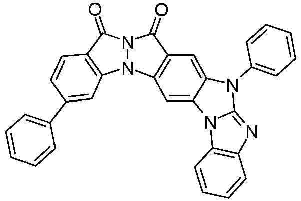 |
| 59 | 60 |
| 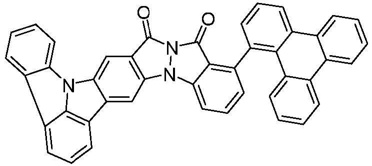 | 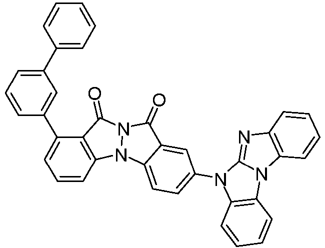 |
| 61 | 62 |
| 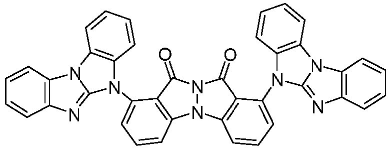 | 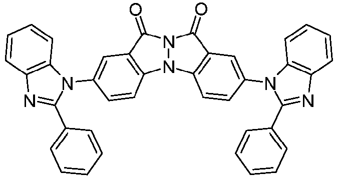 |
| 63 | 64 |
| 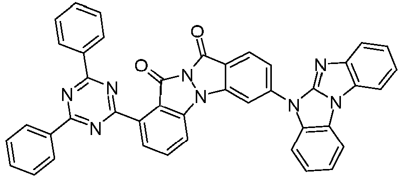 | 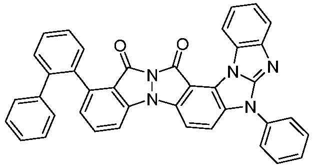 |
| 65 | 66 |
| 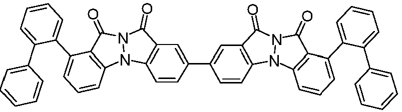 | 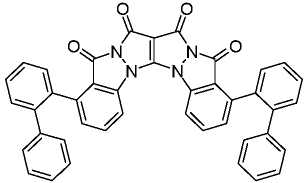 |
| 67 | 68 |
| 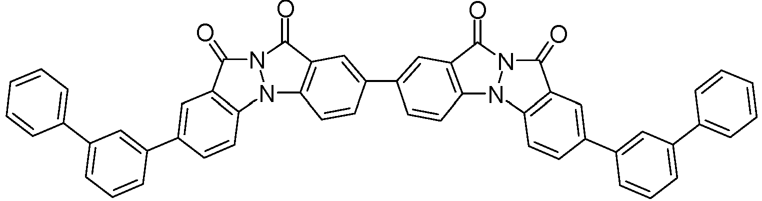 | |
| 69 | |
| 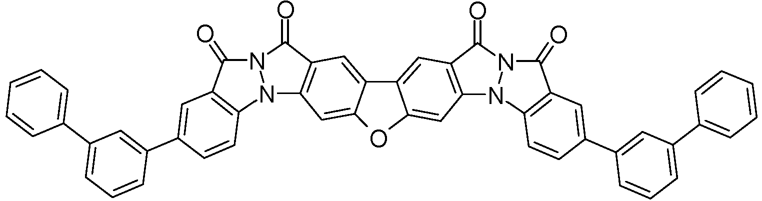 | |
| 70 | |
| 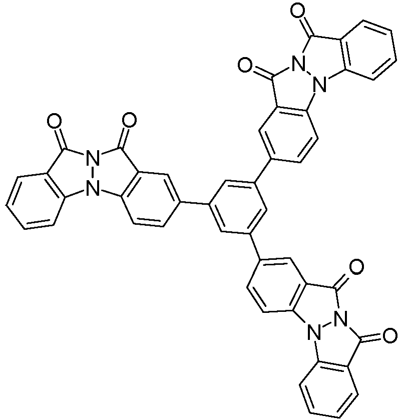 | 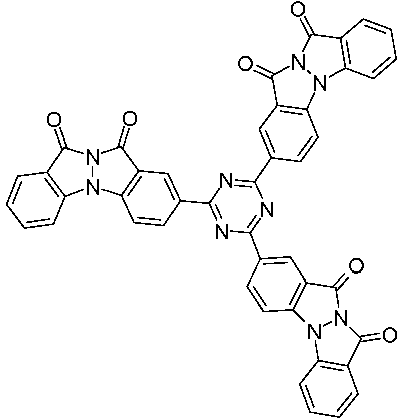 |
| 71 | 72 |
| 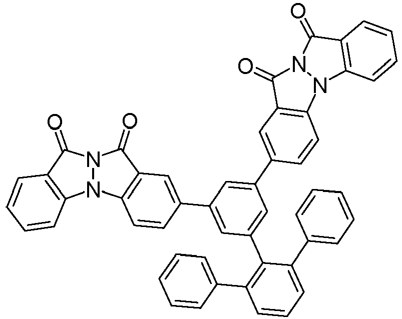 | 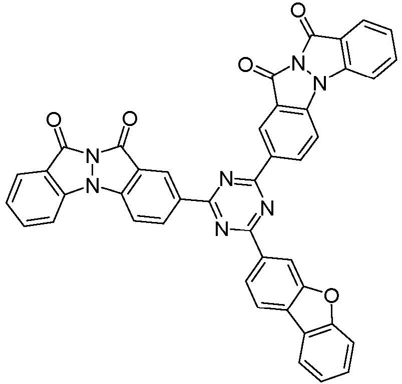 |
| 73 | 74 |
| 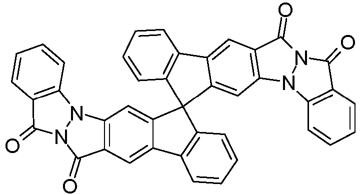 | 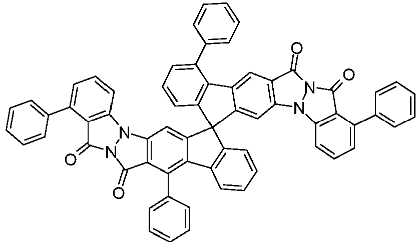 |
| 75 | 76 |
| 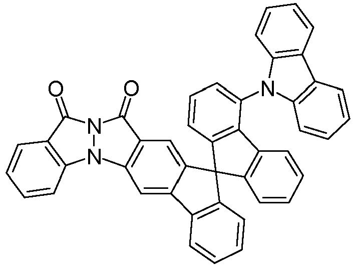 | 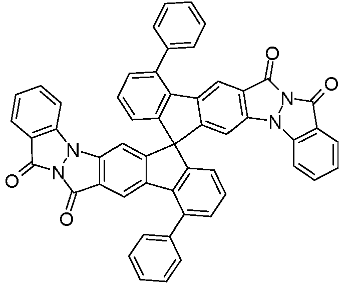 |
| 77 | 78 |
| 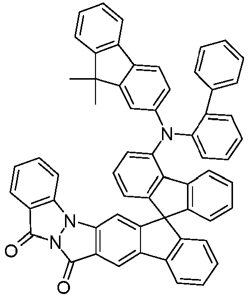 | 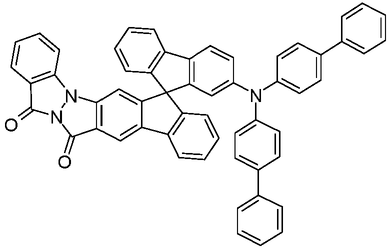 |
| 79 | 80 |
| 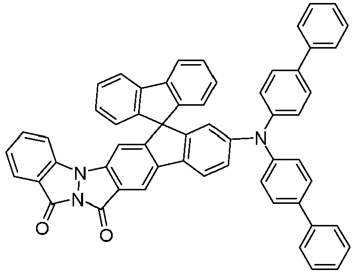 | 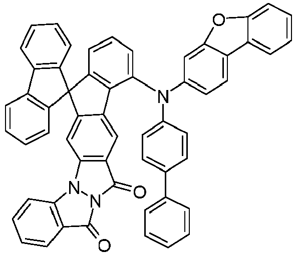 |
| 81 | 82 |
| 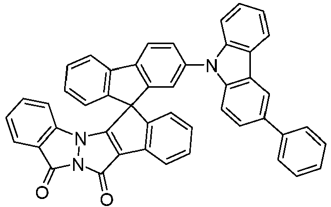 | 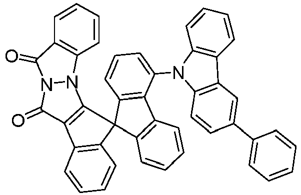 |
| 83 | 84 |
| 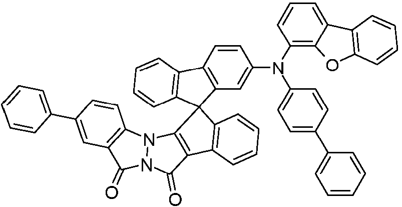 | 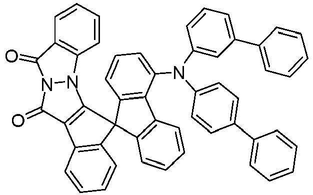 |
| 85 | 86 |
| 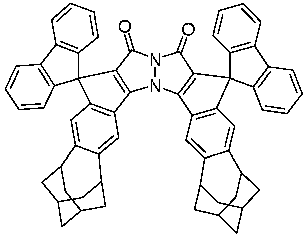 | 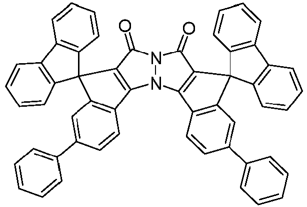 |
| 87 | 88 |
| 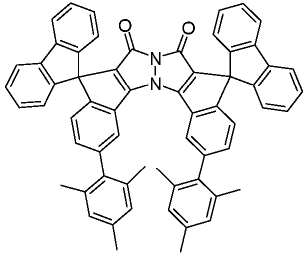 | 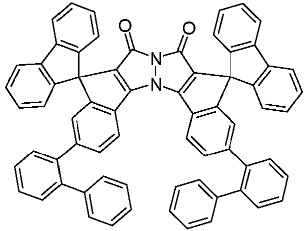 |
| 89 | 90 |
| 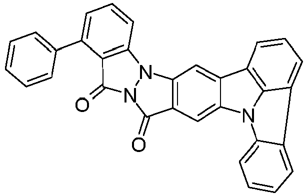 | 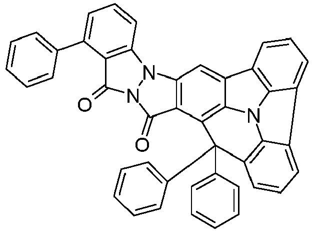 |
| 91 | 92 |
| 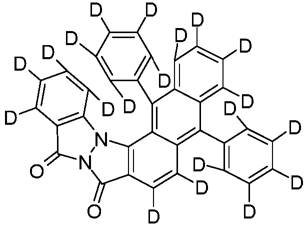 | 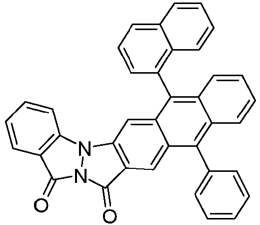 |
| 93 | 94 |
| 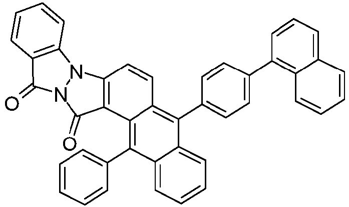 | 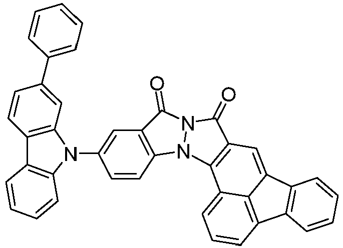 |
| 95 | 96 |
| 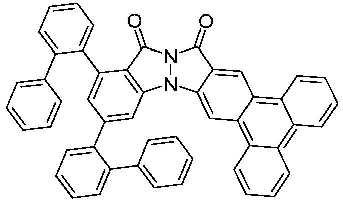 | 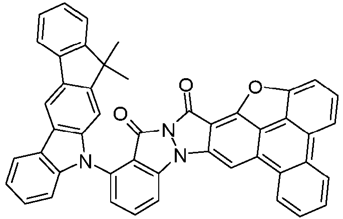 |
| 97 | 98 |
| 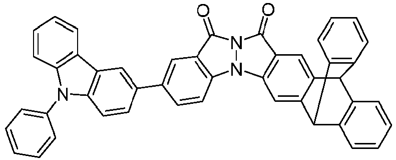 | 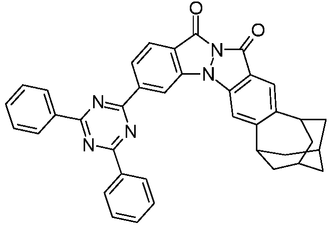 |
| 99 | 100 |
| 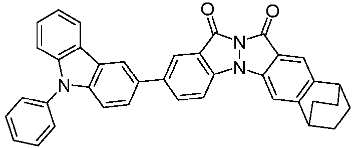 | 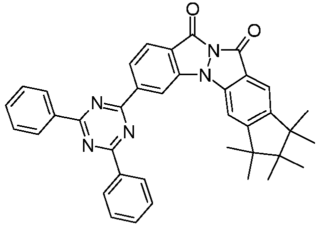 |
| 101 | 102 |
| 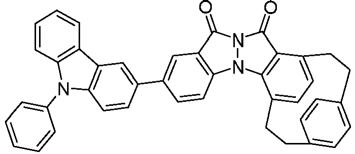 | 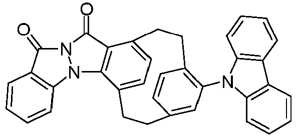 |
| 103 | 104 |
| 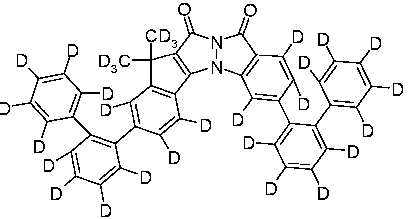 | 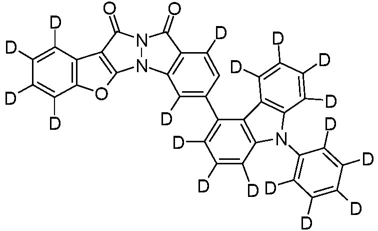 |
| 105 | 106 |
| 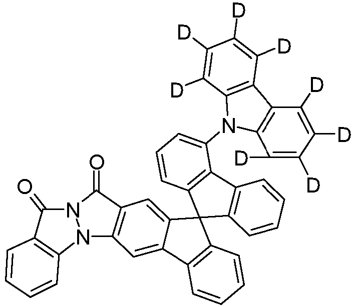 | 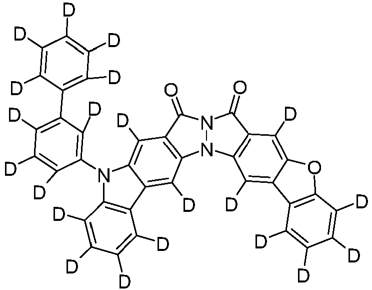 |
| 107 | 108 |
| 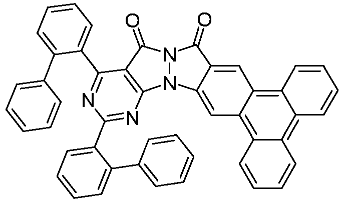 | 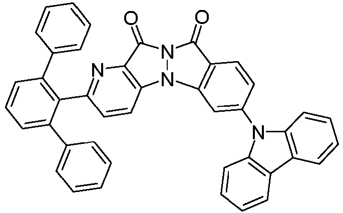 |
| 109 | 110 |
| 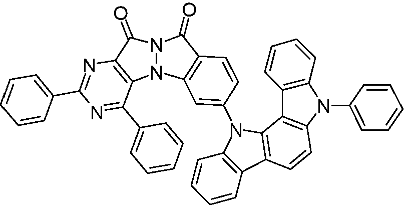 | 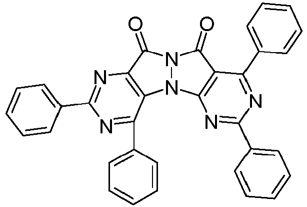 |
| 111 | 112 |
| 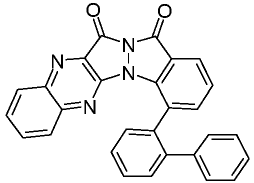 | 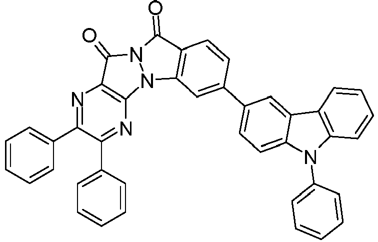 |
| 113 | 114 |
| 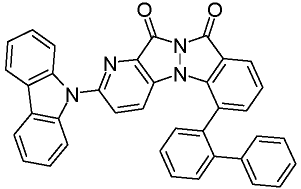 | 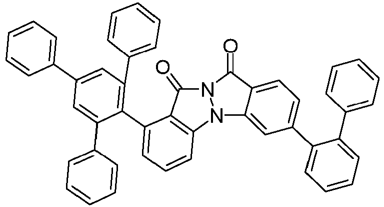 |
| 115 | 116 |
| 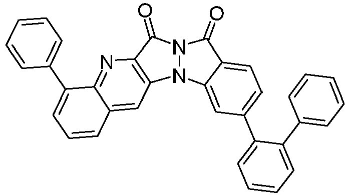 | 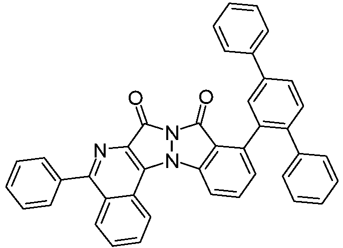 |
| 117 | 118 |
| 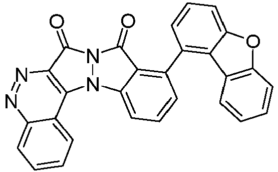 | 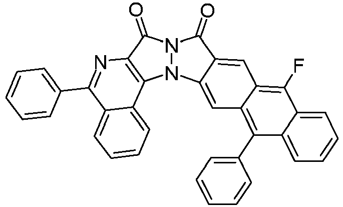 |
| 119 | 120 |
| 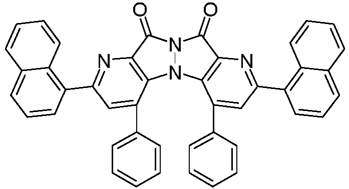 | 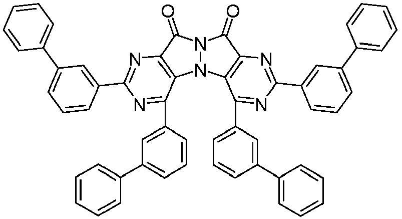 |
| 121 | 122 |
| 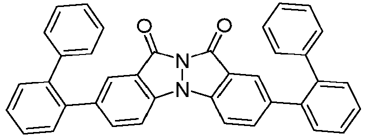 | 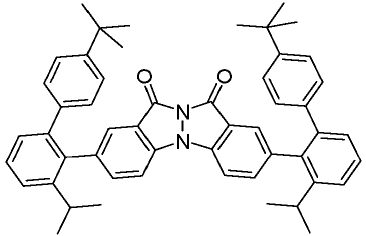 |
| 123 | 124 |
| 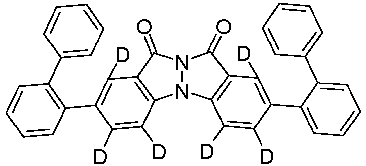 | 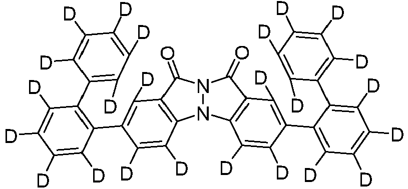 |
| 125 | 126 |
| 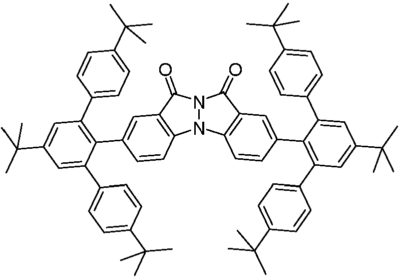 | 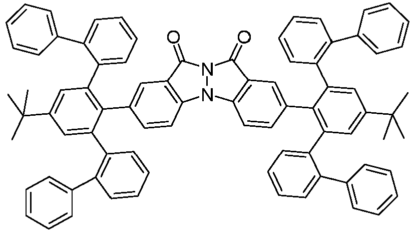 |
| 127 | 128 |
| 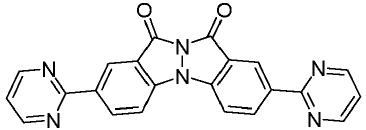 | 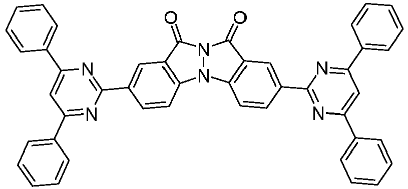 |
| 129 | 130 |
| 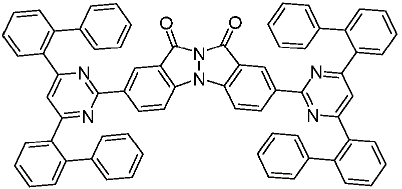 | 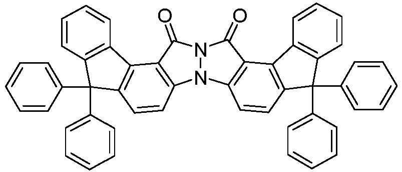 |
| 131 | 132 |
| 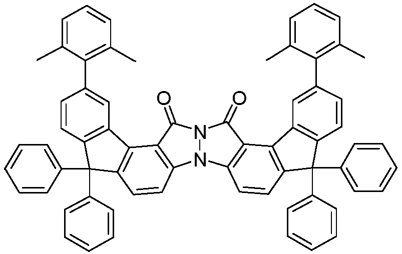 | 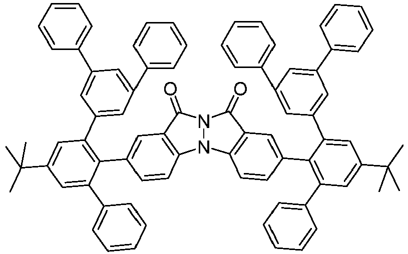 |
| 133 | 134 |
| 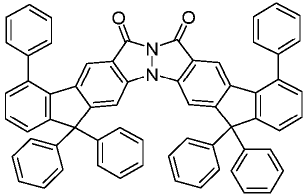 | 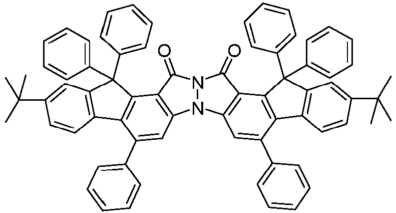 |
| 135 | 136 |
| 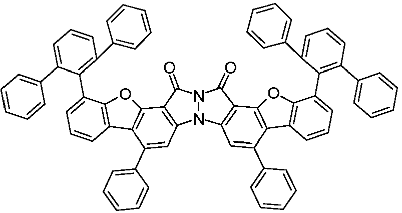 | 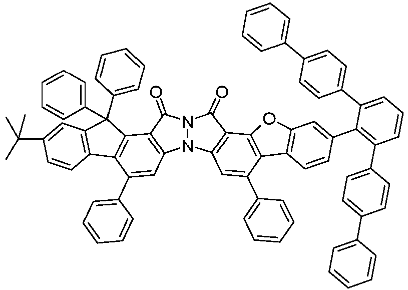 |
| 137 | 138 |
| 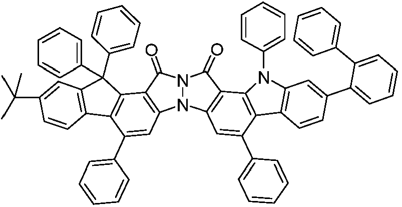 | 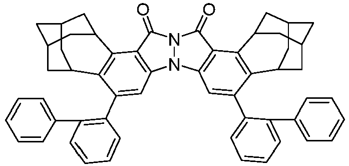 |
| 139 | 140 |
| 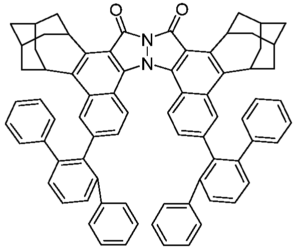 | 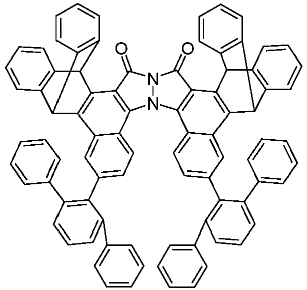 |
| 141 | 142 |
| 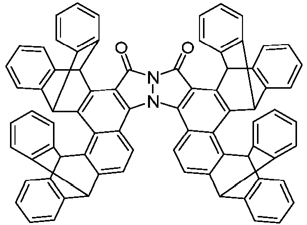 | 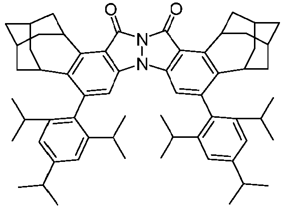 |
| 143 | 144 |
| 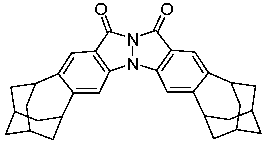 | 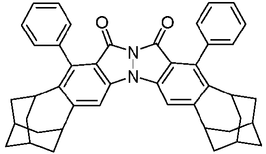 |
| 145 | 146 |
| 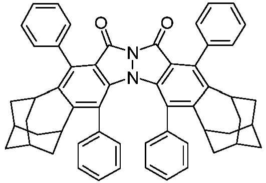 | 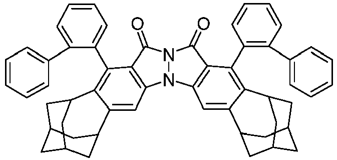 |
| 147 | 148 |
| 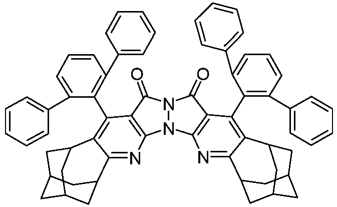 | 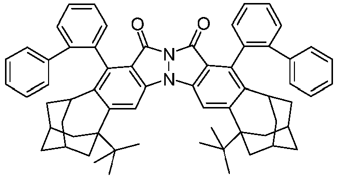 |
| 149 | 150 |
| 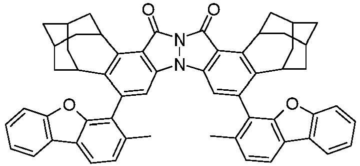 | 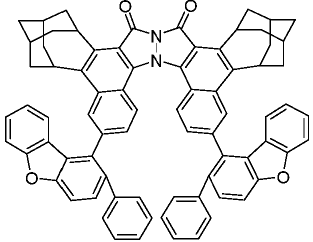 |
| 151 | 152 |

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der erfindungsgemäßen Verbindungen, bei dem ein Grundgerüst mit mindestens einer der Gruppen W^{a} oder einem Vorläufer einer der Gruppe W^{a} synthetisiert wird und ein aromatischer oder heteroaromatischer Rest mittels einer nukleophilen aromatischen Substitutionsreaktion oder einer Kupplungsreaktion eingeführt wird.

Geeignete Verbindungen, umfassend ein Grundgerüst mit einer Gruppe W^{a} können vielfach kommerziell erhalten werden, wobei die in den Beispielen dargelegten Ausgangsverbindungen durch bekannte Verfahren erhältlich sind, so dass hierauf verwiesen wird.

Diese Verbindungen können durch bekannte Kupplungsreaktionen mit weiteren Verbindungen umgesetzt werden, wobei die notwendigen Bedingungen hierfür dem Fachmann bekannt sind und ausführliche Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die erfindungsgemäßen Verbindungen in hoher Reinheit, bevorzugt mehr als 99 % (bestimmt mittels ¹H-NMR und/oder HPLC) erhalten.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten Strukturen der Formel (I) und bevorzugten Ausführungsformen dieser Formel oder erfindungsgemäße Verbindungen, wobei ein oder mehrere Bindungen der erfindungsgemäßen Verbindungen oder der Strukturen der Formel (I) und bevorzugten Ausführungsformen dieser Formel zum Polymer, Oligomer oder Dendrimer vorhanden sind. Je nach Verknüpfung der Strukturen der Formel (I) und bevorzugten Ausführungsformen dieser Formel bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (I) bzw. die zuvor und nachfolgend ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen bevorzugt, umfassend Strukturen gemäß den Formel (I) bzw. die zuvor und nachfolgend ausgeführten bevorzugten Ausführungsformen bevorzugt, die eine Glasübergangstemperatur von mindestens 70 °C, besonders bevorzugt von mindestens 110 °C, ganz besonders bevorzugt von mindestens 125 °C und insbesondere bevorzugt von mindestens 150 °C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung bzw. eine Zusammensetzung, enthaltend mindestens eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Falls die weitere Verbindung ein Lösungsmittel umfasst, so wird diese Mischung hierin als Formulierung bezeichnet. Die weitere Verbindung kann aber auch mindestens eine weitere organische oder anorganische Verbindung sein, die ebenfalls in der elektronischen Vorrichtung eingesetzt wird, beispielsweise ein Emitter und/oder ein Matrixmaterial, wobei sich diese Verbindungen von den erfindungsgemäßen Verbindungen unterscheiden. Geeignete Emitter und Matrixmaterialien sind hinten im Zusammenhang mit der organischen Elektrolumineszenzvorrichtung aufgeführt. Die weitere Verbindung kann auch polymer sein.

Nochmals ein weiterer Gegenstand der vorliegenden Erfindung ist daher eine Zusammensetzung enthaltend mindestens eine Verbindung nach Formel (I) 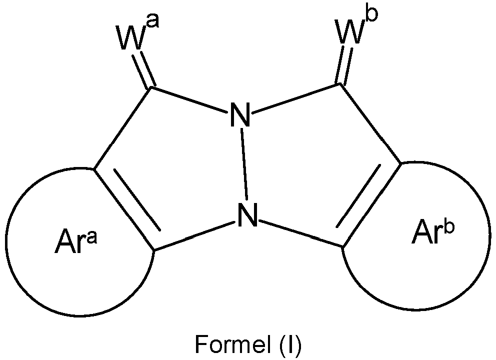 wobei die verwendeten Symbole die zuvor genannten Bedeutungen aufweisen, vorzugsweise mindestens eine erfindungsgemäße Verbindung oder ein Oligomer, Polymer oder Dendri¬mer umfassend Strukturen gemäß Formel (I) und wenigstens ein weiteres organisch funktionelles Material. Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, Emittern, die TADF (thermally activated delayed fluorescence) zeigen, Host-Materialien, Elektronentransportmaterialien, Elektroneninjektionsmaterialien, Lochleitermaterialien, Lochinjektionsmaterialien, Elektronenblockiermaterialien, Lochblockiermaterialien, Wide-Band-Gap-Materialien und n-Dotanden.

Ein weiterer Gegenstand der vorliegenden Erfindung ist die Verwendung einer Verbindung nach Formel (I) 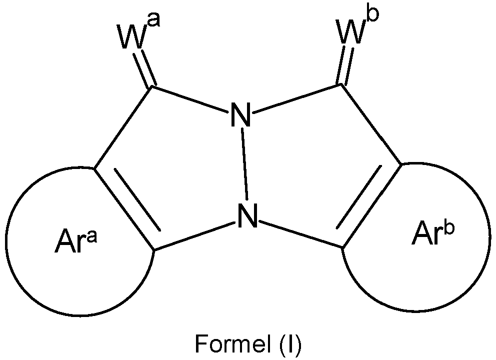 wobei die verwendeten Symbole die zuvor genannten Bedeutungen aufweisen, vorzugsweise einer erfindungsgemäßen Verbindung oder ein Oligomer, Polymer oder Dendri¬mer umfassend Strukturen gemäß Formel (I) in einer elektronischen Vorrichtung, insbesondere in einer organischen Elektrolumineszenzvorrichtung, vorzugsweise als Emitter, besonders bevorzugt als grüner, roter oder blauer Emitter. Hierbei zeigen erfindungsgemäße Verbindungen bevorzugt fluorezierende Eigenschaften und stellen somit bevorzugt fluoreszierenden Emitter bereit.

Vorzugsweise kann vorgesehen sein, dass Strukturen/Verbindungen gemäß Formeln (II-2) bis (II-8), (II-17) bis (II-23), (III-2) bis (III-8), (III-17) bis (III-23), (IV-2) bis (IV-8), (IV-17) bis (IV-23), (V-1), (V-2), (V-5) und/oder (V-6) als Emitter eingesetzt werden.

Ferner können Verbindungen nach Formel (I) oder ein Oligomer, Polymer oder Dendri¬mer umfassend Strukturen gemäß Formel (I) als Host-Materialien und/oder Elektronentransportmaterialien eingesetzt werden. Vorzugsweise kann vorgesehen sein, dass Strukturen/Verbindungen mit einer Anthracen-Gruppe (Ar-76) bis (Ar-78), bevorzugt (Ar-78) und/oder Verbindungen gemäß Formeln (II-9), (II-10), (II-24), (II-25), (III-9), (III-10), (III-24), (III-25), (IV-9), (IV-10), (IV-24) und/oder (IV-25) eingesetzt werden. als Elektronentransportmaterial und/oder Matrixmaterial eingesetzt werden

Ein nochmals weiterer Gegenstand der vorliegenden Erfindung ist eine elektronische Vorrichtung enthaltend mindestens eine Verbindung nach Formel (I) 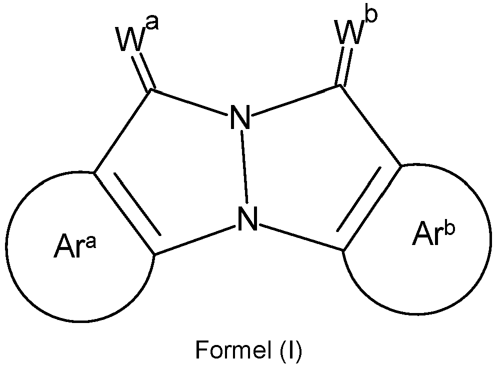 wobei die verwendeten Symbole die zuvor genannten Bedeutungen aufweisen, vorzugsweise einer erfindungsgemäßen Verbindung oder ein Oligomer, Polymer oder Dendrimer umfassend Strukturen gemäß Formel (I). Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus Besonders bevorzugt ist elektronische Vorrichtung ausgewählt aus der Gruppe bestehend aus organischen Elektrolumineszenzvorrichtungen (OLEDs, sOLED, PLEDs, LECs, etc.), vorzugsweise organische lichtemittierenden Dioden (OLEDs), organische lichtemittierenden Dioden auf Basis von kleinen Molekülen (sOLEDs), organische lichtemittierenden Dioden auf Basis von Polymeren (PLEDs), lichtemittierenden elektrochemischen Zellen (LECs), organischen Laserdioden (O-Laser), "organic plasmon emitting devices" (D. M. Koller et al., Nature Photonics 2008, 1-4); organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs) und organischen elektrischen Sensoren, bevorzugt organischen Elektrolumineszenzvorrichtungen (OLEDs, sOLED, PLEDs, LECs, etc.), besonders bevorzugt organische lichtemittierenden Dioden (OLEDs), organische lichtemittierenden Dioden auf Basis von kleiner Moleküle (sOLEDs), organische lichtemittierenden Dioden auf Basis von Polymeren (PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem- Elektrolumineszenzvorrichtung handeln, insbesondere für weiß emittierende OLEDs.

Die erfindungsgemäße Verbindung kann dabei in unterschiedlichen Schichten eingesetzt werden, je nach genauer Struktur. Bevorzugt ist eine organische Elektrolumineszenzvorrichtung, enthaltend eine Stuktur/Verbindung gemäß Formel (I) bzw. die oben ausgeführten bevorzugten Ausführungsformen in einer emittierenden Schicht als Emitter, vorzugsweise roter, grüner oder blauer Emitter. Sehr bevorzugt ist eine organische Elektrolumineszenzvorrichtung, enthaltend eine Stuktur/Verbindung gemäß Formel (I) bzw. die oben ausgeführten bevorzugten Ausführungsformen in einer emittierenden Schicht als fluoreszierender blauer Emitter. Hierbei sind insbesondere Verbindungen Strukturen/Verbindungen gemäß Formeln (II-2) bis (II-8), (II-17) bis (II-23), (III-2) bis (III-8), (III-17) bis (III-23), (IV-2) bis (IV-8), (IV-17) bis (IV-23), (V-1), (V-2), (V-5) und/oder (V-6) bevorzugt.

Wenn die erfindungsgemäße Verbindung als Emitter in einer emittierenden Schicht eingesetzt wird, wird bevorzugt ein geeignetes Matrixmaterial (auch Host Material genannt) eingesetzt, welches als solches bekannt ist.

Eine bevorzugte Mischung aus der erfindungsgemäßen Verbindung und einem Matrixmaterial enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% an Matrixmaterial bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, Dibenzofuranderivate, z. B. gemäß WO 2015/169412, WO 2016/015810, WO 2016/023608, WO 2017/148564 oder WO 2017/148565 oder Biscarbazole, z. B. gemäß JP 3139321 B2.

Weiterhin kann als Co-Host eine Verbindung verwendet werden, die nicht oder nicht in wesentlichem Umfang am Ladungstransport teilnimmt, wie beispielsweise in WO 2010/108579 beschrieben. Insbesondere eignen sich in Kombination mit der erfindungsgemäßen Verbindung als Co-Matrix-Material Verbindungen, welche eine große Bandlücke aufweisen und selber nicht oder zumindest nicht in wesentlichem Maße am Ladungstransport der emittierenden Schicht teilnehmen. Es handelt sich bei solchen Materialien bevorzugt um reine Kohlenwasserstoffe. Beispiele für solche Materialien finden sich beispielsweise in der WO 2009/124627 oder in der WO 2010/006680.

In einer bevorzugten Ausgestaltung wird eine Verbindung enthaltend eine Stuktur/Verbindung gemäß Formel (I) bzw. die oben ausgeführten bevorzugten Ausführungsformen, die als Emitter verwendet wird, vorzugsweise in Kombination mit einem oder mehreren phosphoreszierenden Materialien (Triplettemitter) und/oder einer Verbindung eingesetzt, die ein TADF-Hostmaterial (thermally activated delayed fluorescence) darstellt. Hierbei wird vorzugsweise ein Hyperfluoreszenz-System wie in WO 2012/133188 beschrieben und/oder Hyperphosphoreszenz-System wie in US 2017271611 gebildet. Diese Kombination stellt eine bevorzugte Zusammensetzung gemäß der vorliegenden Erfindung dar.

In WO 2015/091716 A1 und in WO 2016/193243 A1 werden OLEDs offenbart, die in der Emissionsschicht sowohl eine phosphoreszierende Verbindung als auch einen fluoreszierenden Emitter enthalten, wobei die Energie von der phosphoreszierenden Verbindung auf den fluoreszierenden Emitter übertragen wird (Hyperphosphoreszenz). Die phosphoreszierende Verbindung verhält sich in diesem Zusammenhang demnach wie ein Host-Material. Wie der Fachmann weiß, haben Hostmaterialien höhere Singulett und Triplett-Energien im Vergleich zu dem Emittern, damit die Energie des Host-Materials auch möglichst optimal auf den Emitter übertragen werden. Die im Stand der Technik offenbarten Systeme weisen genau solch eine Energierelation auf.

Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439, WO 2018/011186, WO 2018/001990, WO 2018/019687, WO 2018/019688, WO 2018/041769, WO 2018/054798, WO 2018/069196, WO 2018/069197, WO 2018/069273, WO 2018/178001, WO 2018/177981, WO 2019/020538, WO 2019/115423, WO 2019/158453 und WO 2019/179909 entnommen werden. Generell eignen sich alle phosphoreszierenden Komplexe, wie sie gemäß dem Stand der Technik für phosphoreszierende Elektrolumineszenzvorrichtungen verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Eine erfindungsgemäße Verbindung kann vorzugsweise in Kombination mit einem TADF-Hostmaterial und/oder einem TADF-Emitter eingesetzt werden, wie dies zuvor dargelegt ist.

Der als thermisch aktivierte verzögerte Fluoreszenz (TADF = "thermally activated delayed fluorescence") bezeichnete Vorgang wird beispielsweise von B. H. Uoyama et al., Nature 2012, Vol. 492, 234 beschrieben. Um diesen Prozess zu ermöglichen, ist im Emitter ein vergleichsweise kleiner Singulett-Triplett-Abstand ΔE(S₁ - T₁) von zum Beispiel weniger als etwa 2000 cm⁻¹ nötig. Um den an sich spin-verbotenen Übergang T₁ → S₁ zu öffnen, kann neben dem Emitter eine weitere Verbindung in der Matrix vorgesehen werden, die eine starke Spin-Bahn-Kopplung aufweist, sodass über die räumliche Nähe und die damit mögliche Wechselwirkung zwischen den Molekülen ein Inter-System-Crossing ermöglicht wird, oder die Spin-Bahn-Kopplung wird über ein im Emitter enthaltenes Metallatom erzeugt.

Weitere wertvolle Informationen zu Hyperfluoreszenz-Systemen sind unter anderem in WO2012/133188 (Idemitsu), WO2015/022974 (Kyushu Univ.), WO2015/098975 (Idemitsu), WO2020/053150 (Merck) und DE202019005189 (Merck) dargelegt.

Weitere wertvolle Informationen zu Hyperphosphoreszenz-Systemen sind unter anderem in WO2015/091716 A1, WO2016/193243 A1 (BASF), WO01/08230 A1 (Princeton Univ. (Mark Thompson)), US2005/0214575A1 (Fuji), WO2012/079673 (Merck), WO2020/053314 (Merck) und WO2020/053315 (Merck) dargelegt.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Weiterhin ist eine organische Elektrolumineszenzvorrichtung bevorzugt, enthaltend eine Struktur/Verbindung gemäß Formel (I) bzw. die oben ausgeführten bevorzugten Ausführungsformen in einer elektronenleitenden Schicht als Elektronentransportmaterial. Hierbei sind insbesondere Verbindungen mit einer Anthracen-Gruppe, vorzugsweise einer Gruppe gemäß Formeln (Ar-76) bis (Ar-78), bevorzugt (Ar-78) und/oder Strukturen/Verbindungen gemäß Formeln (II-9), (II-10), (II-24), (II-25), (III-9), (III-10), (III-24), (III-25), (IV-9), (IV-10), (IV-24) und/oder (IV-25) bevorzugt.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den Struktur/Verbindung gemäß Formel (I) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Formulierungen zum Auftragen einer Verbindung gemäß Formel (I) oder deren oder deren zuvor dargelegten bevorzugten Ausführungsformen sind neu Ein weiterer Gegenstand der vorliegenden Erfindung ist daher eine Formulierungen, enthaltend mindestens ein Lösungsmittel und eine Verbindung gemäß Formel (I) oder deren zuvor dargelegten bevorzugten Ausführungsformen.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich gegenüber dem Stand der Technik insbesondere durch eine verbesserte Lebensdauer aus. Dabei bleiben die weiteren elektronischen Eigenschaften der Elektrolumineszenzvorrichtungen, wie Effizienz oder Betriebsspannung, mindestens gleich gut. In einer weiteren Varianten zeichnen sich die erfindungsgemäßen Verbindungen und die erfindungsgemäßen organischen Elektrolumineszenzvorrichtungen gegenüber dem Stand der Technik insbesondere durch eine verbesserte Effizienz und/oder Betriebsspannung und höhere Lebensdauer aus.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:
1. Elektronische Vorrichtungen, insbesondere organische Elektrolumineszenzvorrichtungen enthaltend Strukturen/Verbindungen gemäß Formel (I) bzw. die zuvor und nachfolgend ausgeführten bevorzugten Ausführungsformen als Emitter weisen sehr schmal Emissionsbanden mit geringen FWHM-Werten (Full Width Half Maximum) auf und führen zu besonders Farb-reiner Emission, erkennbar an den kleinen CIE-y-Werten. Besonders überraschend ist hierbei, dass sowohl blaue Emitter mit geringen FWHM-Werten als auch Emitter mit geringen FWHM-Werten bereitgestellt werden, die im grünen, gelben oder roten Bereich des Farbspektrums emittieren.
2. Elektronische Vorrichtungen, insbesondere organische Elektrolumineszenzvorrichtungen enthaltend Strukturen/Verbindungen gemäß Formel (I) bzw. die zuvor und nachfolgend ausgeführten bevorzugten Ausführungsformen, insbesondere als Emitter, als Lochleitermaterial und/oder als Elektronentransportmaterial weisen eine sehr gute Lebensdauer auf. Hierbei bewirken diese Verbindungen insbesondere einen geringen Roll-off, d.h. einen geringen Abfall der Leistungseffizienz der Vorrichtung bei hohen Leuchtdichten.
3. Elektronische Vorrichtungen, insbesondere organische Elektrolumineszenzvorrichtungen enthaltend Strukturen/Verbindungen gemäß Formel (I) bzw. die zuvor und nachfolgend ausgeführten bevorzugten Ausführungsformen als Emitter und/oder als Elektronentransportmaterial weisen eine hervorragende Effizienz auf. Hierbei bewirken erfindungsgemäße Strukturen/Verbindungen gemäß Formel (I) bzw. die zuvor und nachfolgend ausgeführten bevorzugten Ausführungsformen eine geringe Betriebsspannung bei Verwendung in elektronischen Vorrichtungen.
4. Die erfindungsgemäßen Strukturen/Verbindungen gemäß Formel (I) bzw. die zuvor und nachfolgend ausgeführten bevorzugten Ausführungsformen zeigen eine sehr hohe Stabilität und Lebensdauer.
5. Mit Strukturen/Verbindungen gemäß Formel (I) bzw. den zuvor und nachfolgend ausgeführten bevorzugten Ausführungsformen kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.
   Excitonenenergie wird von einem Matrix oder Host in der Emissionsschicht typischerweise entweder über den sogenannten Dexter- oder über den Förstertransfer auf den Emitter übertragen. Der Försterenergietransfer (FRET) von einem Host oder einer Matrix auf den erfindungsgemäßen Emitter ist dabei besonders bevorzugt, da dieser besonders effizient ist, was zu elektronischen Vorrichtungen mit besonders guten Leistungsdaten (bspw. Effizienz, Spannung und Lebensdauer) führt. Es zeigt sich, dass der Energieübertrag von einem Host oder einer Matrix auf die erfindungsgemäßen Verbindungen vorzugsweise über den Förstertransfer erfolgt.
6. Strukturen/Verbindungen gemäß Formel (I) bzw. die zuvor und nachfolgend ausgeführten bevorzugten Ausführungsformen weisen eine ausgezeichnete Glasfilmbildung auf.
7. Strukturen/Verbindungen gemäß Formel (I) bzw. die zuvor und nachfolgend ausgeführten bevorzugten Ausführungsformen bilden aus Lösungen sehr gute Filme und zeigen eine ausgezeichnete Löslichkeit.

Diese oben genannten Vorteile gehen nicht mit einer unmäßig hohen Verschlechterung der weiteren elektronischen Eigenschaften einher.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen. Bei Verbinden die mehrere enantiomere, diastereomere oder tautomere Formen aufweisen können wird eine Form stellvertretend gezeigt.

### Literaturbekannte Synthone LS:

| | | | |
|---|---|---|---|
| LS1 | 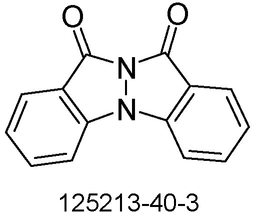 | LS2 | 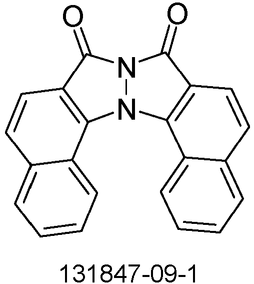 |
| LS3 | 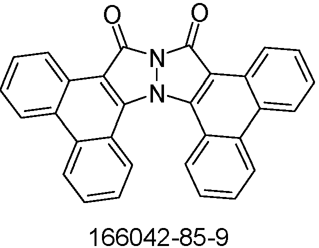 | | |

### Synthese von Synthonen S:

### Literaturbekannte Edukte:

### 1) 1,2-Dihydroindazol-3-one:

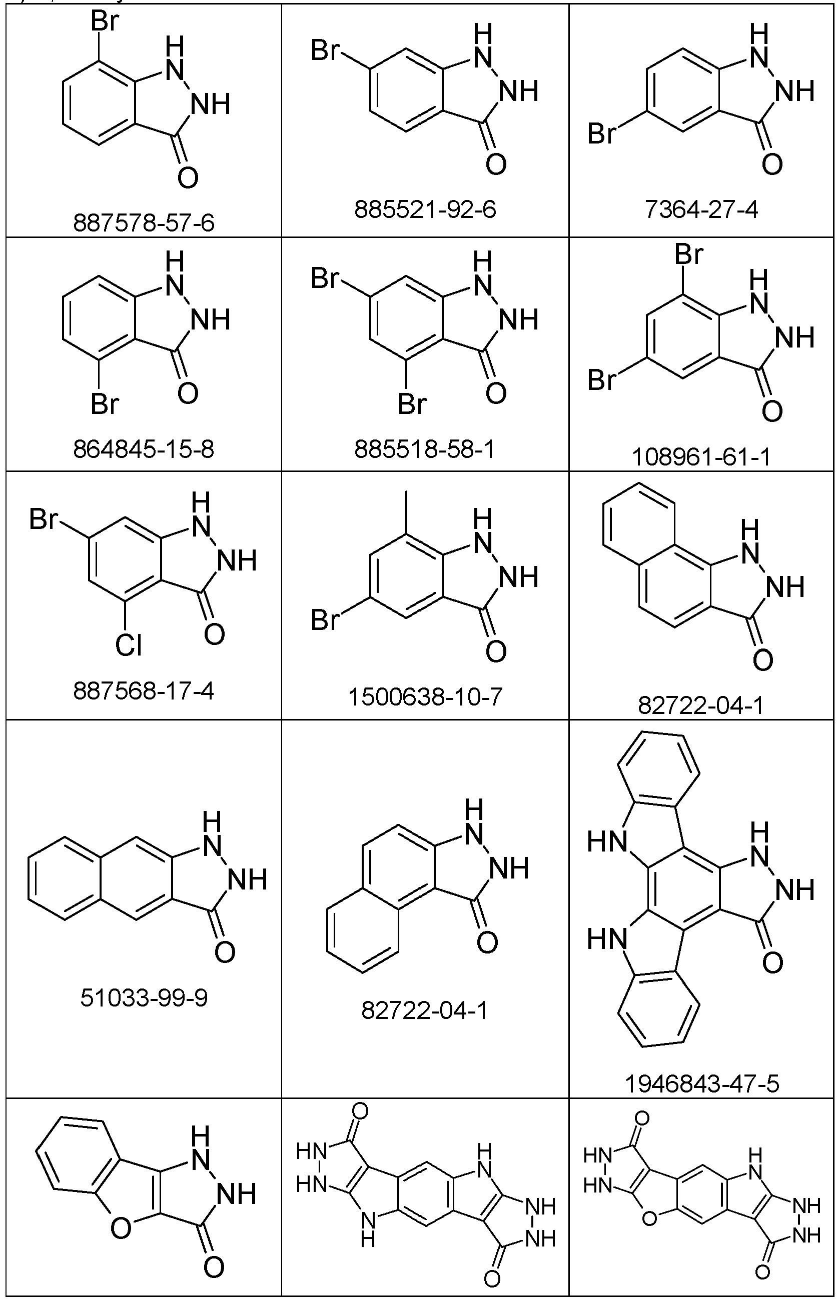

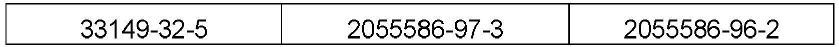

### 2) Säurechloride:

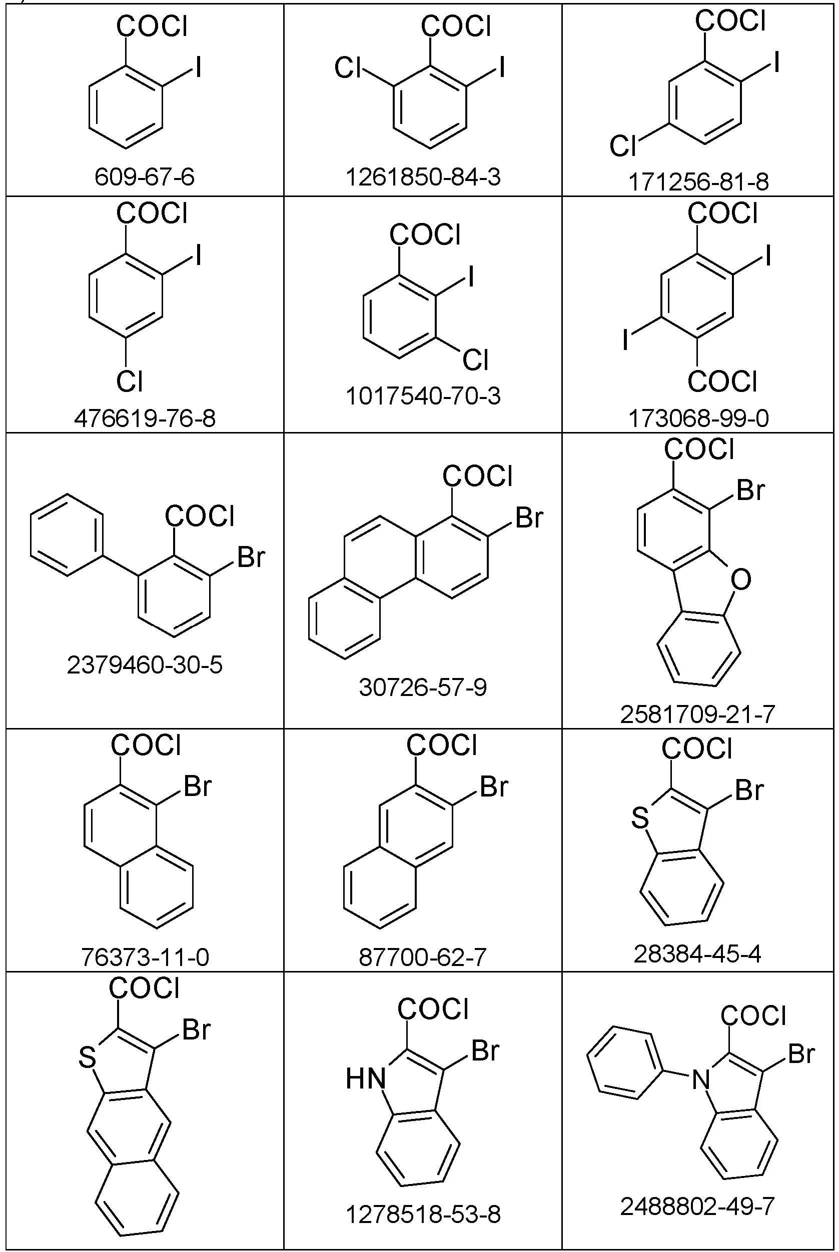

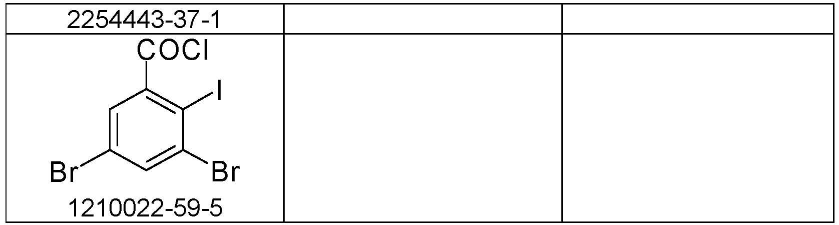

### Beispiel S1:

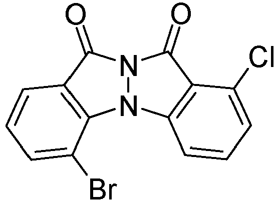

Durchführung nach Z. Wang et al., Chin. J. Chem., 29, 2769, 2011. Ein Gemisch aus 21.3 g (100 mmol) 7-Brom-1,2-dihydro-3H-indazol-3-on [887578-57-6], 39.1 g (130 mmol) 2-Chlor-6-iodbenzoylchlorid [1261850-84-3], 97.8 g (300 mmol) Cäsiumcarbonat, wasserfrei, 3.6 g (20 mmol) 1,10-Phenanthrolin, 1.9 g (10 mmol) Kupferiodid, 200 g Glaskugeln (3 mm Durchmesser) und 800 ml Toluol wird 16 h bei 100 °C gerührt (DC Kontrolle auf vollständigen Umsatz des 7-Brom-1,2-dihydro-3H-indazol-3-ons). Man lässt auf 80 °C erkalten, filtriert über ein mit heißem Toluol vorgeschlämmten Celite-Bett und engt das Filtrat im Vakuum zur Trockene ein. Man nimmt den Rückstand in 500 ml Dichlormethan (DCM) auf, zieht ihn auf ca. 400 g Kieselgel auf, packt das Kieselgel auf eine mit DCM vorgeschlämmte Kieselgelsäule (800 g) und eluiert mit DCM das anti-Dibenzobiman-Regioisomer. Anschließend eluiert man das gewünschte syn-Dibenzobiman-Regioisomer mit DCM: Methyl-tert-butyl-ether (MTBE) 8:2 und kristallisiert den einmal aus Acetonitril um. Ausbeute: 9.5 g (27 mmol), 27 %. Reinheit: 97 % nach ¹H-NMR.

Analog können folgende Verbindungen dargestellt werden:

| Bsp. | Edukte | Produkt | Ausbeute |
|---|---|---|---|
| S2 | 885521-92-6 | 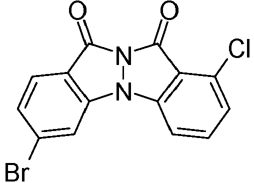 | 30 % |
| | 1261850-84-3 | | |
| S3 | 7364-27-4 | 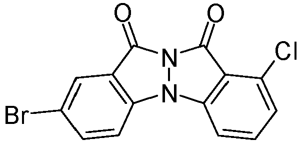 | 30 % |
| | 1261850-84-3 | | |
| S4 | 864845-15-8 | 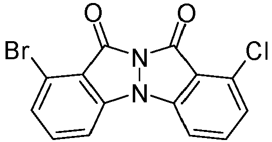 | 28 % |
| | 1261850-84-3 | | |
| S5 | 887578-57-6 | 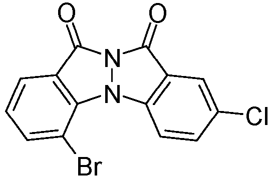 | 32 % |
| | 171256-81-8 | | |
| S6 | 885521-92-6 | 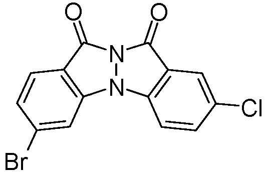 | 35 % |
| | 171256-81-8 | | |
| S7 | 7364-27-4 | 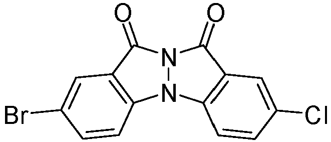 | 33 % |
| | 171256-81-8 | | |
| S8 | 864845-15-8 | 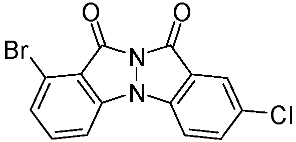 | 32 % |
| | 171256-81-8 | | |
| S9 | 887578-57-6 | 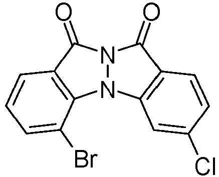 | 29 % |
| | 476619-76-8 | | |
| S10 | 885521-92-6 | 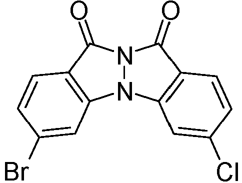 | 38 % |
| | 476619-76-8 | | |
| S11 | 7364-27-4 | 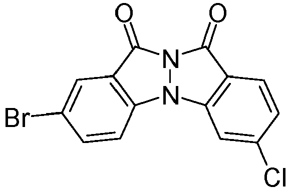 | 35 % |
| | 476619-76-8 | | |
| S12 | 864845-15-8 | 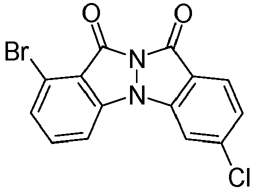 | 33 % |
| | 476619-76-8 | | |
| S13 | 887578-57-6 | 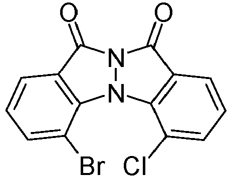 | 20 % |
| | 1017540-70-3 | | |
| S14 | 885521-92-6 | 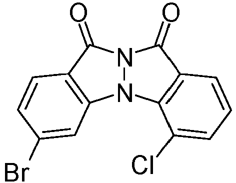 | 26 % |
| | 1017540-70-3 | | |
| S15 | 7364-27-4 | 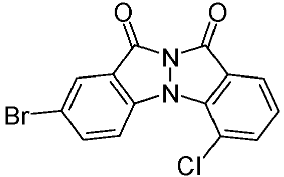 | 29 % |
| | 1017540-70-3 | | |
| S16 | 864845-15-8 | 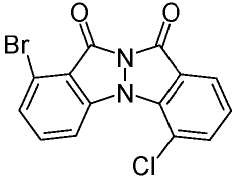 | 27 % |
| | 1017540-70-3 | | |
| S17 | 1500638-10-7 | 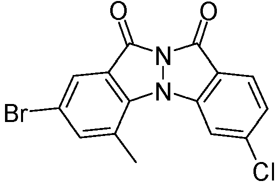 | 30 % |
| | 476619-76-8 | | |
| S18 | 885518-58-1 | 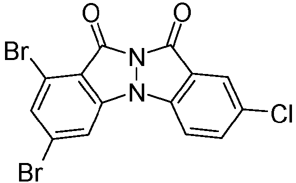 | 34 % |
| | 171256-81-8 | | |
| S19 | 885518-58-1 | 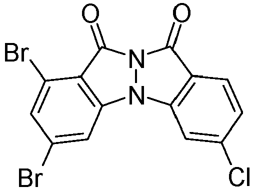 | 31 % |
| | 476619-76-8 | | |
| S20 | 108961-61-1 | 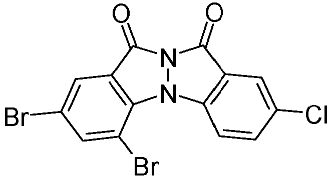 | 27 % |
| | 171256-81-8 | | |
| S21 | 108961-61-1 | 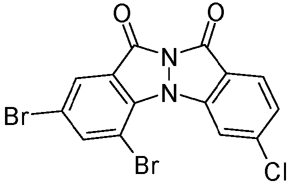 | 26 % |
| | 476619-76-8 | | |
| S22 | 887568-17-4 | 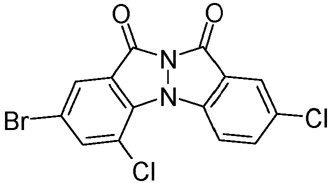 | 28 % |
| | 171256-81-8 | | |
| S23 | 887568-17-4 | 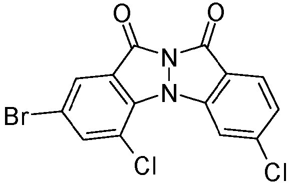 | 23 % |
| | 476619-76-8 | | |
| S24 | 82722-04-1 | 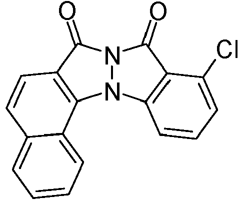 | 30 % |
| | 1261850-84-3 | | |
| S25 | 51033-99-9 | 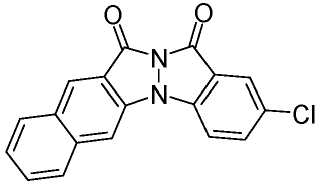 | 35 % |
| | 171256-81-8 | | |
| S26 | 82722-04-1 | 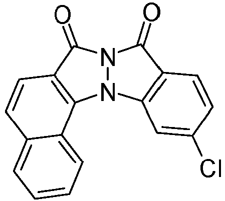 | 32 % |
| | 476619-76-8 | | |
| S27 | 33149-32-5 | 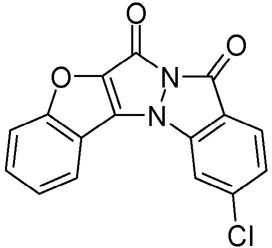 | 31 % |
| | 476619-76-8 | | |
| S28 | 2379460-30-5 | 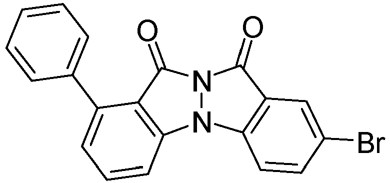 | 32 % |
| | 7364-27-4 | | |
| S29 | 30726-57-9 | 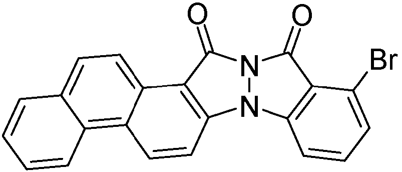 | 26 % |
| | 864845-15-8 | | |
| S30 | 2581709-21-7 | 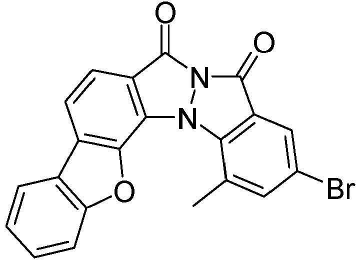 | 29 % |
| | 1500638-10-7 | | |
| S31 | 887578-57-6 | 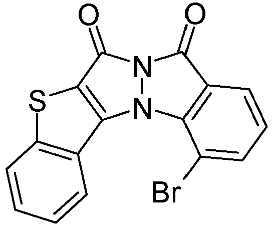 | 23 % |
| | 28384-45-4 | | |
| S32 | 864845-15-8 | 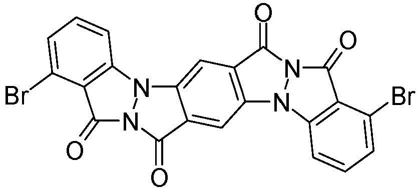 | 17 % |
| | 173068-99-0 50 mmol | | |
| S33 | 885518-58-1 | 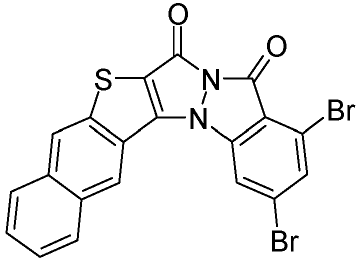 | 33 % |
| | 2254443-37-1 | | |
| S34 | 2488802-49-7 | 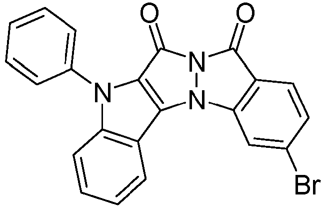 | 32 % |
| | 885521-92-6 | | |
| S35 | 609-67-6 | 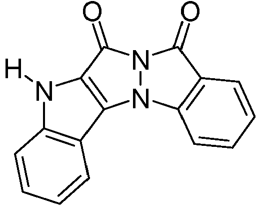 | 21 % |
| | 1278518-53-8 | | |
| S36 | 2055586-96-2 50 mmol | 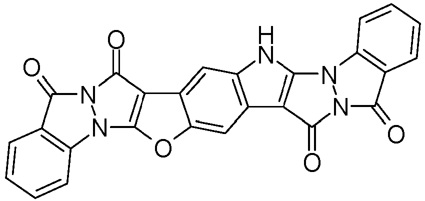 | 16 % |
| | 609-67-6 | | |
| S37 | 609-67-6 | 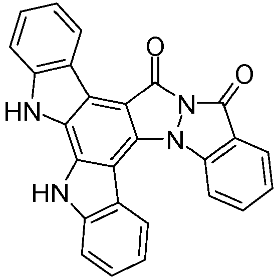 | 29 % |
| | 1946843-47-5 | | |
| S38 | 2055586-97-3 50 mmol | 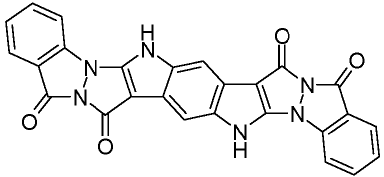 | 16 % |
| | 609-67-6 | | |
| S39 | 887578-57-6 | 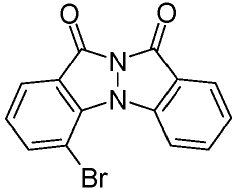 | 28 % |
| | 609-67-6 | | |
| S40 | 885521-92-6 | 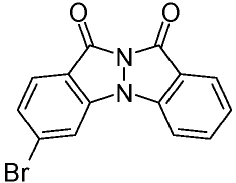 | 36 % |
| | 609-67-6 | | |
| S41 | 7364-27-4 | 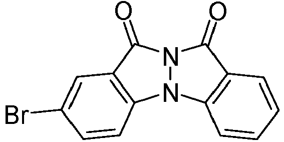 | 35 % |
| | 609-67-6 | | |
| S42 | 864845-15-8 | 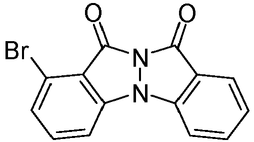 | 31 % |
| | 609-67-6 | | |
| S43 | 108961-61-1 | 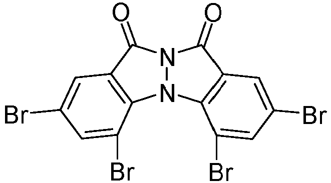 | 17 % |
| | 1210022-59-5 | | |

### Beispiel S50:

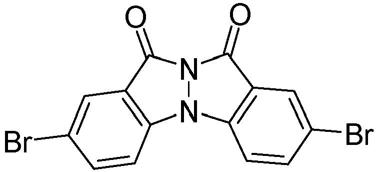

Eine gut gerührte Lösung von 23.6 g (100 mmol) syn-Dibenzobiman [125213-40-3] LS1 in 500 ml DCM wird bei Raumtemperatur unter Lichtausschluß portionsweise mit 37.4 g (210 mmol) N-Bromsuccinimid versetzt und anschließend 12 h nachgerührt. Man wäscht die Reaktionsmischung dreimal mit je 300 ml Wasser, einmal mit 300 ml ges. Kochsalzlösung und trocknet über Natriumsulfat. Man filtriert von Trockenmittel ab, fügt 100 ml Methanol zu, engt das Filtrat am Rotationsverdampfer ein auf ca. 80 ml Volumen ein, saugt vom auskristallisierten Produkt ab und wäscht dieses zweimal mit wenig Methanol. Ausbeute: 26.7 g (68 mmol), 68 %. Reinheit: 97 % nach ¹H-NMR.

Analog können folgende Verbindungen dargestellt werden:

| **Bsp.** | **Edukte** | **Produkt** | **Ausbeute** |
|---|---|---|---|
| S51 | LS2 | 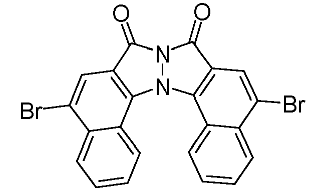 | 63 % |
| S52 | LS3 | 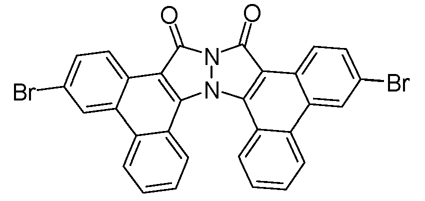 Zusatz von 1 TR HBr in Eisessig (47 Gew.-%ig) | 37 % |
| S53 | S30 110 mmol NBS | 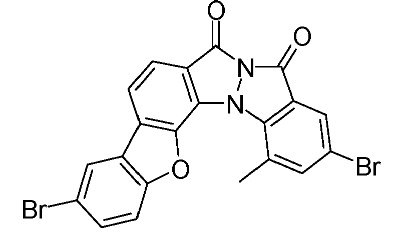 | 54 % |

### Beispiel Int-D1:

### a) Int-B1

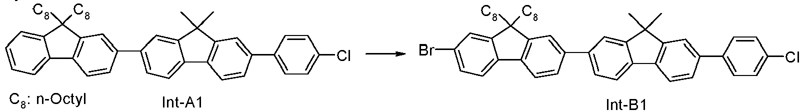

26.0 g (33.7 mmol) Int-A1 [2171483-83-1] werden in 600 ml Dichlormethan gelöst. Unter Lichtausschluss gibt man einen Tropfen HBr (33 %, wässrig) zu, versetzt portionsweise mit 7.2 g (44.0 mmol) N-Bromsuccinimid und rührt 48 h bei Raumtemperatur nach. Nach 48 h versetzt man mit 200 ml wässriger, ges. Natriumhydrogensulfitlösung und 1 h nach. Anschließend werden beide Phasen getrennt, die organische Phase wird dreimal mit je 200 ml Wasser gewaschen und anschließend unter vermindertem Druck eingeengt. Der Rückstand wird mit 300 ml n Heptan versetzt und 1 h unter Rückfluss erhitzt. Nach Erkalten der Mischung wird der farblose Feststoff abfiltriert, zweimal mit je 50 ml n-Heptan gewaschen und im Vakuum getrocknet. Ausbeute: 25.3 g (32 mmol), 97 %. Reinheit: ca. 98 % n. HPLC.

### b) Int-C1:

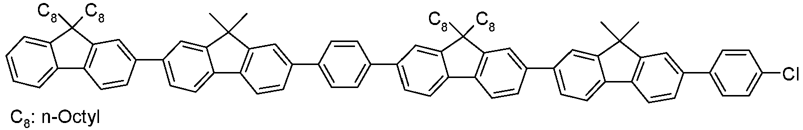

28.0 g (35.5 mmol) Int-B1, 33.6 g (35.5mmol) [2171483-74-0] und 9.8 g (71 mmol) Kaliumcarbonat werden mit 1100 ml THF und 550 ml Wasser versetzt. Anschließend werden 373 mg (1.4 mmol) Triphenylphosphin und 325 mg (0.4 mmol) Tri(dibenzylidenaceton)dipalladium zugegeben und 16 h unter Rückfluss erhitzt. Die erkaltete Reaktionsmischung mit 500 ml Wasser und 500 ml Toluol versetzt, die Phasen werden getrennt. Die wässrige Phase wird zweimal mit je 200 ml Toluol extrahiert, die vereinten organischen Phasen werden einmal mit 500 ml Wasser waschen. Die organische Phase wird über Aluminiumoxid filtriert und anschließend zur Trockene eingeengt. Das Produkt wird durch mehrmalige Kristallisation aus Toluol/n-Heptan 1:10 aufgereinigt und als Feststoff erhalten. Ausbeute: 41.0 g (28.8 mmol) 81 %. Reinheit: ca. 98 % n. HPLC.

### c) Int-D1:

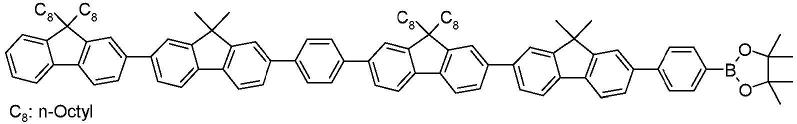

25.3 g (18.7 mmol) Int-C1, 8.6 g (33.7 mmol) Bis-(pinacolato)-diboran, 5.5 g (56 mmol) Kaliumacetat und 830 mg (1.1 mmol) trans-Dichlorobis(tricyclohexyl-phosphin)palladium(II) werden mit 750 ml Dioxan versetzt und 48 h unter Rückfluss erhitzt. Anschließend wird das Reaktionsgemisch mit 1000 ml Toluol und 1000 ml Wasser versetzt und die Phasen getrennt. Die wässrige Phase wird zweimal mit je 200 ml Toluol extrahiert, die vereinten organischen Phasen werden zweimal mit je 500 ml Wasser gewaschen, anschließend über Aluminiumoxid filtriert und zur Trockene eingeengt. Der Rückstand wird mit heißem Ethanol ausgerührt. Ausbeute: 16.0 g (11.1 mmol), 60 %. Reinheit: ca. 99 % n. HPLC.

### Darstellung der erfindungsgemäßen Verbindungen V:

### Beispiel V1

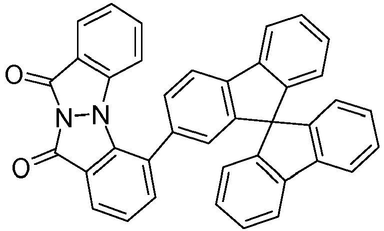

Ein Gemisch aus 27.1 g (100 mmol) S39, 39.6 g (110 mmol) 9,9'-Spiro-bi-fluoren-2-boronsäure [236389-21-2], 21.2 g (100 mmol) Trikaliumphosphat, 1.64 g (4 mmol) S-Phos, 499 mg (2 mmol) Palladium(II)acetat, 300 ml Toluol, 100 ml Dioxan und 300 ml Wasser wird 12 h bei 70 °C gerührt (alternativ kann AmPhosPdCl₂ [887919-35-9] verwendet werden). Nach Erkalten trennt man die org. Phase ab, wäscht diese zweimal mit je 300 ml Wasser und einmal mit 300 ml ges. Kochsalzlösung. Man engt die org. Phase zur Trockene ein, nimmt den Rückstand in Dichlormethan (DCM) auf, filtriert über ein mit DCM vorgeschlämmtes Kieselgelbett ab und engt das Filtrat am Rotationsverdampfer ein, wobei das abrotierte DCM kontinuierlich durch Methanol ersetzt wird. Man saugt vom auskristallisierten Produkt ab, wäscht dieses zweimal mit ja 50 ml Methanol und trocknet im Vakuum. Die weitere Reinigung erfolgt durch Heißextraktionskristallisation (Lösemittelgemische DCM / Acetonitril 1:3 bis 3:1) und fraktionierte Sublimation. Ausbeute: 31.5 g (57 mmol), 57 %. Reinheit: ca. 99.9 % nach HPLC.

Analog können folgende Verbindungen dargestellt werden:

| **Bsp.** | **Edukte** | **Produkt** | **Ausbeute** |
|---|---|---|---|
| V2 | S40 | 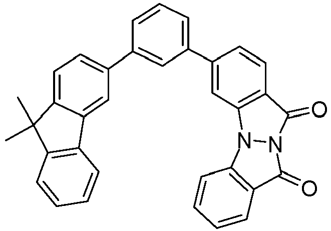 | 67 % |
| | 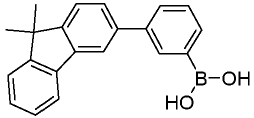 | | |
| | 2363139-27-7 | | |
| V3 | S41 | 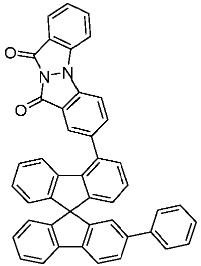 | 70 % |
| | 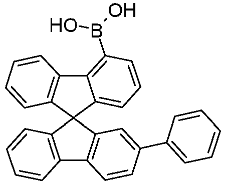 | | |
| | 2201147-30-8 | | |
| V4 | S42 | 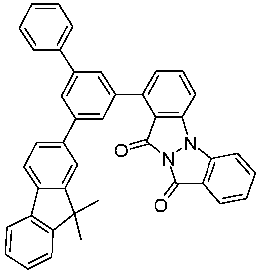 | 51 % |
| | 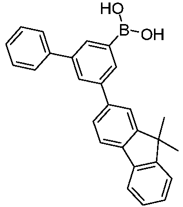 | | |
| | 881911-75-7 | | |
| V5 | S40 | 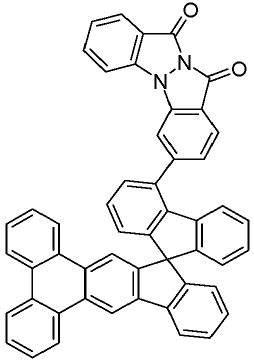 | 56 % |
| | 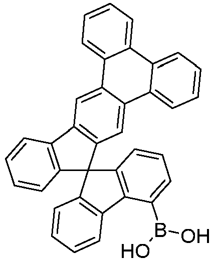 | | |
| | 2088929-31-9 | | |
| V6 | S41 | 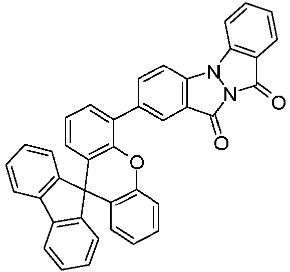 | 65 % |
| | 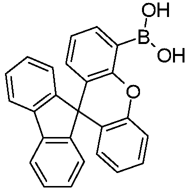 | | |
| | 2418007-78-8 | | |
| V7 | S39 | 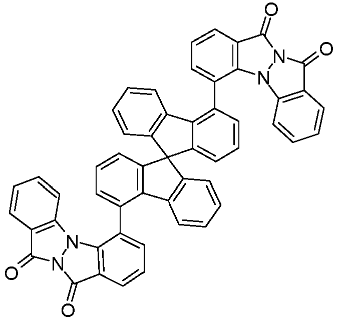 | 43 % |
| | 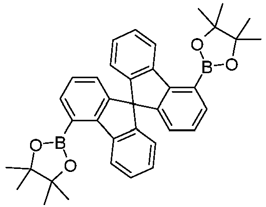 | | |
| | 1257321-47-3 | | |
| | 50 mmol | | |
| V8 | S42 | 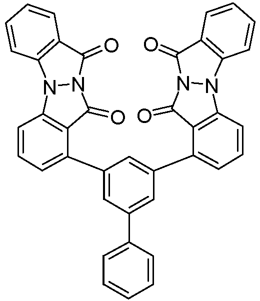 | 58 % |
| | 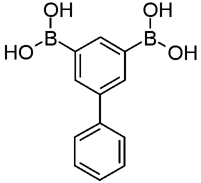 | | |
| | 2249944-12-3 | | |
| | 50 mmol | | |
| V9 | S39 | 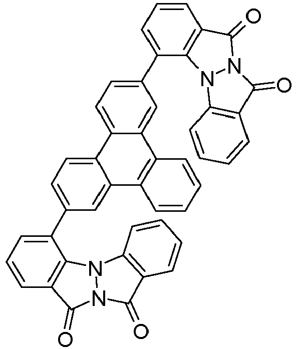 | 61 % |
| | 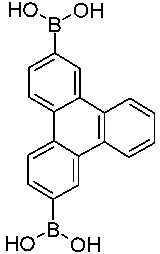 | | |
| | 1326240-20-3 | | |
| | 50 mmol | | |
| V10 | S41 | 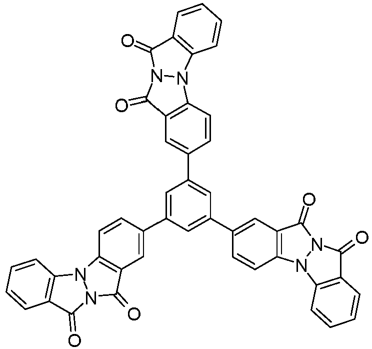 | 44 % |
| | 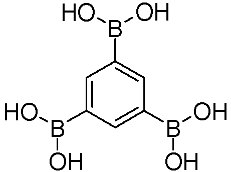 | | |
| | 1216638-44-6 | | |
| | 30 mmol | | |
| V11 | S32 | 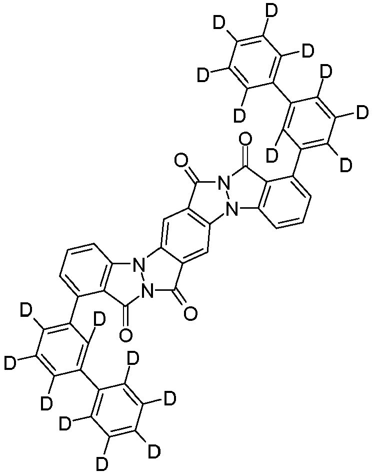 | 40 % |
| | 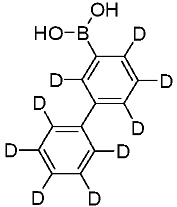 | | |
| | 2368221-45-6 | | |
| | 220 mmol | | |
| V12 | S39 | 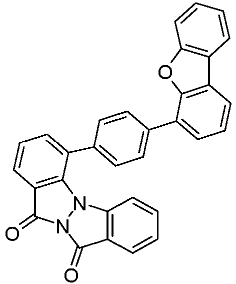 | 43 % |
| | 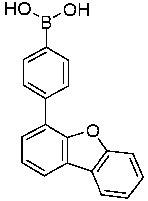 | | |
| | 796071-96-0 | | |
| V13 | S40 | 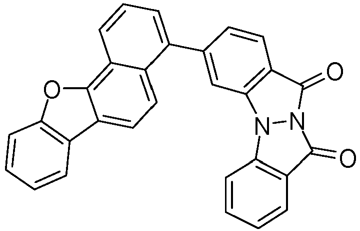 | 69 % |
| | 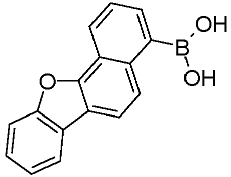 | | |
| | 2345673-16-5 | | |
| V14 | S41 | 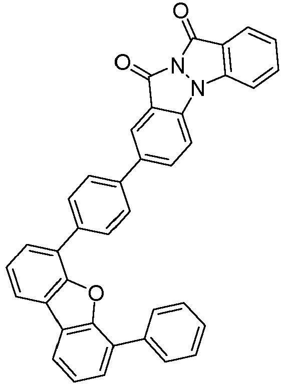 | 65 % |
| | 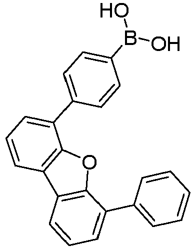 | | |
| | 2247722-85-4 | | |
| V15 | S42 | 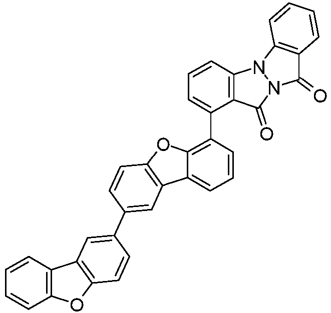 | 56 % |
| | 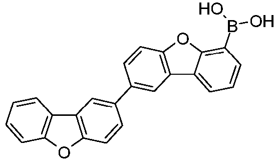 | | |
| | 2411326-31-1 | | |
| V16 | S42 | 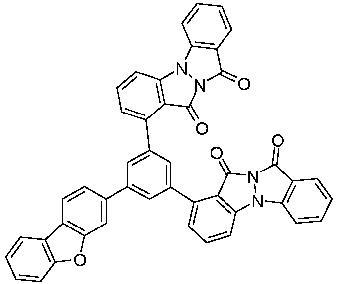 | 47 % |
| | 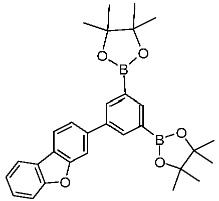 | | |
| | 2375083-92-2 | | |
| | 50 mmol | | |
| V17 | S41 | 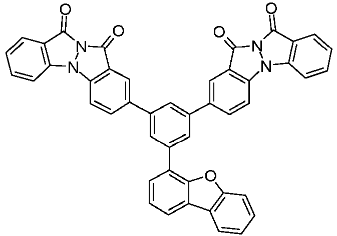 | 61 % |
| | 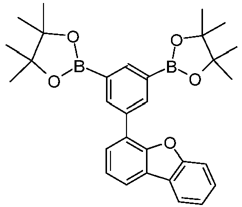 | | |
| | 2375083-93-3 | | |
| | 50 mmol | | |
| V18 | S42 | 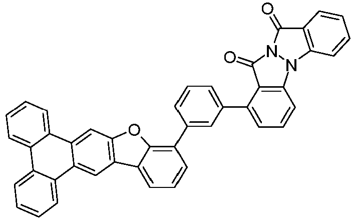 | 53 % |
| | 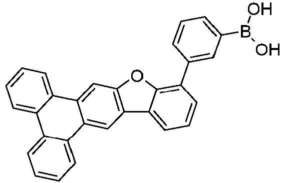 | | |
| | 1644606-71-2 | | |
| V19 | S40 | 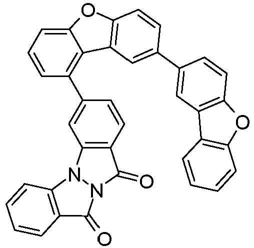 | 67 % |
| | 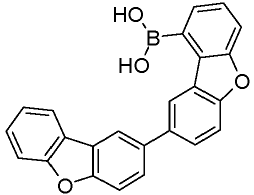 | | |
| | 1822320-55-7 | | |
| V20 | S33 | 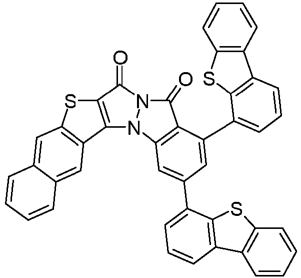 | 51 $ |
| | 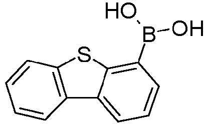 | | |
| | 108847-20-7 | | |
| | 210 mmol | | |
| V21 | S41 | 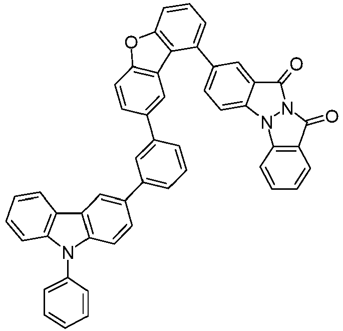 | 70 % |
| | 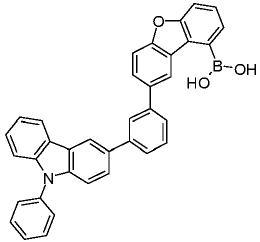 | | |
| | 1822311-37-4 | | |
| V22 | S39 | 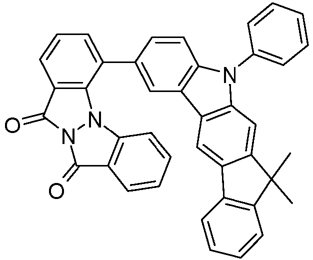 | 44 % |
| | 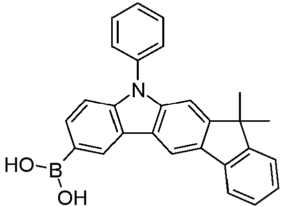 | | |
| | 1379585-25-7 | | |
| V23 | S41 | 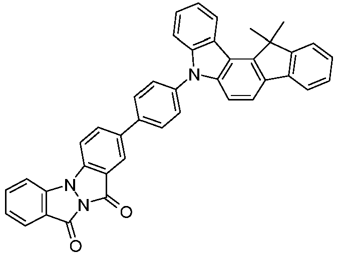 | 69 % |
| | 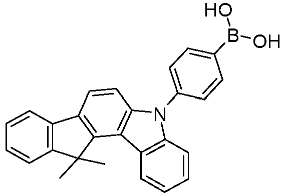 | | |
| | 2241229-08-1 | | |
| V24 | S41 | 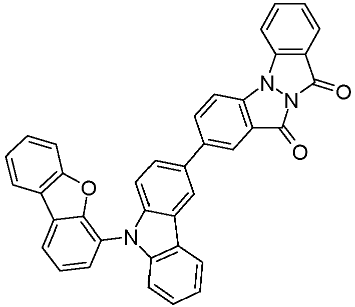 | 67 % |
| | 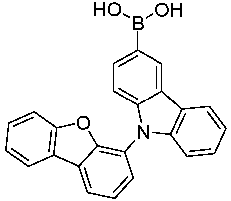 | | |
| | 1547397-15-8 | | |
| V25 | S42 | 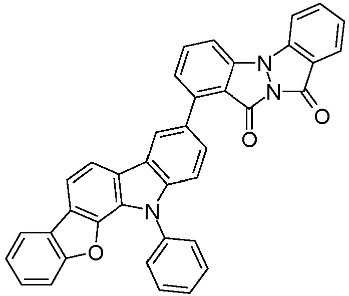 | 55 % |
| | 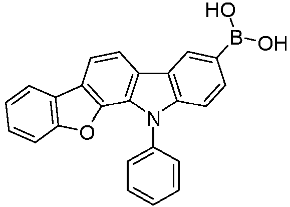 | | |
| | 2128290-87-7 | | |
| V26 | S25 | 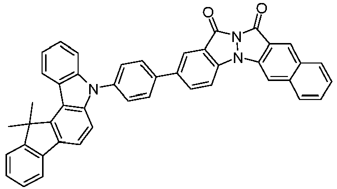 | 67 % |
| | 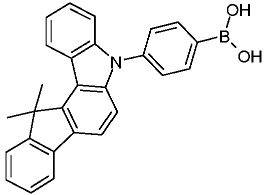 | | |
| | 2241229-08-1 | | |
| V27 | S28 | 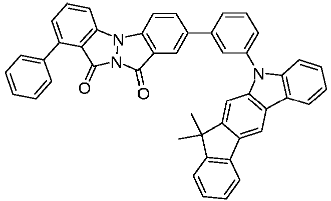 | 66 % |
| | 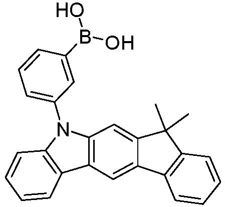 | | |
| | 1369587-64-3 | | |
| V28 | S30 | 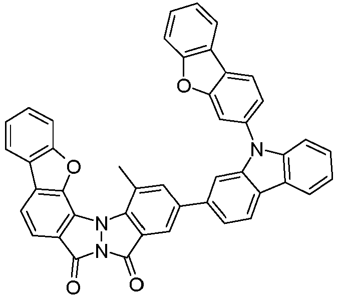 | 53 % |
| | 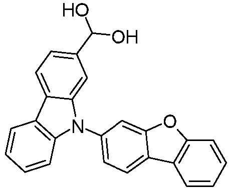 | | |
| | 1637323-05-7 | | |
| V29 | S34 | 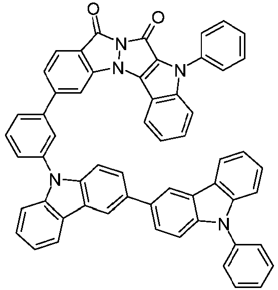 | 72 % |
| | 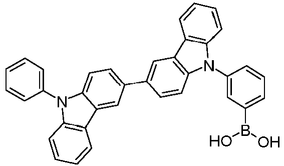 | | |
| | 1398394-64-3 | | |
| V30 | S42 | 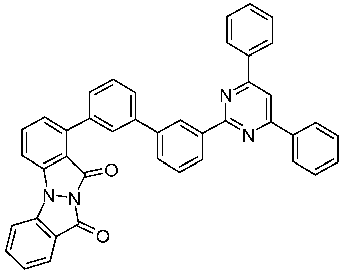 | 56 % |
| | 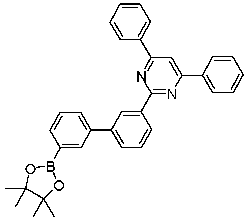 | | |
| | 1802233-05-1 | | |
| V31 | S40 | 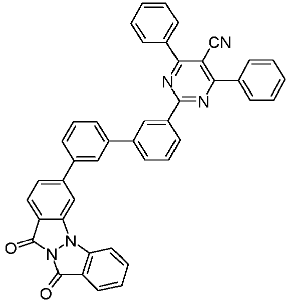 | 68 % |
| | 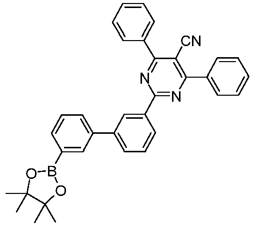 | | |
| | 2319558-93-3 | | |
| V32 | S41 | 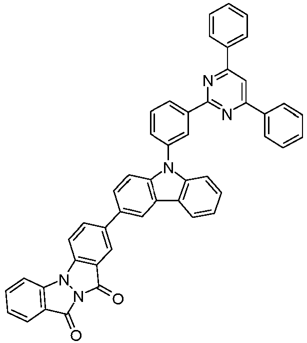 | 64 % |
| | 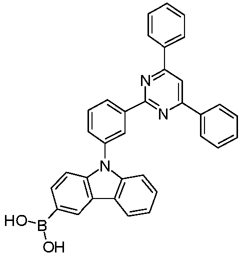 | | |
| | 1813537-09-5 | | |
| V33 | S39 | 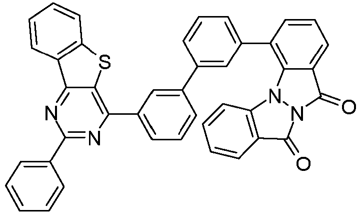 | 47 % |
| | 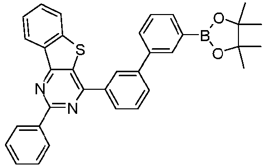 | | |
| | 1801325-80-3 | | |
| V34 | S42 | 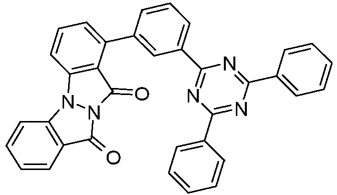 | 54 % |
| | 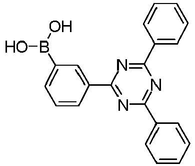 | | |
| | 1612243-82-9 | | |
| V35 | S41 | 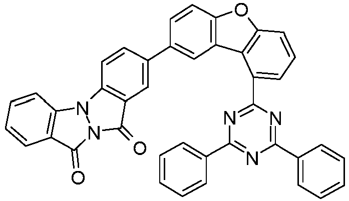 | 69 % |
| | 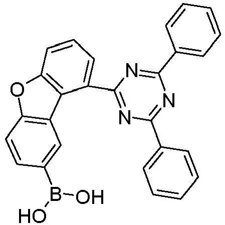 | | |
| | 2138490-96-5 | | |
| V36 | S42 | 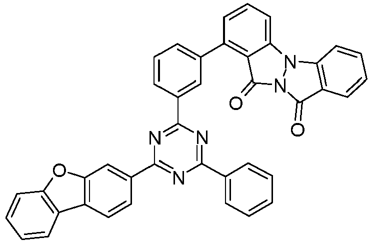 | 56 % |
| | 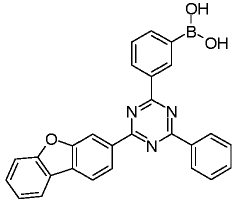 | | |
| | 2305626-41-7 | | |
| V37 | S42 | 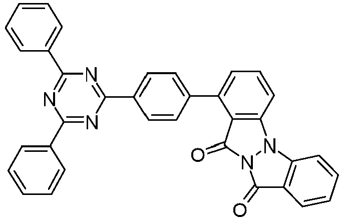 | 55 % |
| | 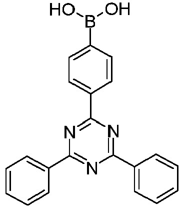 | | |
| | 1313018-07-3 | | |
| V38 | S40 | 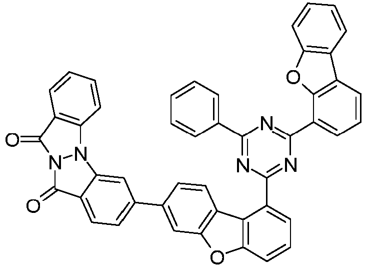 | 71 % |
| | 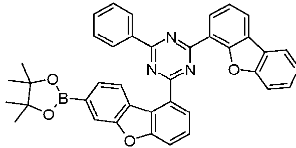 | | |
| | 2268674-03-7 | | |
| V39 | S34 | 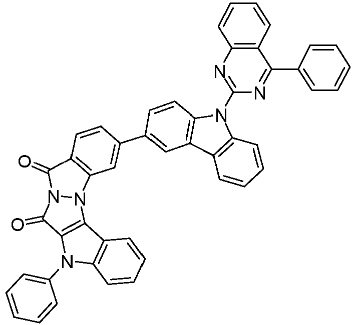 | 71 % |
| | 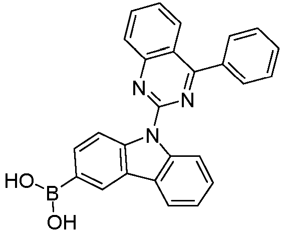 | | |
| | 1642121-58-1 | | |
| V40 | S24 | 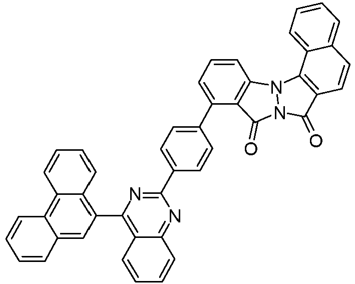 | 56 % |
| | 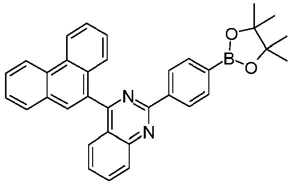 | | |
| | 1637377-61-7 | | |
| V41 | S40 | 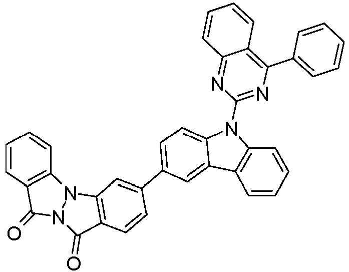 | 73 % |
| | 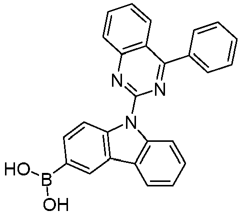 | | |
| | 1642121-58-1 | | |
| V42 | S42 | 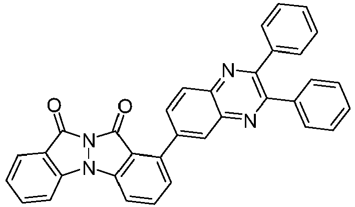 | 57 % |
| | 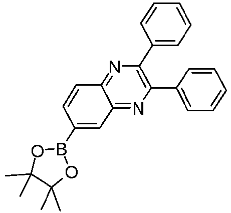 | | |
| | 1133318-06-5 | | |
| V43 | S40 | 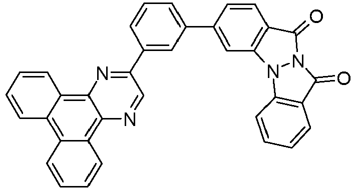 | 70 % |
| | 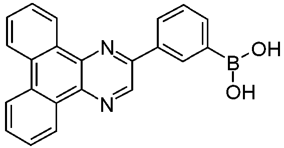 | | |
| | 1609021-60-4 | | |
| V44 | S42 | 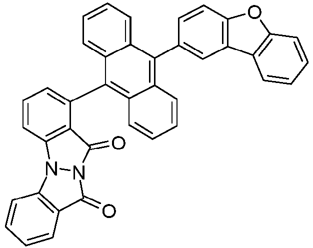 | 52 % |
| | 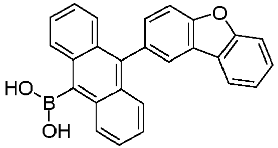 | | |
| | 1188095-78-4 | | |
| V45 | S41 | 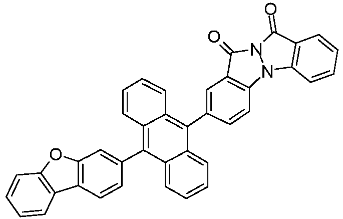 | 67 % |
| | 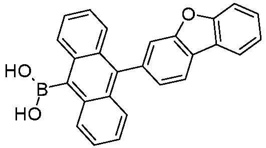 | | |
| | 2241542-34-5 | | |
| V46 | S42 | 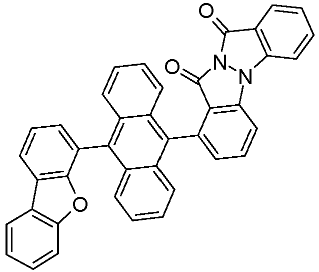 | 55 % |
| | 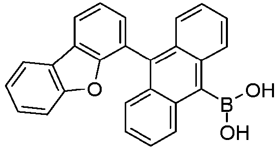 | | |
| | 1304130-07-1 | | |
| V47 | S7 | 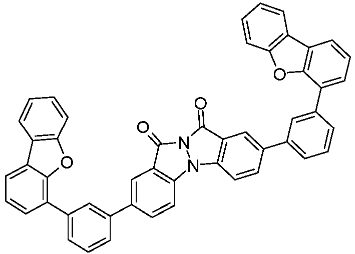 | 58 % |
| | 50 mmol | | |
| | 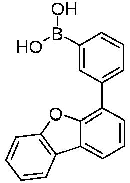 | | |
| | 1271726-52-3 | | |
| V48 | S6 | 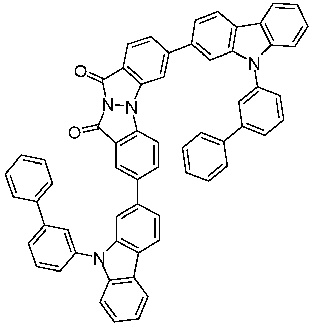 | 50 % |
| | 50 mmol | | |
| | 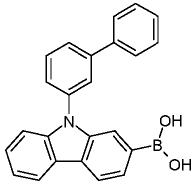 | | |
| | 1835251-21-2 | | |
| V49 | S10 | 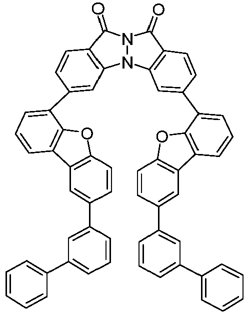 | 48 % |
| | 50 mmol | | |
| | 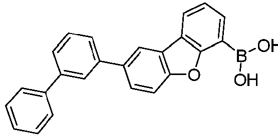 | | |
| | 2305718-67-4 | | |
| V50 | S12 | 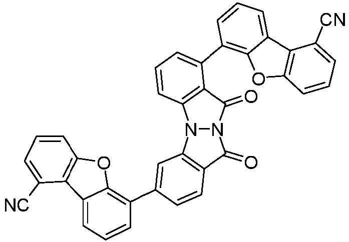 | 53 % |
| | 50 mmol | | |
| | 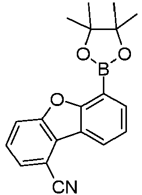 | | |
| | 2458817-52-0 | | |
| V51 | S18 | 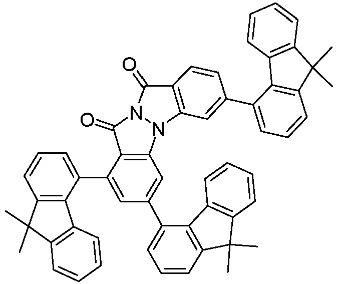 | 46 % |
| | 30 mmol | | |
| | 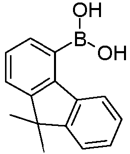 | | |
| | 1246022-50-3 | | |
| V52 | S23 | 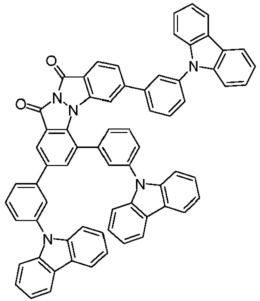 | 39 % |
| | 30 mmol | | |
| | 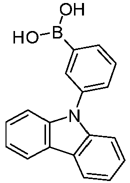 | | |
| | 864377-33-3 | | |
| V53 | S50 | 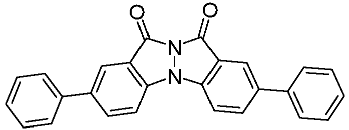 | 70% |
| | 50 mmol | | |
| | 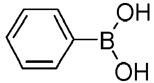 | | |
| | 98-80-6 | | |
| V54 | S51 | 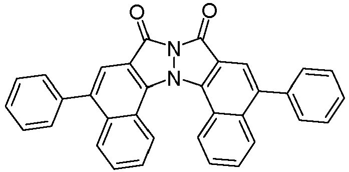 | 67 % |
| | 50 mmol | | |
| | 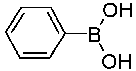 | | |
| | 98-80-6 | | |
| V55 | S52 | 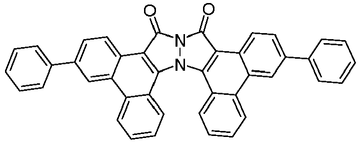 | 74 % |
| | 50 mmol | | |
| | 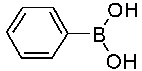 | | |
| | 98-80-6 | | |
| V56 | S50 | 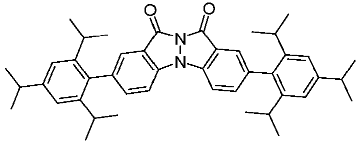 | 46 % |
| | 50 mmol | | |
| | 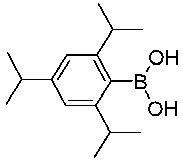 | | |
| | 154549-38-9 | | |
| V57 | S51 | 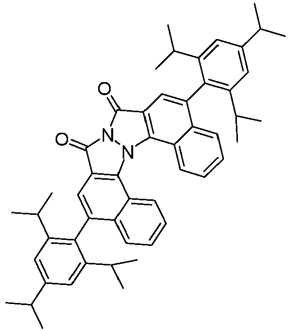 | 43 % |
| | 50 mmol | | |
| | 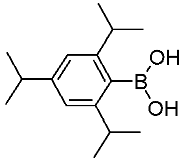 | | |
| | 154549-38-9 | | |
| V58 | S50 | 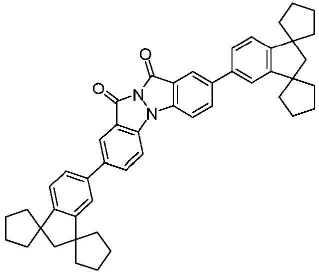 | 67 % |
| | 50 mmol | | |
| | 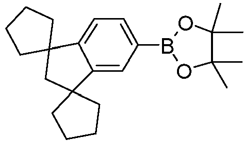 | | |
| | 1912467-76-5 | | |
| V59 | S51 | 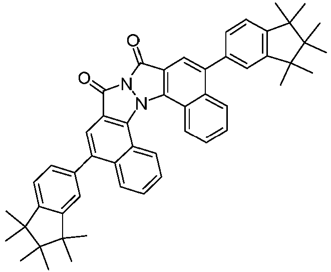 | 59 % |
| | 50 mmol | | |
| | 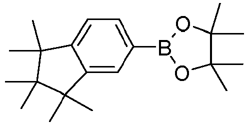 | | |
| | 1562418-16-9 | | |
| V60 | S27 | 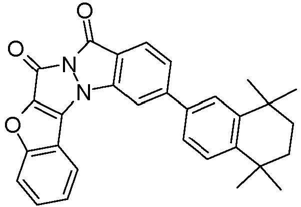 | 60 % |
| | 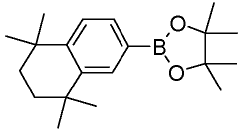 | | |
| | 169126-63-0 | | |
| V61 | S28 | 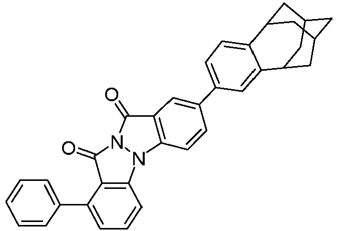 | 65 % |
| | 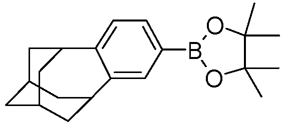 | | |
| | 1801624-63-4 | | |
| V62 | S50 | 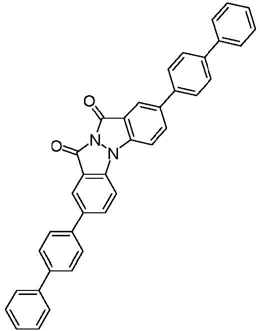 | 57 % |
| | 50 mmol | | |
| | 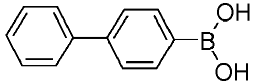 | | |
| | 5122-94-1 | | |
| V63 | S50 | 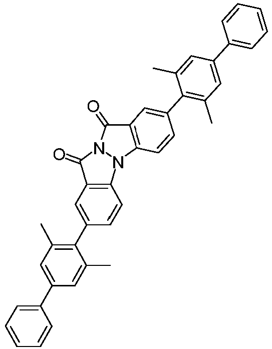 | 53 % |
| | 50 mmol | | |
| | 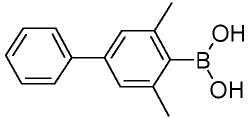 | | |
| | 1057654-43-9 | | |
| V64 | S50 | 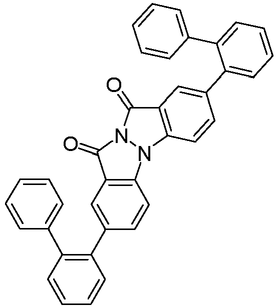 | 51 % |
| | 50 mmol | | |
| | 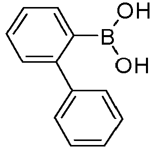 | | |
| | 4688-76-0 | | |
| V65 | S50 | 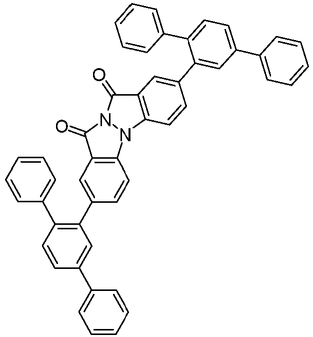 | 62 % |
| | 50 mmol | | |
| | 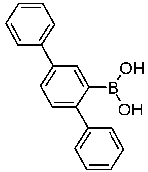 | | |
| | 1205748-34-0 | | |
| V66 | S51 | 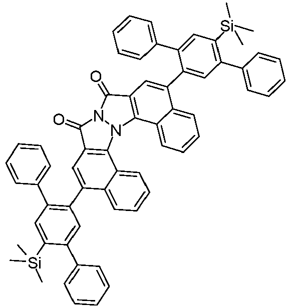 | 41 % |
| | 50 mmol | | |
| | 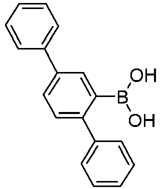 | | |
| | 1602576-91-9 | | |
| V67 | S50 | 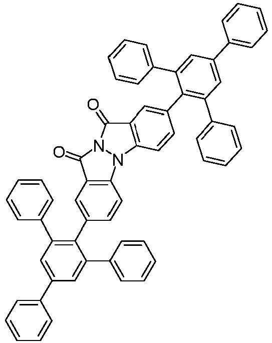 | 47 % |
| | 50 mmol | | |
| | 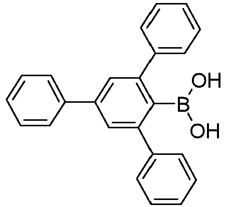 | | |
| | 1004321-87-2 | | |
| V68 | S50 | 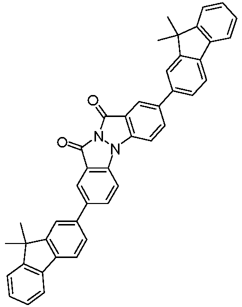 | 63 % |
| | 50 mmol | | |
| | 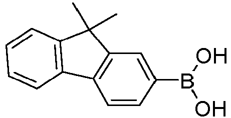 | | |
| | 333432-28-3 | | |
| V69 | S50 | 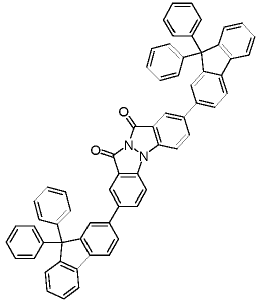 | 60 % |
| | 50 mmol | | |
| | 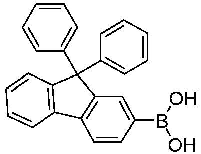 | | |
| | 400607-31-0 | | |
| V70 | S24 | 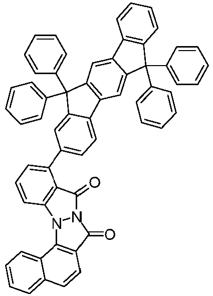 | 59 % |
| | 50 mmol | | |
| | 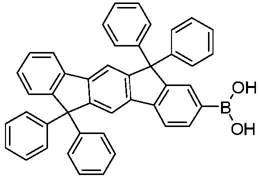 | | |
| | 1207559-33-8 | | |
| V71 | S25 | 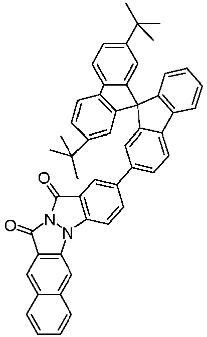 | 72 % |
| | 50 mmol | | |
| | 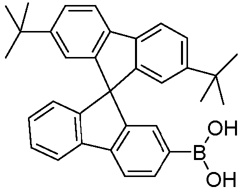 | | |
| | 1241891-39-3 | | |
| V72 | S43 | 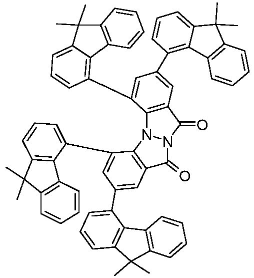 | 31 % |
| | 25 mmol | | |
| | 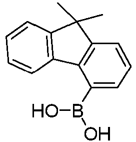 | | |
| | 1246022-50-3 | | |
| V73 | S50 | 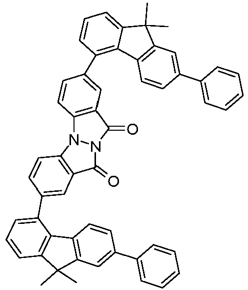 | 61 % |
| | 50 mmol | | |
| | 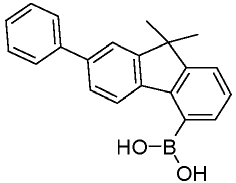 | | |
| | 2597100-63-3 | | |
| V74 | S51 | 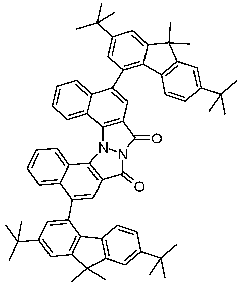 | 59 % |
| | 50 mmol | | |
| | 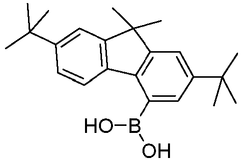 | | |
| | 1092576-56-1 | | |
| V75 | S53 | 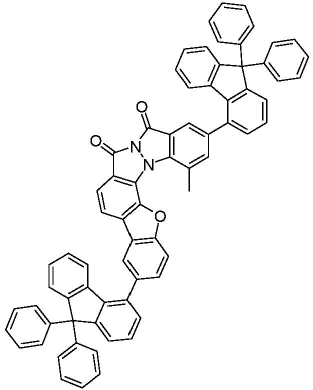 | 51 % |
| | 50 mmol | | |
| | 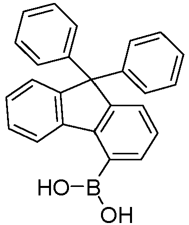 | | |
| | 1224976-40-2 | | |
| V76 | S50 | 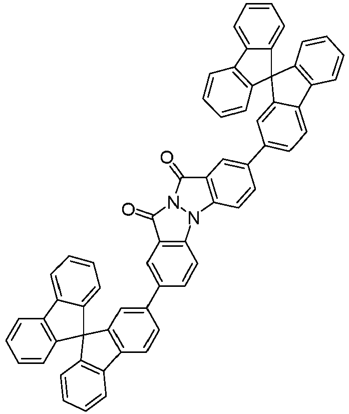 | 66 % |
| | 50 mmol | | |
| | 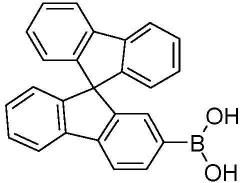 | | |
| | 1421789-05-0 | | |
| V77 | S34 | 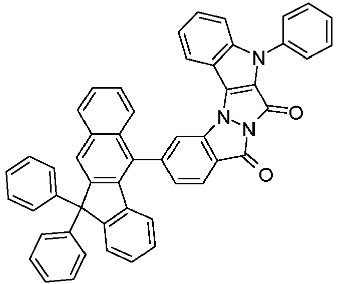 | 70% |
| | 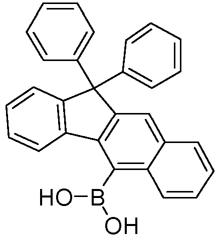 | | |
| | 2540904-37-6 | | |
| V78 | S50 | 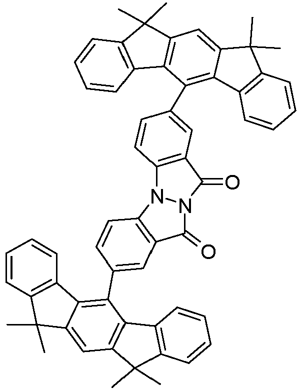 | 50 % |
| | 50 mmol | | |
| | 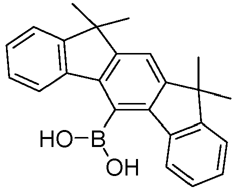 | | |
| | 2364629-32-1 | | |
| V79 | S50 | 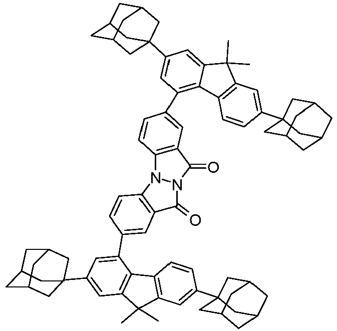 | 49 % |
| | 50 mmol | | |
| | 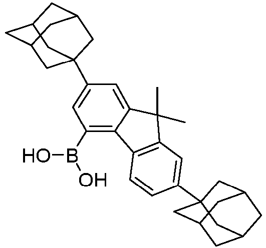 | | |
| | 1630795-54-8 | | |
| V80 | S50 | 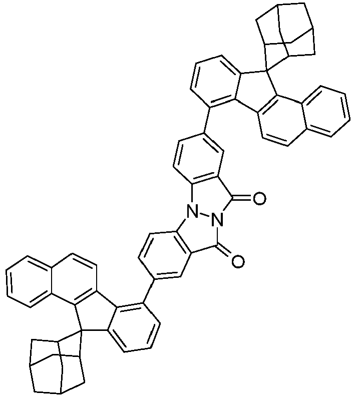 | 50 % |
| | 50 mmol | | |
| | 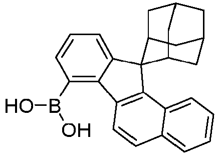 | | |
| | 2549197-79-5 | | |
| V81 | S43 | 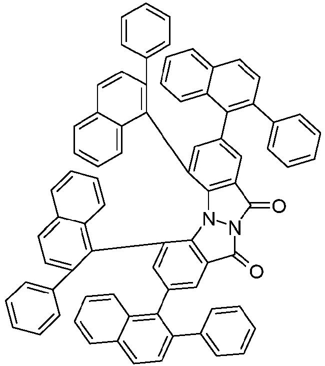 | 21 % |
| | 25 mmol | | |
| | 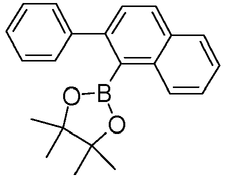 | | |
| | 612086-25-6 | | |
| V82 | S43 | 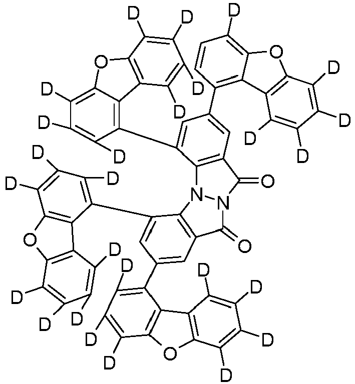 | 23 % |
| | 25 mmol | | |
| | 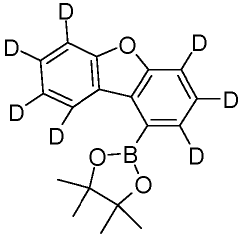 | | |
| | 2605856-02-6 | | |
| V83 | S50 | 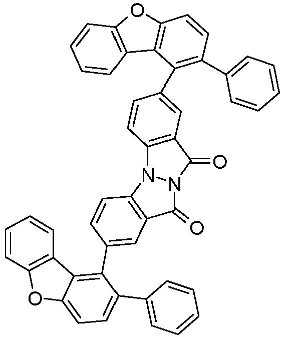 | 60 % |
| | 50 mmol | | |
| | 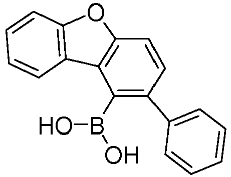 | | |
| | 2402769-87-1 | | |
| V84 | S50 | 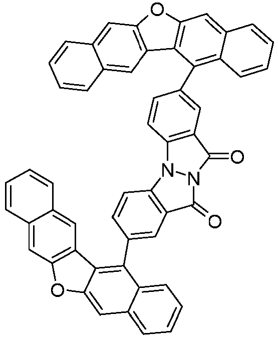 | 51 % |
| | 50 mmol | | |
| | 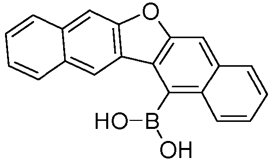 | | |
| | 2049003-24-7 | | |
| V85 | S29 | 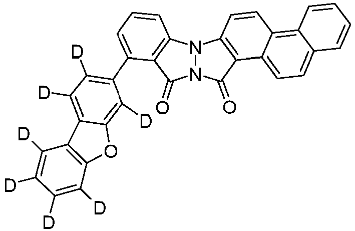 | 59 % |
| | 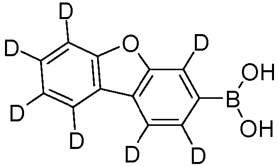 | | |
| | 2377545-64-5 | | |
| V86 | S30 | 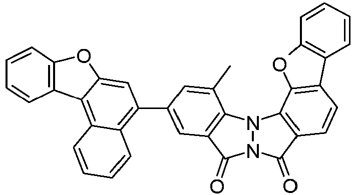 | 62 % |
| | 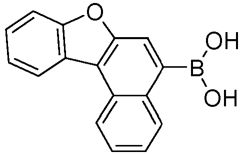 | | |
| | 2035080-71-6 | | |
| V87 | S28 | 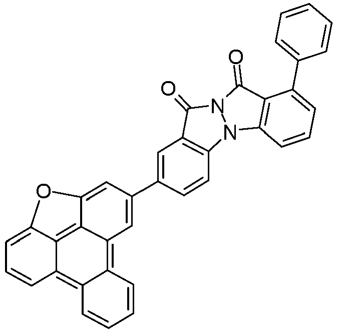 | 60 % |
| | 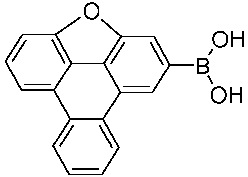 | | |
| | 2606072-21-1 | | |
| V88 | S50 | 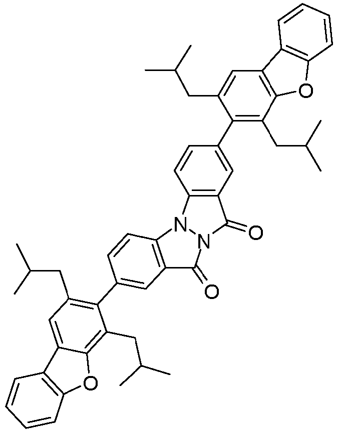 | 54 % |
| | 50 mmol | | |
| | 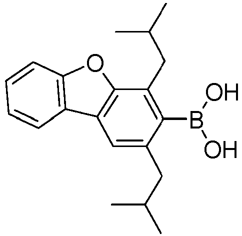 | | |
| | 1651221-10-1 | | |
| V89 | S29 | 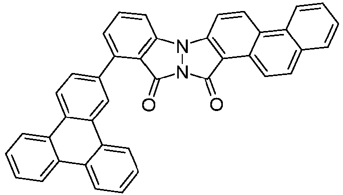 | 59 % |
| | 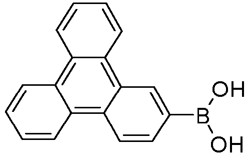 | | |
| | 654664-63-8 | | |
| V90 | S28 | 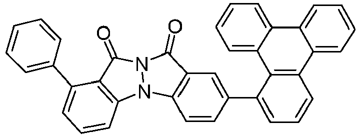 | 68 % |
| | 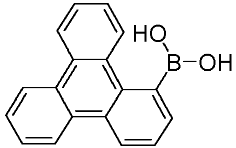 | | |
| | 1430392-46-3 | | |
| V91 | S50 | 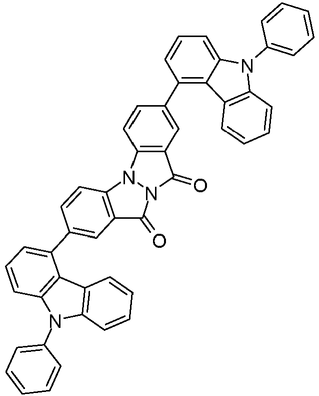 | 51 % |
| | 50 mmol | | |
| | 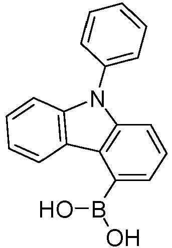 | | |
| | 1370555-65-9 | | |
| V92 | S28 | 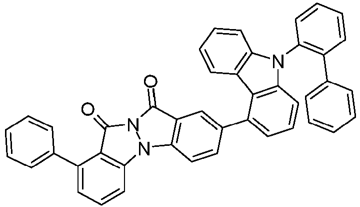 | 62 % |
| | 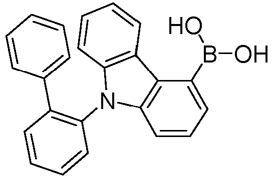 | | |
| | 2361216-20-6 | | |
| V93 | S30 | 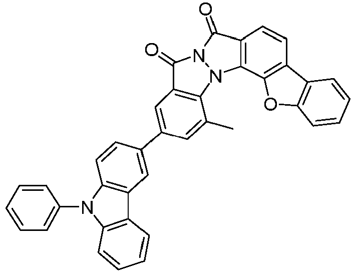 | 67 % |
| | 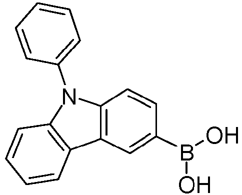 | | |
| | 854952-58-2 | | |
| V94 | S24 | 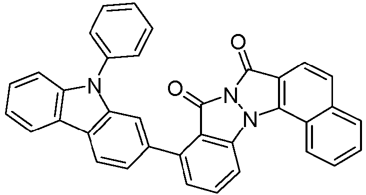 | 53 % |
| | 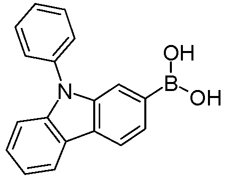 | | |
| | 1001911-63-2 | | |
| V95 | S31 | 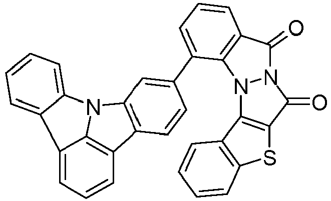 | 43 % |
| | 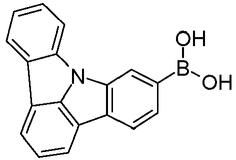 | | |
| | 2193827-55-1 | | |
| V96 | S50 | 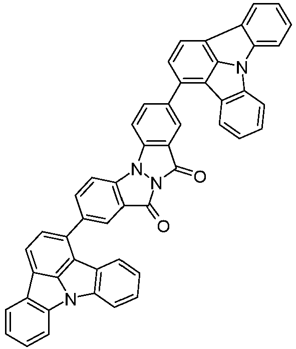 | 61 % |
| | 50 mmol | | |
| | 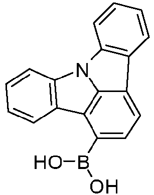 | | |
| | 2144406-20-0 | | |
| V97 | S50 | 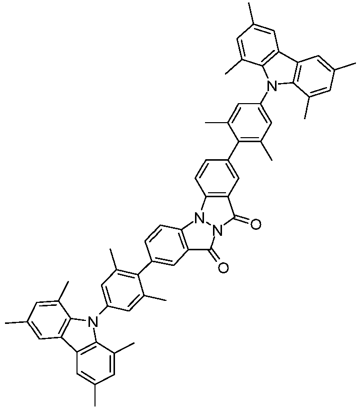 | 50 % |
| | 50 mmol | | |
| | 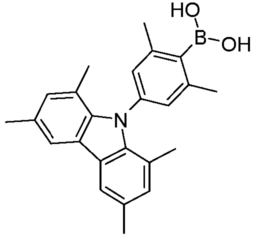 | | |
| | 2595368-09-3 | | |
| V98 | S50 | 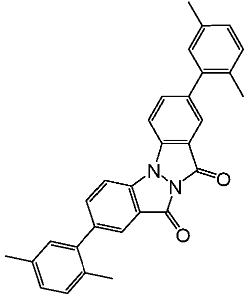 | 66 % |
| | 50 mmol | | |
| | 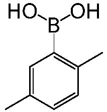 | | |
| | 85199-06-0 | | |
| V99 | S50 | 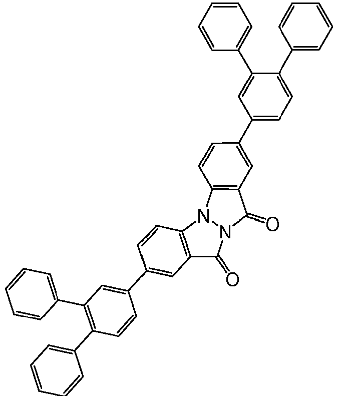 | 61 % |
| | 50 mmol | | |
| | 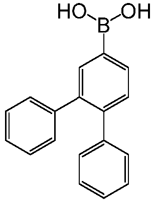 | | |
| | 1246022-49-0 | | |

### Beispiel V200:

### Schritt 1: Brom -Suzuki-Kupplung

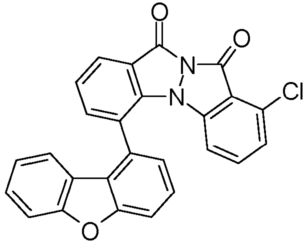

Ein Gemisch aus 35.0 g (100 mmol) S1, g (110 mmol) 1-Dibenzofuranylboronsäure [162607-19-4], 10.6 g (100 mmol) Natriumcarbonat, 1.83 g (6 mmol) Tri-o-tolylphosphin, 225 mg (1 mmol) Palladium(II)acetat, 400 ml Toluol, 200 ml Dioxan und 400 ml Wasser wird 24 h unter Rückfluss erhitzt. Nach Erkalten trennt man die org. Phase ab, wäscht diese zweimal mit je 300 ml Wasser und einmal mit 300 ml ges. Kochsalzlösung. Man engt die org. Phase zur Trockene ein, nimmt den Rückstand in Dichlormethan (DCM) auf, filtriert über ein mit DCM vorgeschlämmtes Kieselgelbett ab und engt das Filtrat am Rotationsverdampfer ein, wobei das abrotierte DCM kontinuierlich durch Methanol ersetzt wird. Man saugt vom auskristallisierten Produkt ab, wäscht dieses zweimal mit ja 50 ml Methanol und trocknet im Vakuum. Das Rohprodukt wird aus Acetonitril umkristallisiert. Ausbeute: 30.3 g (69 mmol), 69 %. Reinheit: ca. 98 % ig nach ¹H-NMR.

### Schritt 2: Chlor-Suzuki-Kupplung

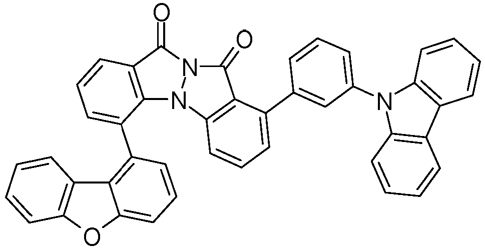

Durchführung analog zu Beispiel V1, Einsatz von 3-(9H-Carbazol-9-yl)phenylboronsäure [864377-33-3]. Ausbeute: 30.3 g (47 mmol), 47 %. Reinheit: ca. 99.9 % nach HPLC.

Analog können folgende Verbindungen dargestellt werden:

| **Bsp.** | **Edukte** | **Produkt** | **Ausbeute** |
|---|---|---|---|
| V201 | S2 | 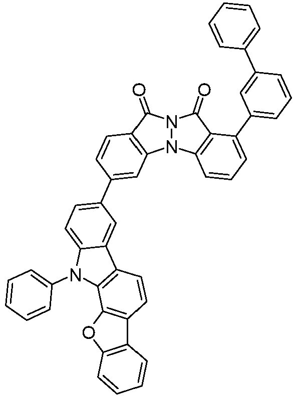 | 35 % |
| | 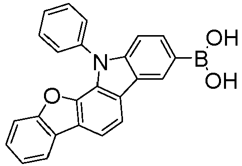 | | |
| | 2128290-87-7 | | |
| | 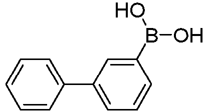 | | |
| | 5122-95-2 | | |
| V202 | S3 | 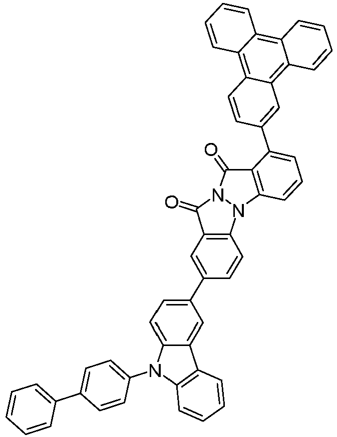 | 31 % |
| | 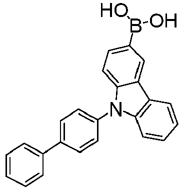 | | |
| | 1028648-22-7 | | |
| | 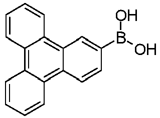 | | |
| | 654664-63-8 | | |
| V203 | S4 | 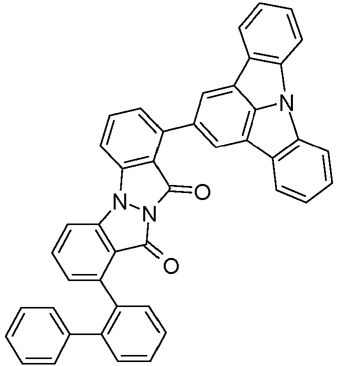 | 30 % |
| | 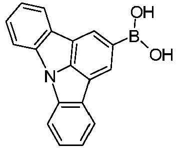 | | |
| | 1174032-85-9 | | |
| | 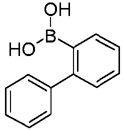 | | |
| | 4688-76-0 | | |
| V204 | S5 | 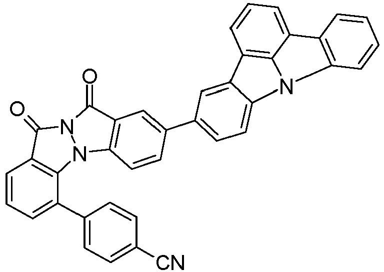 | 28 % |
| | 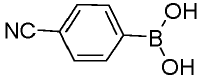 | | |
| | 126747-14-6 | | |
| | 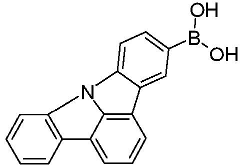 | | |
| | 1174032-92-8 | | |
| V205 | S6 | 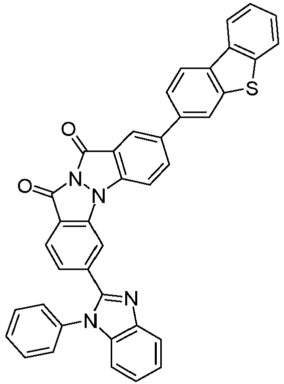 | 46 % |
| | 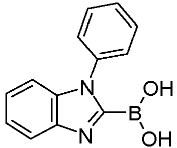 | | |
| | 1214723-25-7 | | |
| | 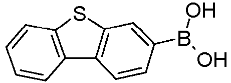 | | |
| | 108847-24-1 | | |
| V206 | S7 | 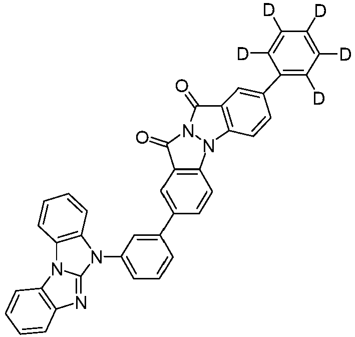 | 45 % |
| | 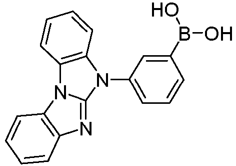 | | |
| | 1401068-23-2 | | |
| | 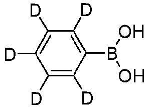 | | |
| | 215527-70-1 | | |
| V207 | S8 | 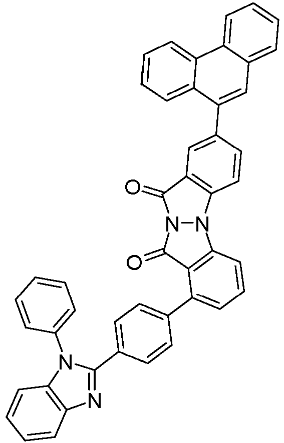 | 41 % |
| | 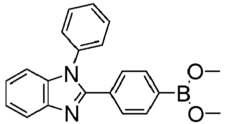 | | |
| | 2395777-93-0 | | |
| | 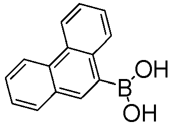 | | |
| | 68572-87-2 | | |
| V208 | S9 | 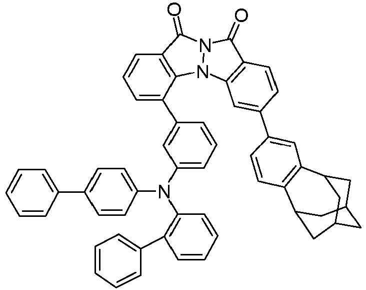 | 36 % |
| | 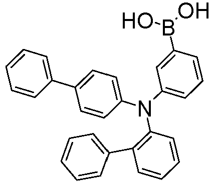 | | |
| | 1978347-56-6 | | |
| | 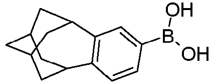 | | |
| | 1801624-63-4 | | |
| V209 | S10 | 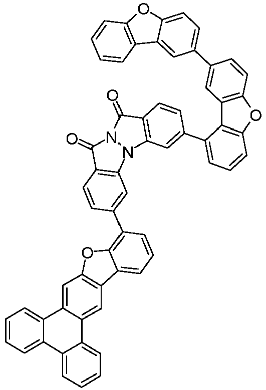 | 45% |
| | 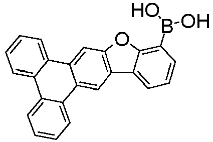 | | |
| | 2454395-23-2 | | |
| | 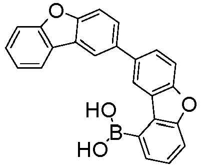 | | |
| | 1822320-55-7 | | |
| V210 | S11 | 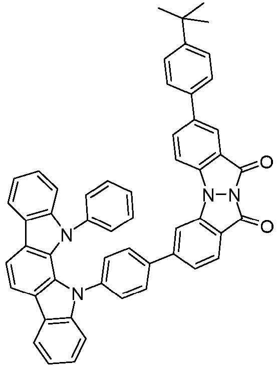 | 44 % |
| | 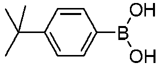 | | |
| | 123324-71-0 | | |
| | 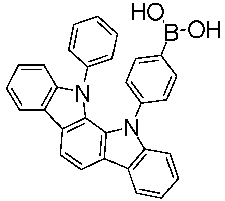 | | |
| | 2222276-71-1 | | |
| V211 | S12 | 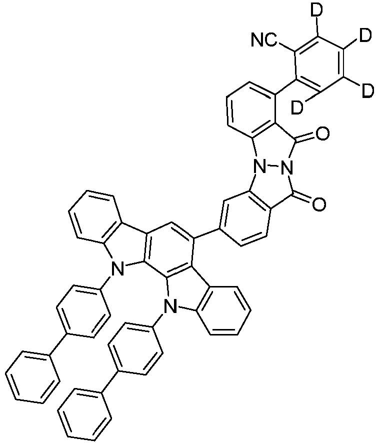 | 41 % |
| | 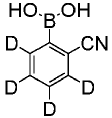 | | |
| | 2241870-66-4 | | |
| | 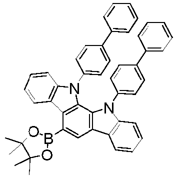 | | |
| | 1900681-96-0 | | |
| V212 | S13 | 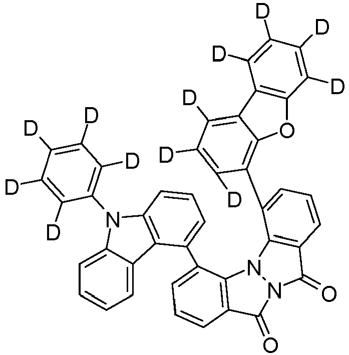 | 22 % |
| | 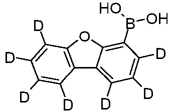 | | |
| | 2230311-62-1 | | |
| | 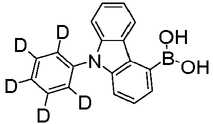 | | |
| | 2271286-98-5 | | |
| V213 | S14 | 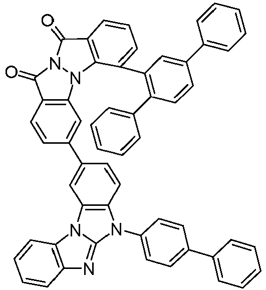 | 37 % |
| | 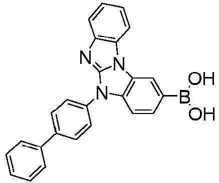 | | |
| | 2217618-64-7 | | |
| | 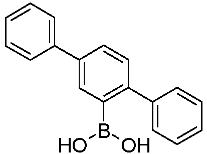 | | |
| | 1205748-34-0 | | |
| V214 | S15 | 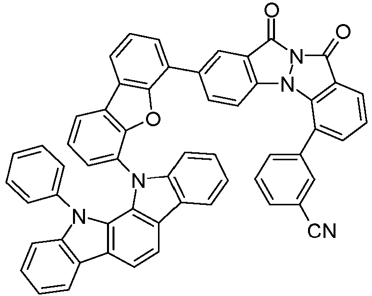 | 35 % |
| | 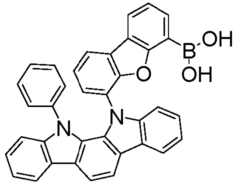 | | |
| | 1609088-39-2 | | |
| | 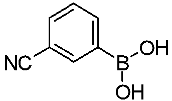 | | |
| | 150255-96-2 | | |
| V215 | S16 | 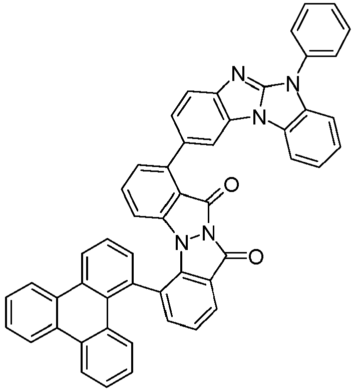 | 31 % |
| | 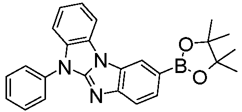 | | |
| | 2093171-71-0 | | |
| | 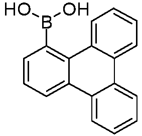 | | |
| | 1430392-46-3 | | |
| V216 | S17 | 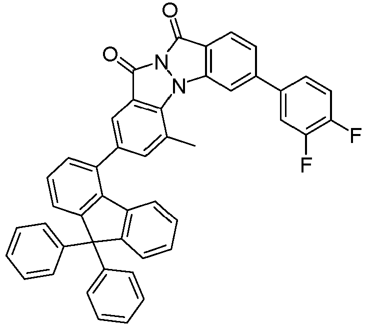 | 43 % |
| | 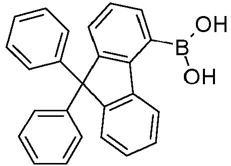 | | |
| | 1224976-40-2 | | |
| | 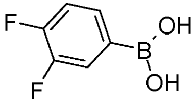 | | |
| | 168267-41-2 | | |
| V217 | S18 | 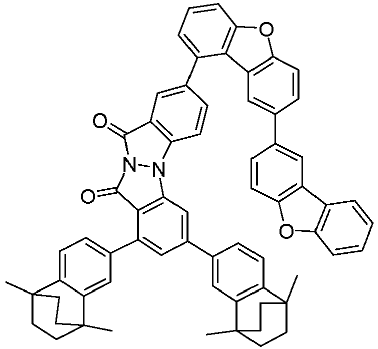 | 35 % |
| | 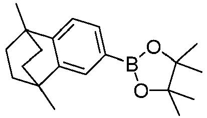 | | |
| | 1801624-61-2 | | |
| | 200 mmol | | |
| | 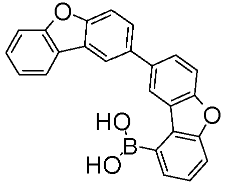 | | |
| | 1822320-55-7 | | |
| V218 | S19 | 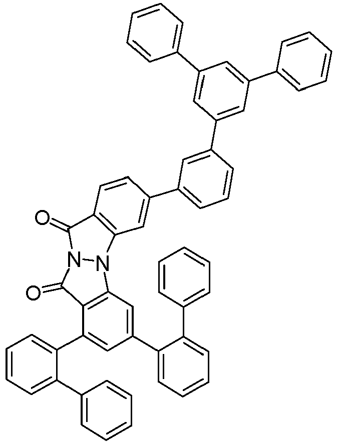 | 33 % |
| | 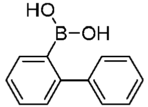 | | |
| | 4688-76-0 | | |
| | 200 mmol | | |
| | 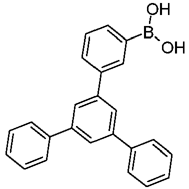 | | |
| | 1233200-59-3 | | |
| V219 | S20 | 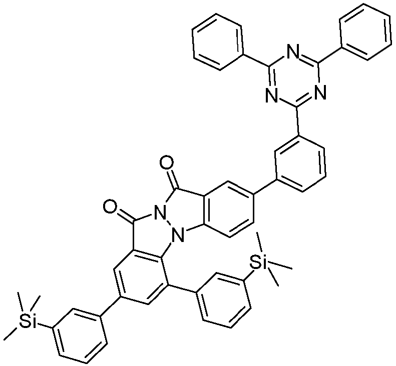 | 29 % |
| | 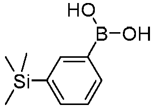 | | |
| | 177171-16-3 | | |
| | 200 mmol | | |
| | 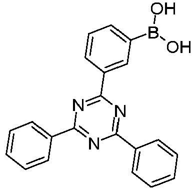 | | |
| | 1612243-82-9 | | |
| V220 | S21 | 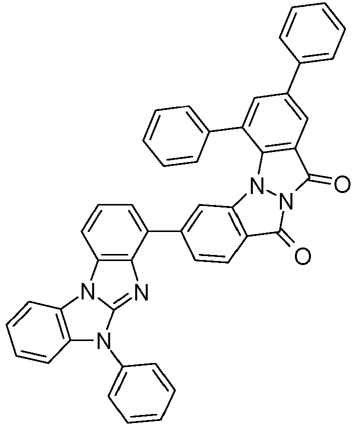 | 37 % |
| | 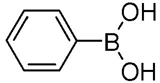 | | |
| | 98-80-6 | | |
| | 200 mmol | | |
| | 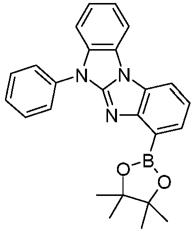 | | |
| | 2111193-19-0 | | |
| V221 | S22 | 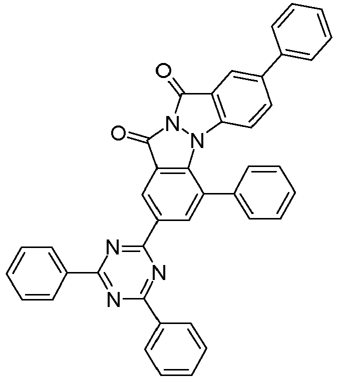 | 35 % |
| | 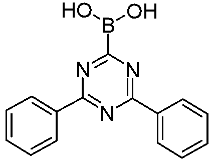 | | |
| | 1251825-65-6 | | |
| | 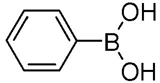 | | |
| | 98-80-6 | | |
| | 210 mmol | | |
| V222 | S23 | 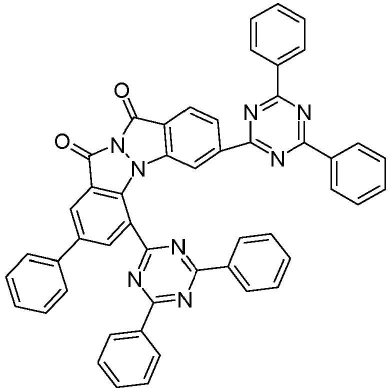 | 31 % |
| | 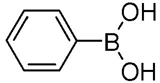 | | |
| | 98-80-6 | | |
| | 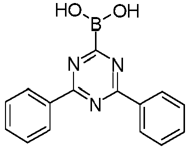 | | |
| | 1251825-65-6 | | |
| | 220 mmol | | |
| V223 | S7 | 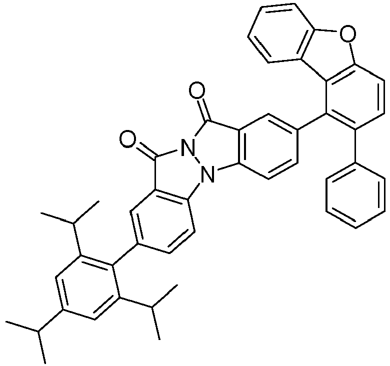 | 48 % |
| | 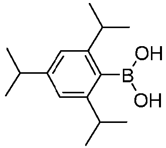 | | |
| | 154549-38-9 | | |
| | 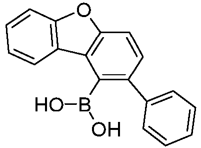 | | |
| | 2402769-87-1 | | |
| V224 | S7 | 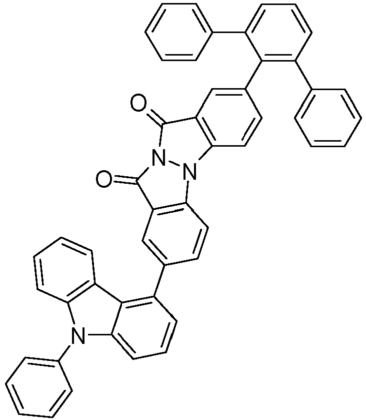 | 47 % |
| | 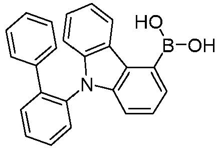 | | |
| | 2361216-20-6 | | |
| | 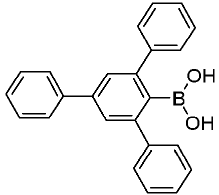 | | |
| | 1004321-87-2 | | |

### Beispiel V300:

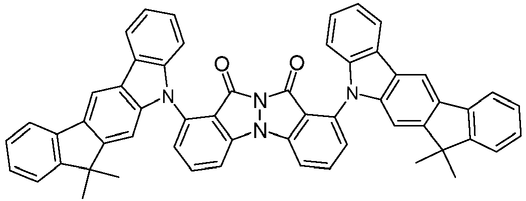

Ein Gemisch aus 13.6 g (50 mmol) S4, 31.2 g (110 mmol) 5,7-Dihydro-7,7-dimethylindeno[2,1-b]carbazol [1257220-47-5], 14.4 g (150 mmol) Natrium-tert-butanolat, 2.65 g (4 mmol) rac-BINAP (alternativ kann S-Phos, X-Phos, Tri-tert-butylphosphin eingesetzt werden), 674 mg (3 mmol) Palladium(II)acetat, 100 g Glaskugeln (3 mm Durchmesser) und 300 ml Toluol wird 24 h bei 110 °C gerührt. Man saugt noch heiß über ein mit heißem Toluol vorgeschlämmtes Kieselgel-Bett ab, engt das Filtrat im Vakuum zur Trockene ein und rührt der Feststoff mit 300 ml Methanol heiß aus. Man saugt noch warm vom Feststoff ab, wäscht diesen zweimal mit 50 ml Methanol nach und trocknet im Vakuum. Die weitere Reinigung erfolgt durch Heißextraktionskristallisation (Lösemittelgemische DCM / Acetonitril 1:3 bis 3:1) und fraktionierte Sublimation. Ausbeute: 20.1 g (25 mol), 50 %. Reinheit: ca. 99.9 % nach HPLC.

Analog können folgende Verbindungen dargestellt werden:

| **Bsp.** | **Edukte** | **Produkt** | **Ausbeute** |
|---|---|---|---|
| V301 | S2 | 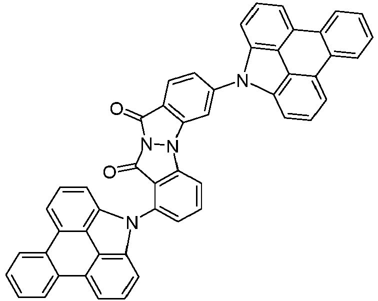 | 46 % |
| | 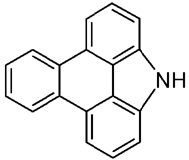 | | |
| | 109606-75-9 | | |
| V302 | S3 | 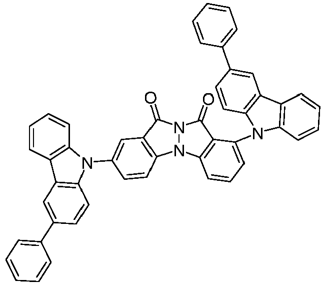 | 44 % |
| | 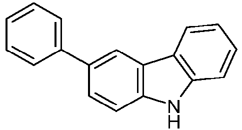 | | |
| | 103012-26-6 | | |
| V303 | S10 | 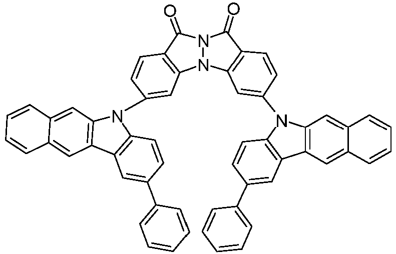 | 40 % |
| | 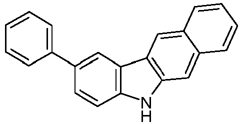 | | |
| | 1819346-21-8 | | |
| V304 | S35 | 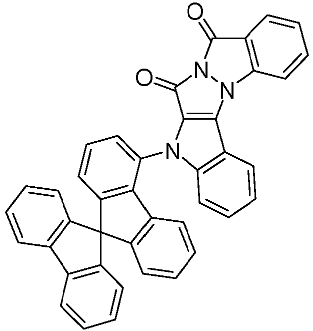 | 57 % |
| | 100 mmol | | |
| | 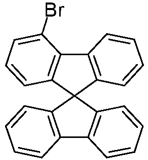 | | |
| | 1161009-88-6 | | |
| | 110 mmol | | |
| V305 | S35 | 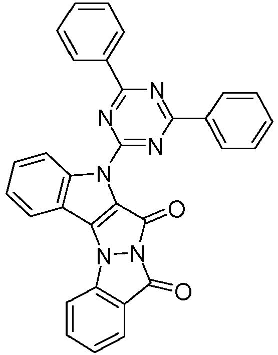 | 59 % |
| | 100 mmol | | |
| | 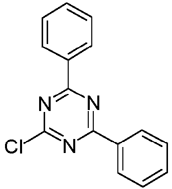 | | |
| | 3842-55-5 | | |
| | 120 mmol | | |
| | 110 mmol NaO-t-Bu | | |
| V306 | S35 | 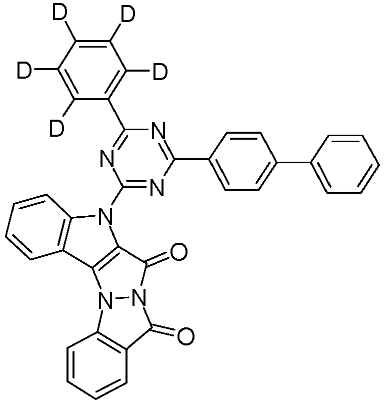 | 56 % |
| | 100 mmol | | |
| | 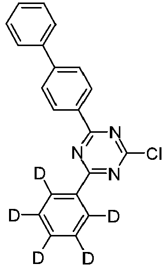 | | |
| | 2401923-63-3 | | |
| | 130 mmol | | |
| | 110 mmol NaO-t-Bu Dimethylacetamid statt Toluol | | |
| V307 | S35 | 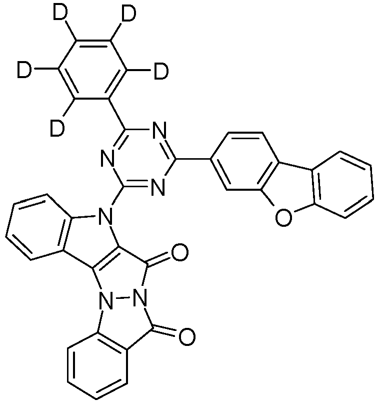 | 54 % |
| | 100 mmol | | |
| | 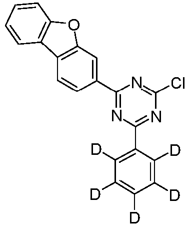 | | |
| | 2226747-65-3 | | |
| | 130 mmol | | |
| | 110 mmol NaO-t-Bu Dimethylacetamid statt Toluol | | |
| V308 | S36 | 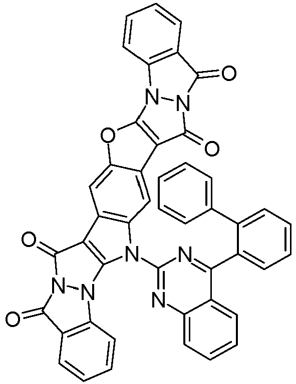 | 60 % |
| | 100 mmol | | |
| | 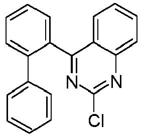 | | |
| | 1852465-53-2 | | |
| | 120 mmol | | |
| | 110 mmol NaO-t-Bu Dimethylacetamid statt Toluol | | |
| V309 | S37 | 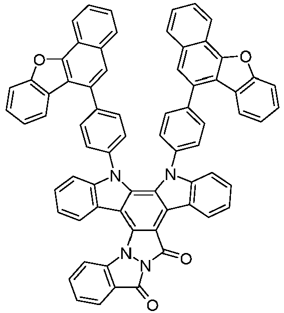 | 34 % |
| | 50 mmol | | |
| | 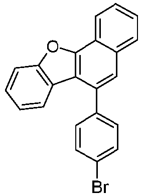 | | |
| | 2541485-90-7 | | |
| | 110 mmol | | |
| V310 | S38 | 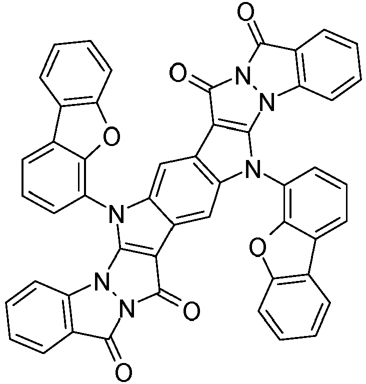 | 47 % |
| | 50 mmol | | |
| | 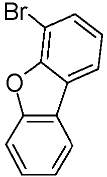 | | |
| | 89827-45-2 | | |
| | 110 mmol | | |

### Beispiel L1:

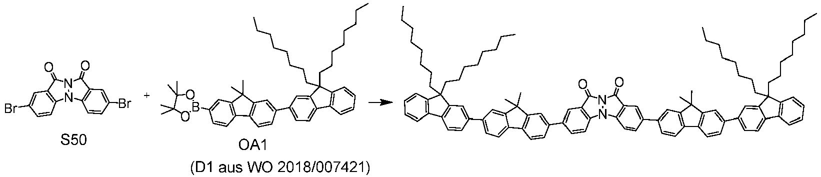

8.27 g (21 mmol) S50, 32.60 g (46 mmol) OA1 und 4.5 g (42.4 mmol) Natriumcarbonat werden in 600 ml eines Gemischs aus Toluol/Ethanol/Wasser (2:1:1) suspendiert und mit Argon entgast. Nach Zugabe von 613 mg (0.53 mmol) Tetrakis(triphenylphosphin)-palladium(0) wird die Mischung bei 110°C gerührt. Nach sechs Stunden lässt man die Reaktionsmischung auf Raumtemperatur erkalten und fügt weitere 200 ml Toluol zu. Die organische Phase wird abgetrennt, zweimal mit je 300 ml Wasser, einmal mit 300 ml ges. Kochsalzlösung gewaschen, über Natriumsulfat getrocknet und über ein mit Toluol vorgeschlämmtes Celite-Bett filtriert. Das Filtrat wird zur Trockene eingeengt, der Rückstand wird in 300 ml Dichlormethan gelöst, mit 200 ml Methanol versetzt und am Rotationsverdampfer auf ca. 300 ml eingeengt. Der ausgefallene Feststoff wird abgesaugt und dreimal mit je 50 ml Methanol gewaschen. Der erhaltene Feststoff wird mittels Säulenchromatographie und mehrmaliger Umkristallisation aus Toluol/n-Heptan bis zu einer HPLC-Reinheit von >99.9% aufgereinigt und abschließend bei 10⁻⁶ mbar und 250 °C getempert. Ausbeute: 6.85 g (4.9 mmol), 24%.

Folgende Verbindungen können in analoger Weise synthetisiert werden:

| **L** | **Edukt 1** | **Edukt 2** | **Produkt** |
|---|---|---|---|
| L2 | S50 | CAS 2171483-75-1 | 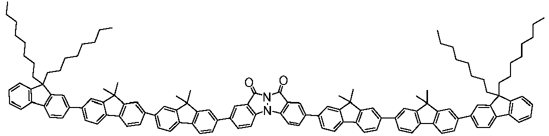 |
| L3 | S50 | G10 aus WO2018/0 07421 | 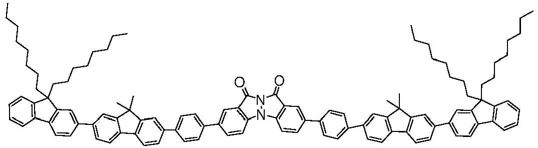 |
| L4 | S50 | Int-D1 | 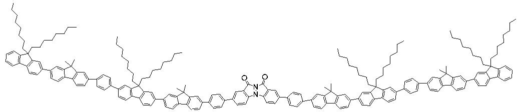 |
| L5 | S51 | Int-D1 | 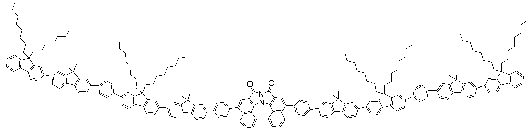 |
| L6 | S52 | Int-D1 | 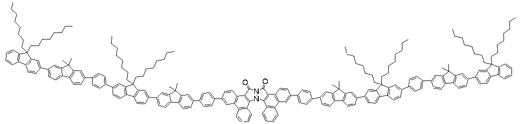 |

### Messung von Photolumineszenspektren (PL-Speltren):

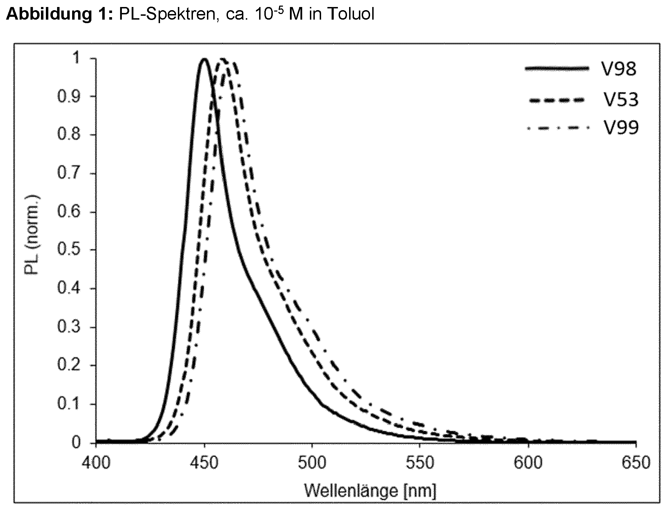

Abbildung 1 zeigt PL-Spektren der erfindungsgemäßen Verbindungen V53, V98 und V99, gemessen mit einem PL-Spektrometer der Fa. Hitachi, F-4500 PL, in ca. 10⁻⁵ molarer, entgaster Toluol-Lösung bei Raumtemperatur (ca. 25 °C).

Die PL-Spektren weisen sehr schmale Emissionsbanden mit geringen FWHM Werten (< 0.18 eV) auf und führen zu besonders farbreiner Emission.

### Herstellung von OLED-Bauteilen

### 1) Vakuum-prozessierte Devices:

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, verwendete Materialien) angepasst wird.

In den folgenden Beispielen werden die Ergebnisse verschiedener OLEDs vorgestellt. Gereinigte Glasplättchen (Reinigung in Miele Laborspülmaschine, Reiniger Merck Extran), die mit strukturiertem ITO (Indium Zinn Oxid) der Dicke 50 nm beschichtet sind, werden 25 Minuten mit UV-Ozon vorbehandelt (UV-Ozon Generator PR-100, Firma UVP) und innerhalb 30 min. zur verbesserten Prozessierung mit 20 nm PEDOT:PSS beschichtet (Poly(3,4-ethylendioxy-thiophen)poly(styrolsulfonat), bezogen als CLEVIOS^{™} P VP AI 4083 von Heraeus Precious Metals GmbH Deutschland, aus wässriger Lösung aufgeschleudert) und anschließend bei 180 °C 10 min. lang ausgeheizt. Diese beschichteten Glasplättchen bilden die Substrate, auf welche die OLEDs aufgebracht werden.

### 1a) Blaue und Grüne Fluoreszenz-OLED- Bauteile - BF und GF:

Alle Materialien werden in einer Vakuumkammer thermisch aufgedampft. Dabei besteht die Emissionsschicht (EML) immer aus mindestens einem Matrixmaterial (Hostmaterial, Wirtsmaterial) SMB (s. Tabelle 1) und einem emittierenden Dotierstoff (Dotand, Emitter) V, der dem Matrixmaterial bzw. den Matrixmaterialien durch Co-Verdampfung in einem bestimmten Volumenanteil beigemischt wird. Eine Angabe wie SMB:V (97:3%) bedeutet hierbei, dass das Material SMB in einem Volumenanteil von 97% und der Dotand V in einem Anteil von 3% in der Schicht vorliegt. Analog können die erfindungsgemäßen Verbindungen V44, V45 und V46 als Host für grüne fluoreszierende Bauteile verwendet werden. Analog kann auch die Elektronentransportschicht aus einer Mischung zweier Materialien bestehen, s. Tabelle 1. Die zur Herstellung der OLEDs verwendeten Materialien sind in Tabelle 7 gezeigt.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten und die Halbwertsbreite auf halber Peakhöhe (FWHM: Full Width Half Maximum) bestimmt.

### Die OLEDs haben folgenden Schichtaufbau:

### Substrat

Lochinjektionsschicht 1 (HIL1) aus HTM1 dotiert mit 5 % NDP-9 (kommerziell erhältlich von der Fa. Novaled), 20 nm
Lochtransportschicht 1 (HTL1) aus HTM1, 140 nm
Lochtransportschicht 2 (HTL2), aus HTM2, 10 nm
Emissionsschicht (EML), s. Tabelle 1
Elektronentransportschicht (ETL2), s. Tabelle 1
Elektronentransportschicht (ETL1), s. Tabelle 1
Elektroneninjektionsschicht (EIL) aus ETM2, 1 nm
Kathode aus Aluminium, 100 nm

**Tabelle 1: Aufbau Blaue BF und Grüne GF Fluoreszenz-OLED-Bauteile**

| **Bsp.** | **EML** | **ETL2** | **ETL1** |
|---|---|---|---|
| **BF1** | SMB1:V64 (97:3%) | --- | ETM1:ETM2 (50:50%) |
| | 20 nm | | 30 nm |
| **BF2** | SMB2:V65 (97:3%) | --- | ETM1:ETM2 (50:50%) |
| | 20 nm | | 30 nm |
| **BF3** | SMB3:V67 (97:3%) | ETM1 5 nm | ETM1:ETM2 (50:50%) |
| | 20 nm | | 30 nm |
| **BF4** | SMB1:V83 (97:3%) | V5 5 nm | V5:ETM2 (50:50%) |
| | 20 nm | | 30 nm |
| **BF5** | SMB1:V53 (97:3%) | --- | ETM1:ETM2 (50:50%) |
| | 20 nm | | 30 nm |
| **BF6** | SMB1:V98 (97:3%) | --- | ETM1:ETM2 (50:50%) |
| | 20 nm | | 30 nm |
| **BF7** | SMB1:V99 (97:3%) | --- | ETM1:ETM2 (50:50%) |
| | 20 nm | | 30 nm |
| **GF1** | V44:V54 (95:5%) | --- | ETM1:ETM2 (50:50%) |
| | 20 nm | | 30 nm |
| **GF2** | V45:V57 (95:5%) | V4 5 nm | V4:ETM2 (50:50%) |
| | 20 nm | | 30 nm |

**Tabelle 2: Ergebnisse Blaue Fluoreszenz-OLED- Bauteile**

| **Bsp.** | **EQE (%) 1000 cd/m²** | **Spannung (V) 1000 cd/m²** | **CIE (x/y)** |
|---|---|---|---|
| **BF1** | 6.8 | 3.5 | 0.16/0.06 |
| **BF2** | 7.5 | 3.4 | 0.16/0.10 |
| **BF3** | 6.7 | 3.3 | 0.15/0.08 |
| **BF4** | 7.2 | 3.1 | 0.15/0.09 |
| **BF5** | 6.3 | 3.5 | 0.15/0.11 |
| **BF6** | 7.4 | 3.6 | 0.15/0.08 |
| **BF7** | 7.0 | 3.5 | 0.16/0.13 |
| **GF1** | 28.0 | 3.0 | 0.26/0.64 |
| **GF2** | 25.6 | 2.8 | 0.24/0.59 |

### 1b) Phosphoreszenz-OLED-Bauteile - BP, GP, RP:

Die erfindungsgemäßen Verbindungen lassen sich unter anderem als elektronenleitendes Hostmaterial eTMM in der Emissionsschicht EML einer phosphoreszierenden OLED und als Elektronentransportmaterial in der HBL und ETL einsetzen.

Hierfür werden alle Materialien in einer Vakuumkammer thermisch aufgedampft. Dabei besteht die Emissionsschicht immer aus mindestens einem bzw. mehreren Matrixmaterialien M (Hostmaterialien, Wirtsmaterialien) und einem phosphoreszierenden Dotierstoff Ir, der dem Matrixmaterial bzw. den Matrixmaterialien durch Co-Verdampfung in einem bestimmten Volumenanteil beigemischt wird. Eine Angabe wie M1:M2:Ir (55%:35%:10%) bedeutet hierbei, dass das Material M1 in einem Volumenanteil von 55%, M2 in einem Volumenanteil von 35% und Ir in einem Volumenanteil von 10% in der Schicht vorliegt. Analog kann auch die Elektronentransportschicht aus einer Mischung zweier Materialien bestehen. Der genaue Aufbau der OLEDs ist Tabelle 3 zu entnehmen. Die zur Herstellung der OLEDs verwendeten Materialien sind in Tabelle 7 gezeigt.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet.

### Die OLEDs haben folgenden Schichtaufbau:

### Substrat

Lochinjektionsschicht 1 (HIL1) aus HTM1 dotiert mit 5 % NDP-9 (kommerziell erhältlich von der Fa. Novaled), 20 nm
Lochtransportschicht 1 (HTL1) aus HTM1, 170 nm für Blaue, 40 nm für Grüne/Gelbe und 90 nm für Rot Bauteile
Lochtransportschicht 2 (HTL2), s. Tabelle 3
Emissionsschicht (EML), s. Tabelle 3
Elektronentransportschicht (ETL2), s. Tabelle 3
Elektronentransportschicht (ETL1), s. Tabelle 3
Elektroneninjektionsschicht (EIL) aus ETM2, 1 nm
Kathode aus Aluminium, 100 nm

**Tabelle 3: Aufbau Phosphoreszenz-OLED-Bauteile**

| **Bsp.** | **HTL2 Dicke** | **EML Dicke** | **ETL2 Dicke** | **ETL1 Dicke** |
|---|---|---|---|---|
| Blaue OLED's | | | | |
| BP1 | HTM3 10 nm | M3:M4:IrB1 (30%:60%:10%) | ETM3 10 nm | V1:ETM2 (70%:30%) |
| | | 30 nm | | 30 nm |
| BP2 | HTM3 10 nm | M3:M4:IrB1 (30%:60%:10%) | ETM3 10 nm | V6:ETM2 (60%:40%) |
| | | 30 nm | | 30 nm |

| Grüne und Gelbe OLED's | | | | |
|---|---|---|---|---|
| GP1 | HTM2 20 nm | V9:M1:IrG1 (50%:40%:10%) | ETM1 5 nm | ETM1:ETM2 (50%:50%) |
| | | 40 nm | | 30 nm |
| GP2 | HTM2 20 nm | V12:M2:IrG2 (50%:35%:15%) | ETM1 5 nm | ETM1:ETM2 (50%:50%) |
| | | 40 nm | | 30 nm |
| GP3 | HTM2 20 nm | V24:M1:IrG3 (45%:45%:10%) | ETM1 5 nm | ETM1:ETM2 (50%:50%) |
| | | 40 nm | | 30 nm |
| GP4 | HTM2 20 nm | V32:M2:IrG3 (45%:45%:10%) | V3 5 nm | ETM1:ETM2 (50%:50%) |
| | | 40 nm | | 30 nm |
| GP5 | HTM2 20 nm | V35:M1:IrG3 (45%:45%:10%) | V5 5 nm | V5:ETM2 (60%:40%) |
| | | 40 nm | | 30 nm |

| Rote OLED's | | | | |
|---|---|---|---|---|
| RP1 | HTM2 10 nm | V94:IrR1 (97%:3%) | ETM1 10 nm | ETM1:ETM2 (50%:50%) |
| | | 35 nm | | 30 nm |

**Tabelle 4: Ergebnisse Phosphoreszenz-OLED-Bauteile**

| **Bsp.** | **EQE (%) 1000 cd/m²** | **Spannung (V) 1000 cd/m²** | **CIE x/y 1000 cd/m²** |
|---|---|---|---|
| Blaue OLED's | | | |
| BP1 | 20.6 | 3.5 | 0.16/0.36 |
| BP2 | 22.3 | 3.4 | 0.16/0.36 |

| Grüne und Gelbe OLED's | | | |
|---|---|---|---|
| GP1 | 23.2 | 3.3 | 0.34/0.62 |
| GP2 | 21.9 | 3.2 | 0.35/0.65 |
| GP3 | 27.7 | 3.2 | 0.49/0.51 |
| GP4 | 28.3 | 3.1 | 0.49/0.51 |
| GP5 | 28.5 | 3.0 | 0.50/0.50 |

| Rote OLED's | | | |
|---|---|---|---|
| RP1 | 18.6 | 2.7 | 0.70/0.30 |

### 2) Lösungs-prozessierte Bauteile:

### 2a) Lösungsprozessierte Phosphoreszenz-OLED-Bauteile - Sol-GP und Sol-RP:

Die erfindungsgemäßen Iridium-Komplexe können auch aus Lösung verarbeitet werden und führen dort zu prozesstechnisch wesentlich einfacheren OLEDs, im Vergleich zu den vakuumprozessierten OLEDs, mit dennoch guten Eigenschaften. Die Herstellung solcher Bauteile lehnt sich an die Herstellung polymerer Leuchtdioden (PLEDs) an, die in der Literatur bereits vielfach beschrieben ist (z. B. in der WO 2004/037887). Der Aufbau setzt sich zusammen aus:
Der verwendete Aufbau ist damit wie folgt:
- Substrat,
- ITO (50 nm),
- Lochinjektionsschicht (60 nm),
- Interlayer (20 nm), aus HTL-S1
- Emissionsschicht (60 nm), s. Tabelle 5
- Lochblockierschicht (10 nm), aus ETM-1
- Elektronentransportschicht (40 nm), aus ETM-1/ETM-2 (50:50)
- Kathode (Al) (100 nm)

Dazu werden Substrate der Firma Technoprint (Sodalimeglas) verwendet, auf welche die ITO-Struktur (Indium-Zinn-Oxid, eine transparente, leitfähige Anode) aufgebracht wird. Die Substrate werden im Reinraum mit DI Wasser und einem Detergens (Deconex 15 PF) gereinigt und dann durch eine UV/Ozon-Plasmabehandlung aktiviert. Danach wird ebenfalls im Reinraum eine 20 nm Lochinjektionsschicht (PEDOT:PSS von Clevios^{™}) durch Spin-Coating aufgebracht. Die benötigte Spinrate hängt vom Verdünnungsgrad und der spezifischen Spin-Coater-Geometrie ab. Um Restwasser aus der Schicht zu entfernen, werden die Substrate für 10 Minuten bei 180 °C an Luft auf einer Heizplatte ausgeheizt. Die verwendete Interlayer dient dem Lochtransport, wobei in diesem Fall wird HTL-S1 von Merck verwendet (WO2013156130) wird. Der Feststoffgehalt beträgt ca. 5 g/l um eine Schichtdicke von 20 nm mittels Spincoating zu erzielen. Die Interlayer wird in einer Inertgasatmosphäre, im vorliegenden Fall Argon, 30 Minuten bei 220 °C auf einer Heizplatte ausgeheizt. Die Interlayer kann alternativ auch durch eine oder mehrere Schichten ersetzt werden, die lediglich die Bedingung erfüllen müssen, durch den nachgelagerten Prozessierungsschritt der EML-Abscheidung aus Lösung nicht wieder abgelöst zu werden. Zur Herstellung der Emissionsschicht werden die Triplettemitter Ir zusammen mit den Matrixmaterialien in Toluol oder Chlorbenzol gelöst. Der typische Feststoffgehalt solcher Lösungen liegt zwischen 16 und 25 g/L, wenn, wie hier, die für eine Device typische Schichtdicke von 60 nm mittels Spincoating erzielt werden soll. Die lösungsprozessierten Devices enthalten eine Emissionsschicht aus Ma:Mb:Ir (w%:x%:z%), s. Tabelle 5. Die Emissionsschicht wird in einer Inertgasatmosphäre, im vorliegenden Fall Argon, aufgeschleudert und 10 min bei 160 °C ausgeheizt. Darüber wird die Lochblockierschicht und die Elektronentransportschicht aufgedampft (Aufdampfanlagen von Lesker o.a., typischer Aufdampfdruck 5 x 10⁻⁶ mbar). Zuletzt wird eine Kathode aus Aluminium (hochreines Metall von Aldrich) aufgedampft. Um das Device vor Luft und Luftfeuchtigkeit zu schützen, wird die Vorrichtung abschließend verkapselt und dann charakterisiert. Die genannten OLED-Beispiele sind noch nicht optimiert. Tabelle 5 fasst die erhaltenen Daten zusammen.

**Tabelle 5: Ergebnisse mit aus Lösung prozessierten Materialien**

| **Bsp.** | **Emissionsschicht** | **EQE** (%) 1000 cd/m² | **Spannung** (V) 1000 cd/m² | **CIE x/y** |
|---|---|---|---|---|
| | | | | 1000 cd/m² |
| Sol-GP1 | V22:M5:IrG-Sol1 (20%:58%:22%) | 22.0 | 4.4 | 0.34/0.62 |
| Sol-GP2 | V200:M5:IrG-Sol1 (20%:58%:22%) | 22.7 | 4.3 | 0.34/0.63 |
| Sol-GP3 | V213:M5:IrG-Sol1 (30%:50%:20%) | 22.5 | 4.3 | 0.34/0.62 |
| Sol-RP1 | V307:M5:IrR1 (25%:57%:18%) | 17.7 | 3.8 | 0.70/0.30 |

### 2b) Lösungsprozessierte Fluoreszenz-OLED-Bauteile - Sol-BF:

Die erfindungsgemäßen Fluoreszenzemitter L können ebenfalls aus Lösung verarbeitet werden und führen zu guten Eigenschaften. Die Herstellung solcher Bauteile erfolgt analog zu den lösungsprozessierten Phosphoreszenz-OLED-Bauteile - GP, RP, wobei beim Aufbau die im folgenden beschriebene Anpassung gemacht werden:
- Substrat,
- ITO (50 nm),
- Lochinjektionsschicht (20 nm),
- Interlayer (20 nm), aus HTL-S1
- Emissionsschicht (40 nm), aus Fluoreszenzemitter L und Matrix MS
- Lochblockierschicht (10 nm), aus ETM-1
- Elektronentransportschicht (40 nm), aus ETM-1/ETM-2 (50:50)
- Kathode (Al) (100 nm).

Zur Herstellung der Emissionsschicht werden die erfindungsgemäßen Fluoreszenzemitter L (5 Gew.-%) zusammen mit dem Matrixmaterial MS (95 Gew.-%) in Toluol oder Chlorbenzol gelöst. Der typische Feststoffgehalt solcher Lösungen liegt bei 14 g/L, wenn, wie hier, die für eine Device typische Schichtdicke von 40 nm mittels Spincoating erzielt werden soll. Die Emissionsschicht wird in einer Inertgasatmosphäre, im vorliegenden Fall Argon, aufgeschleudert und 10 min bei 160 °C ausgeheizt. Tabelle 6 fasst die erhaltenen Daten zusammen.

**Tabelle 6: Ergebnisse mit aus Lösung prozessierten Materialien**

| **Bsp.** | **Emissionsschicht** | | **EQE** (%) 1000 cd/m² | **Spannung** (V) 1000 cd/m² | **CIE x/y** |
|---|---|---|---|---|---|
| | Host | Emitter | | | 1000 cd/m² |
| Sol-BF1 | MS | L1 | 3.9 | 4.3 | 0.14/0.14 |
| Sol-BF2 | MS | L2 | 4.3 | 4.3 | 0.14/0.14 |
| Sol-BF3 | MS | L3 | 4.5 | 4.4 | 0.14/0.13 |
| Sol-BF4 | MS | L4 | 5.9 | 4.4 | 0.14/0.13 |

**Tabelle 7: Strukturformeln der verwendeten Materialien**

| | |
|---|---|
| 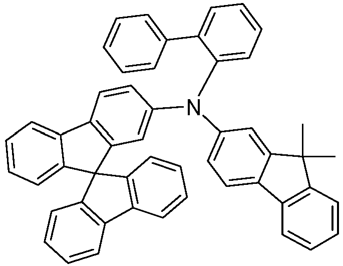 HTM1 136463-07-5 | 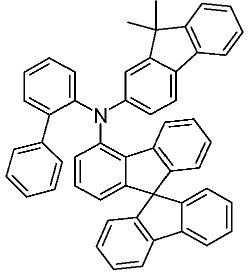 HTM2 1450933-44-4 |
| 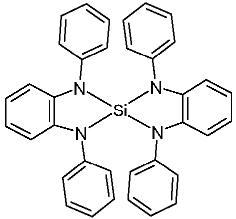 HTM3 1206465-62-4 | 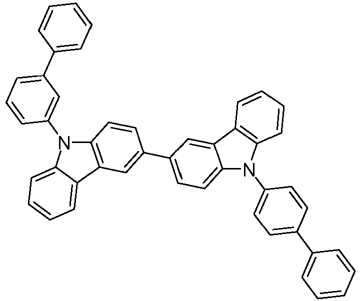 |
| | M1 1643479-47-3 |
| 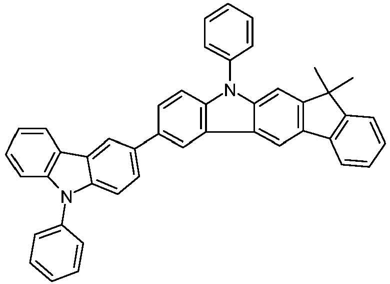 M2 1357150-54-9 | 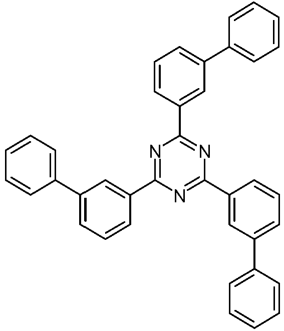 M3 = ETM3 1201800-83-0 |
| 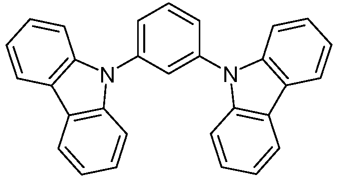 M4 550378-78-4 | 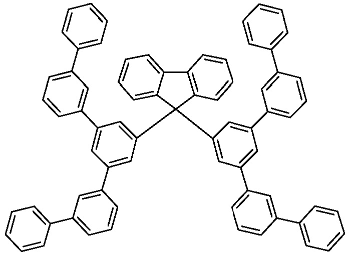 M5 1246496-85-4 |
| 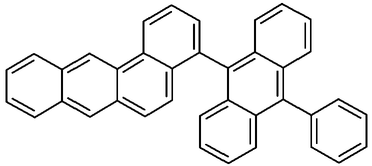 SMB1 [1087346-88-0] | 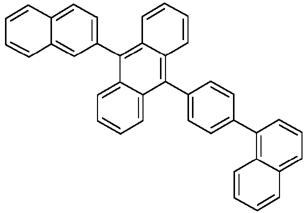 SMB2 [667940-34-3] |
| 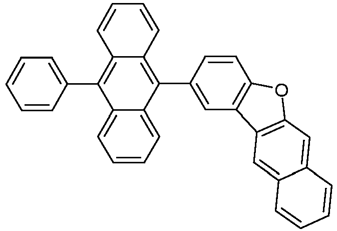 SMB3 [1627916-48-6] | 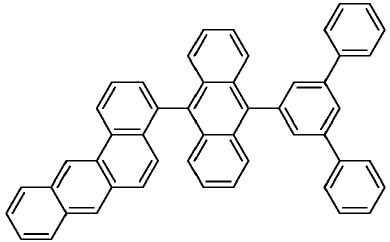 MS [1273319-88-2] |
| 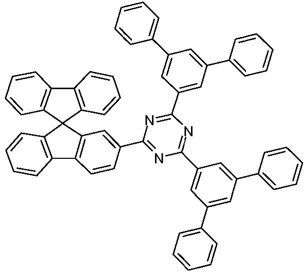 ETM1 1233200-52-6 | 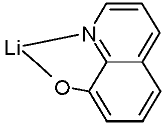 ETM2 25387-93-3 |
| 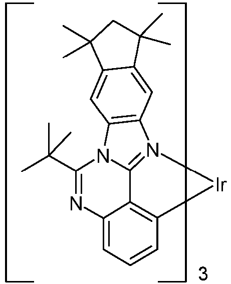 IrB1 1541114-98-0 | 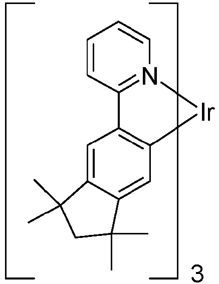 IrG1 1562420-772 |
| 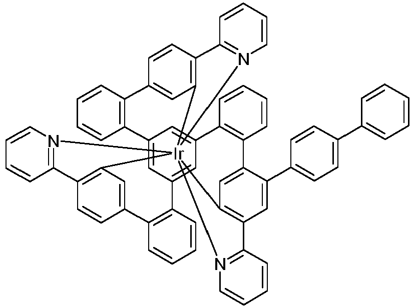 IrG2 2245866-06-0 | 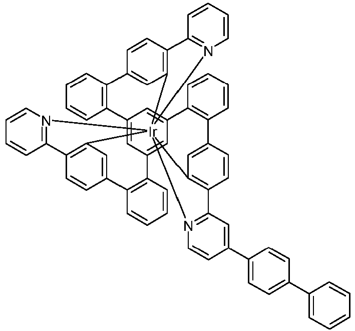 IrG3 2245945-28-0 |
| 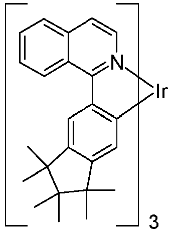 IrR1 1562420-79-4 | 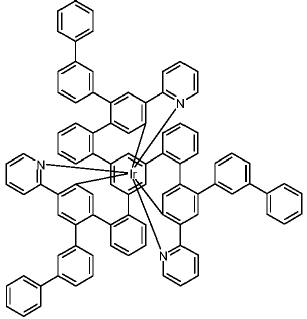 IrG-Sol1 1989601-89-9 |
| 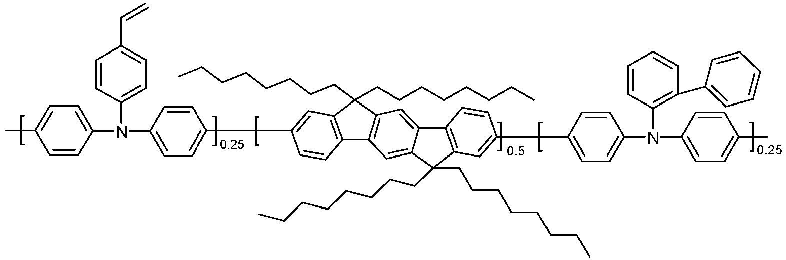 HTL-S1 WO2013156130 | |

Die in den Tabellen 1, 3, 5 und 6 in Bezug auf die erfindungsgemäßen Materialien dargelegten Abkürzungen, wie beispielsweise L1, L2, L3, L4, V1, V4, V5,V6, V9, V12, V22, V24, V32, V35, V44, V45, V54, V57, V64, V65, V67, V83, V94, V200, V213 und V307 etc. beziehen sich auf die in den Synthesebeispielen zuvor näher ausgeführten Verbindungen.

## Patentansprüche

1. Verbindung umfassend mindestens eine Struktur der Formel (I), 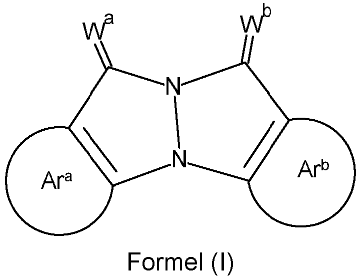
wobei der Ring Ar^{a} bei jedem Auftreten gleich oder verschieden für ein aromatisches oder heteroaromatisches Ringsystem mit 5 bis 60 aromatischen Ringatomen steht, das mit einem oder mehreren Resten Ar oder R^{a} substituiert sein kann, der Ring Ar^{b} bei jedem Auftreten gleich oder verschieden für ein aromatisches oder heteroaromatisches Ringsystem mit 5 bis 60 aromatischen Ringatomen steht, das mit einem oder mehreren Resten Ar oder R^{b} substituiert sein kann;
und für die weiteren verwendeten Symbole und Indizes gilt:
W^{a}. W^{b} ist bei jedem Auftreten gleich oder verschieden O oder S;
Ar ist bei jedem Auftreten gleich oder verschieden ein aromatisches oder heteroaromatisches Ringsystem mit 5 bis 60 aromatischen Ringatomen, das mit einem oder mehreren Resten R substituiert sein kann, hierbei kann die Gruppe Ar mit mindestens einer Gruppe Ar, R, R^{a}, R^{b} oder einer weiteren Gruppe ein Ringsystem bilden;
R, R^{a}, R^{b} ist bei jedem Auftreten gleich oder verschieden H, D, OH, F, Cl, Br, I, CN, NO₂, N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R', OSO₂Ar¹, OSO₂R¹, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann; oder Heteroarylthiogruppe mit 5 bis 60 aromatischen Ringatomen, die durch einen oder mehrere Reste R¹ substituiert sein kann, oder eine Diarylamino-, Arylheteroarylamino-, Diheteroarylaminogruppe mit 5 bis 60 aromatischen Ringatomen, die durch einen oder mehrere Reste R¹ substituiert sein kann, oder eine Arylalkyl- oder Heteroarylalkylgruppe mit 5 bis 60 aromatischen Ringatomen und 1 bis 10 C-Atomen im Alkylrest, die durch einen oder mehrere Reste R¹ substituiert sein kann; dabei können zwei Reste R, R^{a}, R^{b} auch miteinander oder einer weiteren Gruppe ein Ringsystem bilden;
Ar' ist bei jedem Auftreten gleich oder verschieden ein aromatisches oder heteroaromatisches Ringsystem mit 5 bis 60 aromatischen Ringatomen, das mit einem oder mehreren Resten R¹ substituiert sein kann, dabei können zwei Reste Ar', welche an dasselbe C-Atom, Si-Atom, N-Atom, P-Atom oder B-Atom binden, auch durch eine Einfachbindung oder eine Brücke, ausgewählt aus B(R¹), C(R¹)₂, Si(R¹)₂, C=O, C=NR¹, C=C(R¹)₂, O, S, S=O, SO₂, N(R¹), P(R¹) und P(=O)R¹, miteinander verbrückt sein;
R¹ ist bei jedem Auftreten gleich oder verschieden H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch - R²C=CR²-, -C=C-, Si(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei oder mehrere Reste R¹ miteinander ein Ringsystem bilden, dabei können einer oder mehrere Reste R¹ mit einem weiteren Teil der Verbindung ein Ringsystem bilden;
Ar" ist bei jedem Auftreten gleich oder verschieden ein aromatisches oder heteroaromatisches Ringsystem mit 5 bis 30 aromatischen Ringatomen, das mit einem oder mehreren Resten R² substituiert sein kann, dabei können zwei Reste Ar", welche an dasselbe C-Atom, Si-Atom, N-Atom, P-Atom oder B-Atom binden, auch durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verbrückt sein;
R² ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann, dabei können zwei oder mehrere Substituenten R² miteinander ein Ringsystem bilden;;
wobei Verbindungen der Formeln (A), (B) und (C) 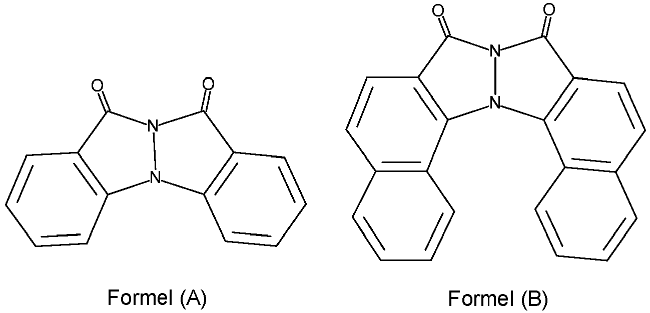 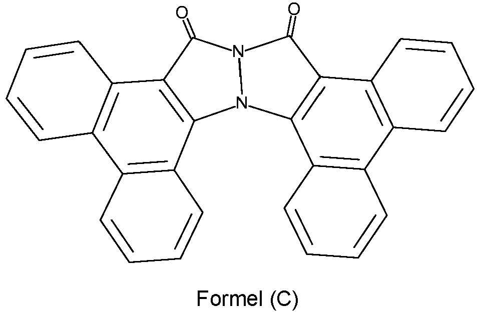
vom Schutz ausgenommen sind.

2. Verbindung nach Anspruch 1, umfassend mindestens eine Struktur der Formel (II-1) bis (II-42), 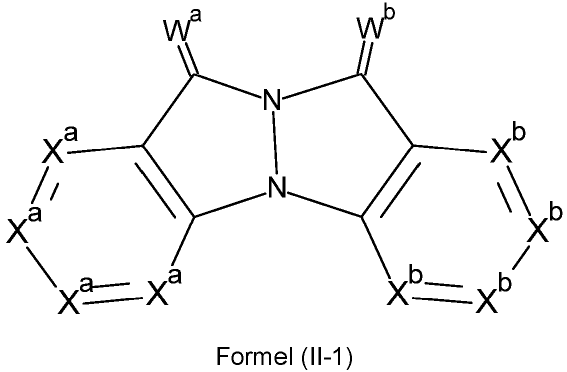 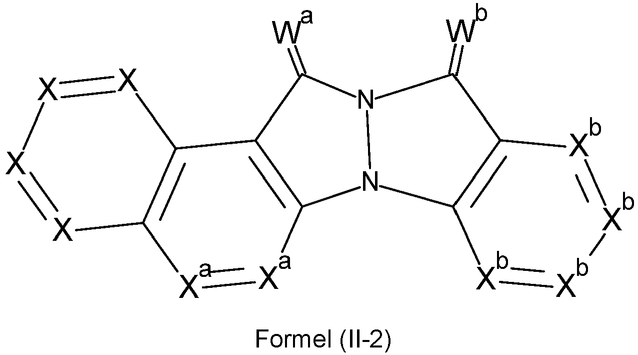 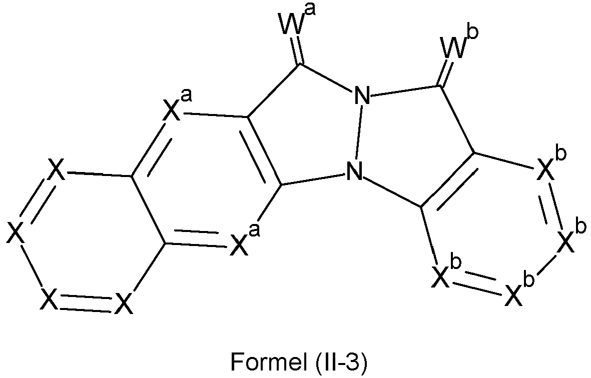 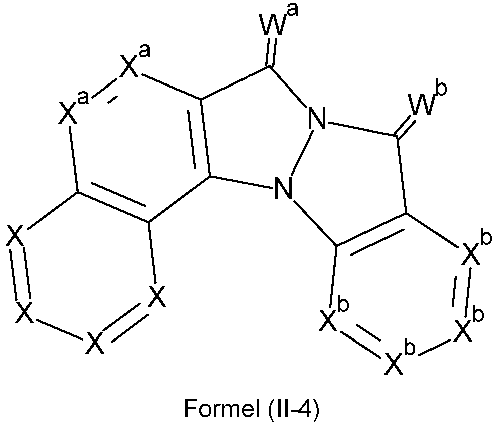 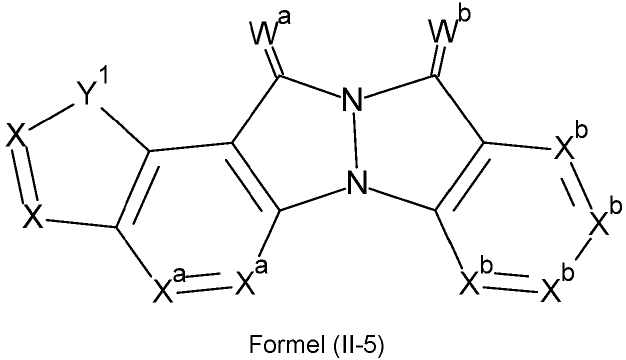 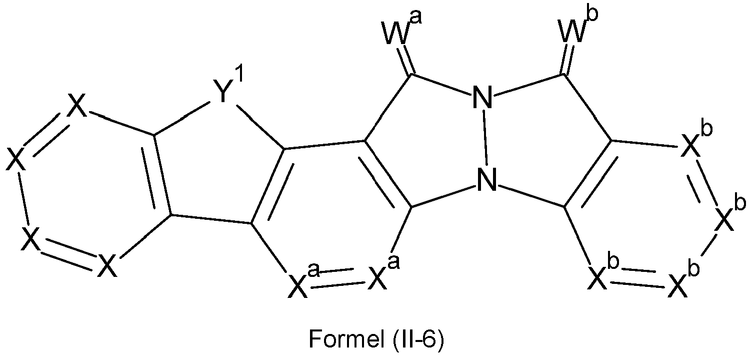 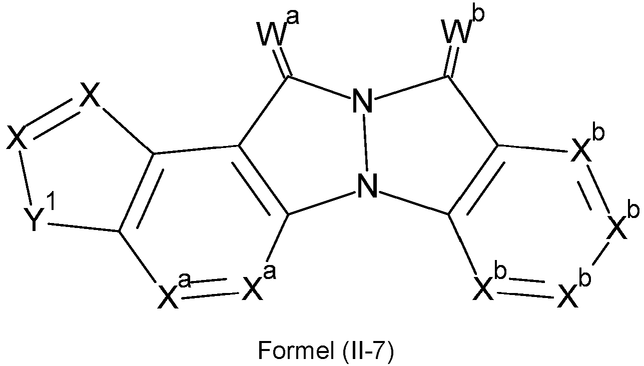 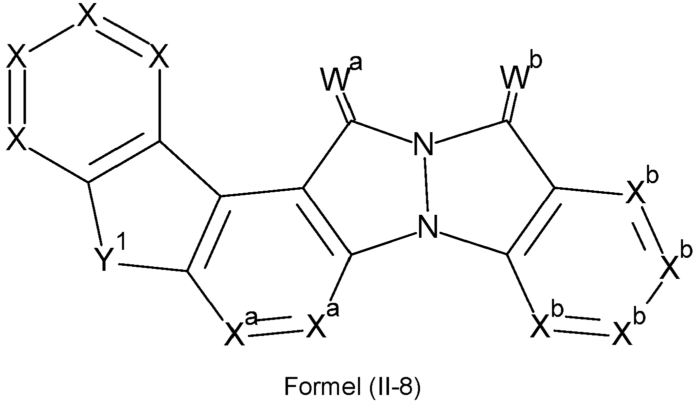 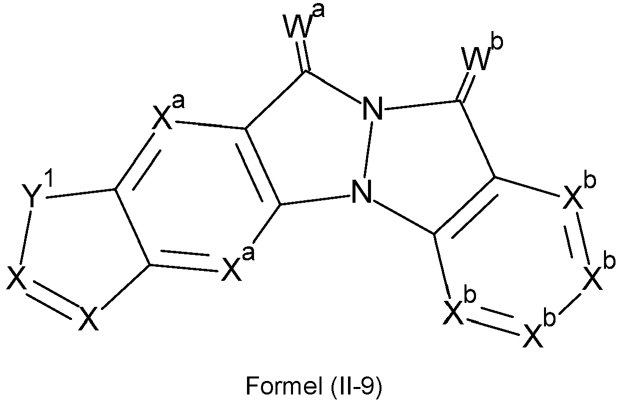 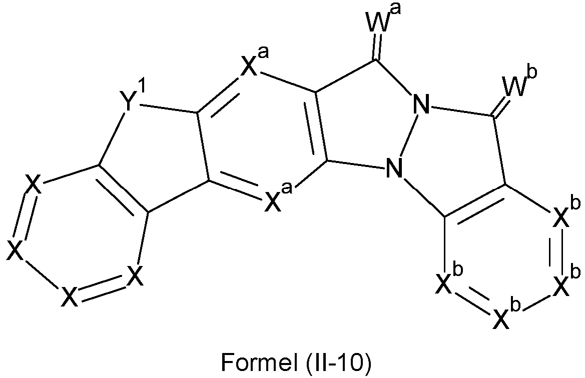 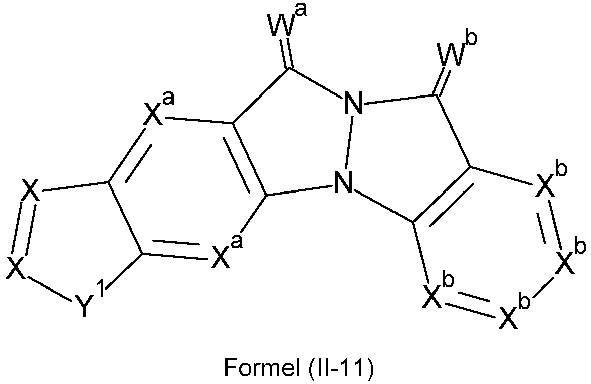 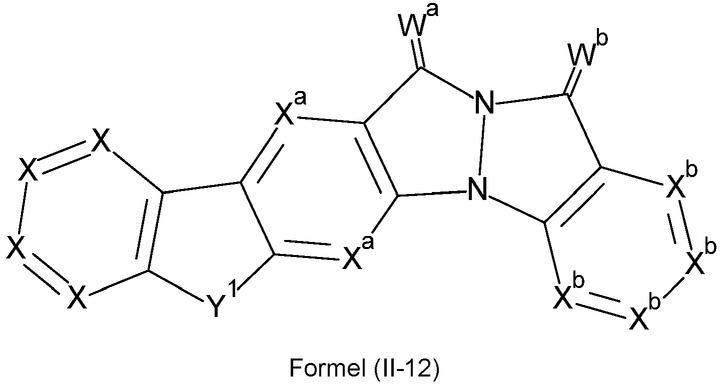 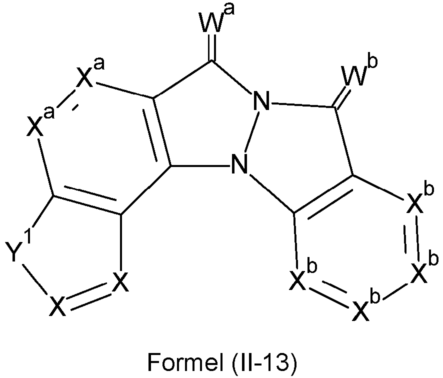 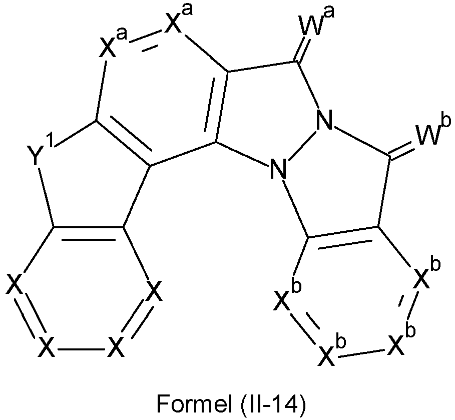 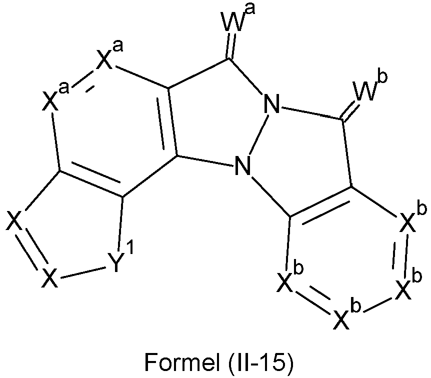 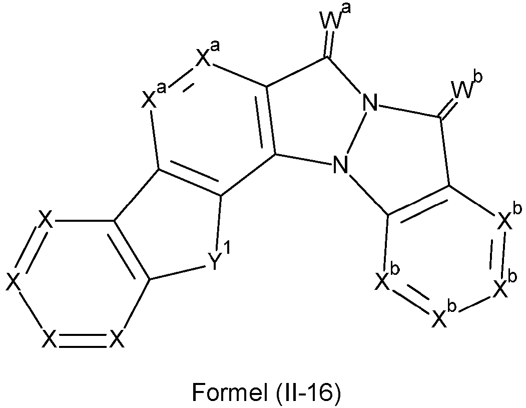 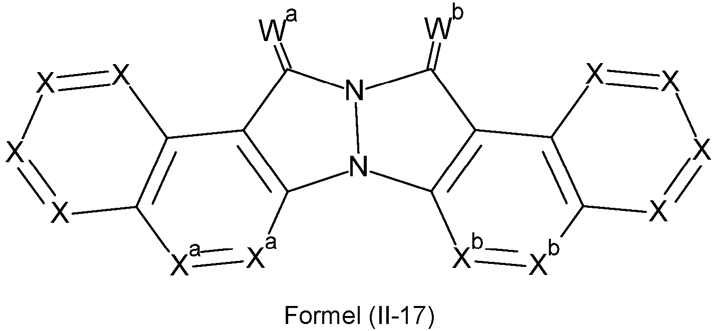 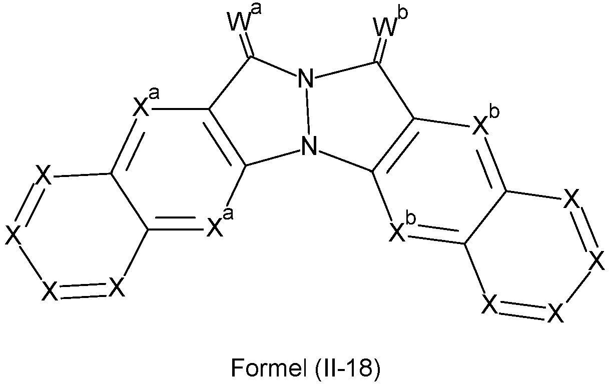 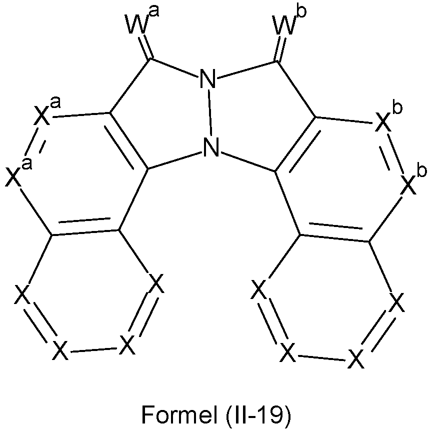 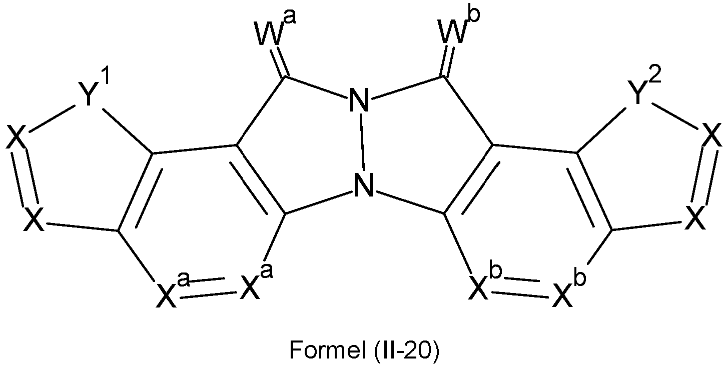 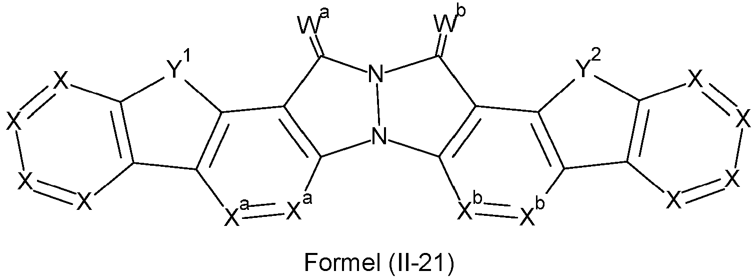 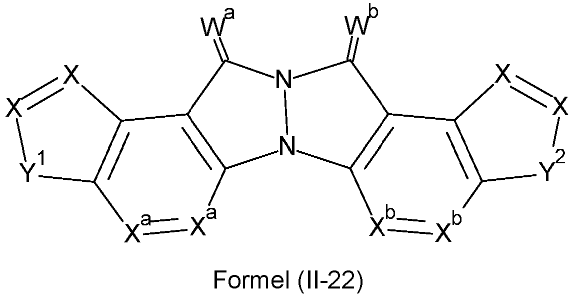 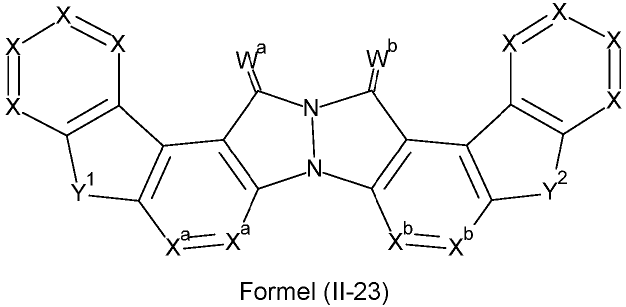 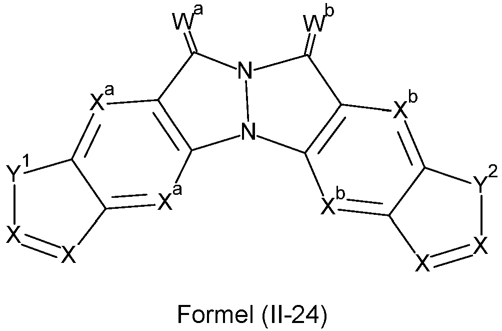 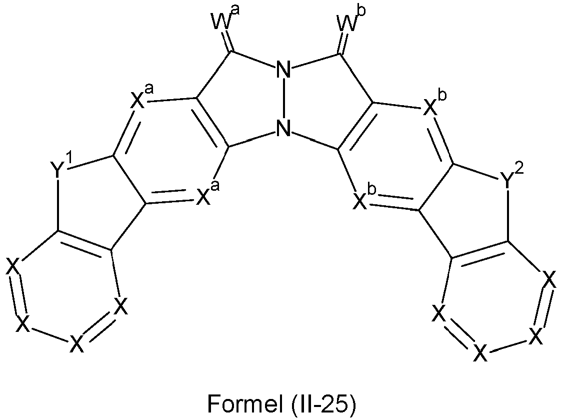 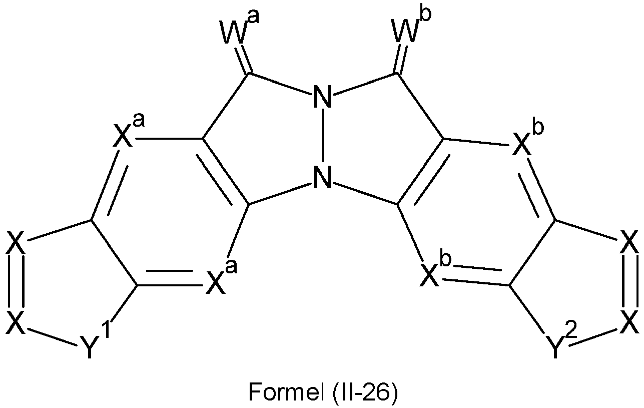 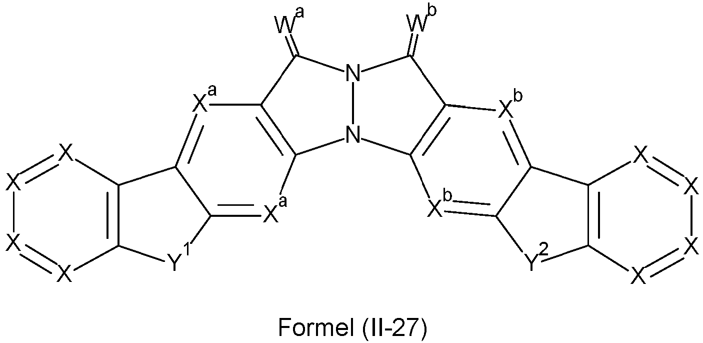 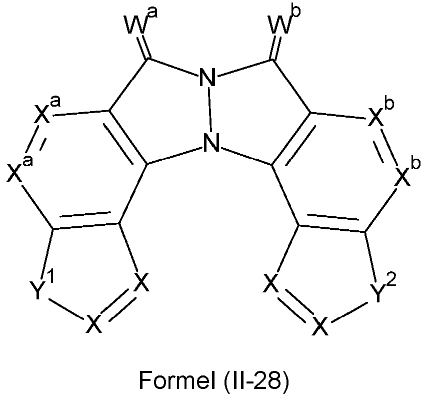 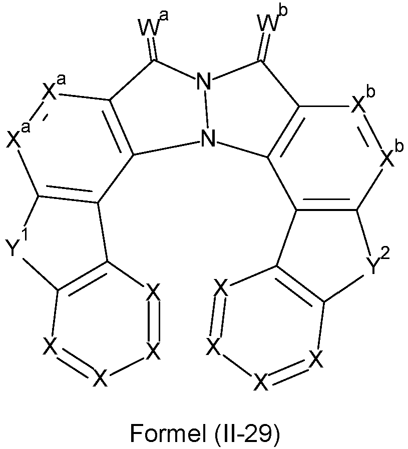 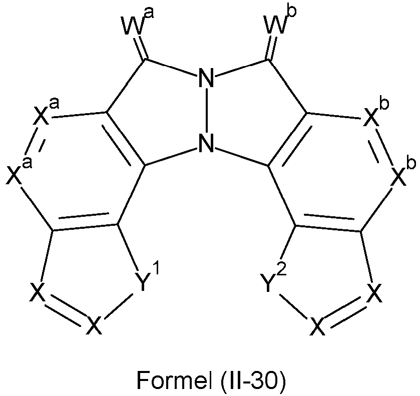 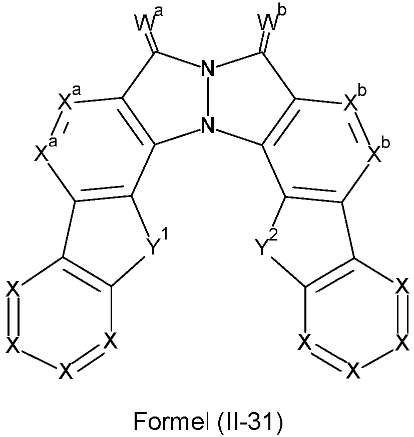 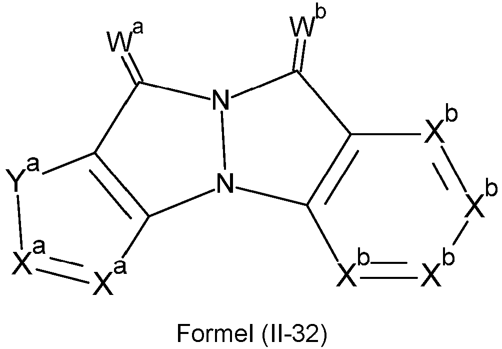 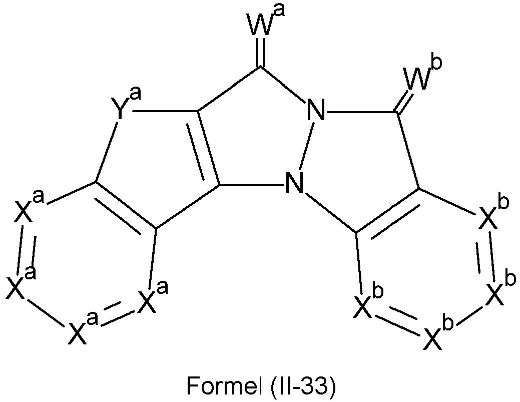 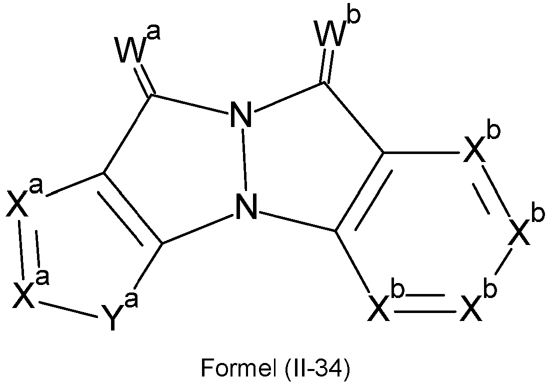 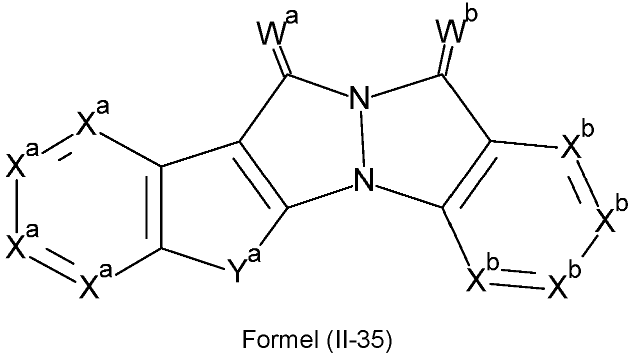 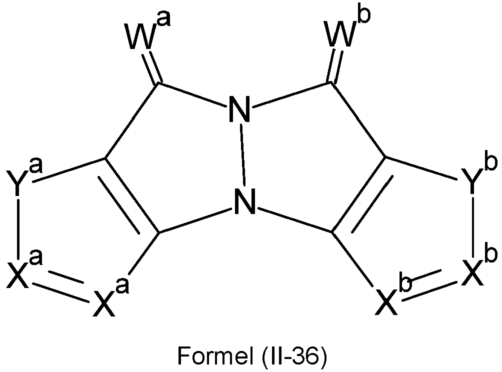 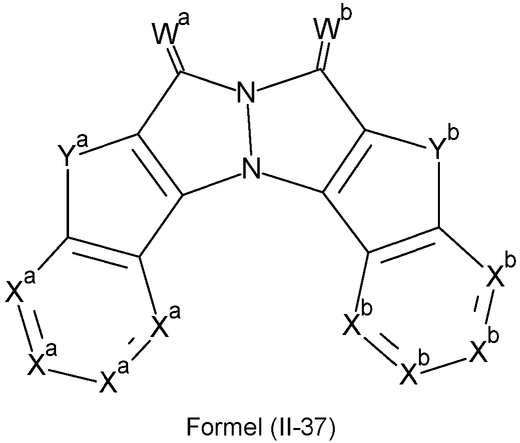 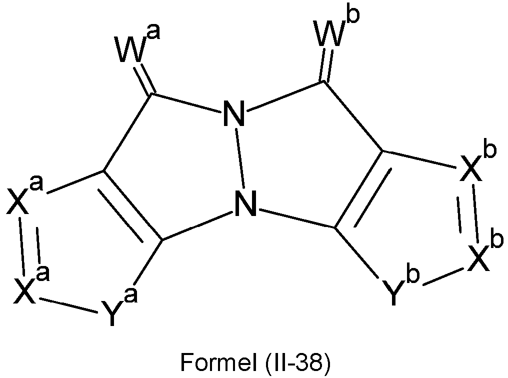 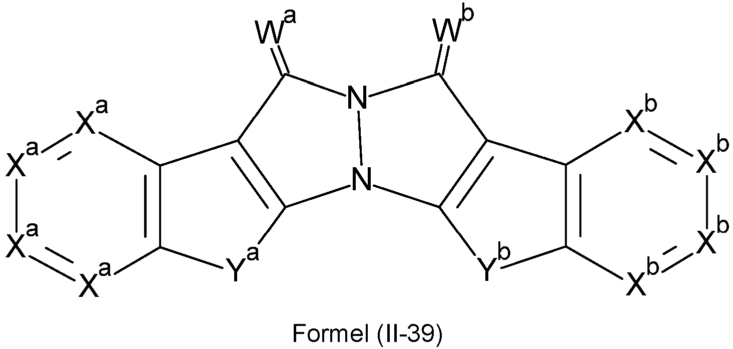 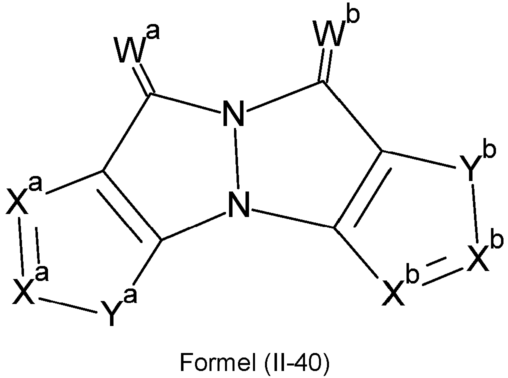 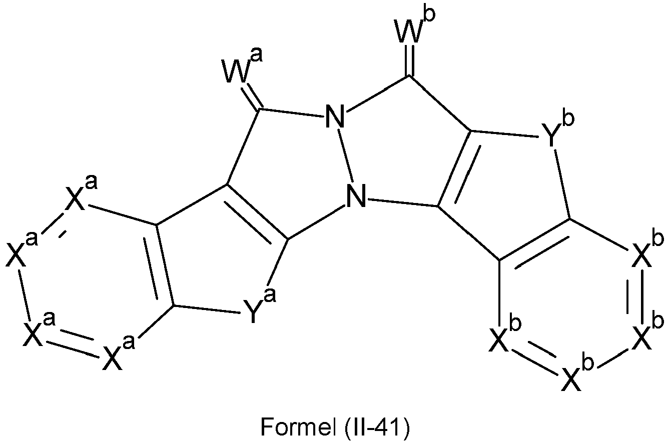 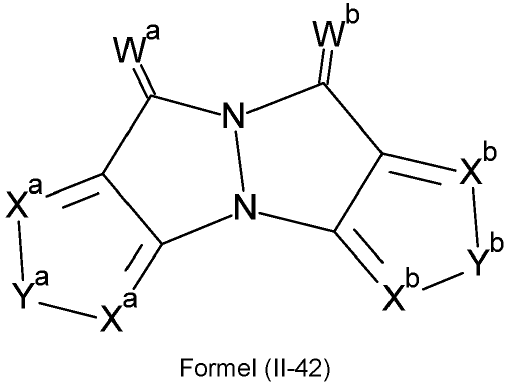 wobei die Symbole W^{a} und W^{b} die in Anspruch 1 genannten Bedeutungen aufweisen und für die weiteren Symbole gilt:
Y^{a} ist bei jedem Auftreten gleich oder verschieden N(Ar), N(R^{a}), P(Ar), P(R^{a}), P(=O)Ar, P(=O)R^{a}, P(=S)Ar, P(=S)R^{a}, B(Ar), B(R^{a}), Al(Ar), Al(R^{a}), Ga(Ar), Ga(R^{a}), C=O, C(R^{a})₂, Si(R^{a})₂, Ge(R^{a})₂, C=NR^{a}, C=NAr, C=C(R^{a})₂, C=C(R^{a})(Ar), O, S, Se, S=O, oder SO₂, vorzugsweise N(Ar), N(R^{a}), B(Ar), B(R^{a}), P(=O)R^{a}, P(=O)Ar, C=O, C(R^{a})₂, C=C(R^{a})₂, C=C(R^{a})(Ar), Si(R^{a})₂, O, S, Se, S=O oder SO₂, besonders bevorzugt N(Ar), C(R^{a})₂, O oder S, wobei R^{a} die in Anspruch 1 dargelegte Bedeutung aufweist;
Y^{b} ist bei jedem Auftreten gleich oder verschieden N(Ar), N(R^{b}), P(Ar), P(R^{b}), P(=O)Ar, P(=O)R^{b}, P(=S)Ar, P(=S)R^{b}, B(Ar), B(R^{b}), Al(Ar), Al(R^{a}), Ga(Ar), Ga(R^{b}), C=O, C(R^{b})₂, Si(R^{b})₂, Ge(R^{b})₂, C=NR^{b}, C=NAr, C=C(R^{b})₂, C=C(R^{b})(Ar), O, S, Se, S=O, oder SO₂, vorzugsweise N(Ar), N(R^{b}), B(Ar), B(R^{b}), P(=O)R^{b}, P(=O)Ar, C=O, C(R^{b})₂, C=C(R^{b})₂, C=C(R^{b})(Ar), Si(R^{b})₂, O, S, Se, S=O oder SO₂, besonders bevorzugt N(Ar), C(R^{b})₂, O oder S, wobei R^{b} die in Anspruch 1 dargelegte Bedeutung aufweist;
Y¹, Y² ist bei jedem Auftreten gleich oder verschieden N(Ar), N(R), P(Ar), P(R), P(=O)Ar, P(=O)R, P(=S)Ar, P(=S)R, B(Ar), B(R), Al(Ar), Al(R), Ga(Ar), Ga(R), C=O, C(R)₂, Si(R)₂, Ge(R)₂, C=NR, C=NAr, C=C(R)₂, C=C(R)(Ar), O, S, Se, S=O, oder SO₂, vorzugsweise N(Ar), N(R), B(Ar), B(R), P(=O)R, P(=O)Ar, C=O, C(R)₂, C=C(R)₂, C=C(R)(Ar), Si(R)₂, O, S, Se, S=O oder SO₂, besonders bevorzugt N(Ar), C(R)₂, O oder S, wobei R die in Anspruch 1 dargelegte Bedeutung aufweist;
X steht bei jedem Auftreten gleich oder verschieden für N oder CR, mit der Maßgabe, dass nicht mehr als zwei der Gruppen X in einem Cyclus für N stehen, wobei R die in Anspruch 1 dargelegte Bedeutung aufweist;
X^{a} steht bei jedem Auftreten gleich oder verschieden für N oder CR^{a}, mit der Maßgabe, dass nicht mehr als zwei der Gruppen X^{a} in einem Cyclus für N stehen, wobei R^{a} die in Anspruch 1 dargelegte Bedeutung aufweist;
X^{b} steht bei jedem Auftreten gleich oder verschieden für N oder CR^{b}, mit der Maßgabe, dass nicht mehr als zwei der Gruppen X^{b} in einem Cyclus für N stehen, wobei R^{b} die in Anspruch 1 dargelegte Bedeutung aufweist.

3. Verbindung Anspruch 1 oder 2, umfassend mindestens eine Struktur der Formeln (III-1) bis (III-41), 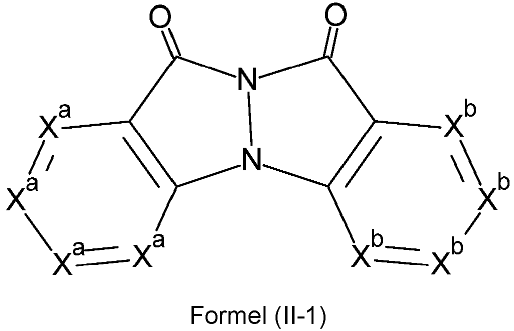 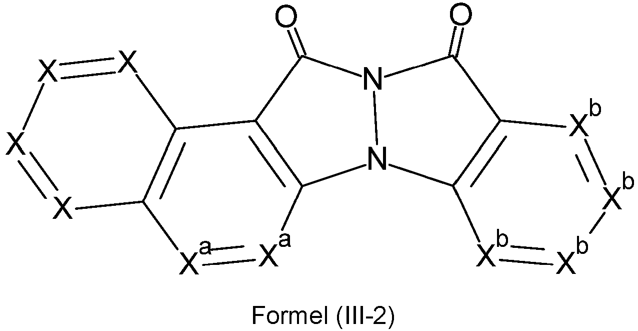 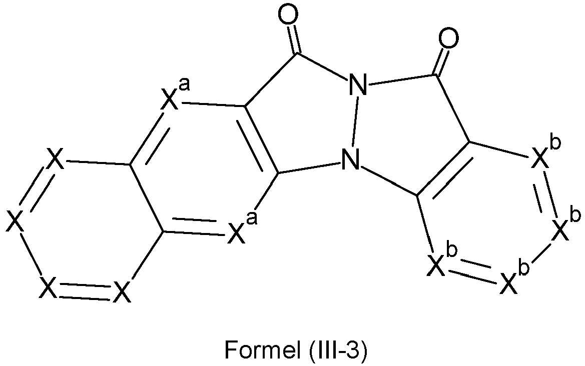 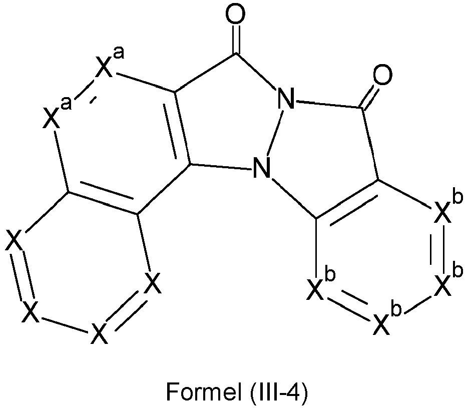 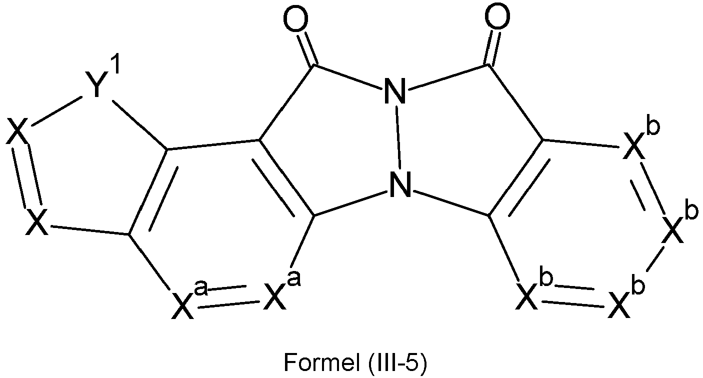 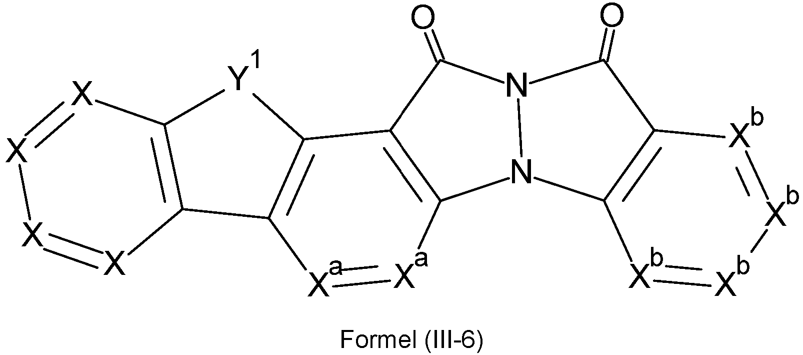 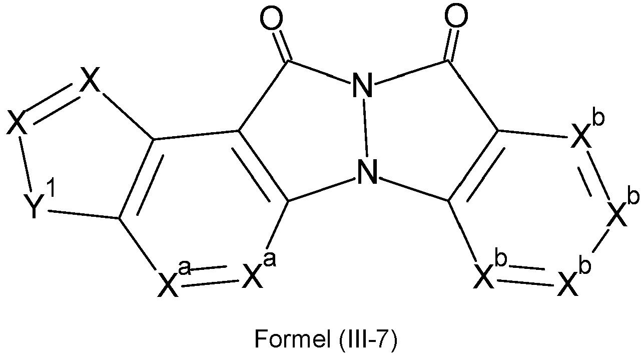 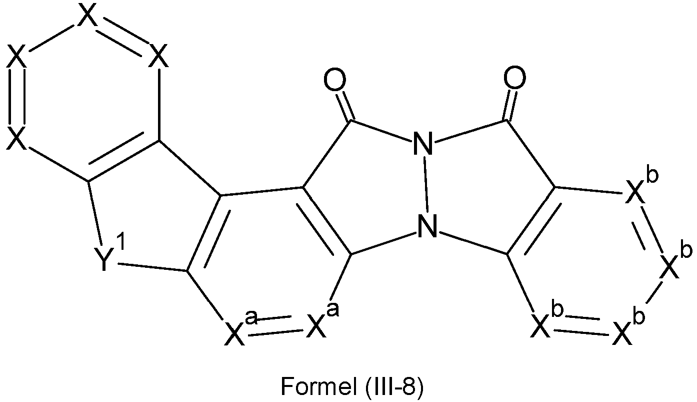 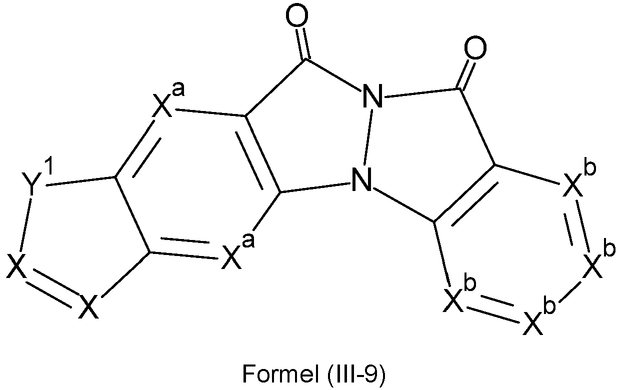 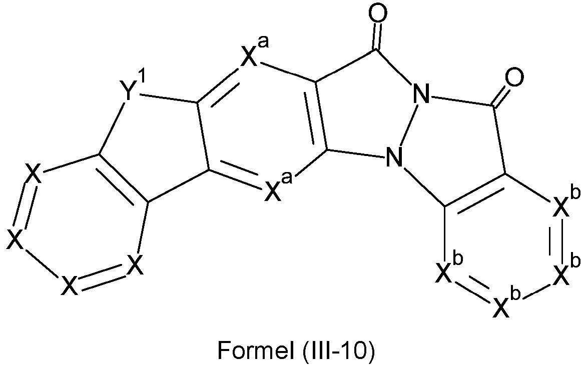 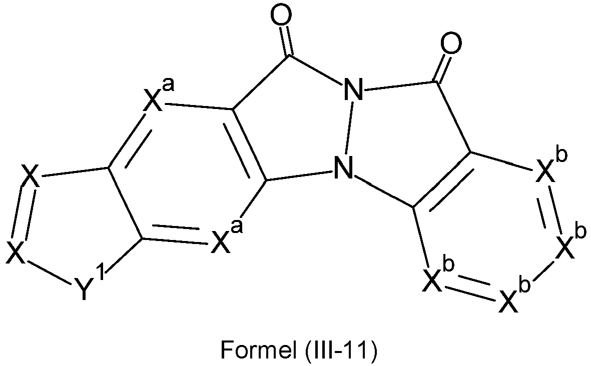 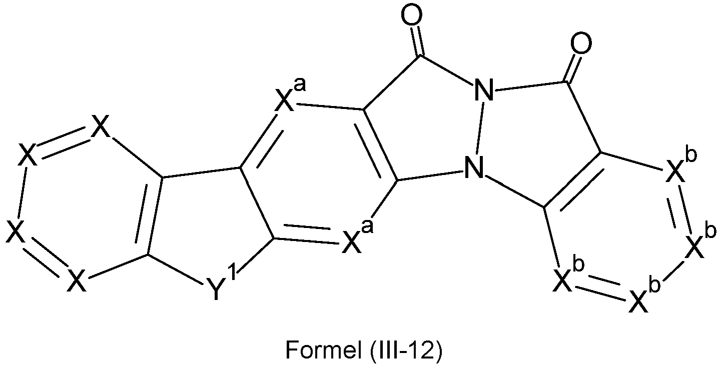 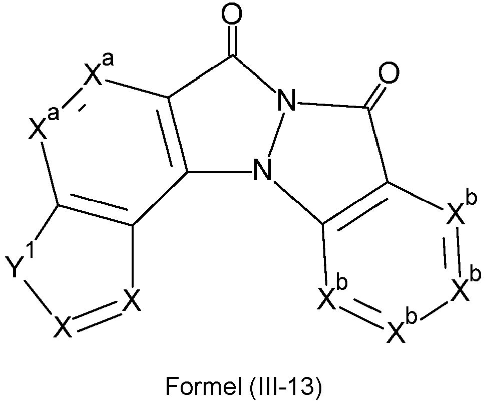 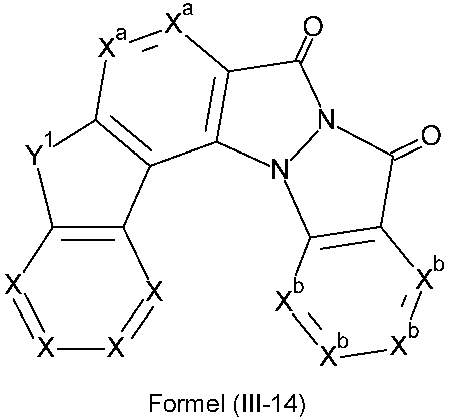 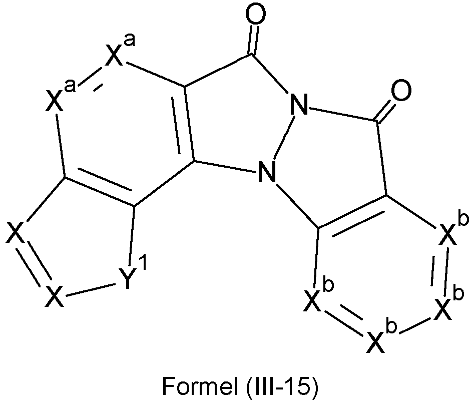 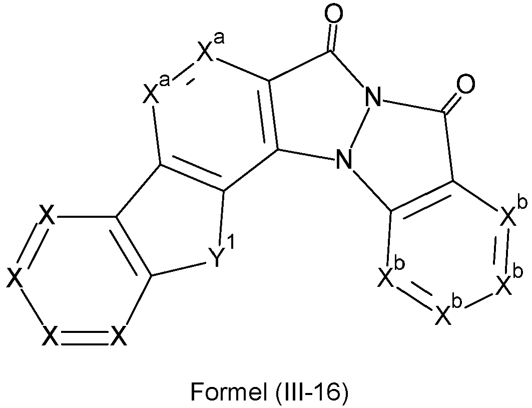 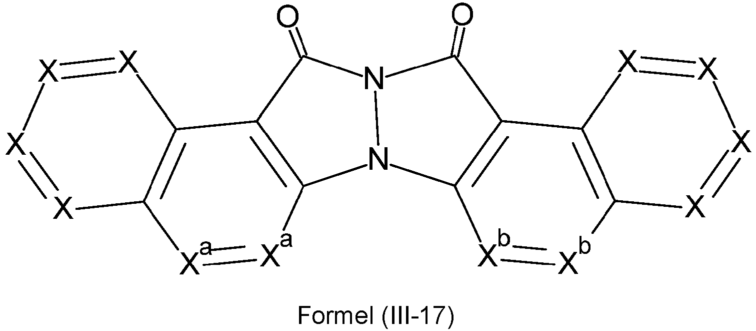 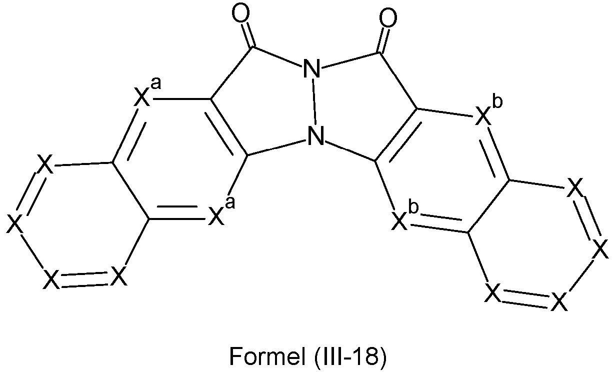 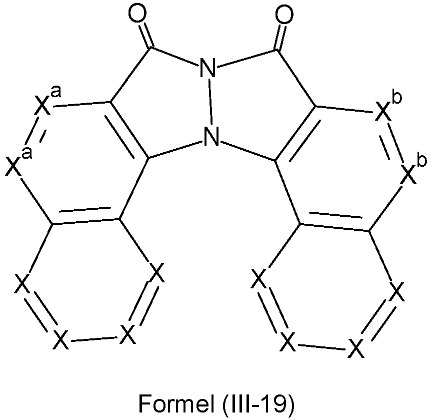 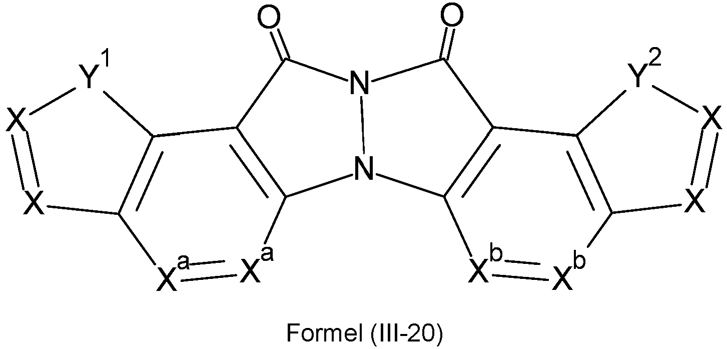 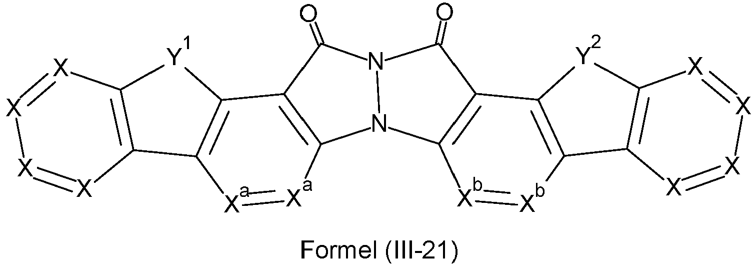 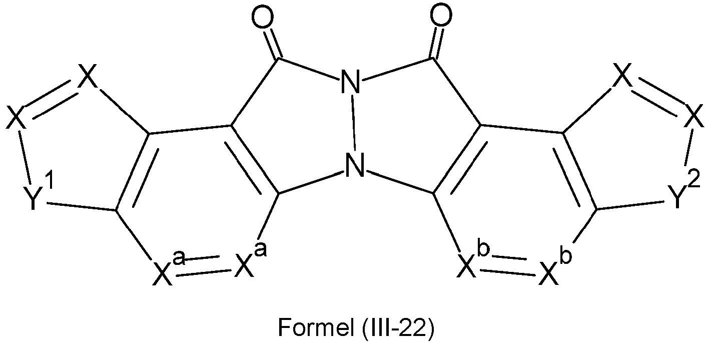 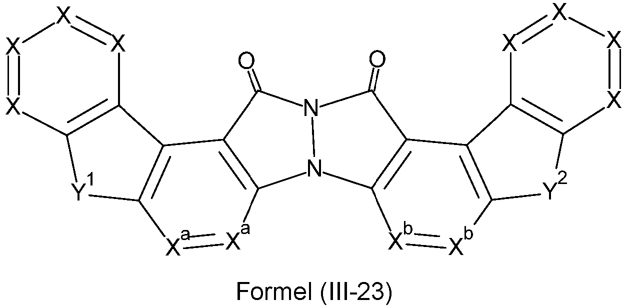 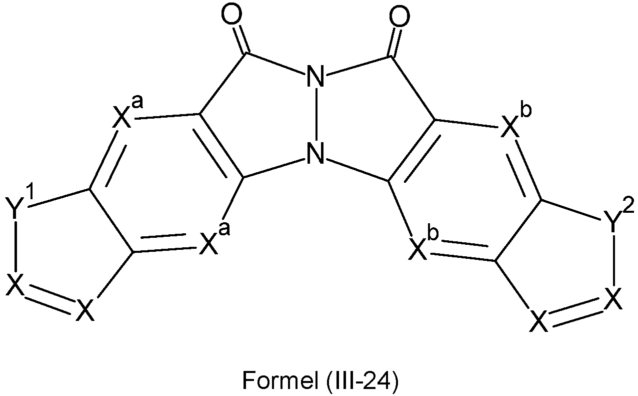 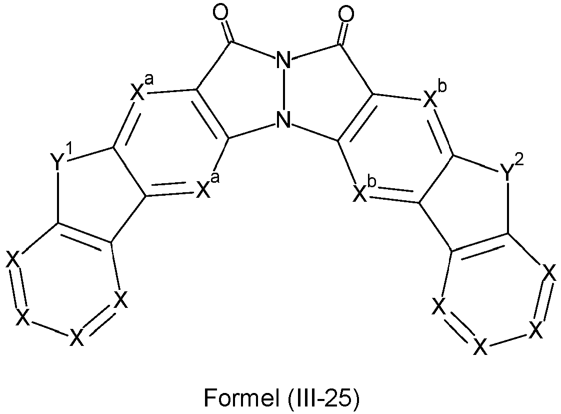 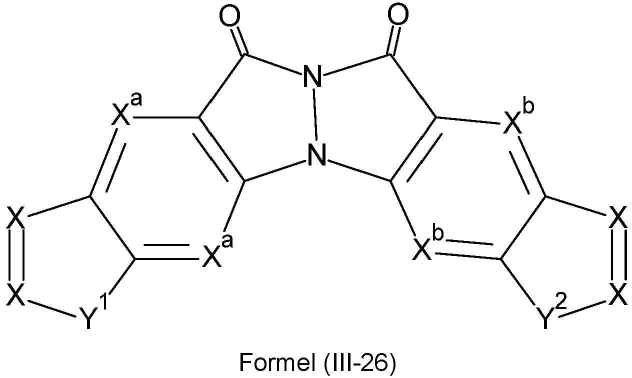 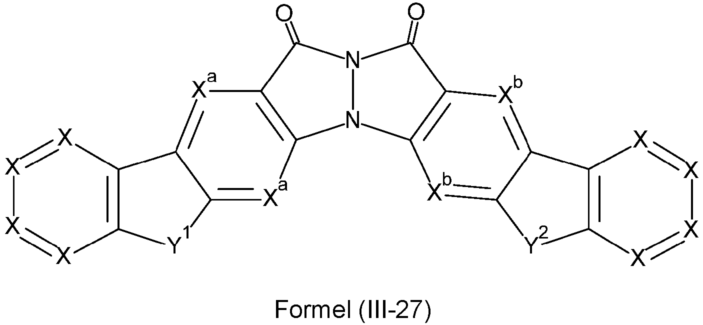 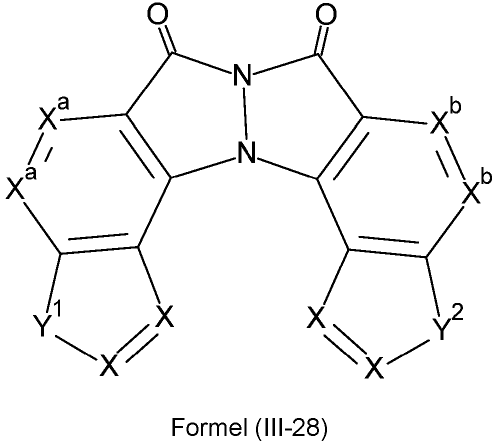 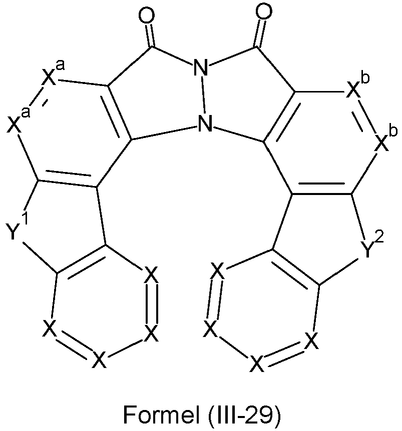 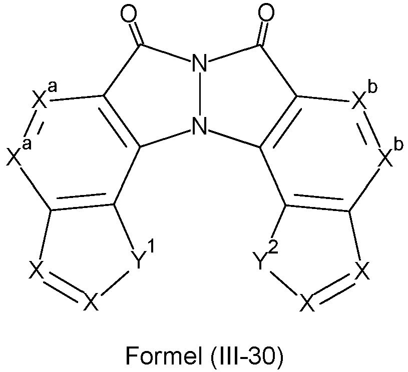 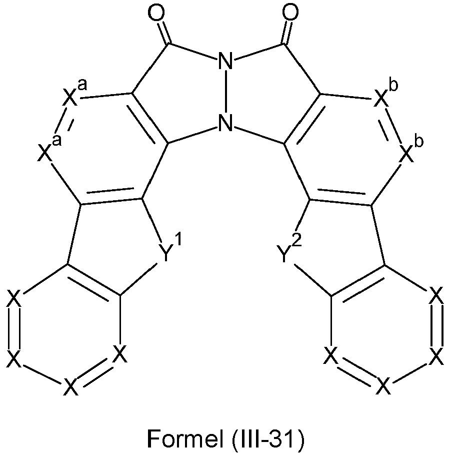 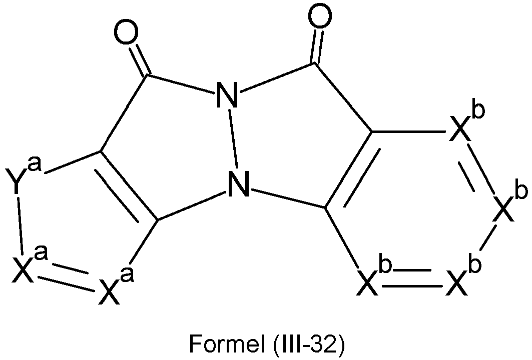 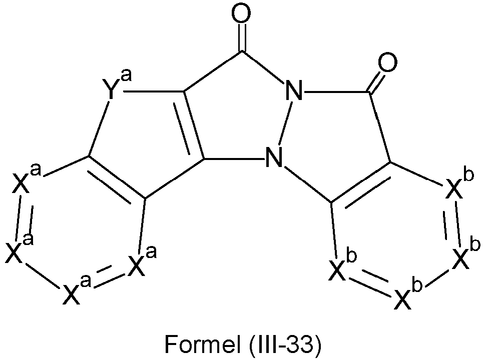 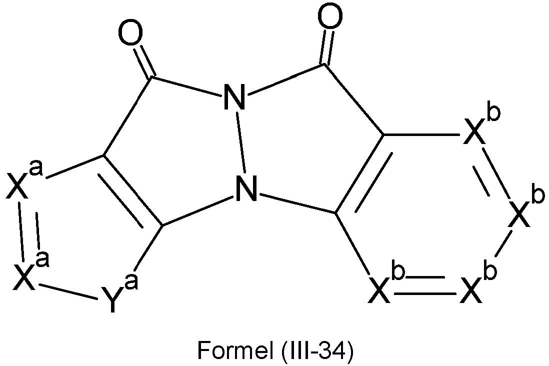 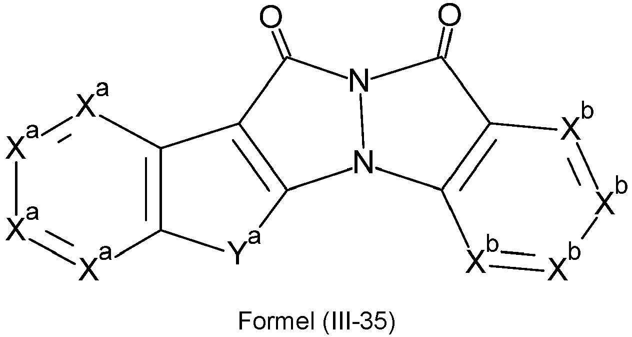 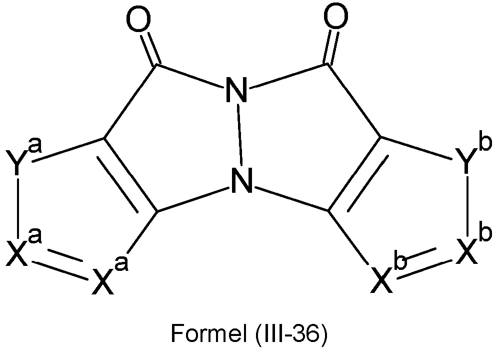 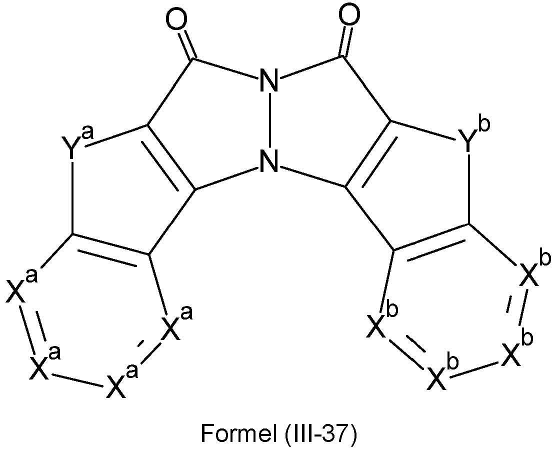 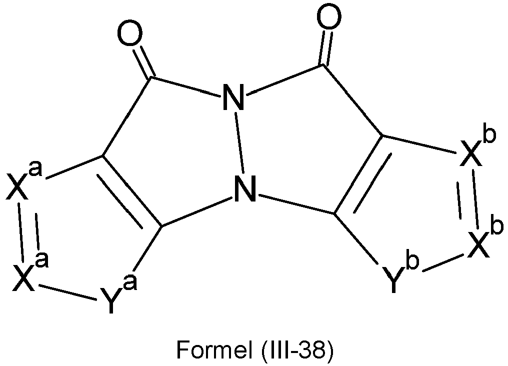 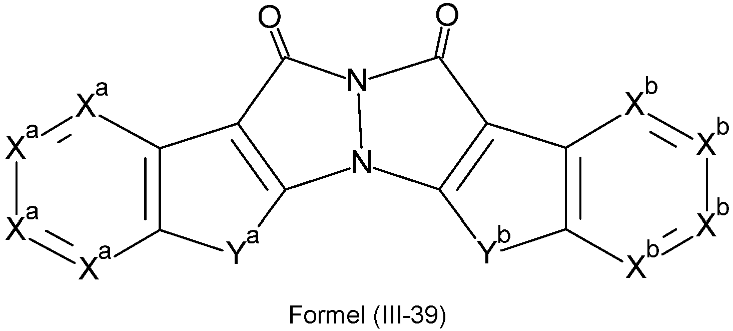 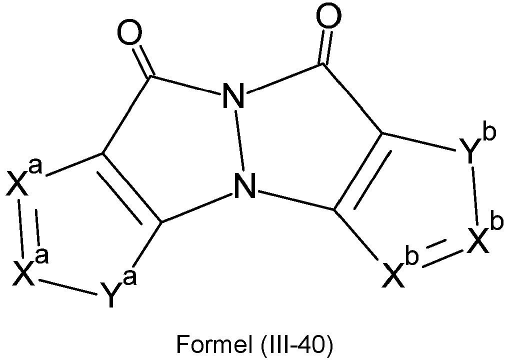 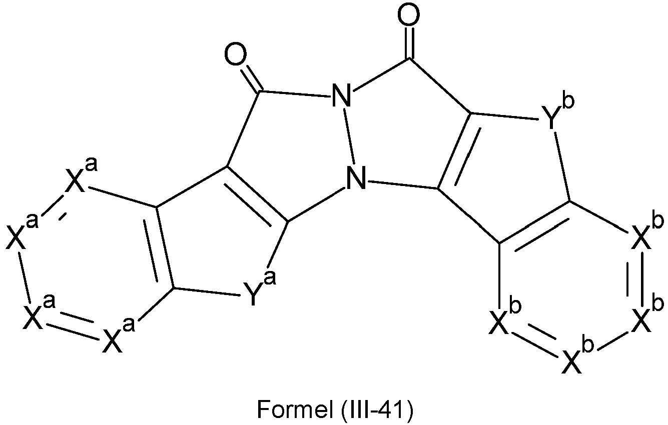 wobei die Symbole Y¹, Y², Y^{a}, Y^{b}, X, X^{a} und X^{b} die in Anspruch 2 genannten Bedeutungen aufweisen.

4. Verbindung nach einem oder mehreren der Ansprüche 1 bis 3, umfassend mindestens eine Struktur der Formeln (IV-1) bis (IV-41), 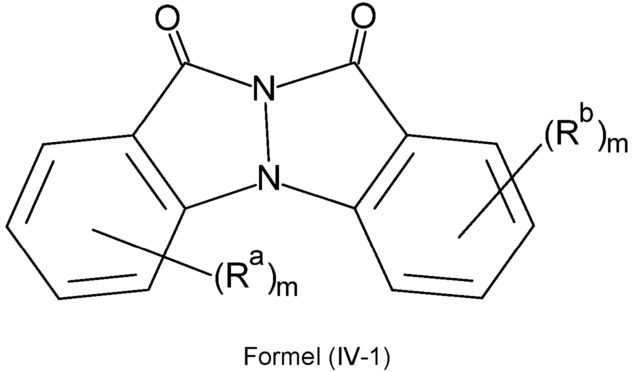 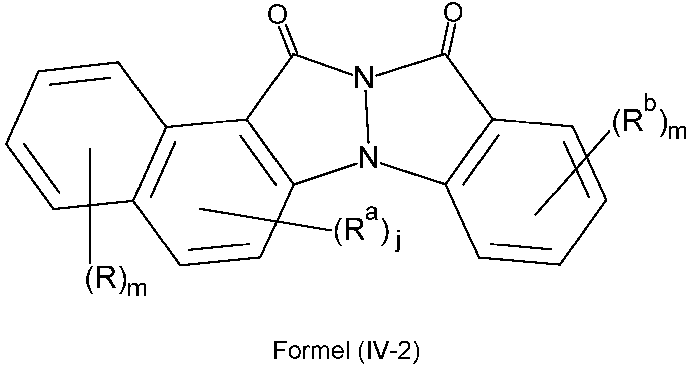 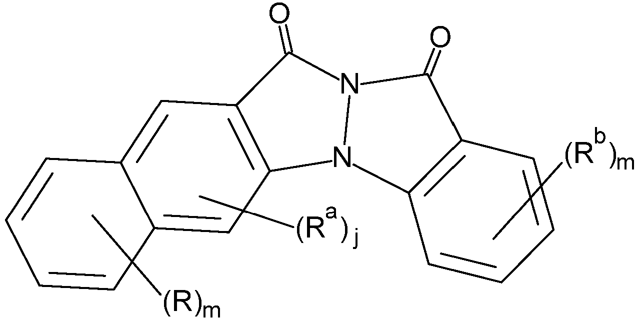 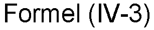 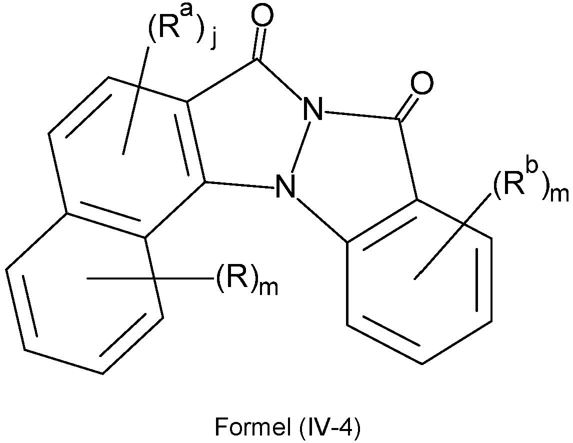 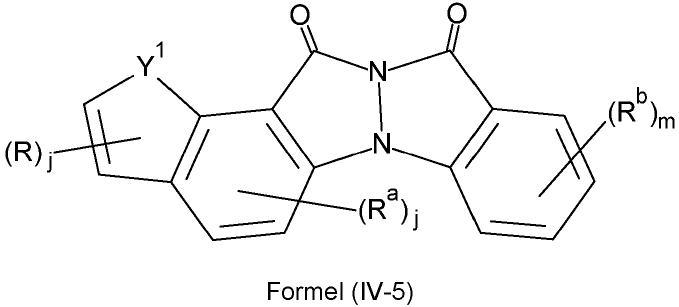 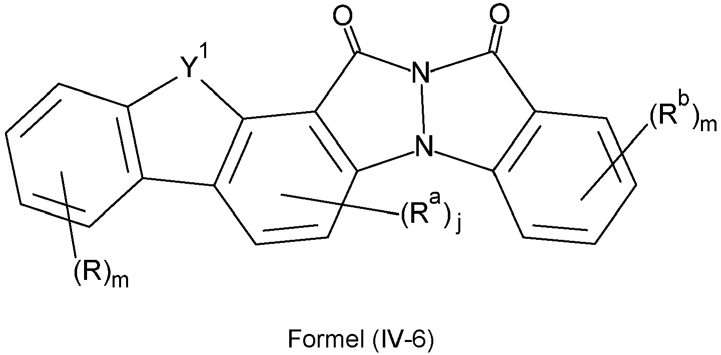 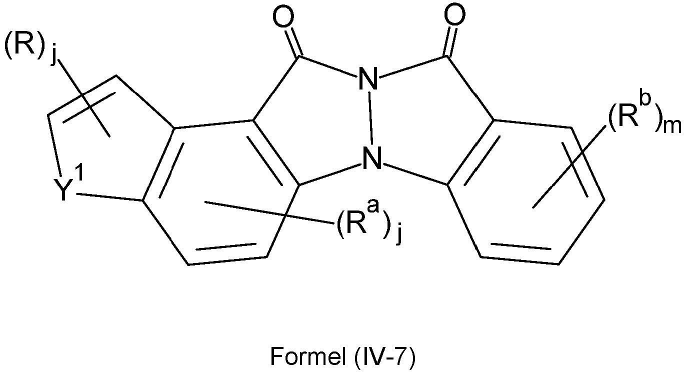 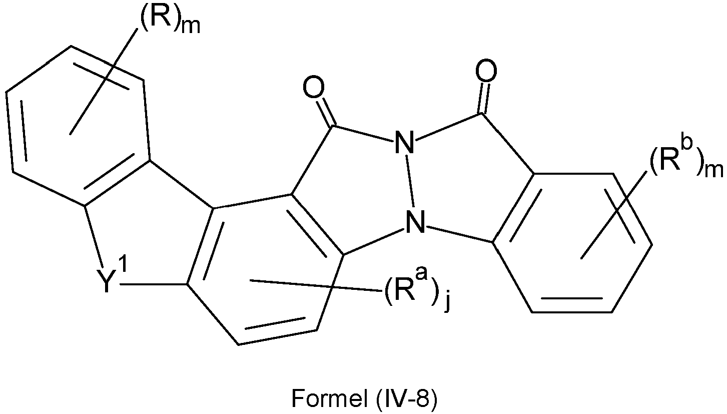 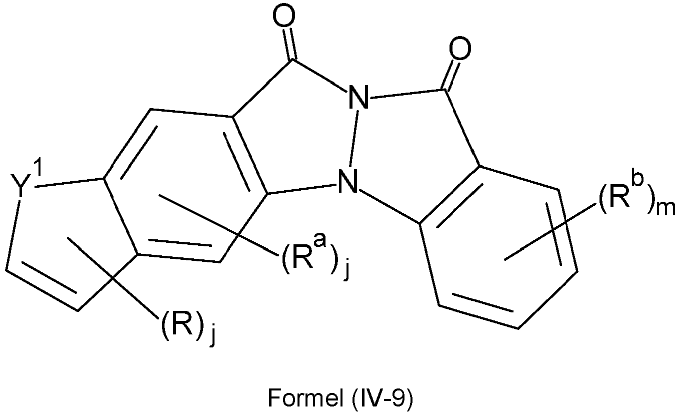 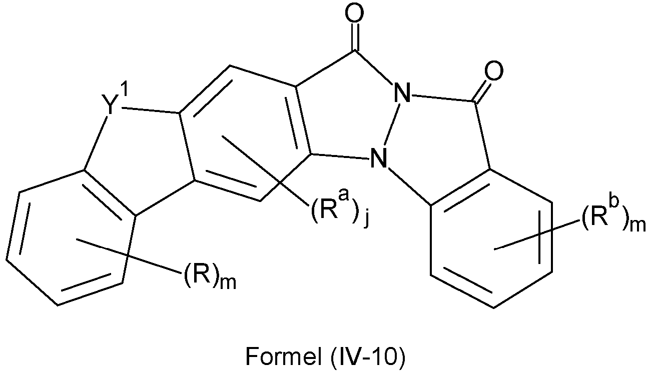 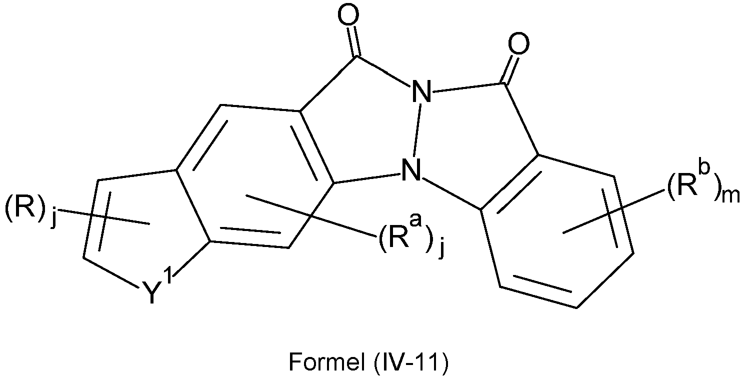 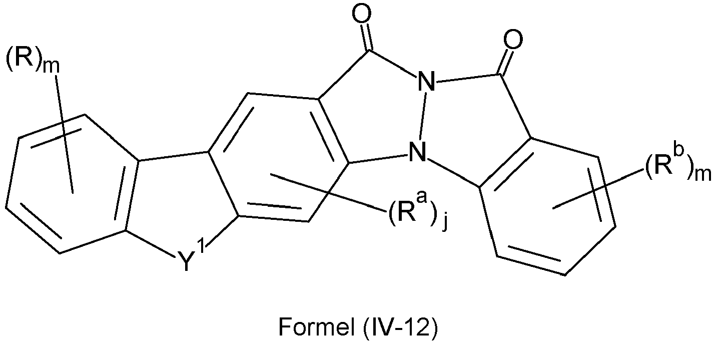 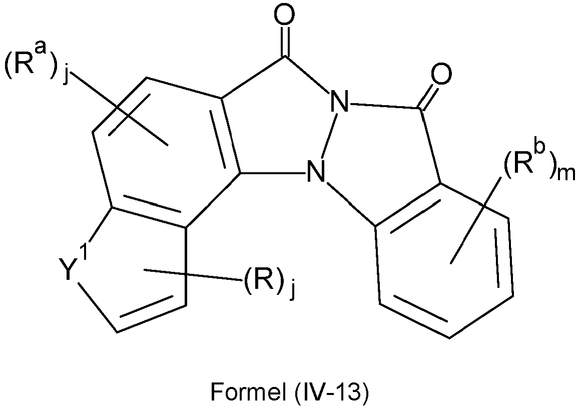 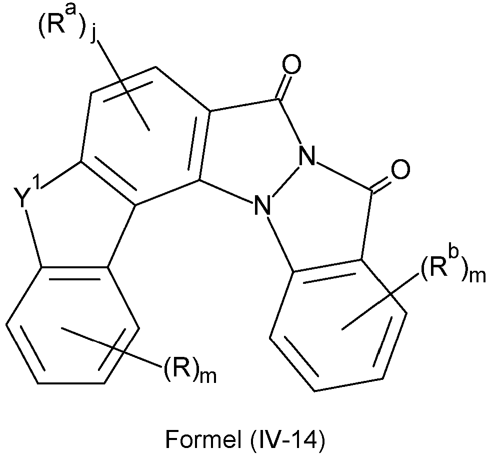 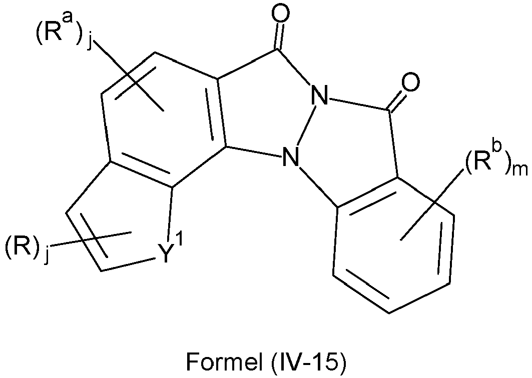 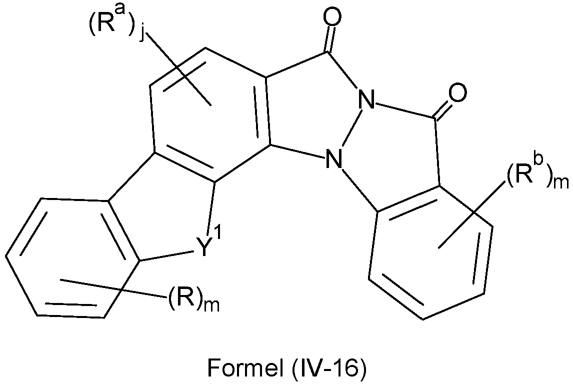 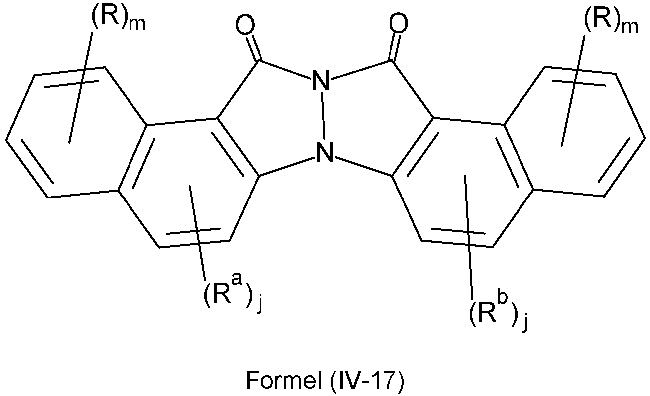 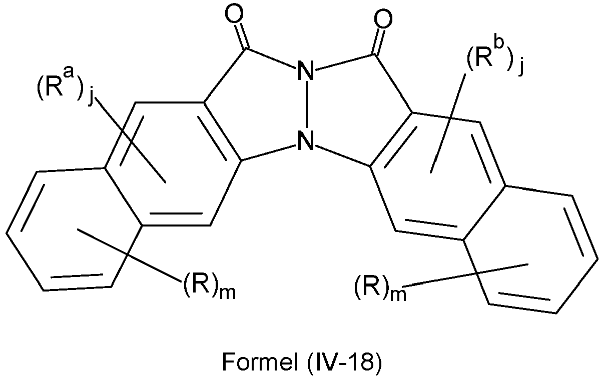 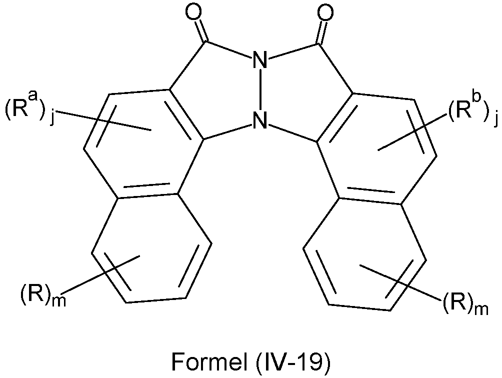 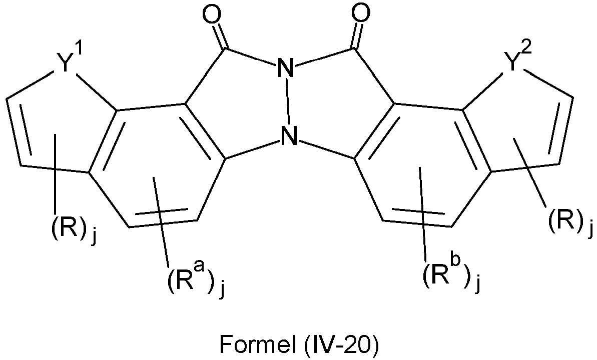 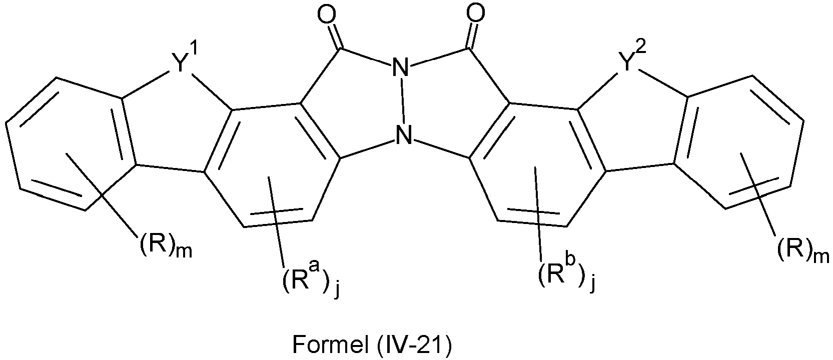 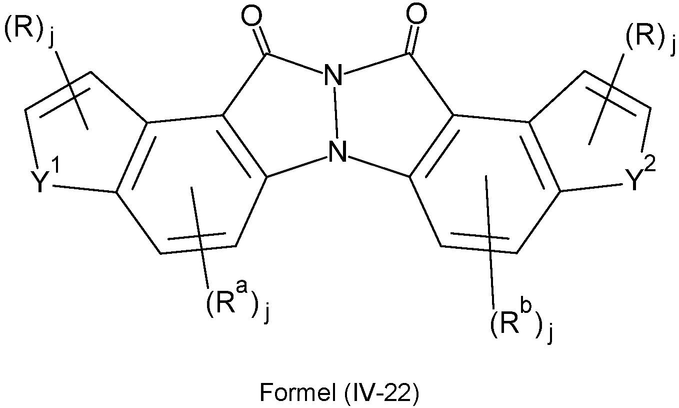 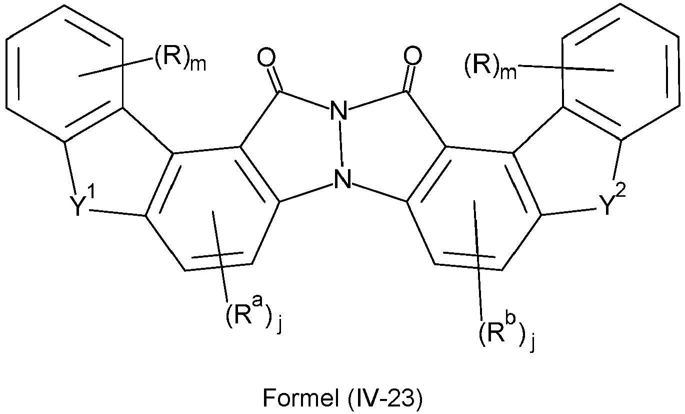 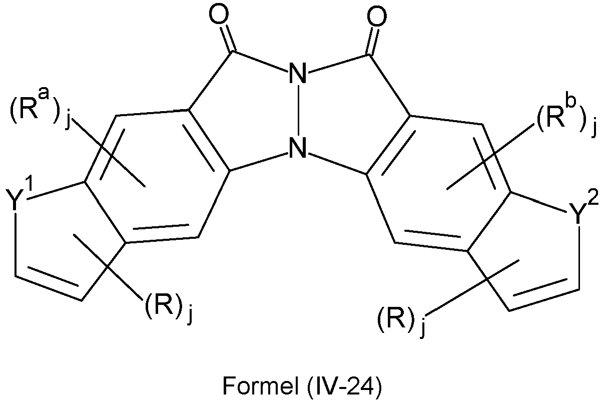 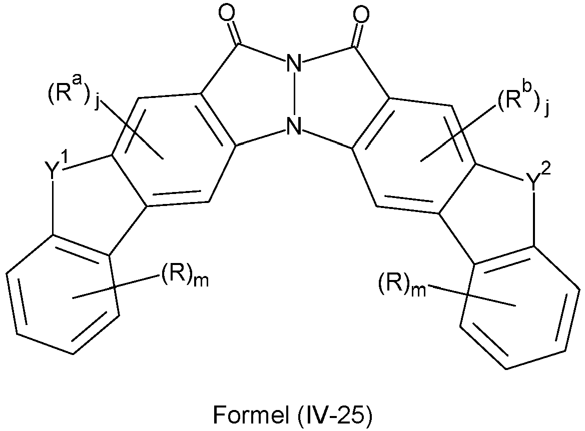 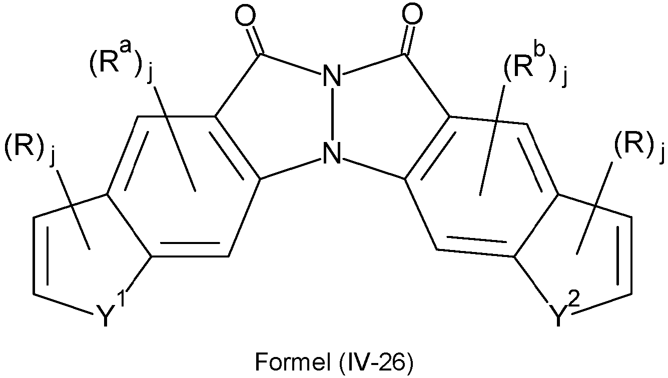 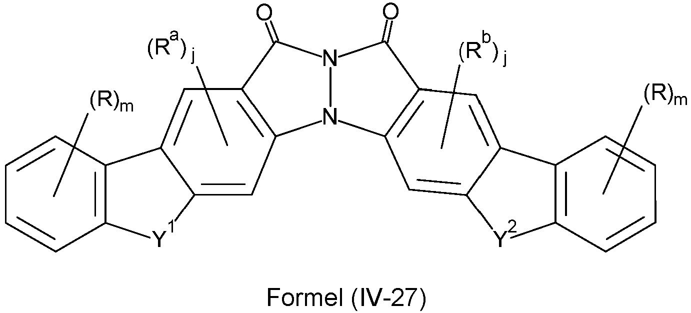 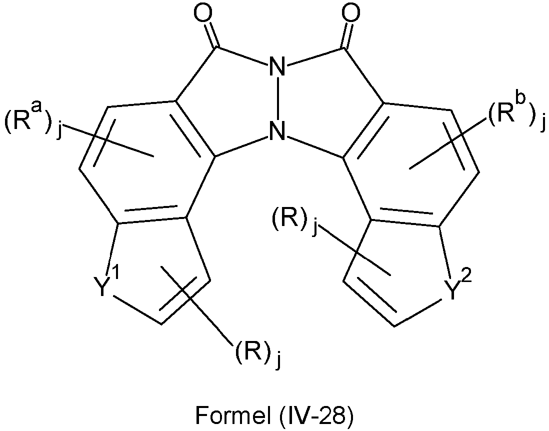 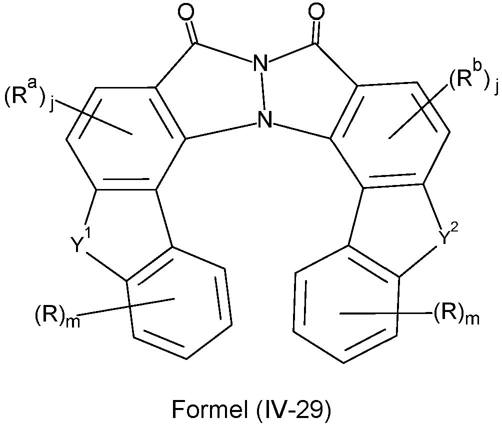 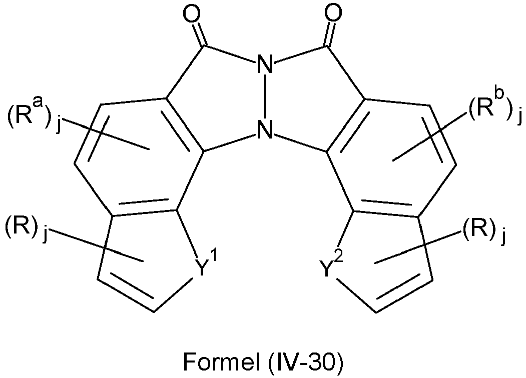 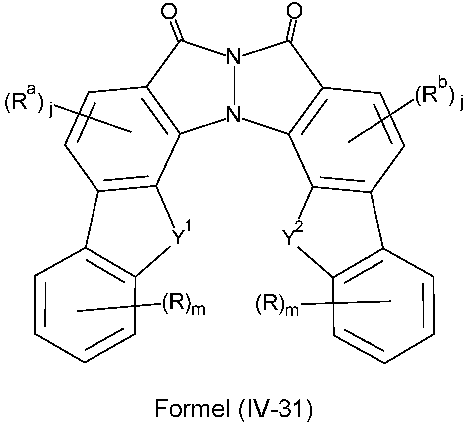 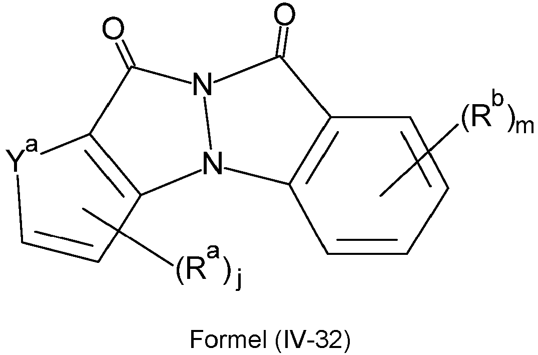 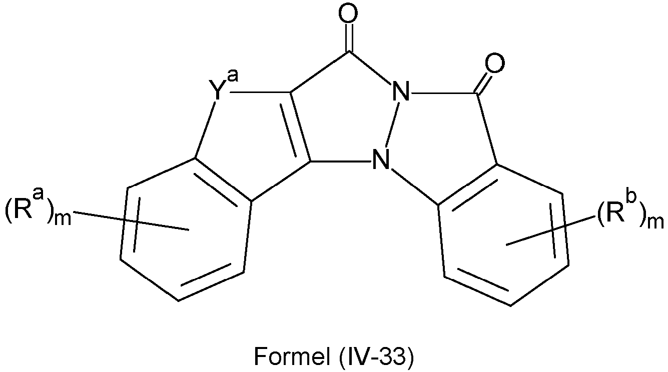 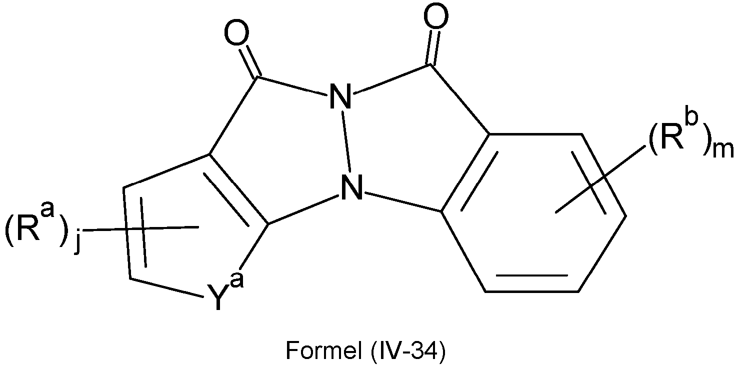 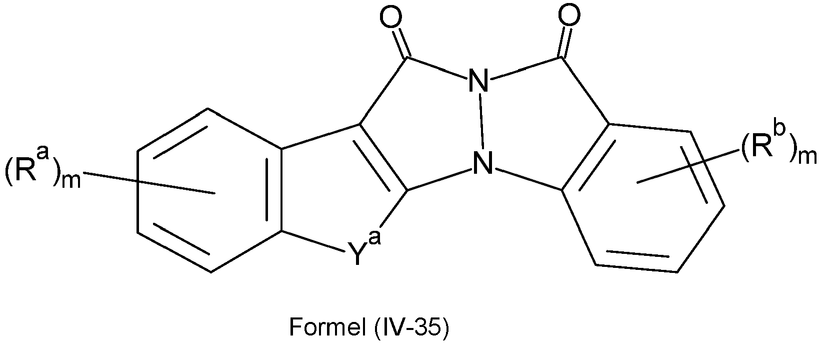 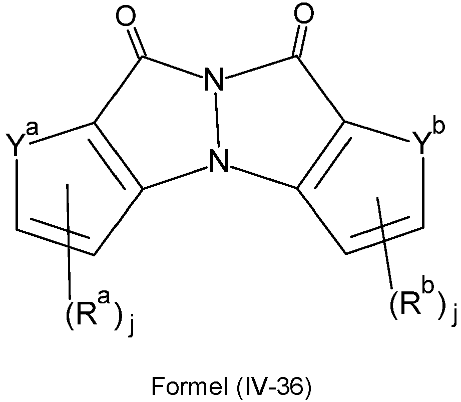 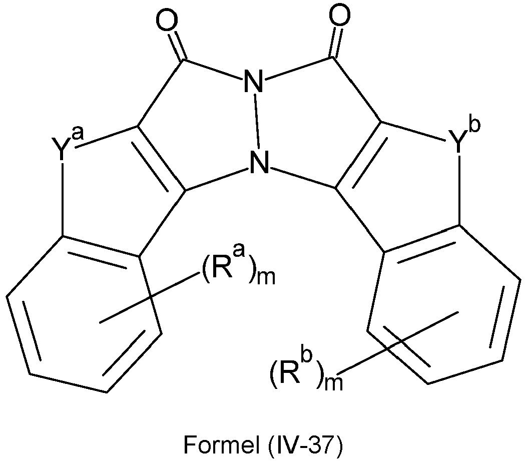 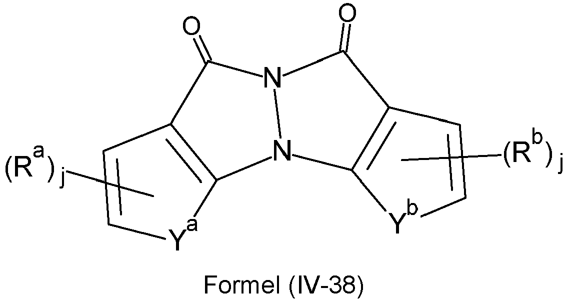 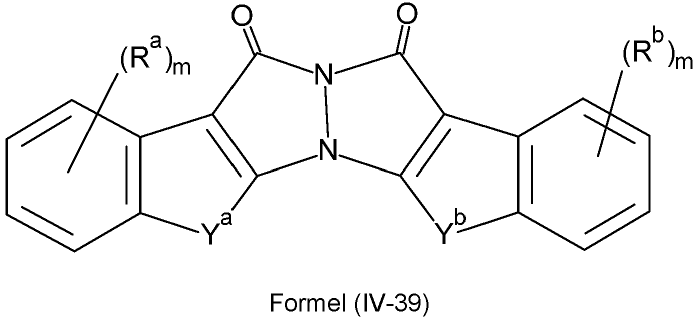 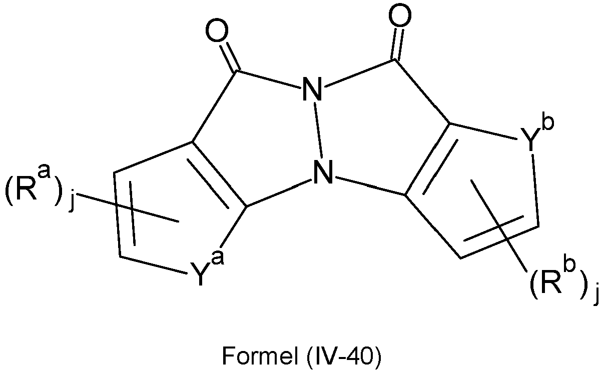 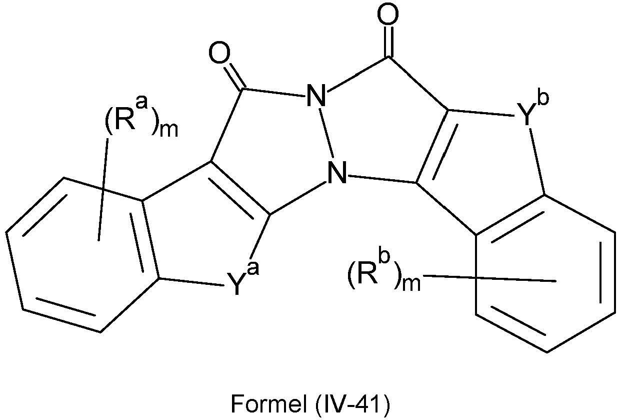 wobei die Symbole R, R^{a} und R^{b} die in Anspruch 1 genannten Bedeutungen aufweisen, die Symbole , Y¹, Y², Y^{a} und Y^{b} die in Anspruch 2 genannten Bedeutungen aufweisen und die weiteren Symbole die folgende Bedeutung aufweisen:
m ist 0, 1, 2, 3 oder 4;
j ist 0, 1 oder 2.

5. Verbindung nach einem oder mehreren der Ansprüche 1 bis 4, umfassend mindestens eine Struktur der Formeln (V-1) bis (V-8), 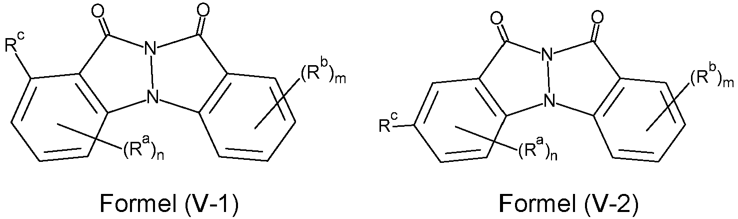 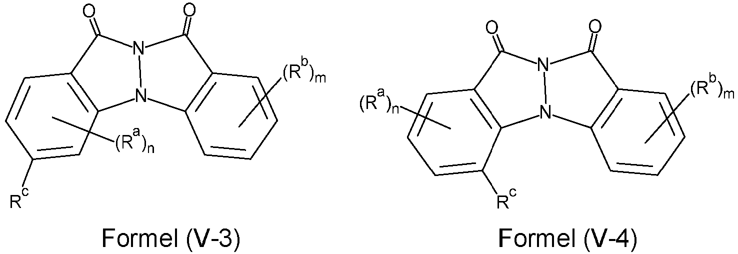 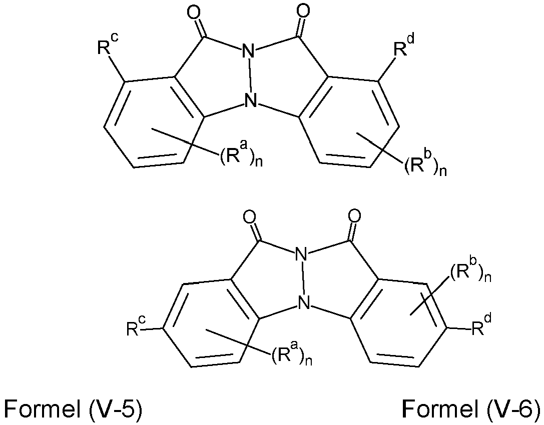 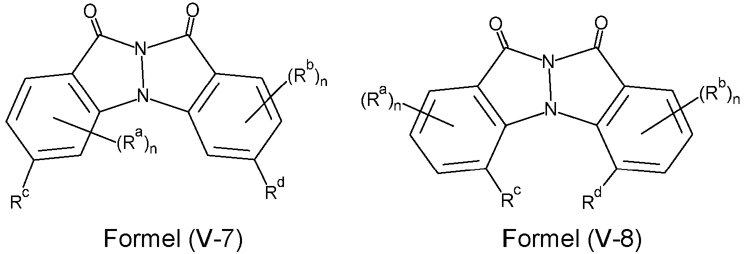 wobei die Symbole R^{a} und R^{b} die in Anspruch 1 genannten Bedeutungen aufweisen und die weiteren Symbole die folgende Bedeutung aufweisen:
R^{c}, R^{d} ist bei jedem Auftreten gleich oder verschieden N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, eine geradkettige Alkyl-, Alkoxy-oder Thioalkoxygruppe mit 1 bis 40 C-Atomen oder eine Alkenyl-oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann; oder Heteroarylthiogruppe mit 5 bis 60 aromatischen Ringatomen, die durch einen oder mehrere Reste R¹ substituiert sein kann, oder eine Diarylamino-, Arylheteroarylamino-, Diheteroarylaminogruppe mit 5 bis 60 aromatischen Ringatomen, die durch einen oder mehrere Reste R¹ substituiert sein kann, oder eine Arylalkyl-oder Heteroarylalkylgruppe mit 5 bis 60 aromatischen Ringatomen und 1 bis 10 C-Atomen im Alkylrest, die durch einen oder mehrere Reste R¹ substituiert sein kann; dabei ein Rest R^{c} mit einem R est R, R^{a}, R^{b}, R^{d} oder einer weiteren Gruppe ein Ringsystem bilden; dabei ein Rest R^{d} mit einem R est R, R^{a}, R^{b}, R^{c} oder einer weiteren Gruppe ein Ringsystem bilden;
m ist 0, 1, 2, 3 oder 4;
n ist 0, 1, 2 oder 3.

6. Verbindung nach einem oder mehreren der Ansprüche 1 bis 5, **dadurch gekennzeichnet, dass** mindestens ein R est R, R^{a}, R^{b}, R^{c}, R^{d} für eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, steht.

7. Verbindung nach einem oder mehreren der Ansprüche 1 bis 6, **dadurch gekennzeichnet, dass** mindestens zwei Reste R, R^{a}, R^{b}, R^{c}, R^{d} mit den weiteren Gruppen, an die die zwei R este R, R^{a}, R^{b}, R^{c}, R^{d} binden, einen kondensierten Ring bilden, wobei die zwei Reste R, R^{a}, R^{b}, R^{c}, R^{d} mindestens eine Struktur der Formeln (RA-1) bis (RA-12) formen 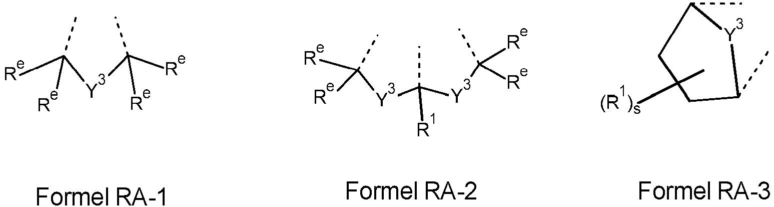 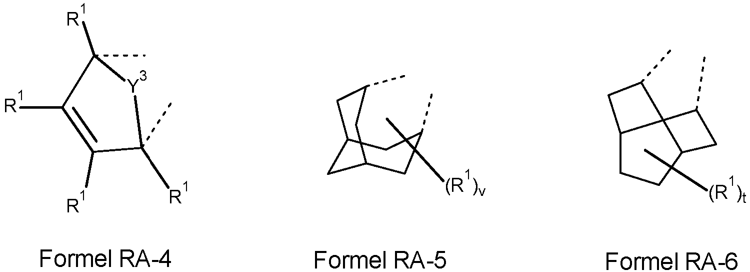 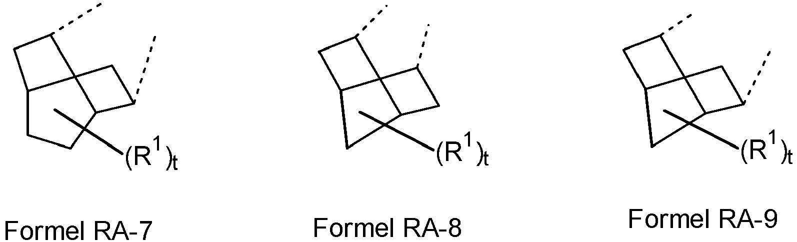 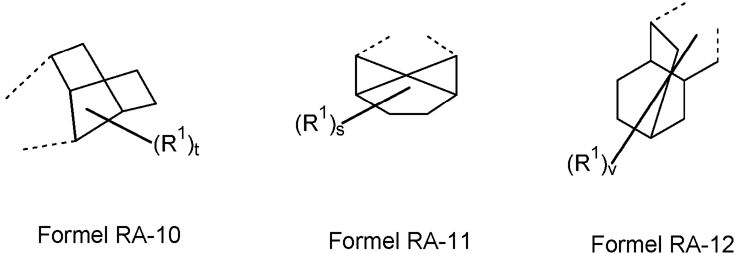 wobei R¹ die zuvor dargelegte Bedeutung hat, die gestrichelten Bindungen die Anbindungsstellen darstellen, über die die zwei Reste R, R^{a}, R^{b}, R^{c}, R^{d} an die weiteren Gruppen binden, darstellen, und die weiteren Symbole die folgende Bedeutung aufweisen:
Y³ ist bei jedem Auftreten gleich oder verschieden C(R¹)₂, (R¹)₂C-C(R¹)₂, (R¹)C=C(R¹), NR¹, NAr', O oder S;
R^{e} ist bei jedem Auftreten gleich oder verschieden F, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy-oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R² substituiert sein kann, wobei eine oder mehrere nicht benachbarte CH₂-Gruppen durch R²C=CR², C≡C, Si(R²)₂, C=O, C=S, C=Se, C=NR², -C(=O)O-, -C(=O)NR²-, NR², P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann; dabei können zwei Reste R^{e} auch miteinander oder ein Rest R^{e} mit einem Rest R¹ oder mit einer weiteren Gruppe ein Ringsystem bilden;
s ist 0, 1, 2, 3, 4, 5 oder 6;
t ist 0, 1, 2, 3, 4, 5, 6, 7 oder 8;
v ist 0, 1, 2, 3, 4, 5, 6, 7, 8 oder 9.

8. Verbindung nach einem oder mehreren der Ansprüche 1 bis 7, **dadurch gekennzeichnet, dass** mindestens zwei Reste R, R^{a}, R^{b}, R^{c}, R^{d} mit den weiteren Gruppen, an die die zwei Reste R, R^{a}, R^{b}, R^{c}, R^{d} binden, einen kondensierten Ring bilden, wobei die zwei Reste R, R^{a}, R^{b}, R^{c}, R^{d} der Strukturen der Formel (RB) formen, 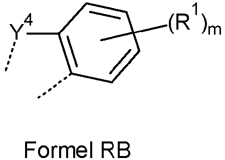 wobei R¹ die in Anspruch 1 dargelegte Bedeutung aufweist, die gestrichelten Bindungen die Anbindungsstellen darstellen, über die die zwei Reste R, R^{a}, R^{b}, R^{c}, R^{d} an die weiteren Gruppen binden, der Index m 0, 1, 2, 3 oder 4 und Y⁴ C(R¹)₂, NR¹, NAr', BR¹, BAr', O oder S ist.

9. Verbindung nach einem oder mehreren der Ansprüche 1 bis 8, umfassend mindestens eine Struktur der Formeln (VI-1) bis (VI-22), wobei die Verbindungen mindestens einen kondensierten Ring aufweisen, 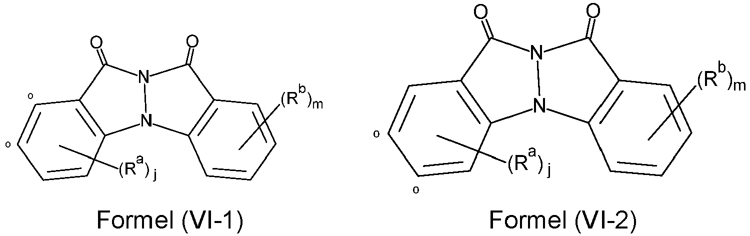 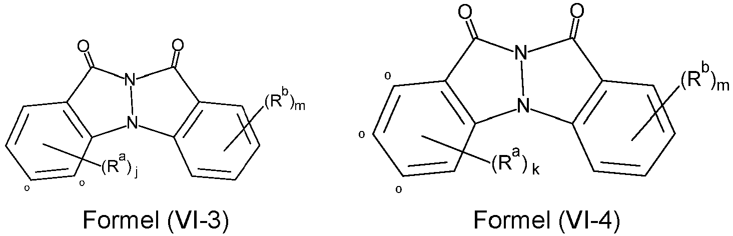 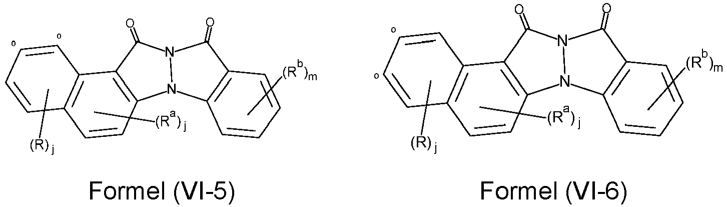 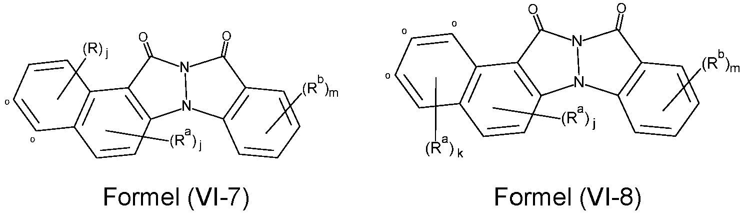 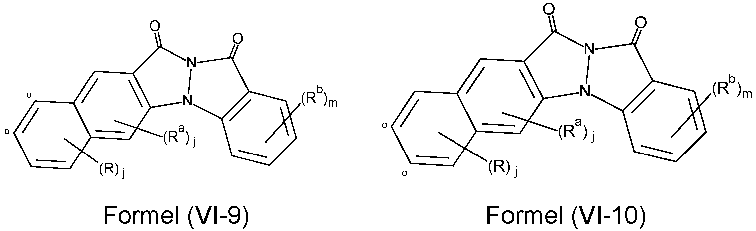 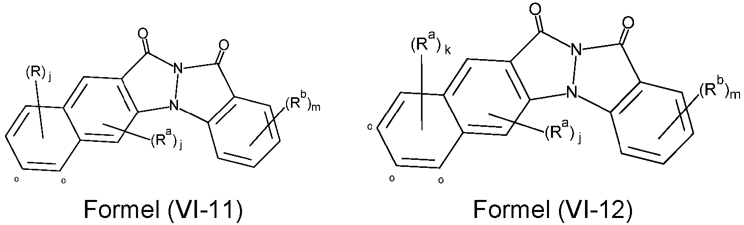 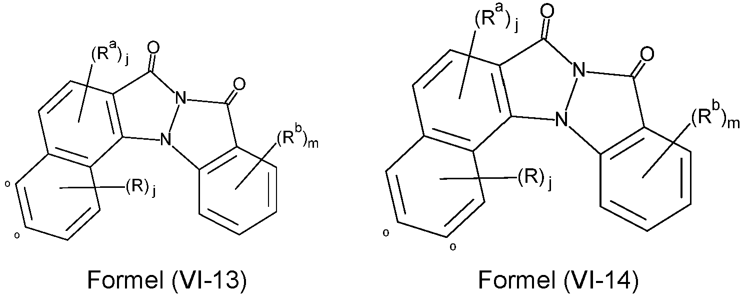 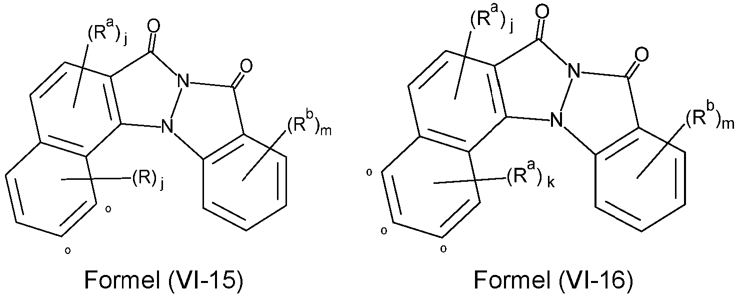 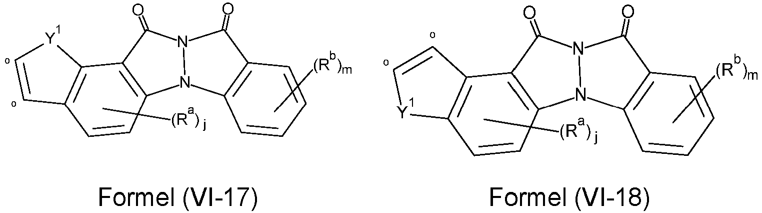 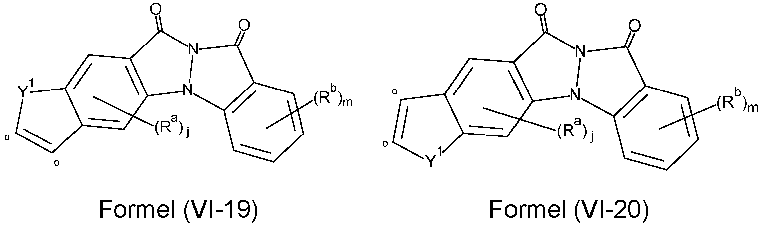 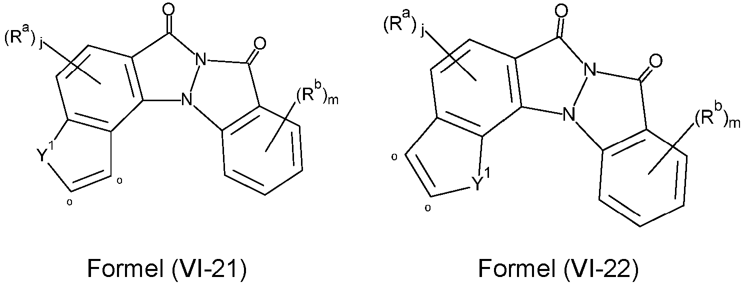 wobei die Symbole R, R^{a}, R^{b}, Y¹ und Y² die in Anspruch 1 beziehungsweise Anspruch 2 genannten Bedeutungen aufweisen, das Symbol o für die Anbindungsstellen steht und die weiteren Symbole die folgende Bedeutung haben:
m ist 0, 1, 2, 3 oder 4;
n ist 0, 1, 2 oder 3;
j ist 0, 1 oder 2.

10. Verbindung nach einem oder mehreren der Ansprüche 1 bis 9, **dadurch gekennzeichnet, dass** mindestens ein Substituent R, R^{a}, R^{b} gleich oder verschieden bei jedem Auftreten ausgewählt ist aus der Gruppe bestehend aus H, D, einer verzweigte oder cyclische Alkyl-, Alkoxy- oder Thioalkoxygruppe mit 3 bis 20 C-Atomen oder einem aromatischen oder heteroaromatischen Ringsystem ausgewählt aus den Gruppen der folgenden Formeln Ar-1 bis Ar-78, 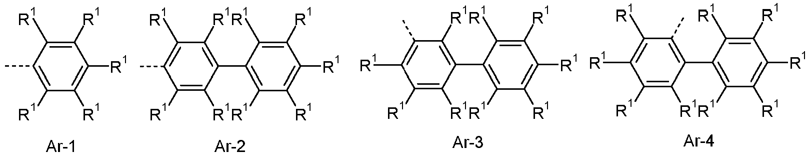 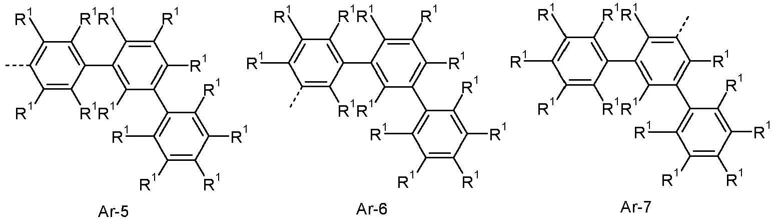 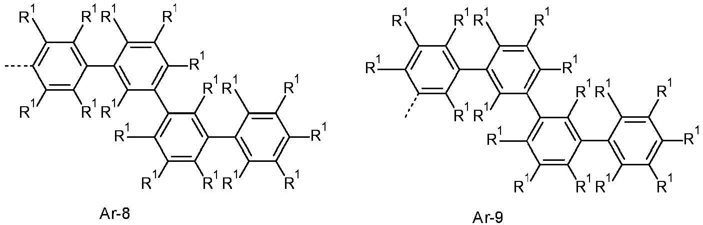 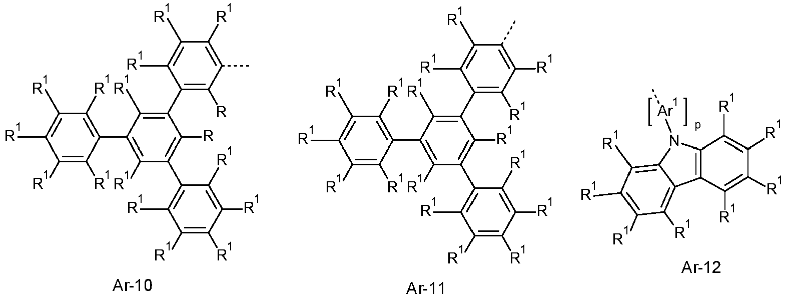 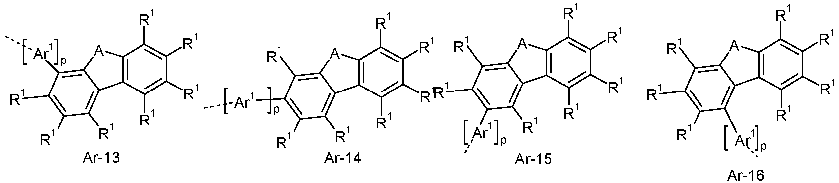 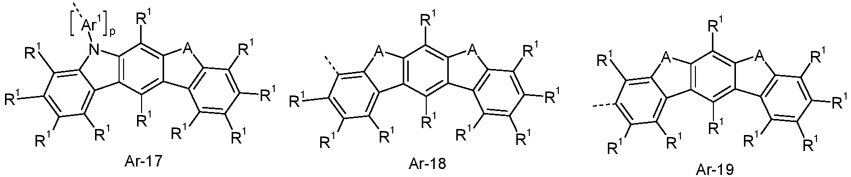 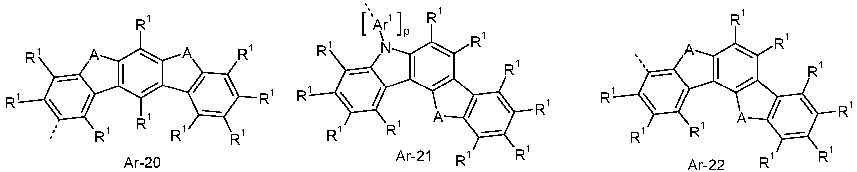 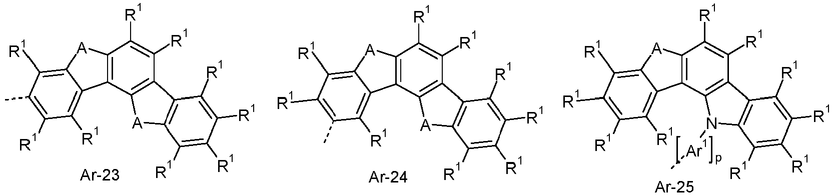 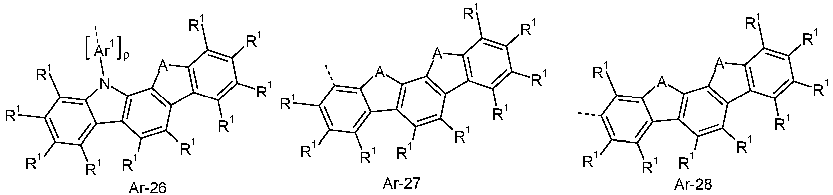 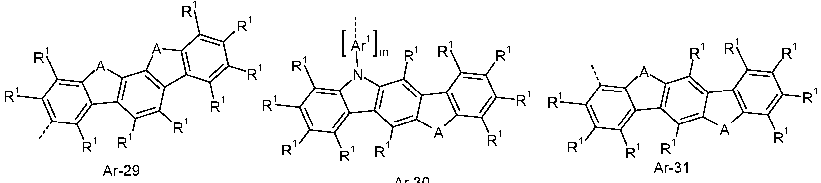 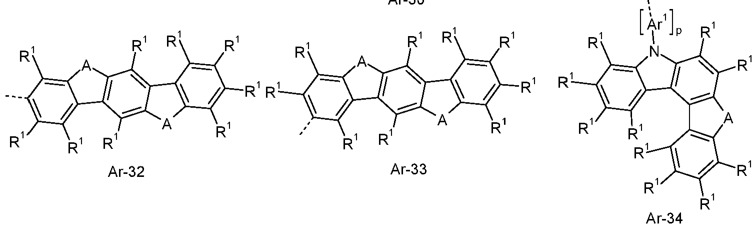 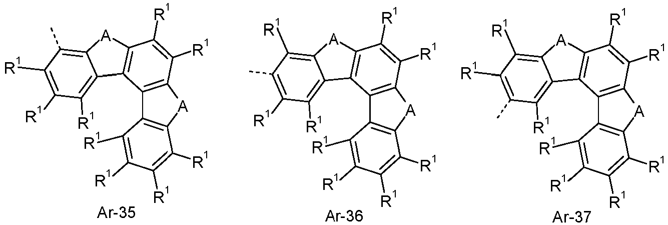 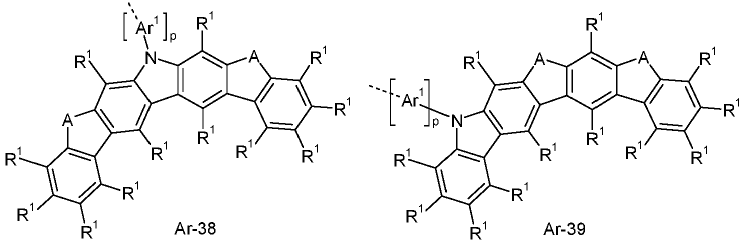 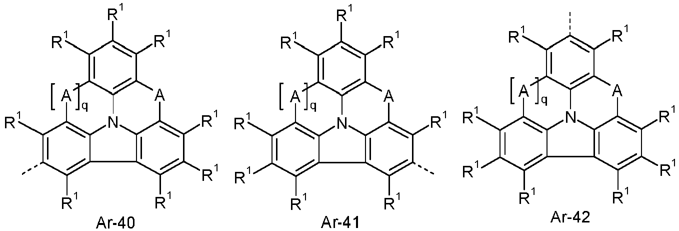 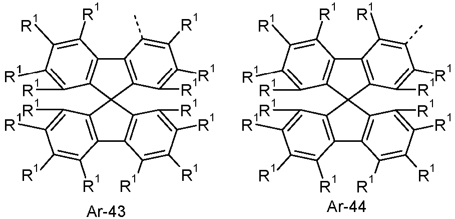 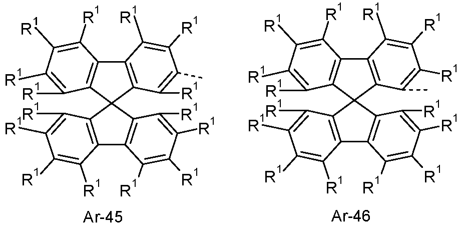 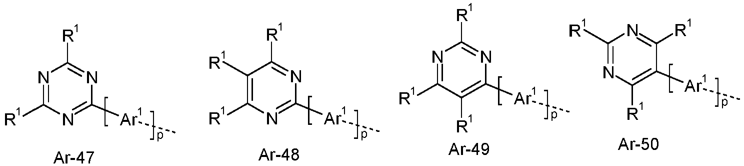 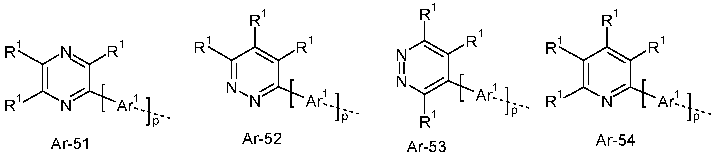 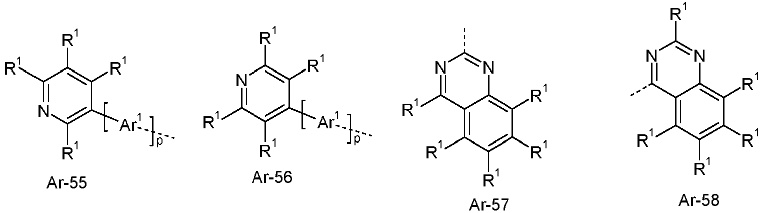 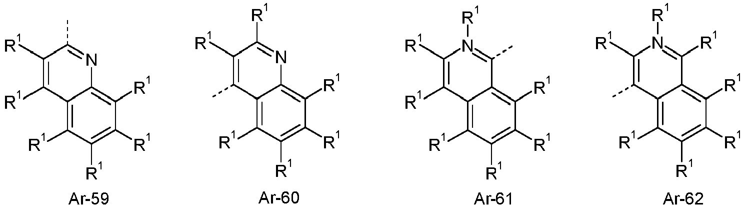 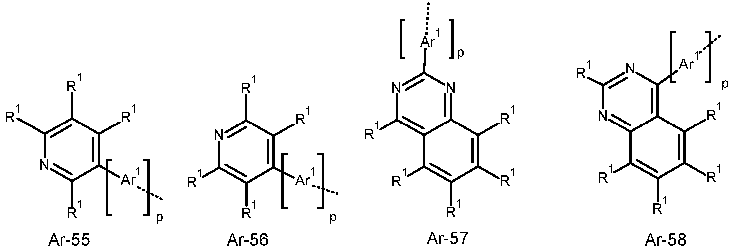 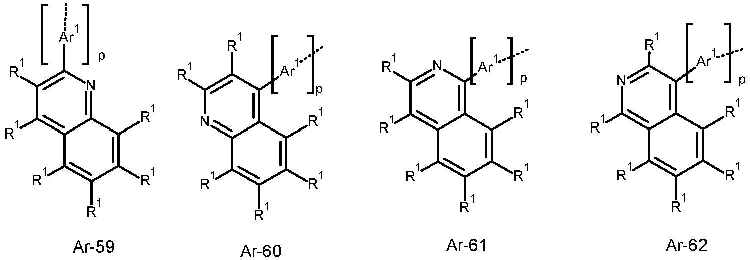 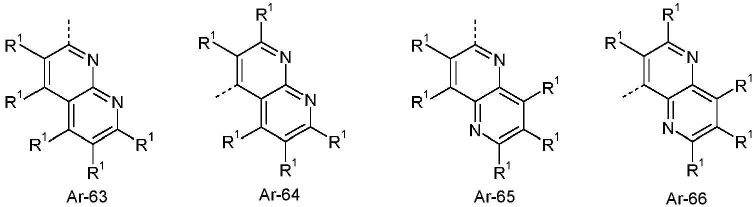 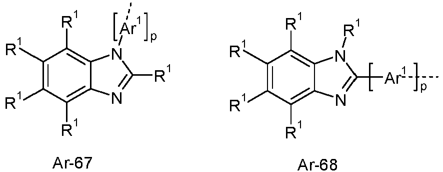 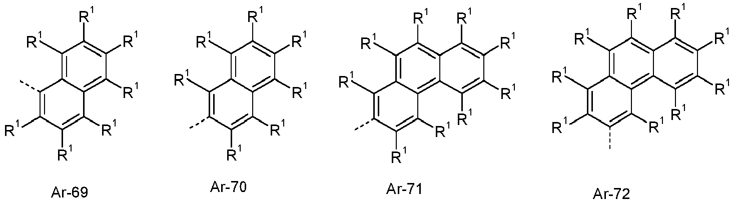 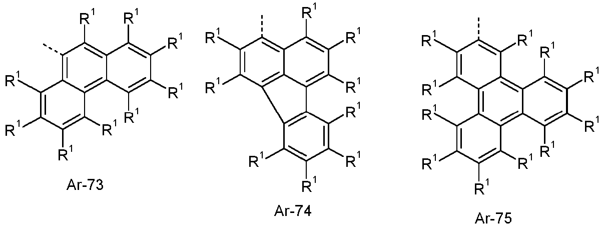 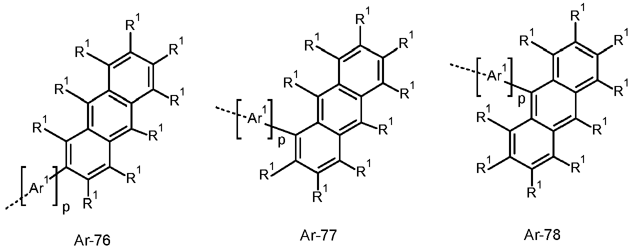 wobei R¹ die oben genannten Bedeutungen aufweist, die gestrichelte Bindung die Bindung an die entsprechende Gruppe darstellt und weiterhin gilt:
Ar¹ ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
A ist bei jedem Auftreten gleich oder verschieden C(R¹)₂, NR¹, O oder S;
p ist 0 oder 1, wobei p = 0 bedeutet, dass die Gruppe Ar¹ nicht vorhanden ist und dass die entsprechende aromatische bzw. heteroaromatische Gruppe direkt an den entsprechenden Rest gebunden ist;
q ist 0 oder 1, wobei q = 0 bedeutet, dass an dieser Position keine Gruppe A gebunden ist und an die entsprechenden Kohlenstoffatome statt dessen Reste R¹ gebunden sind.

11. Verbindung gemäß mindestens einem der vorhergehenden Ansprüche 5 bis 10, **dadurch gekennzeichnet, dass** der Substituent R^{c}, R^{d} gleich oder verschieden bei jedem Auftreten ein aromatisches oder heteroaromatisches Ringsystem mit 6 bis 30 aromatischen Ringatomen darstellt, welches mit einem oder mehreren Resten R¹ substituiert sein kann, welches ausgewählt ist aus den Gruppen der in Anspruch 10 dargelegten Formeln (Ar-1) bis (Ar-78).

12. Verbindung gemäß mindestens einem der vorhergehenden Ansprüche 1 bis 11, **dadurch gekennzeichnet, dass** die Verbindung genau zwei oder genau drei Strukturen gemäß Formel (I), (II-1) bis (II-42), (III-1) bis (III-41), (IV-1) bis (IV-41), (V-1) bis (V-8) und/oder (VI-1) bis (VI-22) umfasst.

13. Oligomer, Polymer oder Dendrimer enthaltend eine oder mehrere Verbindungen nach einem der Ansprüche 1 bis 11, wobei statt eines Wasserstoffatoms oder eines Substituenten eine oder mehrere Bindungen der Verbindungen zum Polymer, Oligomer oder Dendrimer vorhanden sind.

14. Formulierung, enthaltend mindestens eine Verbindung nach einem oder mehreren der Ansprüche 1 bis 12 oder ein Oligomer, Polymer oder Dendrimer nach Anspruch 13 und mindestens eine weitere Verbindung, wobei die weitere Verbindung bevorzugt ausgewählt ist aus einem oder mehreren Lösemitteln.

15. Zusammensetzung, enthaltend mindestens eine Verbindung nach Formel (I) 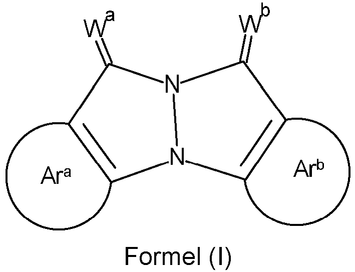 wobei die verwendeten Symbole die in Anspruch 1 genannten Bedeutungen aufweisen, oder ein Oligomer, Polymer oder Dendrimer nach Anspruch 13 und mindestens eine weitere Verbindung ausgewählt aus der Gruppe bestehend aus fluoreszierenden Emittern, phosphoreszierenden Emittern, Emittern, die TADF zeigen, Hostmaterialien, Elektronentransportmaterialien, Elektroneninjektionsmaterialien, Lochleitermaterialien, Lochinjektionsmaterialien, Elektronenblockiermaterialien und Lochblockiermaterialien.

16. Verfahren zur Herstellung einer Verbindung nach einem oder mehreren der Ansprüche 1 bis 12, **dadurch gekennzeichnet, dass** ein Grundgerüst mit mindestens einer der Gruppen W^{a} oder einem Vorläufer einer der Gruppe W^{a} synthetisiert wird und ein aromatischer oder heteroaromatischer Rest mittels einer nukleophilen aromatischen Substitutionsreaktion oder einer Kupplungsreaktion eingeführt wird.

17. Verwendung einer Verbindung nach Formel (I) 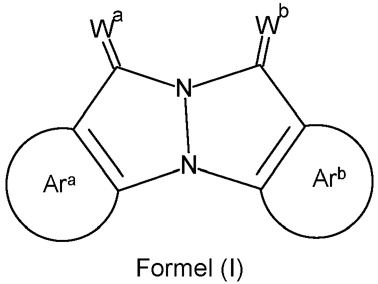 wobei die verwendeten Symbole die in Anspruch 1 genannten Bedeutungen aufweisen, oder ein Oligomer, Polymer oder Dendrimer nach Anspruch 13 in einer elektronischen Vorrichtung

18. Elektronische Vorrichtung enthaltend mindestens eine Verbindung nach Formel (I) 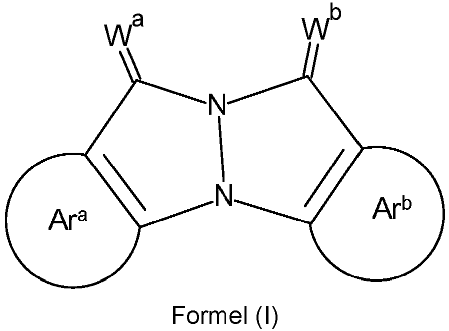 wobei die verwendeten Symbole die in Anspruch 1 genannten Bedeutungen aufweisen, oder ein Oligomer, Polymer oder Dendrimer nach Anspruch 13.

## Claims

1. Compound including at least one structure of the formula (I) 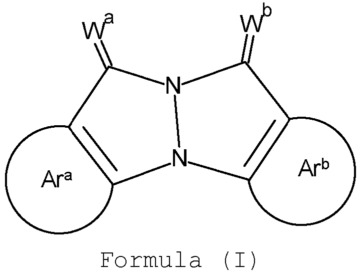
where the ring Ar^{a} is the same or different at each instance and is an aromatic or heteroaromatic ring system which has 5 to 60 aromatic ring atoms and may be substituted by one or more Ar or R^{a} radicals, the ring Ar^{b} is the same or different at each instance and is an aromatic or heteroaromatic ring system which has 5 to 60 aromatic ring atoms and may be substituted by one or more Ar or R^{b} radicals;
and where the further symbols and indices used are as follows:
W^{a}, W^{b} is the same or different at each instance and is O or S;
Ar is the same or different at each instance and is an aromatic or heteroaromatic ring system which has 5 to 60 aromatic ring atoms and may be substituted by one or more R radicals; the Ar group here may form a ring system with at least one Ar, R, R^{a}, R^{b} group or a further group;
R, R^{a}, R^{b} is the same or different at each instance and is H, D, OH, F, Cl, Br, I, CN, NO₂, N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P (=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, a straight-chain alkyl, alkoxy or thioalkoxy group having 1 to 40 carbon atoms or an alkenyl or alkynyl group having 2 to 40 carbon atoms or a branched or cyclic alkyl, alkoxy or thioalkoxy group having 3 to 20 carbon atoms, where the alkyl, alkoxy, thioalkoxy, alkenyl or alkynyl group may in each case be substituted by one or more R¹ radicals, where one or more nonadjacent CH₂ groups may be replaced by R¹C=CR¹, C≡C, Si(R¹)₂, C=O, C=S, C=Se, C=NR¹, -C(=O)O-, -C(=O)NR¹-, NR¹, P(=O)(R¹), -O-, -S-, SO or SO₂, or an aromatic or heteroaromatic ring system which has 5 to 60 aromatic ring atoms and may be substituted in each case by one or more R¹ radicals, or an aryloxy or heteroaryloxy group which has 5 to 60 aromatic ring atoms and may be substituted by one or more R¹ radicals; or heteroarylthio group which has 5 to 60 aromatic ring atoms and may be substituted by one or more R¹ radicals, or a diarylamino, arylheteroarylamino, diheteroarylamino group which has 5 to 60 aromatic ring atoms and may be substituted by one or more R¹ radicals, or an aralkyl or heteroarylalkyl group which has 5 to 60 aromatic ring atoms and 1 to 10 carbon atoms in the alkyl radical and may be substituted by one or more R¹ radicals; at the same time, two R, R^{a}, R^{b} radicals together or with a further group may also form a ring system;
Ar' is the same or different at each instance and is an aromatic or heteroaromatic ring system which has 5 to 60 aromatic ring atoms and may be substituted by one or more R¹ radicals; at the same time, it is possible for two Ar' radicals bonded to the same carbon atom, silicon atom, nitrogen atom, phosphorus atom or boron atom also to be joined together via a bridge by a single bond or a bridge selected from B (R¹), C(R¹)₂, Si(R¹)₂, C=O, C=NR¹, C=C(R¹)₂, O, S, S=O, SO₂, N(R¹), P(R¹) and P(=O)R¹;
R¹ is the same or different at each instance and is H, D, F, Cl, Br, I, CN, NO₂, N(Ar'')₂, N(R²)₂, C(=O)Ar", C(=O)R², P(=O) (Ar")₂, P(Ar")₂, B(Ar")₂, B(R²)₂, C(Ar")₃, C(R²)₃, Si(Ar")₃, Si(R²)₃, a straight-chain alkyl, alkoxy or thioalkoxy group having 1 to 40 carbon atoms or a branched or cyclic alkyl, alkoxy or thioalkoxy group having 3 to 40 carbon atoms or an alkenyl group having 2 to 40 carbon atoms, each of which may be substituted by one or more R² radicals, where one or more nonadjacent CH₂ groups may be replaced by -R²C=CR²-, -C≡C-, Si(R²)₂, C=O, C=S, C=Se, C=NR², -C(=O)O-, -C(=O)NR²-, NR², P(=O)(R²), -O-, -S-, SO or SO₂ and where one or more hydrogen atoms may be replaced by D, F, Cl, Br, I, CN or NO₂, or an aromatic or heteroaromatic ring system which has 5 to 60 aromatic ring atoms, each of which may be substituted by one or more R² radicals, or an aryloxy or heteroaryloxy group which has 5 to 60 aromatic ring atoms and may be substituted by one or more R² radicals, or an aralkyl or heteroaralkyl group which has 5 to 60 aromatic ring atoms and may be substituted by one or more R² radicals, or a combination of these systems; at the same time, two or more R¹ radicals together may form a ring system; at the same time, one or more R¹ radicals may form a ring system with a further part of the compound;
Ar'' is the same or different at each instance and is an aromatic or heteroaromatic ring system which has 5 to 30 aromatic ring atoms and may be substituted by one or more R² radicals; at the same time, it is possible for two Ar'' radicals bonded to the same carbon atom, silicon atom, nitrogen atom, phosphorus atom or boron atom also to be joined together via a bridge by a single bond or a bridge selected from B (R²), C(R²)₂, Si(R²)₂, C=O, C=NR², C=C(R²)₂, O, S, S=O, SO₂, N (R²), P(R²) and P(=O)R²;
R² is the same or different at each instance and is selected from the group consisting of H, D, F, CN, an aliphatic hydrocarbyl radical having 1 to 20 carbon atoms or an aromatic or heteroaromatic ring system which has 5 to 30 aromatic ring atoms and in which one or more hydrogen atoms may be replaced by D, F, Cl, Br, I or CN and which may be substituted by one or more alkyl groups each having 1 to 4 carbon atoms; at the same time, two or more substituents R² together may form a ring system;
with exclusion of compounds of the formulae (A), (B) and (C) 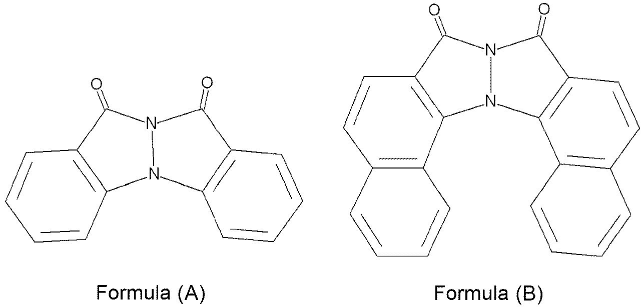 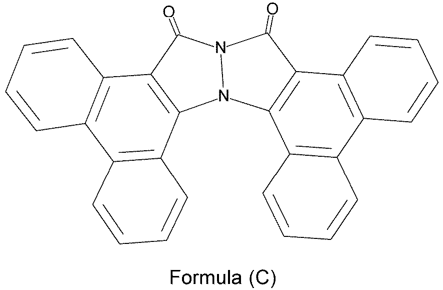
from protection.

2. Compound according to Claim 1, comprising at least one structure of the formula (II-1) to (II-42): 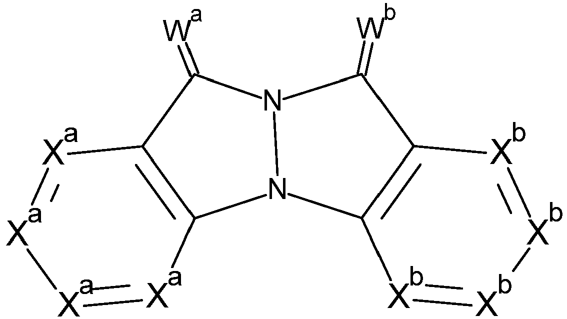 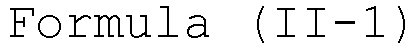 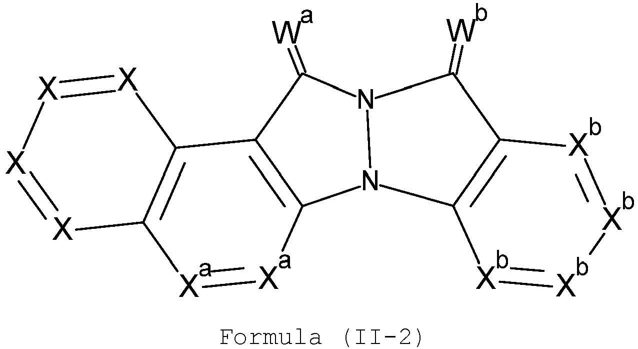 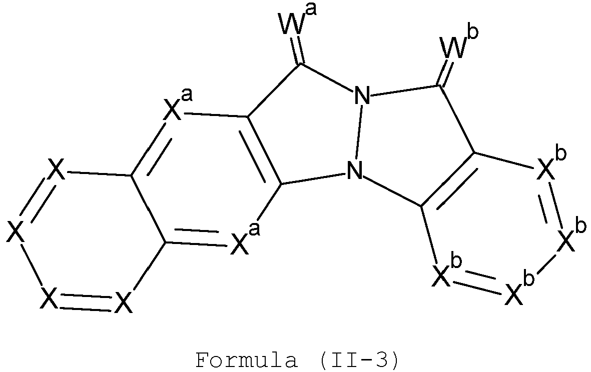 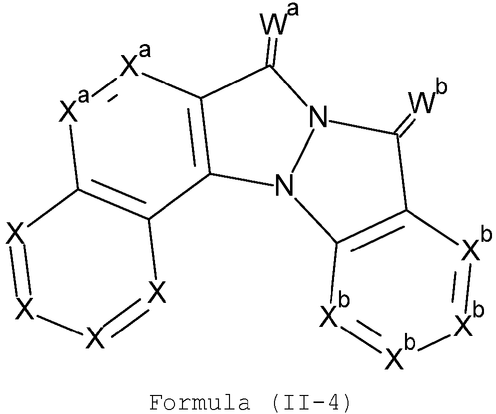 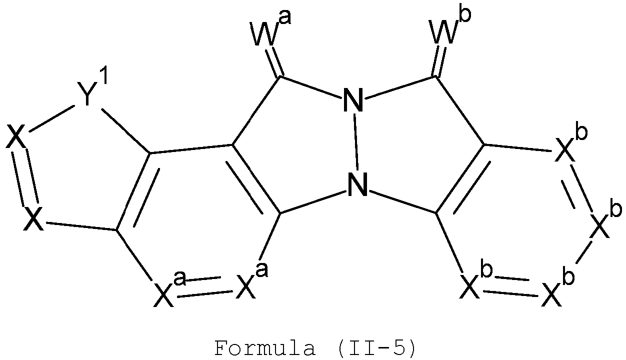 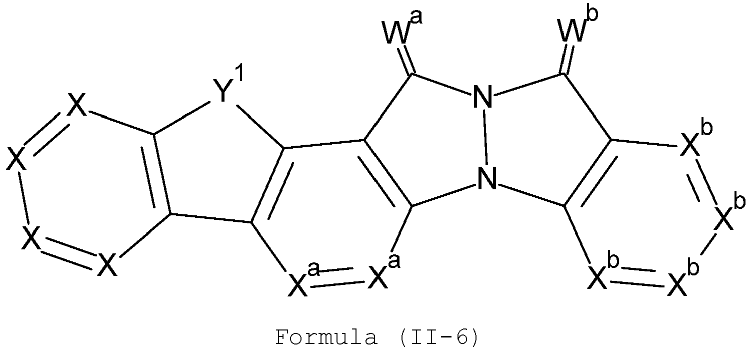 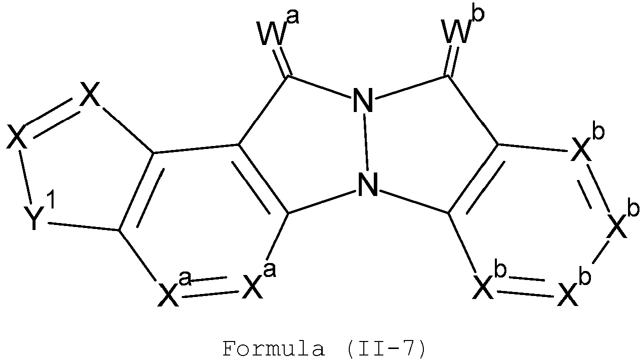 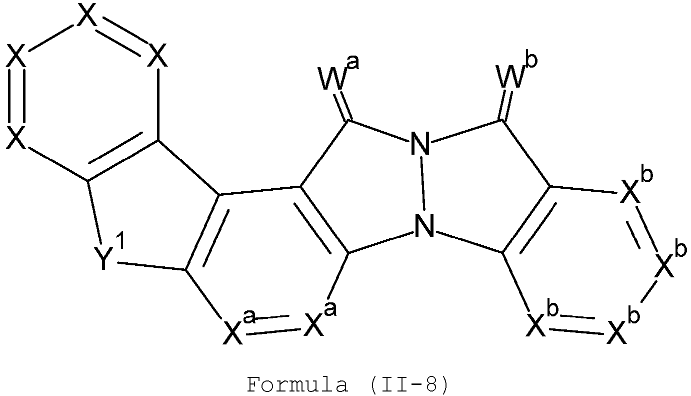 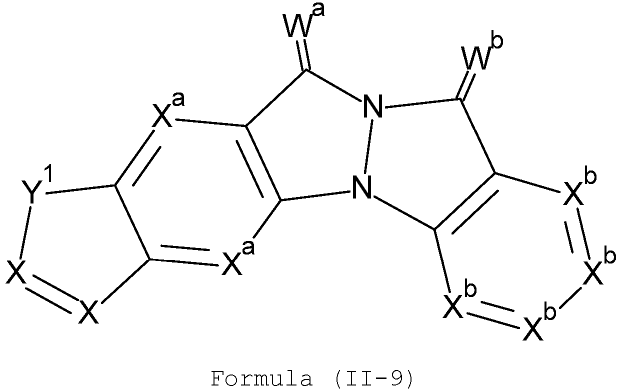 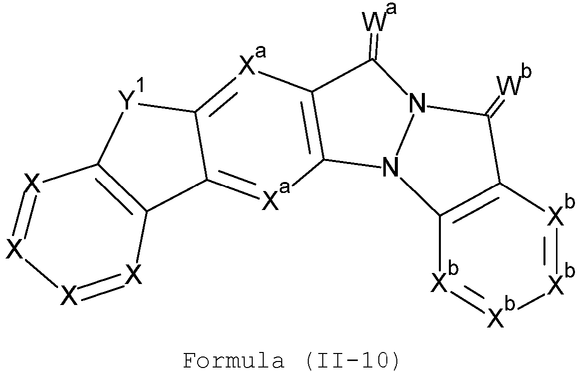 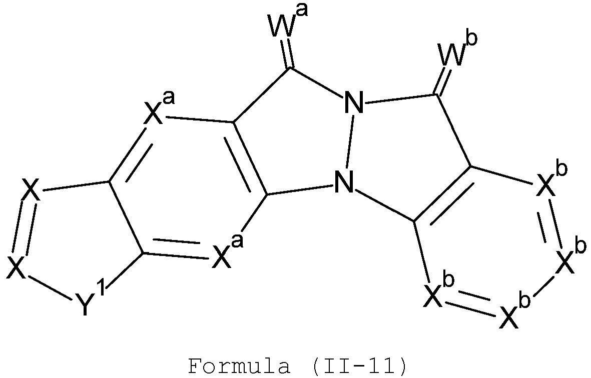 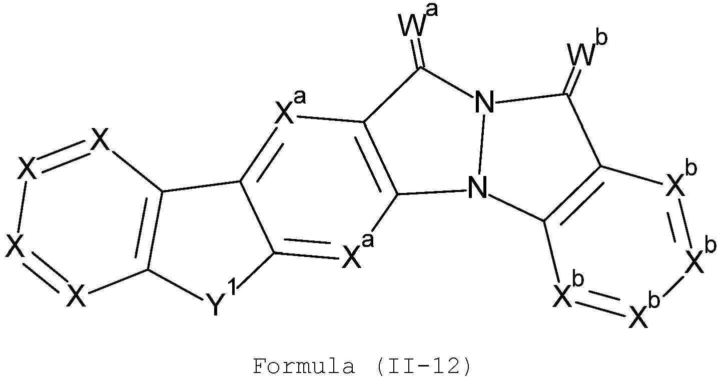 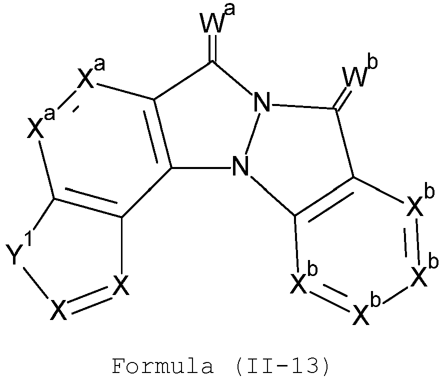 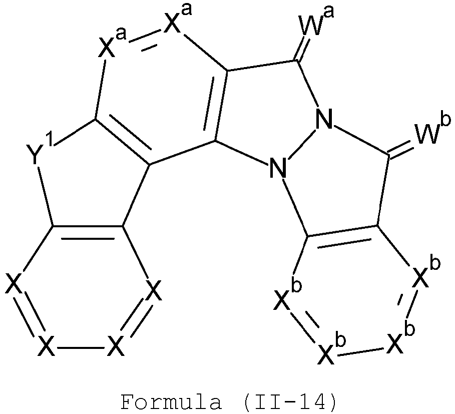 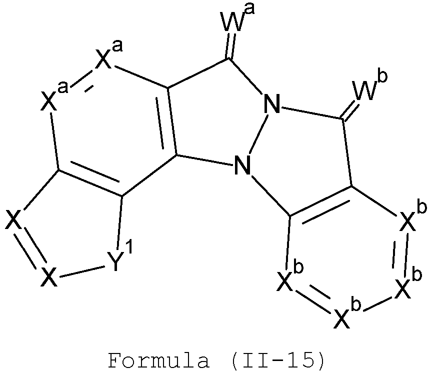 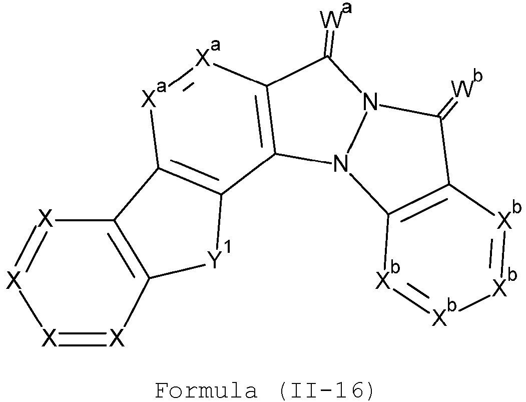 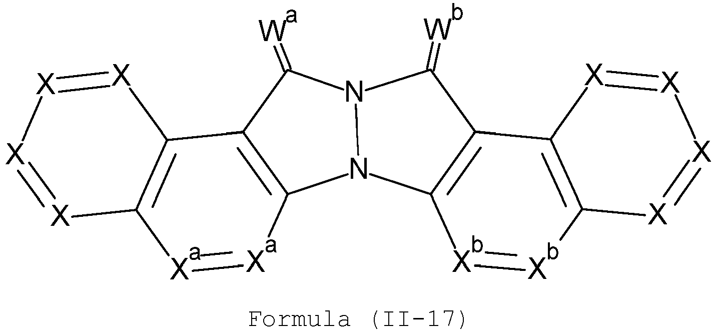 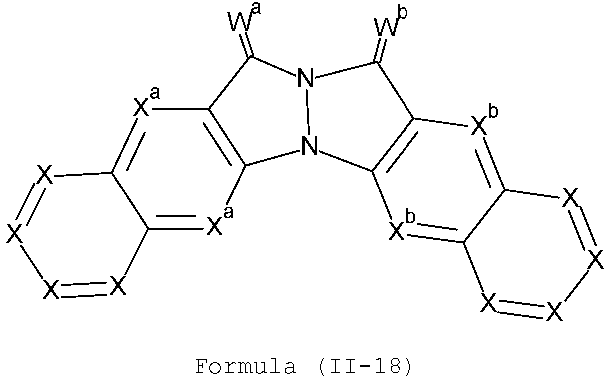 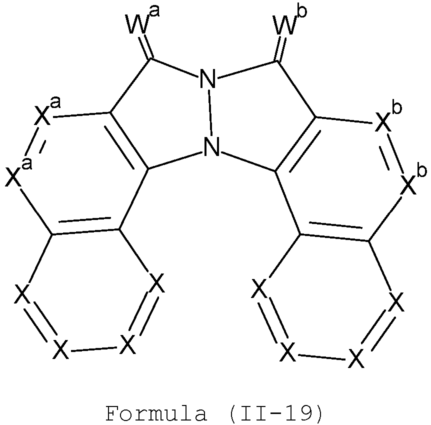 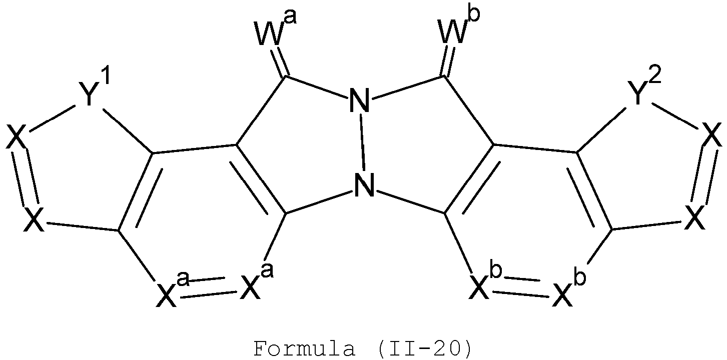 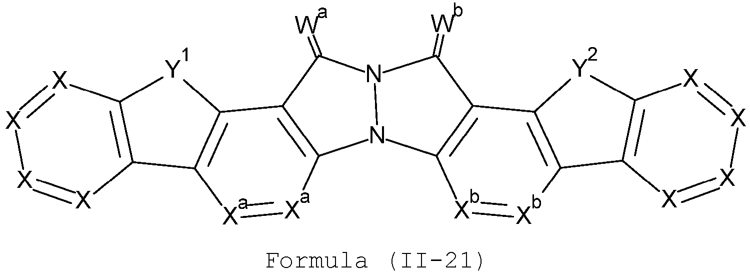 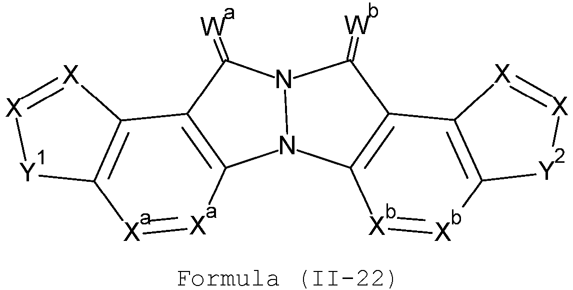 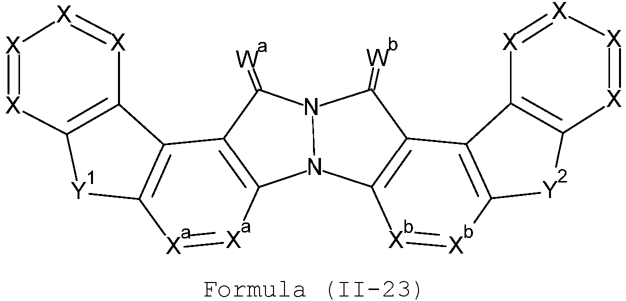 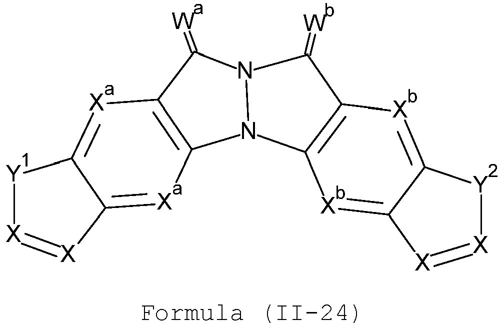 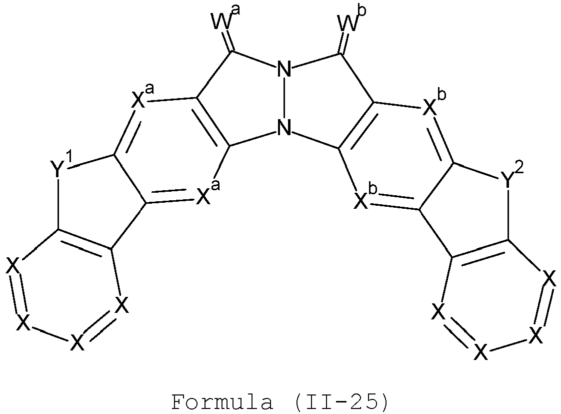 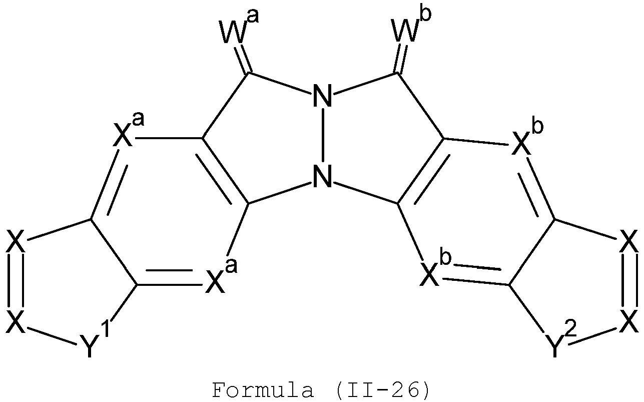 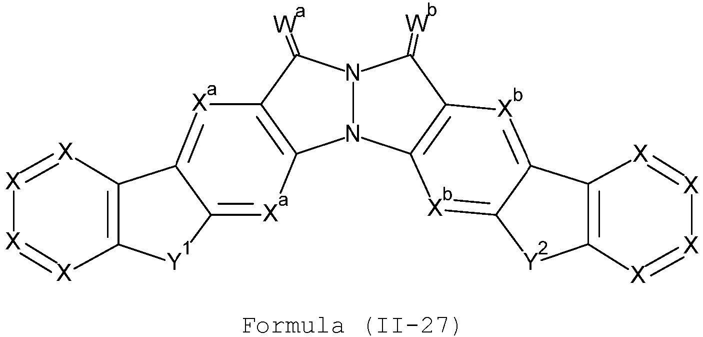 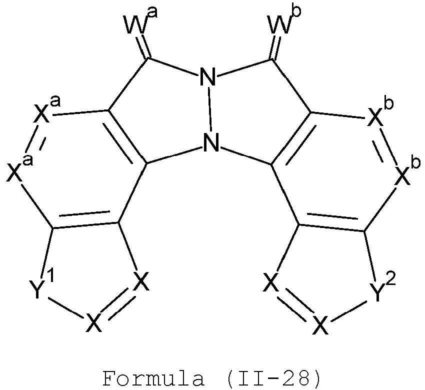 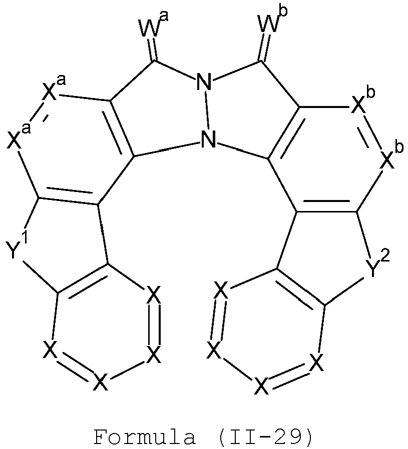 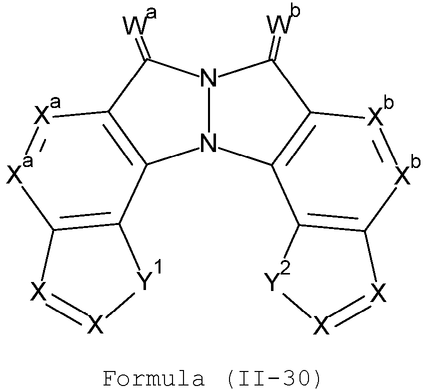 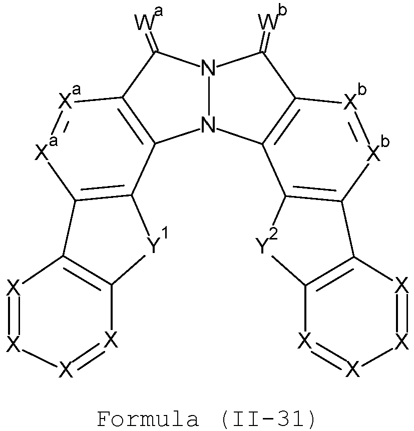 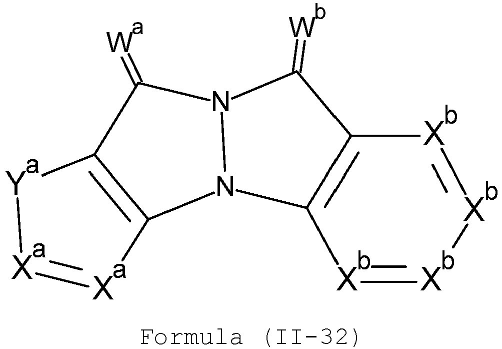 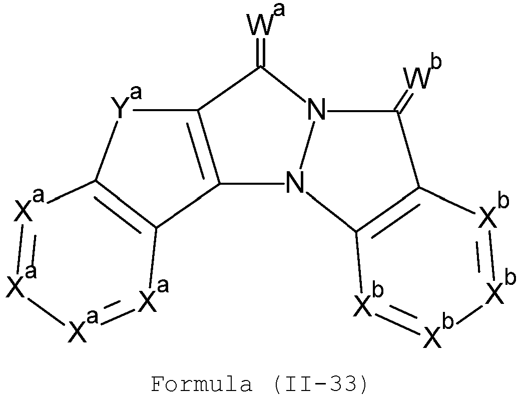 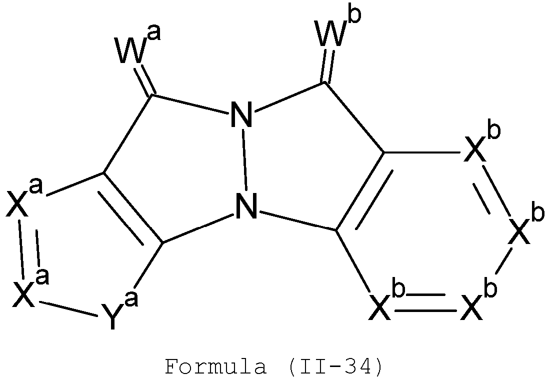 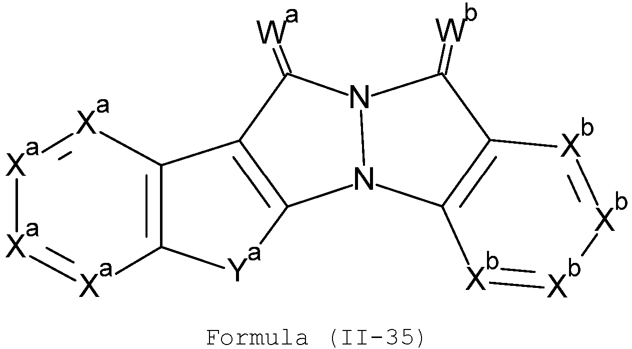 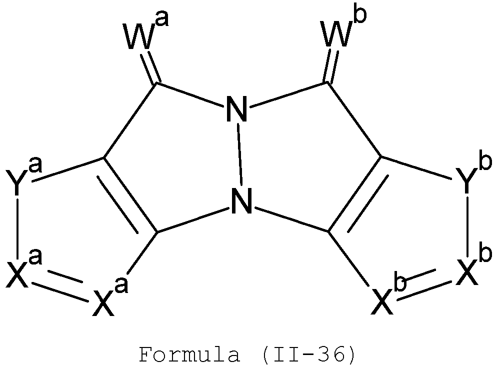 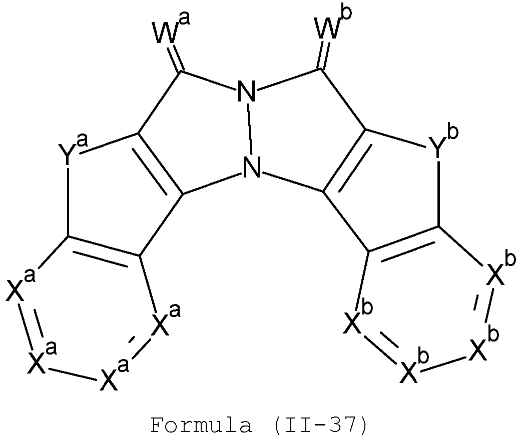 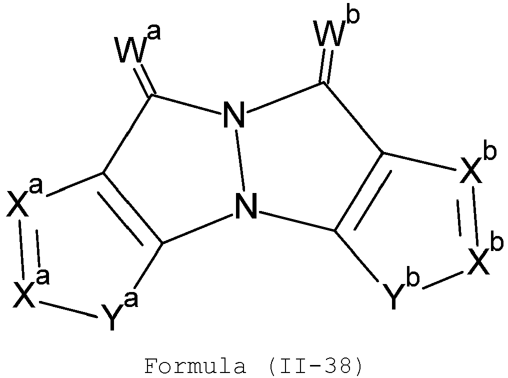 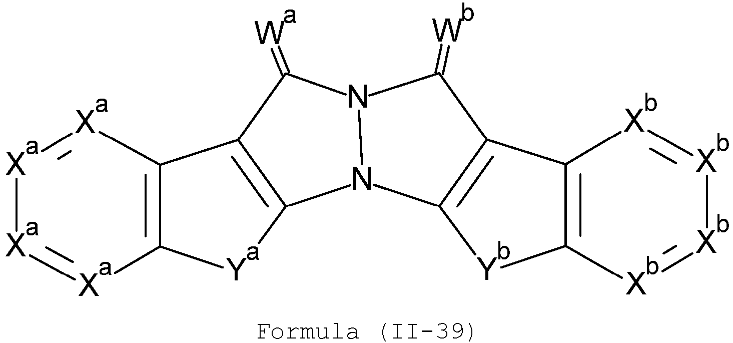 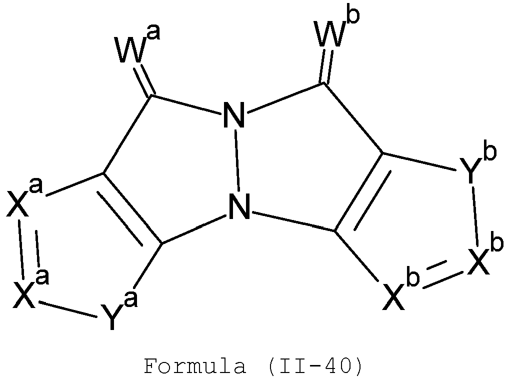 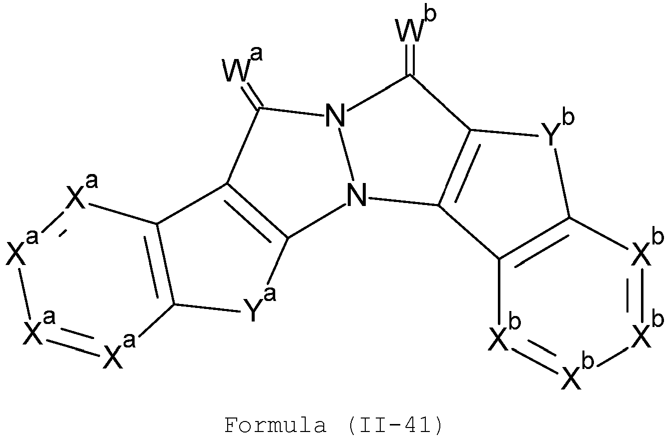 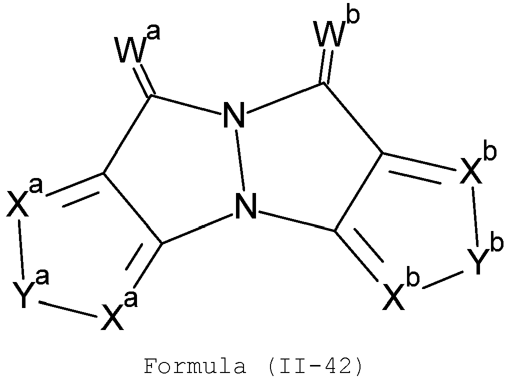 where the symbols W^{a} and W^{b} have the definitions given in Claim 1 and the further symbols are as follows:
Y^{a} is the same or different at each instance and is N(Ar), N(R^{a}), P(Ar), P(R^{a}), P(=O)Ar, P(=O)R^{a}, P(=S)Ar, P(=S)R^{a}, B(Ar), B(R^{a}), Al(Ar), Al(R^{a}), Ga(Ar), Ga(R^{a}), C=O, C(R^{a})₂, Si(R^{a})₂, Ge(R^{a})₂, C=NR^{a}, C=NAr, C=C(R^{a})₂, C=C(R^{a})(Ar), O, S, Se, S=O or SO₂, preferably N(Ar), N(R^{a}), B(Ar), B(R^{a}), P(=O)R^{a}, P(=O)Ar, C=O, C(R^{a})₂, C=C(R^{a})₂, C=C(R^{a})(Ar), Si(R^{a})₂, O, S, Se, S=O or SO₂, more preferably N(Ar), C(R^{a})₂, O or S, where R^{a} has the definition detailed in Claim 1;
Y^{b} is the same or different at each instance and is N(Ar), N(R^{b}), P(Ar), P(R^{b}), P(=O)Ar, P(=O)R^{b}, P(=S)Ar, P(=S)R^{b}, B(Ar), B(R^{b}), Al(Ar), Al(R^{a}), Ga(Ar), Ga(R^{b}), C=O, C(R^{b})₂, Si(R^{b})₂, Ge(R^{b})₂, C=NR^{b}, C=NAr, C=C(R^{b})₂, C=C(R^{b})(Ar), O, S, Se, S=O or SO₂, preferably N(Ar), N(R^{b}), B(Ar), B(R^{b}), P(=O)R^{b}, P(=O)Ar, C=O, C(R^{b})₂, C=C(R^{b})₂, C=C(R^{b})(Ar), Si(R^{b})₂, O, S, Se, S=O or SO₂, more preferably N(Ar), C(R^{b})₂, O or S, where R^{b} has the definition detailed in Claim 1;
Y¹, Y² is the same or different at each instance and is N(Ar), N(R), P(Ar), P(R), P(=O)Ar, P(=O)R, P(=S)Ar, P(=S)R, B(Ar), B(R), Al(Ar), Al(R), Ga(Ar), Ga(R), C=O, C(R)₂, Si(R)₂, Ge(R)₂, C=NR, C=NAr, C=C(R)₂, C=C(R)(Ar), O, S, Se, S=O or SO₂, preferably N(Ar), N(R), B(Ar), B(R), P(=O)R, P(=O)Ar, C=O, C(R)₂, C=C(R)₂, C=C (R) (Ar), Si(R)₂, O, S, Se, S=O or SO₂, more preferably N(Ar), C(R)₂, O or S, where R has the definition detailed in Claim 1;
X is the same or different at each instance and is N or CR, with the proviso that not more than two of the X groups in one cycle are N, where R has the definition detailed in Claim 1;
X^{a} is the same or different at each instance and is N or CR^{a}, with the proviso that not more than two of the X^{a} groups in one cycle are N, where R² has the definition detailed in Claim 1;
X^{b} is the same or different at each instance and is N or CR^{b}, with the proviso that not more than two of the X^{b} groups in one cycle are N, where R^{b} has the definition detailed in Claim 1.

3. Compound according to Claim 1 or 2, including at least one structure of the formulae (III-1) to (III-41): 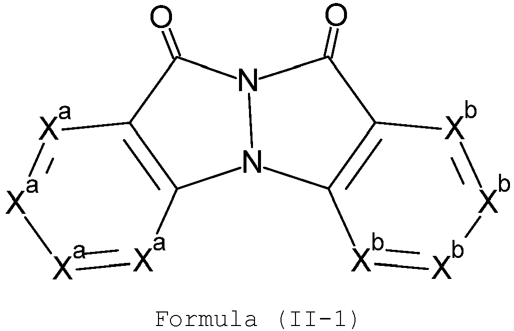 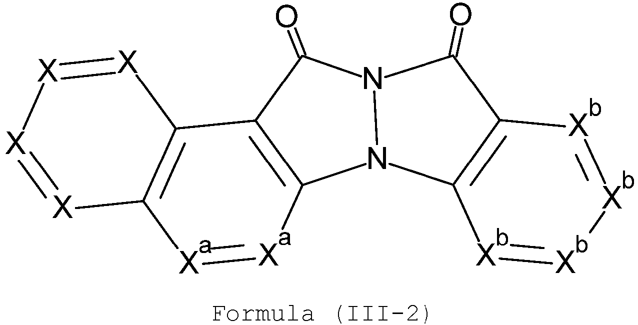 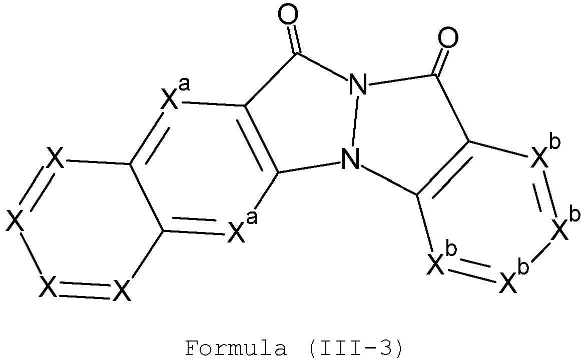 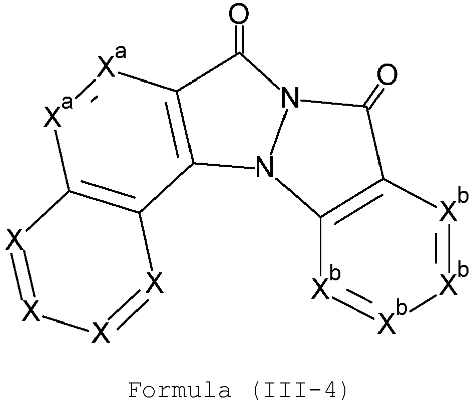 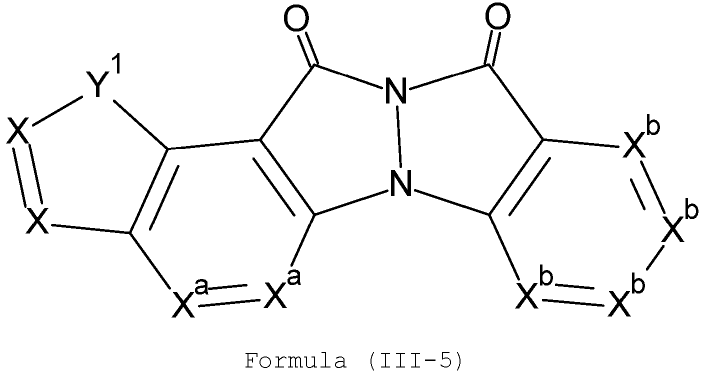 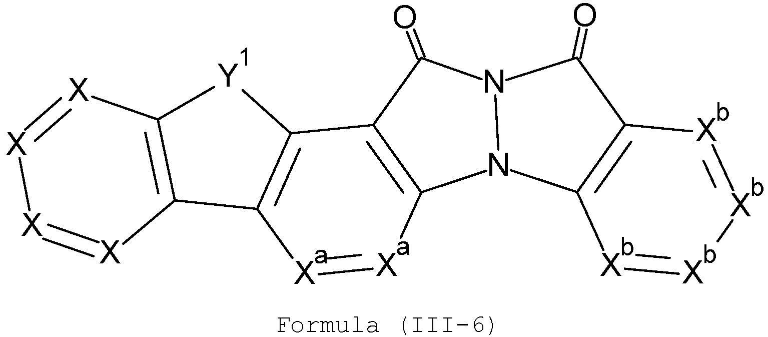 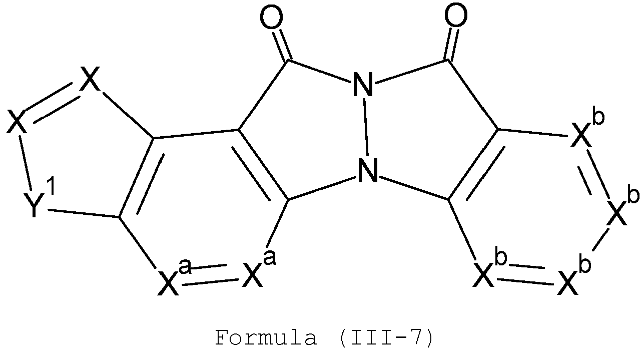 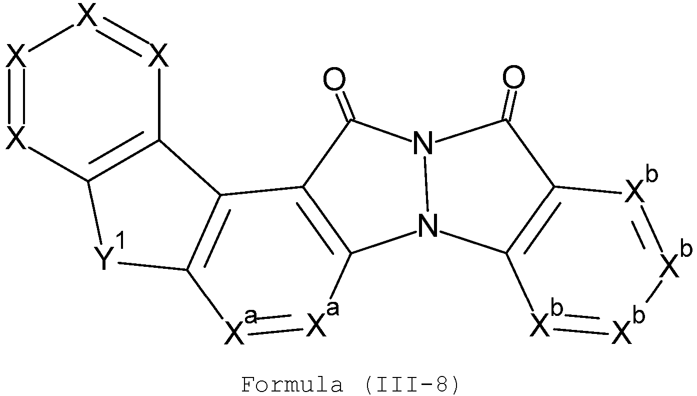 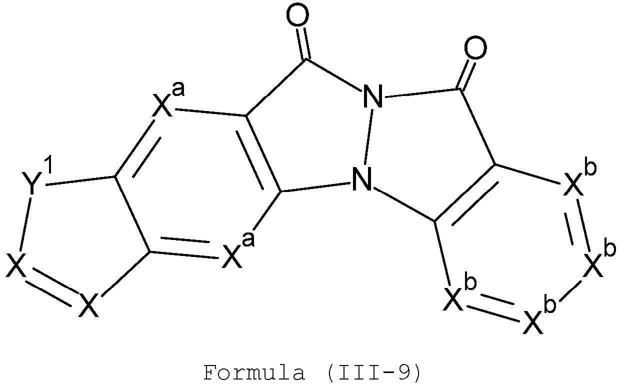 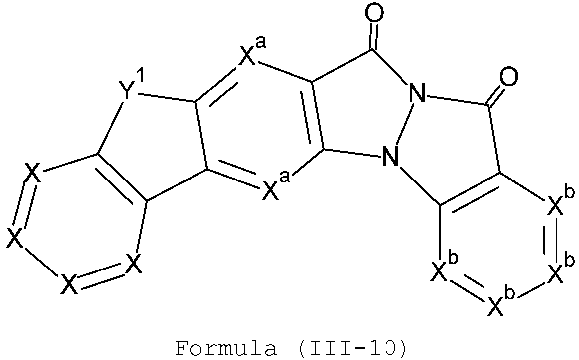 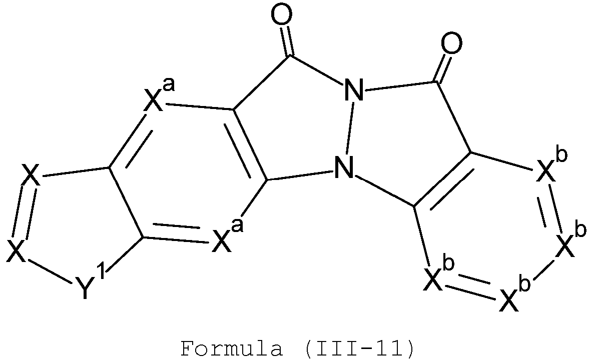 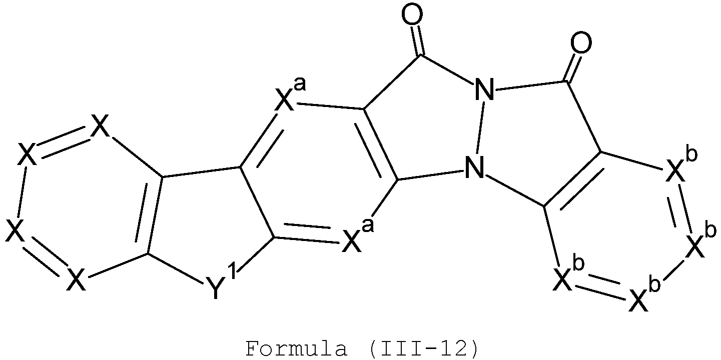 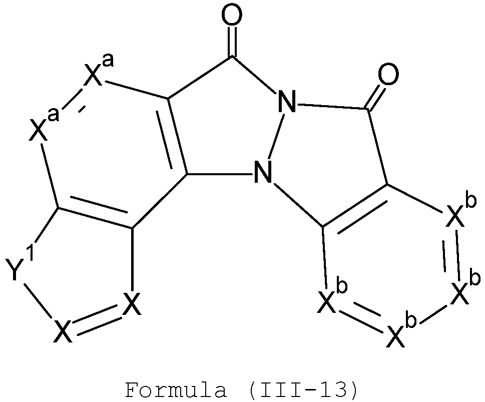 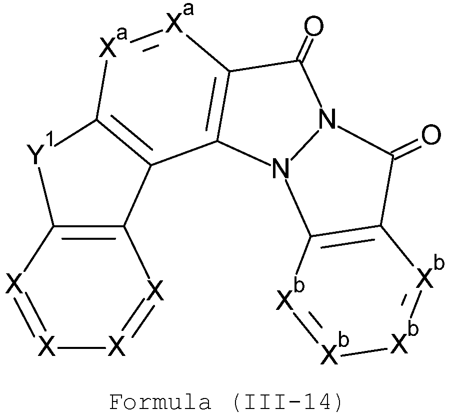 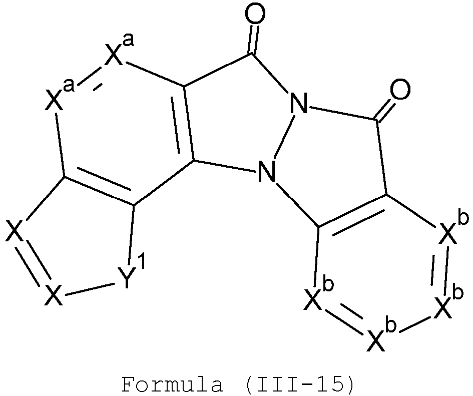 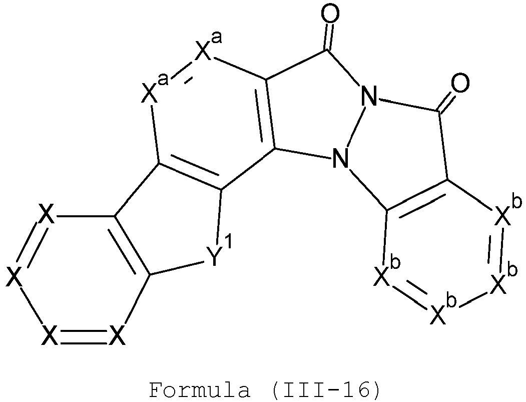 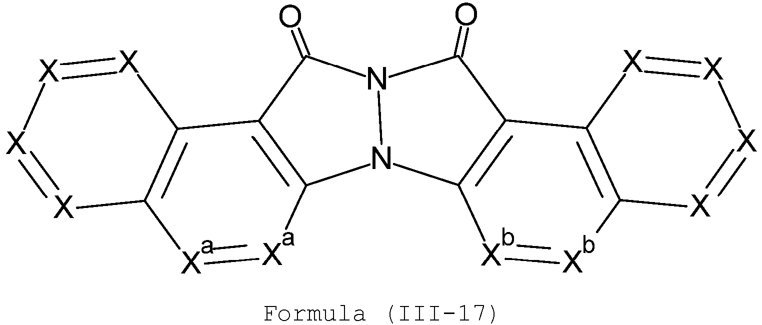 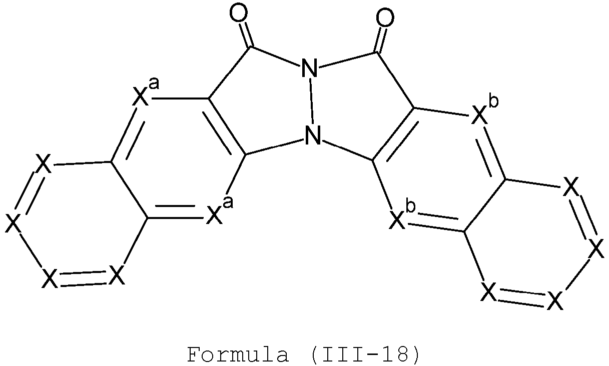 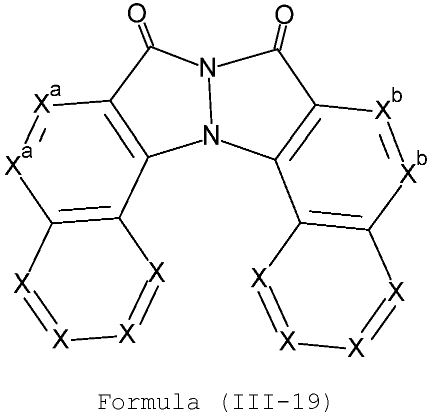 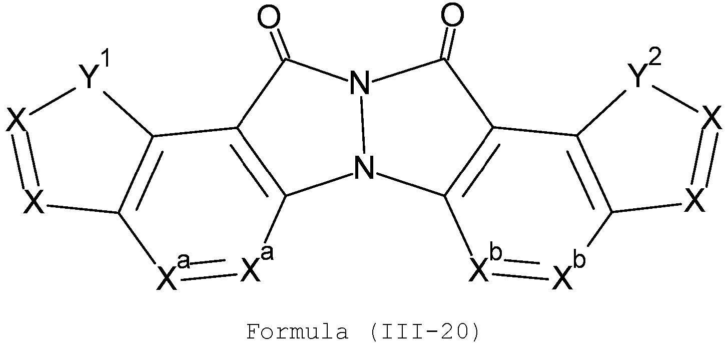 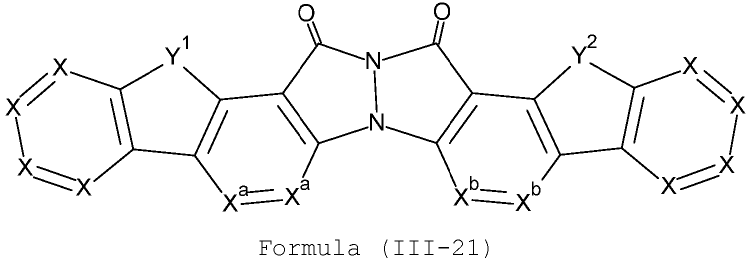 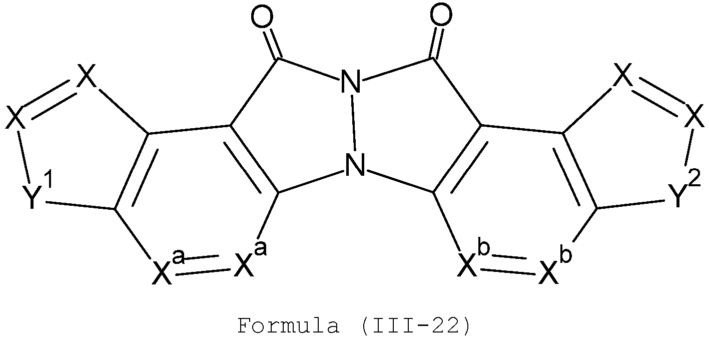 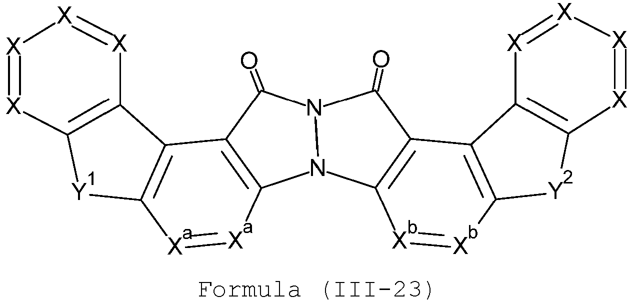 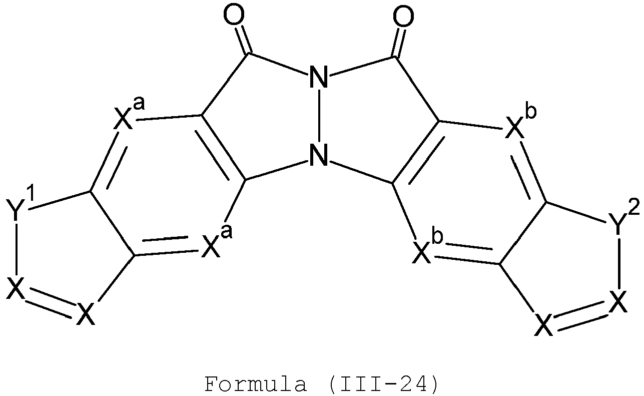 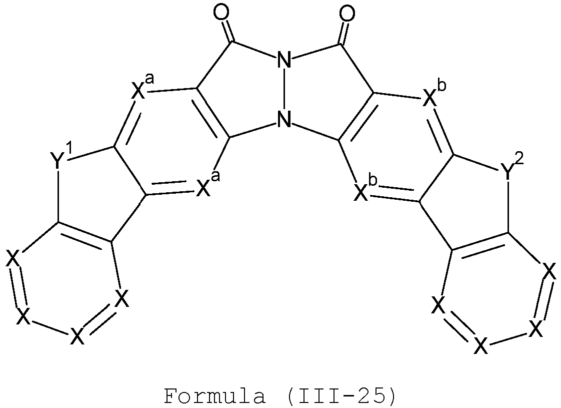 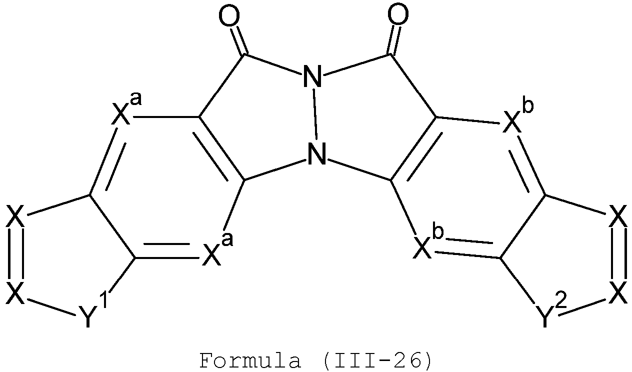 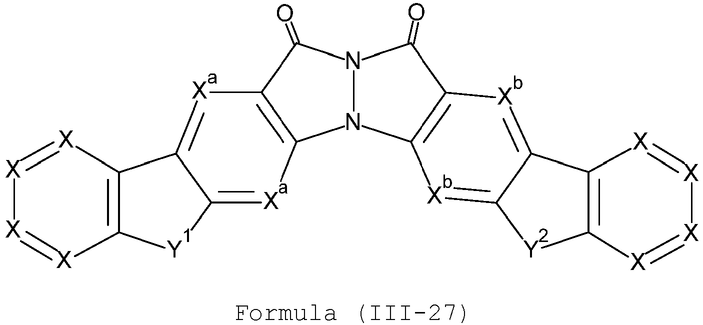 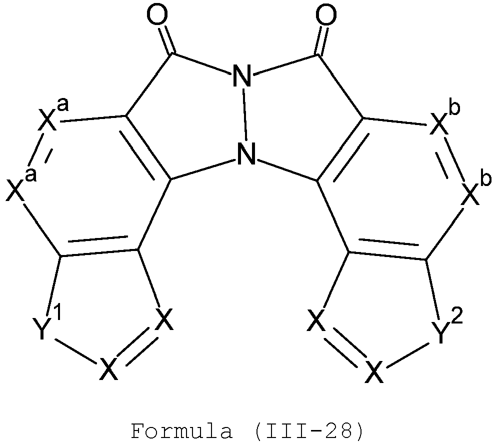 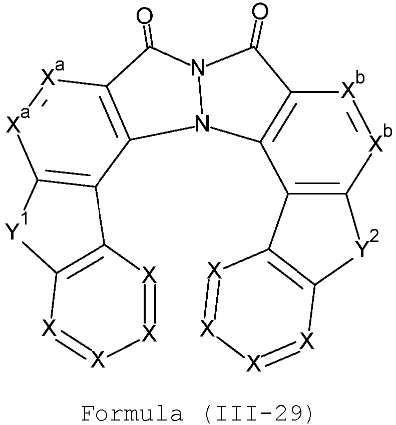 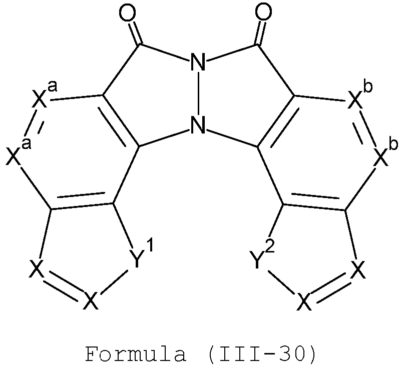 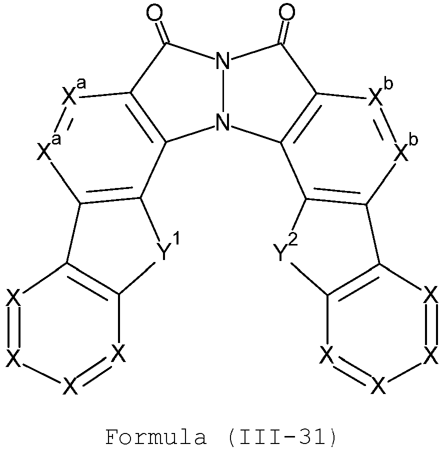 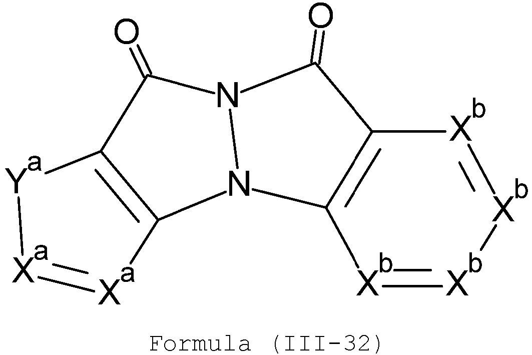 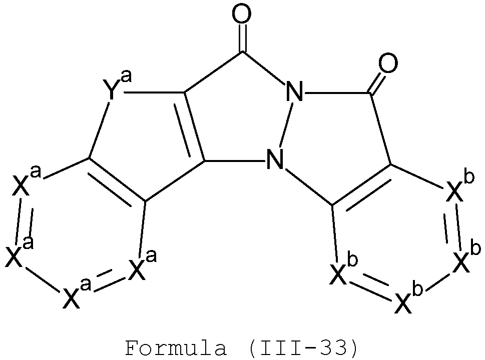 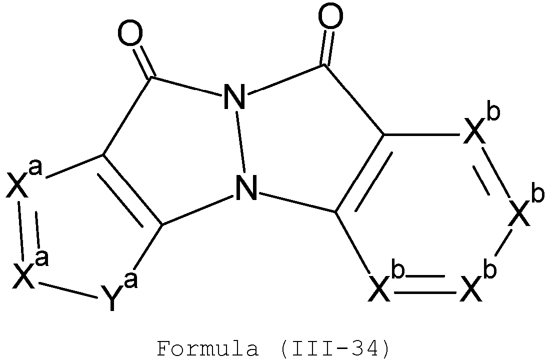 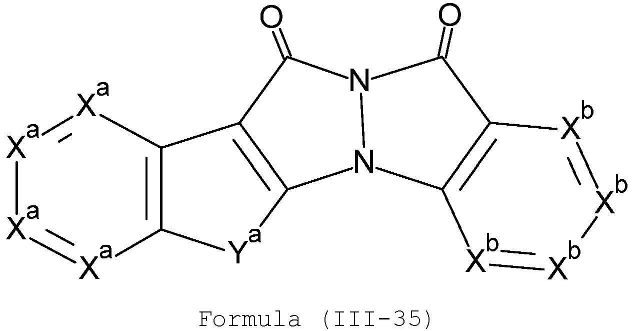 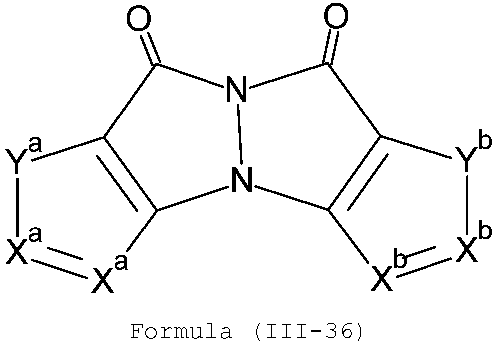 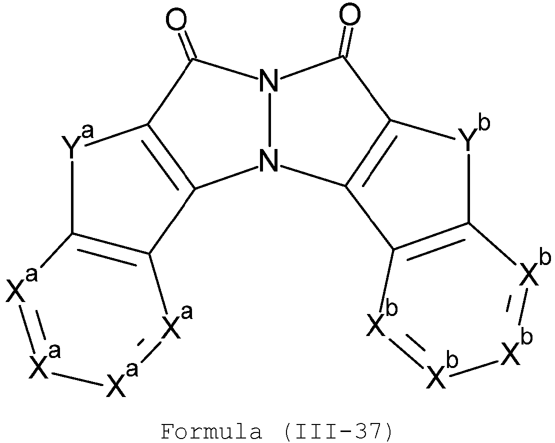 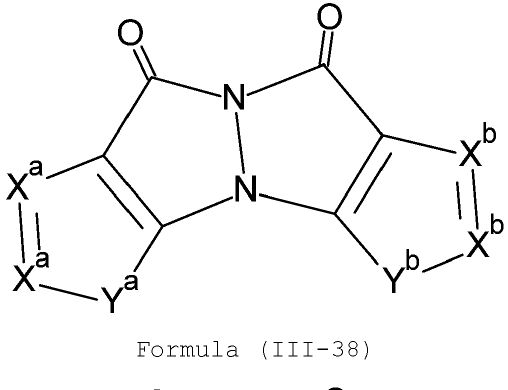 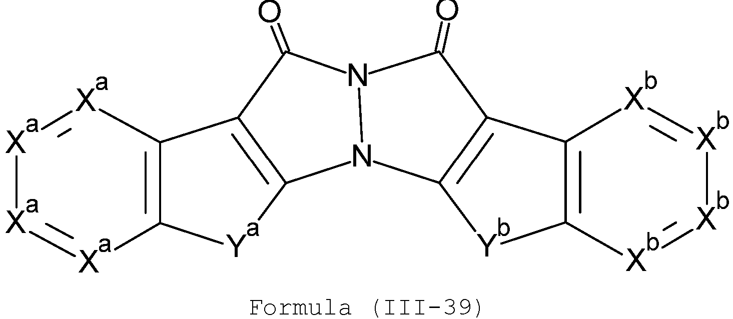 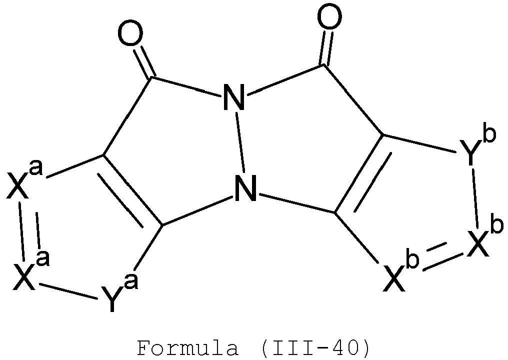 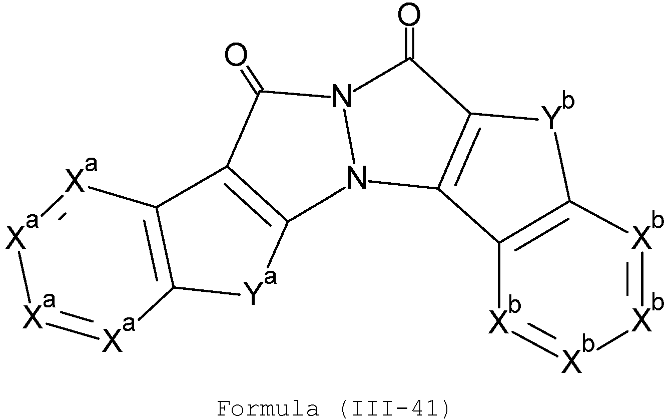 where the symbols Y¹, Y², Y^{a}, Y^{b}, X, X^{a} and X^{b} have the definitions given in Claim 2.

4. Compound according to one or more of Claims 1 to 3, including at least one structure of the formulae (IV-1) to (IV-41): 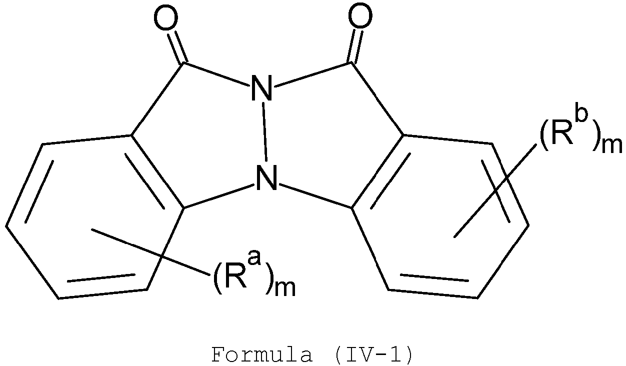 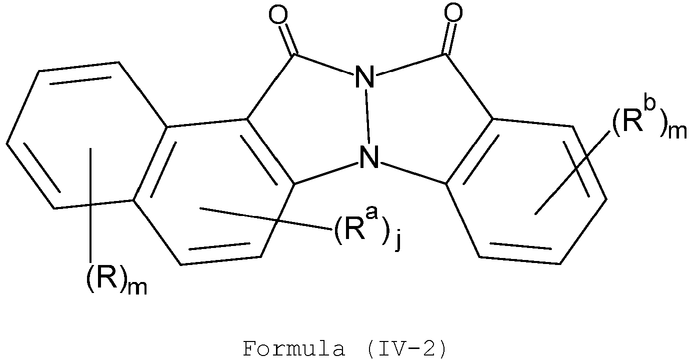 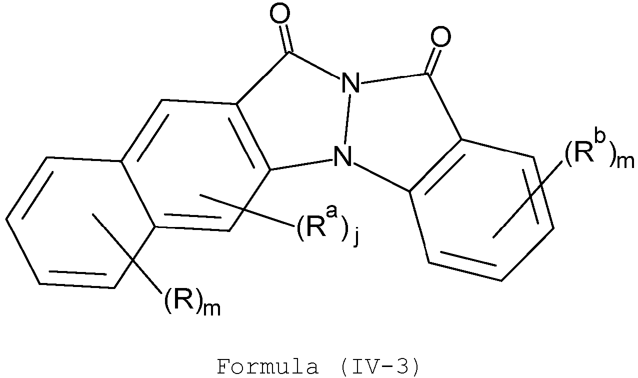 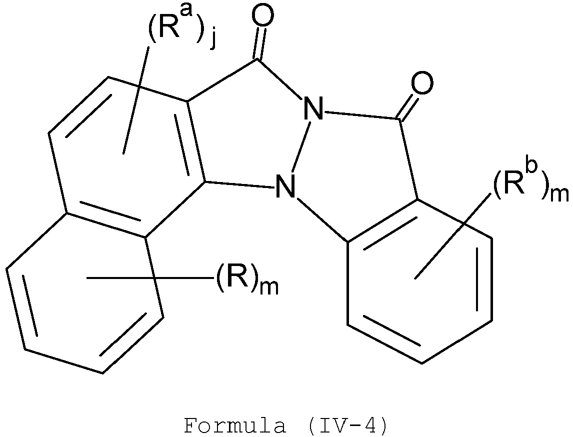 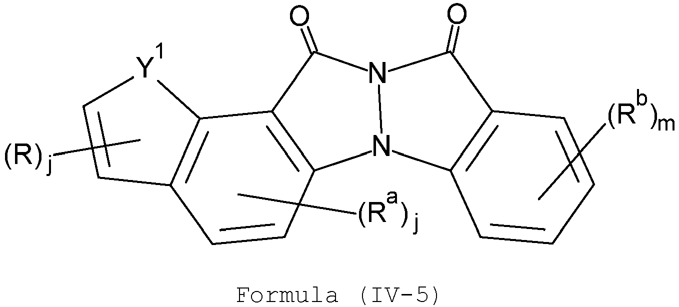 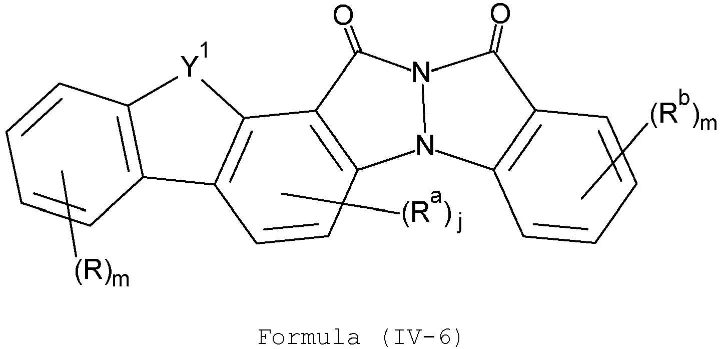 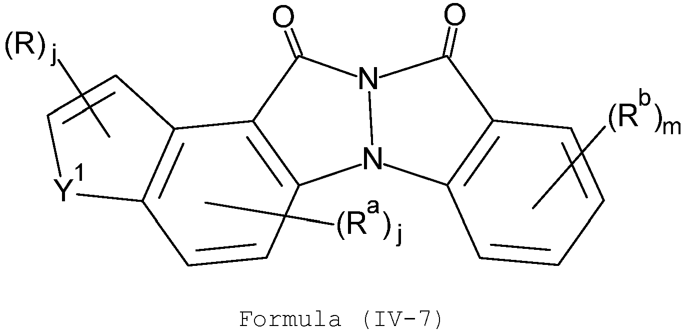 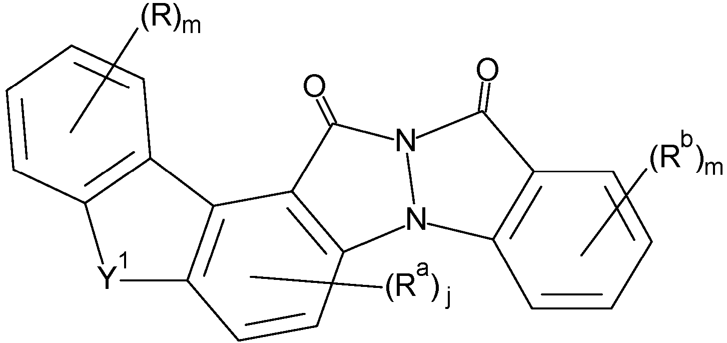 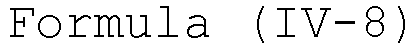 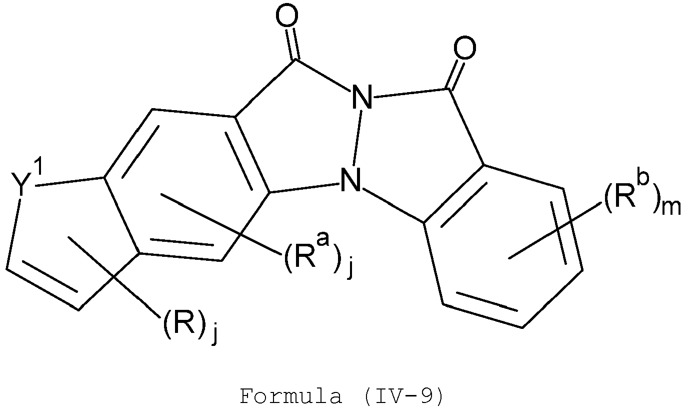 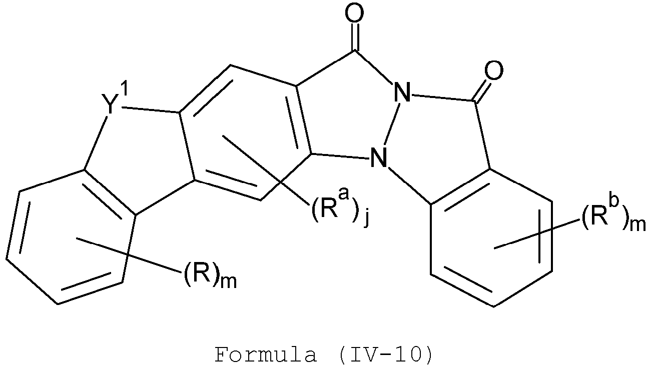 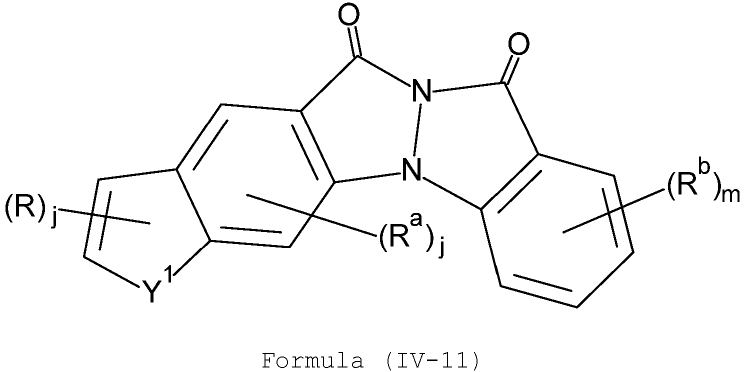 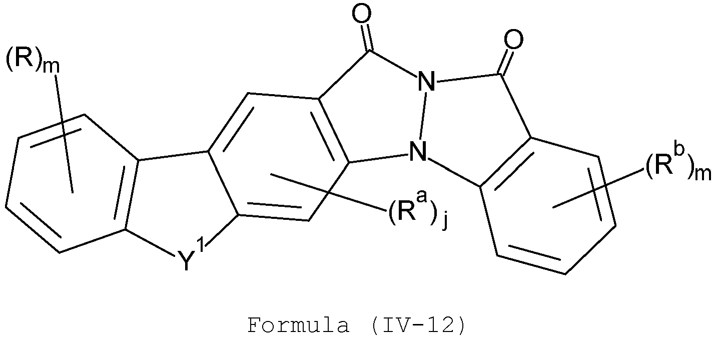 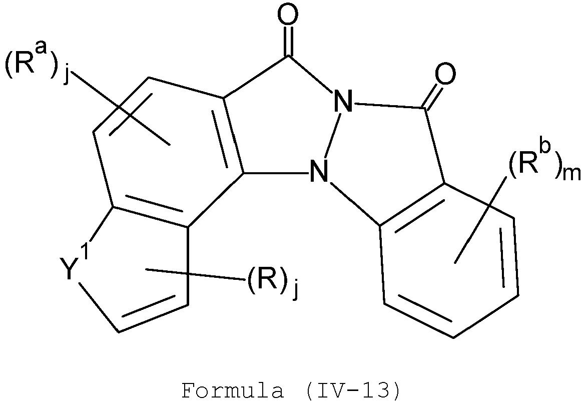 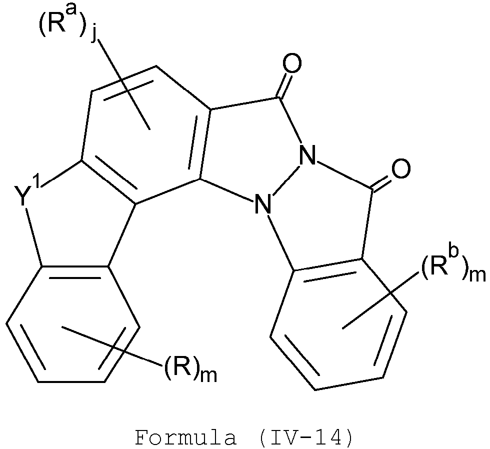 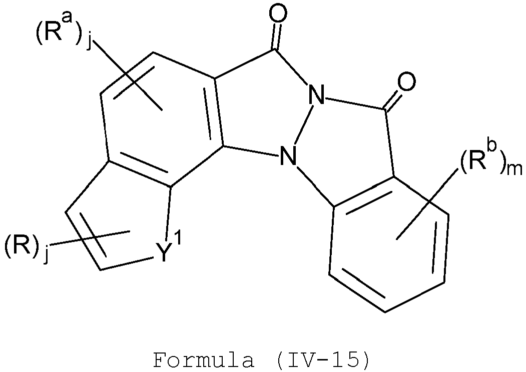 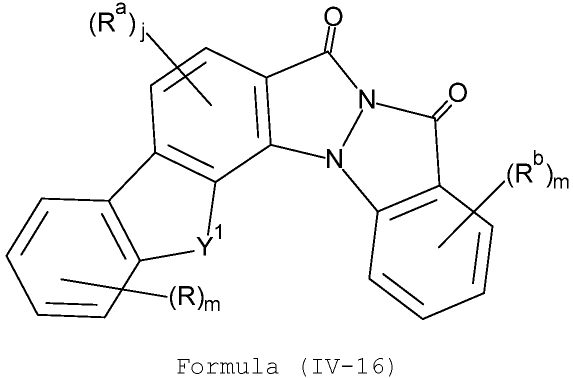 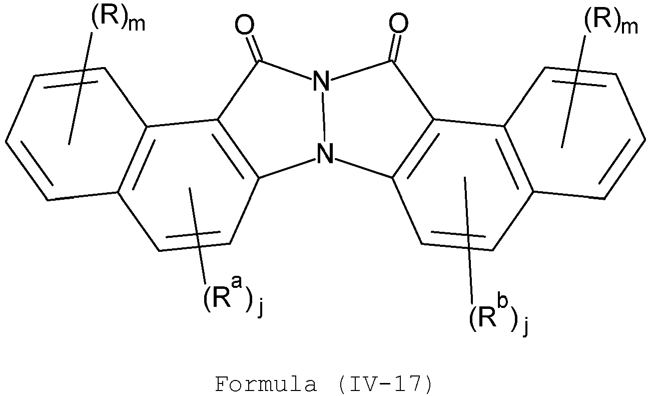 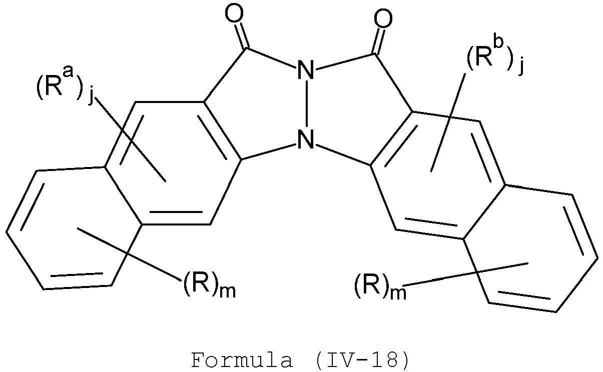 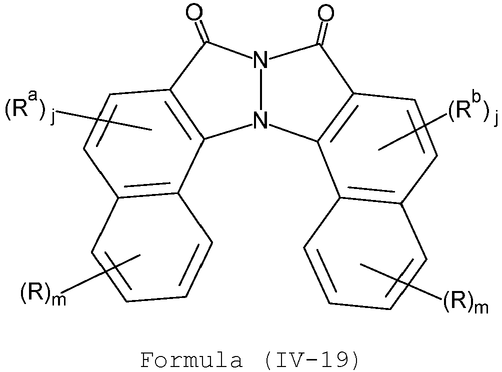 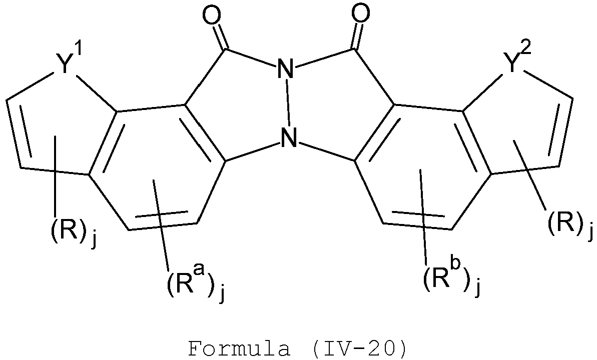 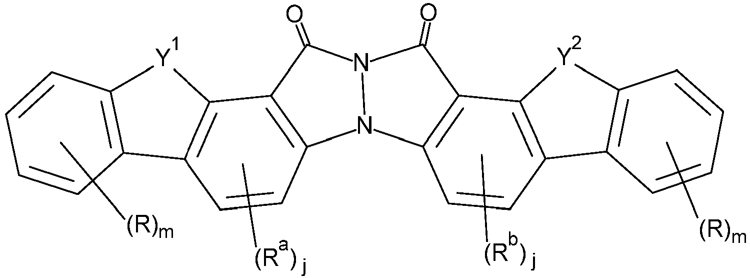 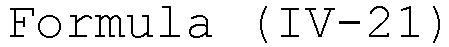 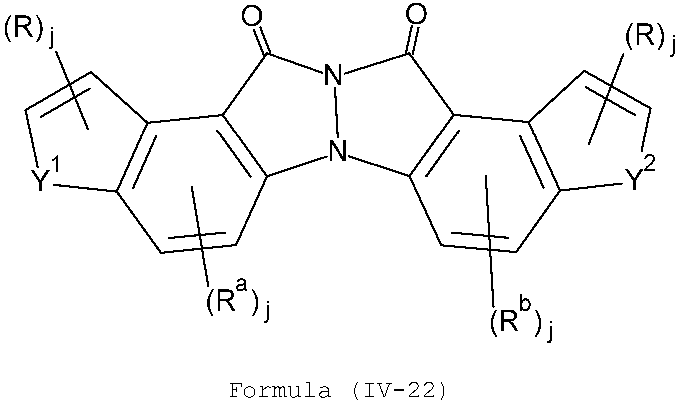 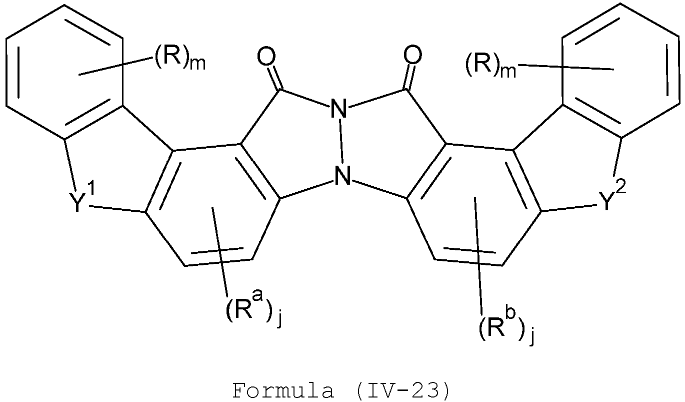 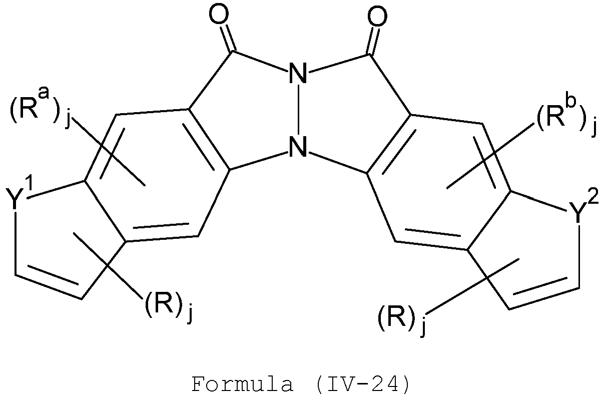 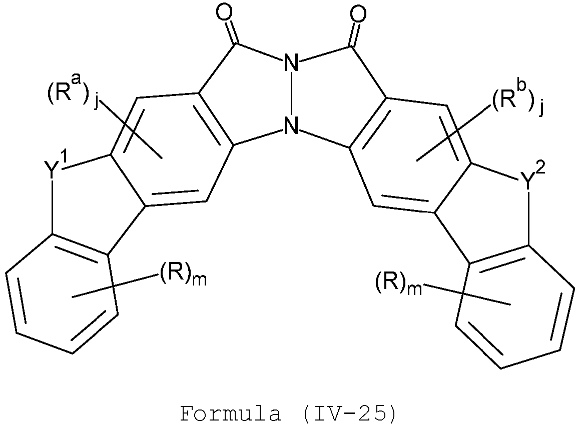 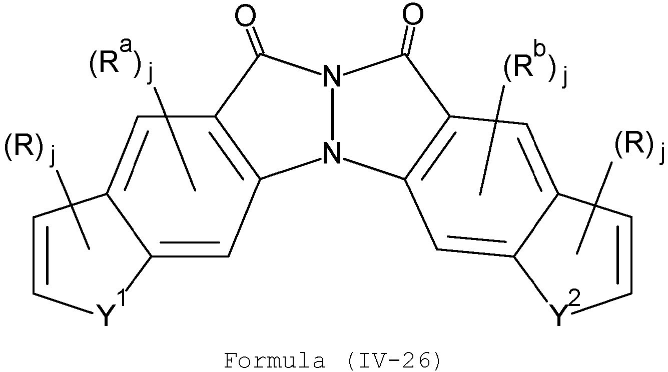 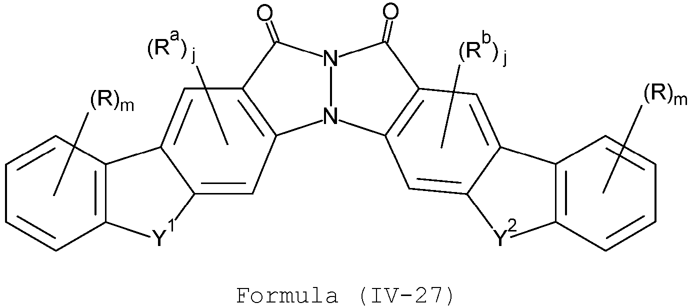 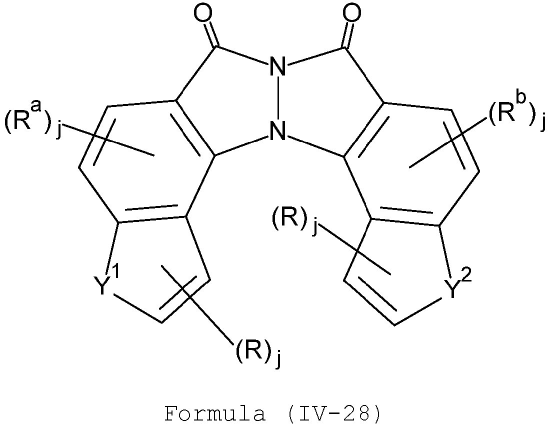 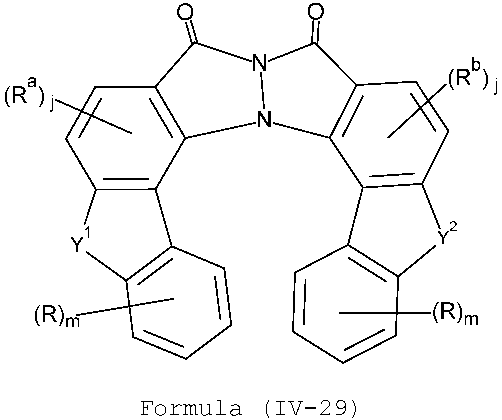 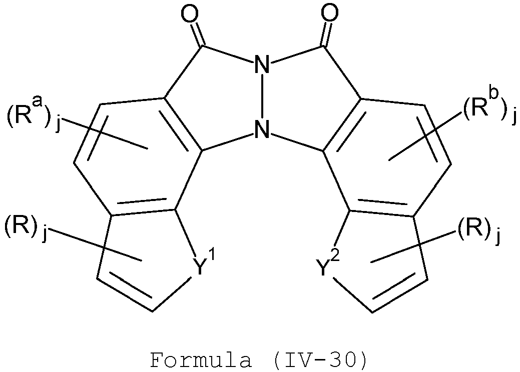 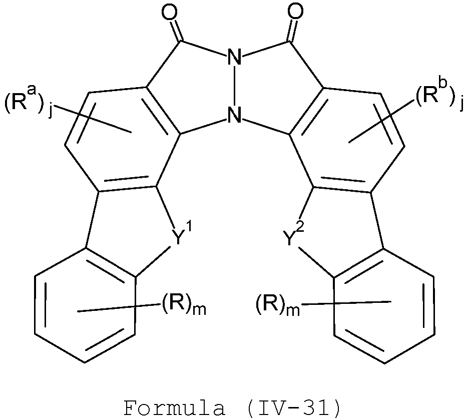 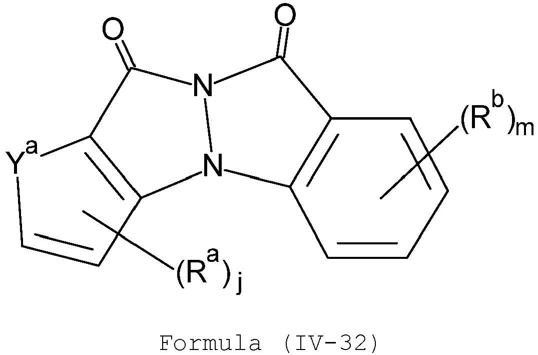 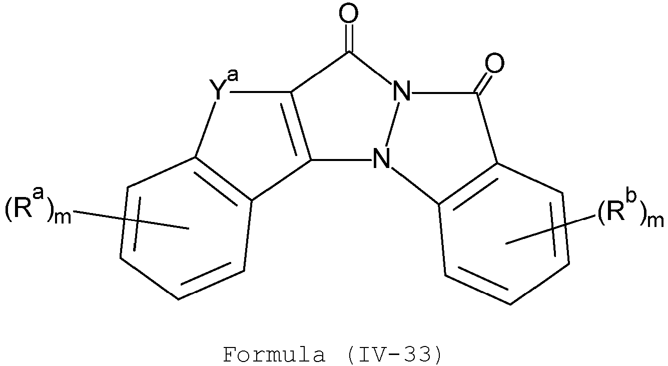 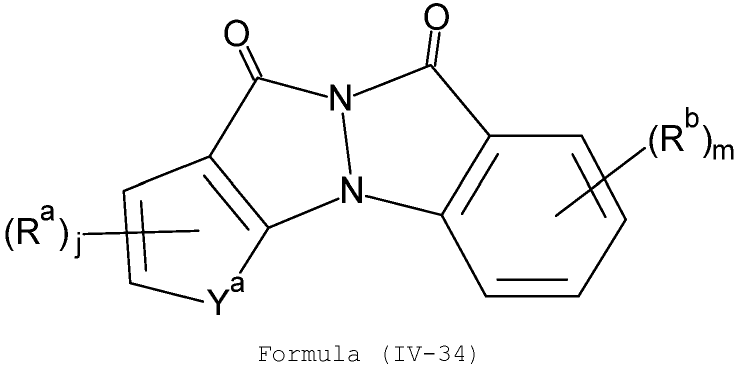 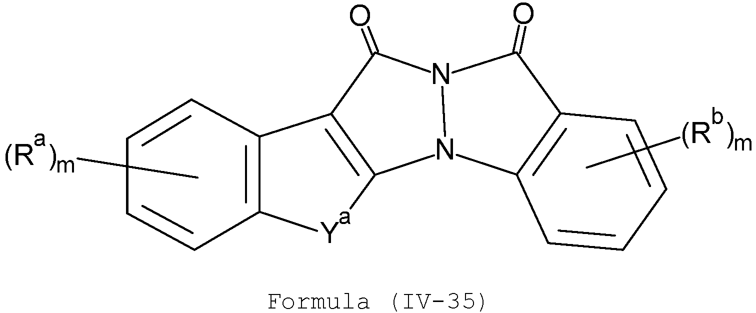 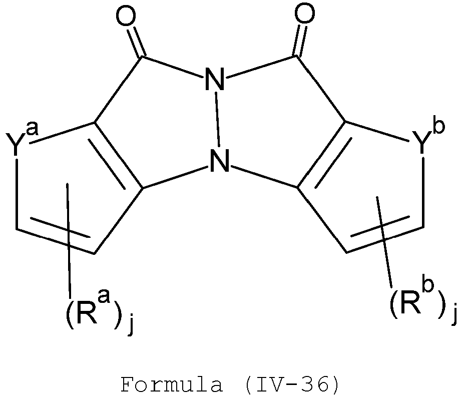 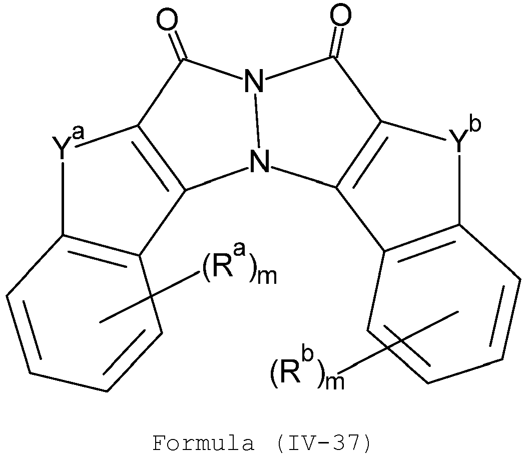 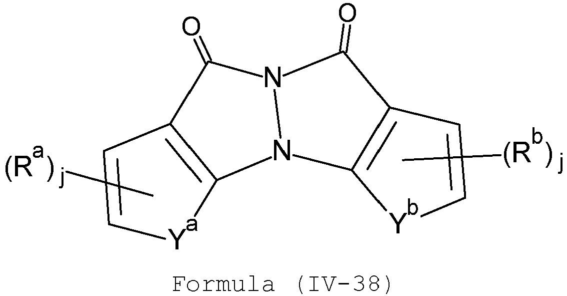 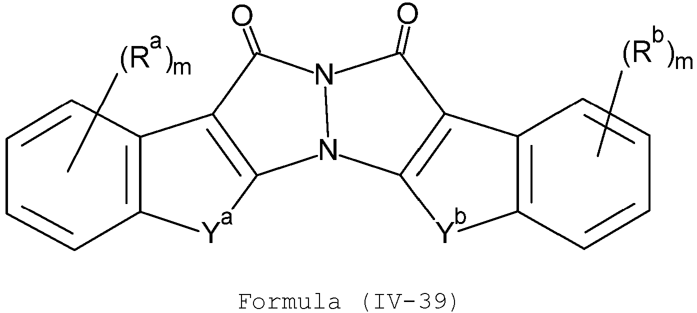 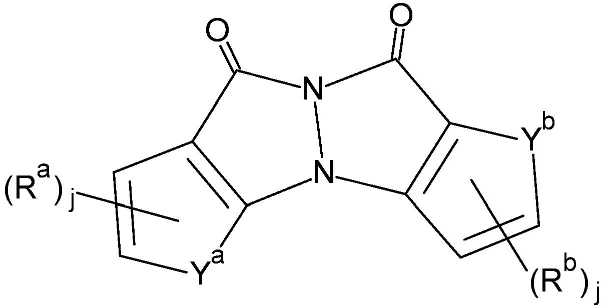 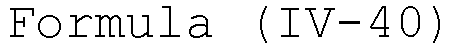 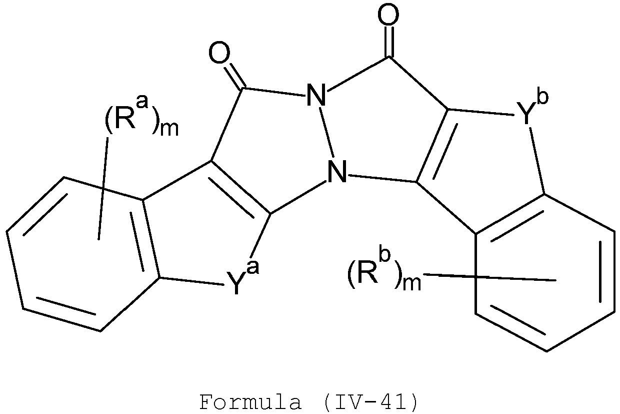 where the symbols R, R^{a} and R^{b} have the definitions given in Claim 1, the symbols Y¹, Y², Y^{a} and Y^{b} have the definitions given in Claim 2, and the further symbols have the following definition:
m is 0, 1, 2, 3 or 4;
j is 0, 1 or 2.

5. Compound according to one or more of Claims 1 to 4, including at least one structure of the formulae (V-1) to (V-8): 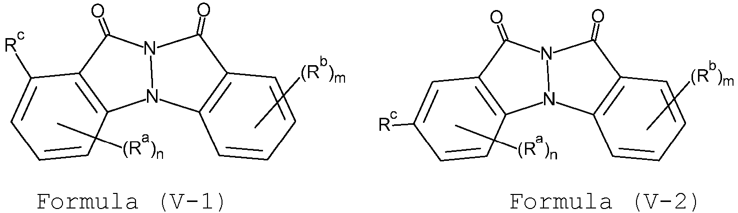 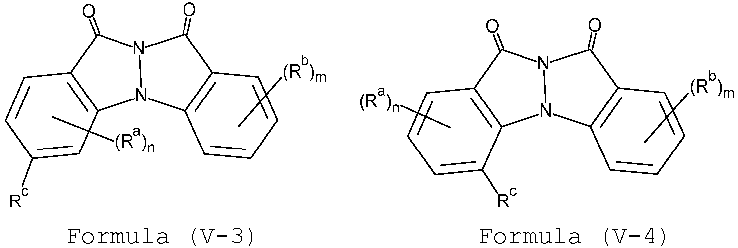 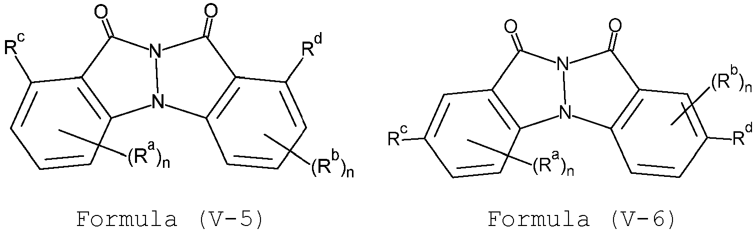 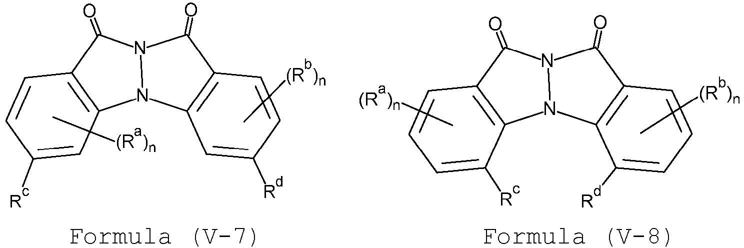 where the symbols R^{a} and R^{b} have the definitions given in Claim 1 and the further symbols have the following definition:
R^{c}, R^{d} is the same or different at each instance and is N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P (=O) (Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, a straight-chain alkyl, alkoxy or thioalkoxy group having 1 to 40 carbon atoms or an alkenyl or alkynyl group having 2 to 40 carbon atoms or a branched or cyclic alkyl, alkoxy or thioalkoxy group having 3 to 20 carbon atoms, where the alkyl, alkoxy, thioalkoxy, alkenyl or alkynyl group may in each case be substituted by one or more R¹ radicals, where one or more nonadjacent CH₂ groups may be replaced by R¹C=CR¹, C≡C, Si(R¹)₂, C=O, C=S, C=Se, C=NR¹, -C(=O)O-, -C(=O)NR¹-, NR¹, P(=O)(R¹), -O-, -S-, SO or SO₂, or an aromatic or heteroaromatic ring system which has 5 to 60 aromatic ring atoms and may be substituted in each case by one or more R¹ radicals, or an aryloxy or heteroaryloxy group which has 5 to 60 aromatic ring atoms and may be substituted by one or more R¹ radicals; or heteroarylthio group which has 5 to 60 aromatic ring atoms and may be substituted by one or more R¹ radicals, or a diarylamino, arylheteroarylamino, diheteroarylamino group which has 5 to 60 aromatic ring atoms and may be substituted by one or more R¹ radicals, or an arylalkyl or heteroarylalkyl group which has 5 to 60 aromatic ring atoms and 1 to 10 carbon atoms in the alkyl radical and may be substituted by one or more R¹ radicals; at the same time, one R^{c} radical may form a ring system with an R, R^{a}, R^{b}, R^{d} radical or a further group; at the same time, an R^{d} radical may form a ring system with an R, R^{a}, R^{b}, R^{c} radical or a further group;
m is 0, 1, 2, 3 or 4;
n is 0, 1, 2 or 3.

6. Compound according to one or more of Claims 1 to 5, **characterized in that** at least one R, R^{a}, R^{b}, R^{c}, R^{d} radical is a straight-chain alkyl, alkoxy or thioalkoxy group having 1 to 40 carbon atoms or an alkenyl or alkynyl group having 2 to 40 carbon atoms or a branched or cyclic alkyl, alkoxy or thioalkoxy group having 3 to 20 carbon atoms, where the alkyl, alkoxy, thioalkoxy, alkenyl or alkynyl group may be substituted in each case by one or more R¹ radicals, where one or more nonadjacent CH₂ groups may be replaced by R¹C=CR¹, C≡C, Si(R¹)₂, C=O, C=S, C=Se, C=NR¹, -C(=O)O-, -C(=O)NR¹-, NR¹, P(=O)(R¹), -O-, -S-, SO or SO₂, or an aromatic or heteroaromatic ring system which has 5 to 60 aromatic ring atoms and may be substituted in each case by one or more R¹ radicals.

7. Compound according to one or more of Claims 1 to 6, **characterized in that** at least two R, R^{a}, R^{b}, R^{c}, R^{d} radicals together with the further groups to which the two R, R^{a}, R^{b}, R^{c}, R^{d} radicals bind form a fused ring, where the two R, R^{a}, R^{b}, R^{c}, R^{d} radicals form at least one structure of the formulae (RA-1) to (RA-12): 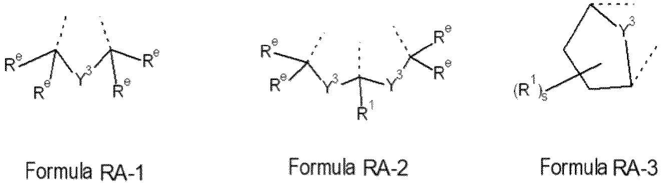 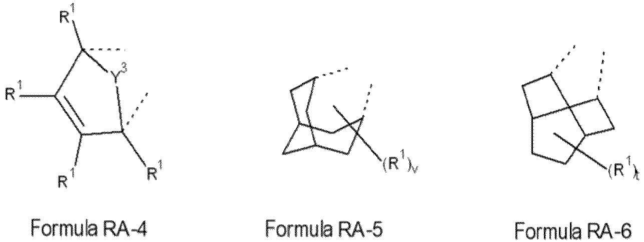 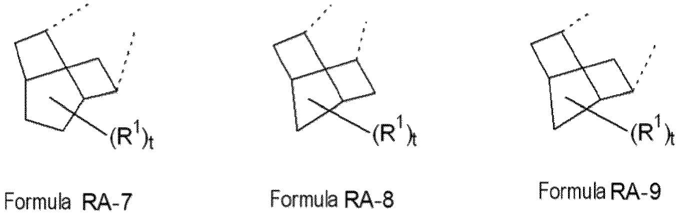 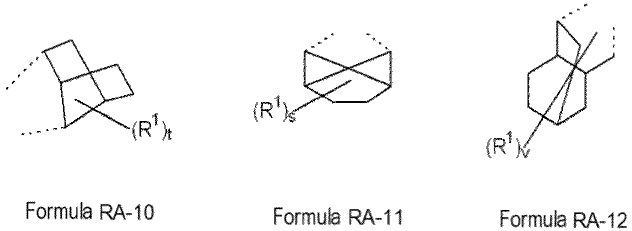 where R¹ has the definition detailed above, the dotted bonds represent the sites of attachment via which the two R, R^{a}, R^{b}, R^{c}, R^{d} radicals bind to the further groups, and the further symbols have the following definition:
Y³ is the same or different at each instance and is C(R¹)₂, (R¹)₂C-C(R¹)₂, (R¹)C=C(R¹), NR¹, NAr', O or S;
R^{e} is the same or different at each instance and is F, a straight-chain alkyl, alkoxy or thioalkoxy group having 1 to 40 carbon atoms or an alkenyl or alkynyl group having 2 to 40 carbon atoms or a branched or cyclic alkyl, alkoxy or thioalkoxy group having 3 to 20 carbon atoms, where the alkyl, alkoxy, thioalkoxy, alkenyl or alkynyl group may in each case be substituted by one or more R² radicals, where one or more nonadjacent CH₂ groups may be replaced by R²C=CR², C≡C, Si(R²)₂, C=O, C=S, C=Se, C=NR², -C(=O)O-, -C(=O)NR²-, NR², P(=O)(R¹), -O-, -S-, SO or SO₂, or an aromatic or heteroaromatic ring system which has 5 to 60 aromatic ring atoms and may be substituted in each case by one or more R² radicals, or an aryloxy or heteroaryloxy group which has 5 to 60 aromatic ring atoms and may be substituted by one or more R² radicals; at the same time, two R^{e} radicals together or one R^{e} radical together with an R¹ radical or with a further group may also form a ring system;
s is 0, 1, 2, 3, 4, 5 or 6;
t is 0, 1, 2, 3, 4, 5, 6, 7 or 8;
v is 0, 1, 2, 3, 4, 5, 6, 7, 8 or 9.

8. Compound according to one or more of Claims 1 to 7, **characterized in that** at least two R, R^{a}, R^{b}, R^{c}, R^{d} radicals together with the further groups to which the two R, R^{a}, R^{b}, R^{c}, R^{d} radicals bind form a fused ring, where the two R, R^{a}, R^{b}, R^{c}, R^{d} radicals form structures of the formula (RB): 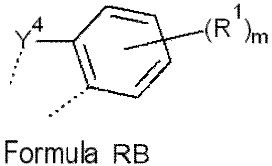 where R¹ has the definition detailed in Claim 1, the dotted bonds represent the sites of attachment via which the two R, R^{a}, R^{b}, R^{c}, R^{d} radicals bind to the further groups, the index m is 0, 1, 2, 3 or 4 and Y⁴ is C(R¹)₂, NR¹, NAr', BR¹, BAr', O or S.

9. Compound according to one or more of Claims 1 to 8, comprising at least one structure of the formulae (VI-1) to (VI-22), where the compounds have at least one fused ring, 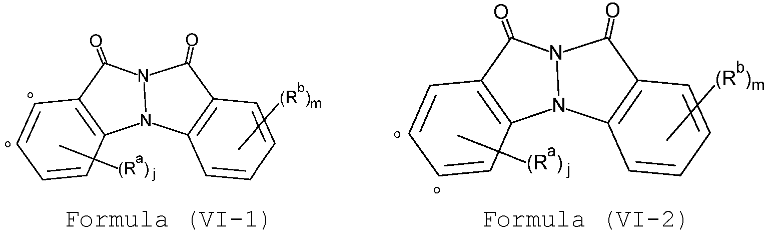 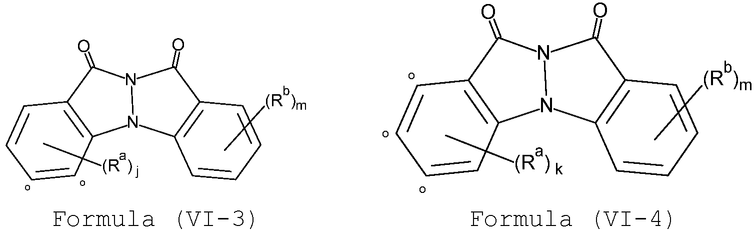 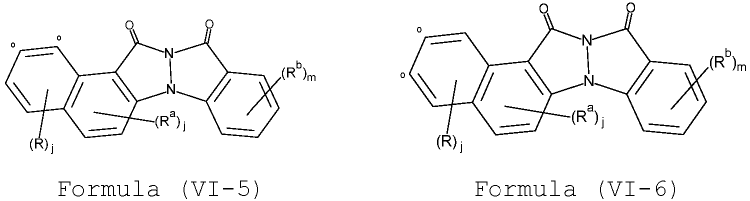 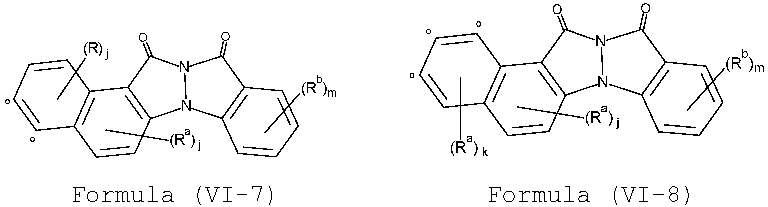 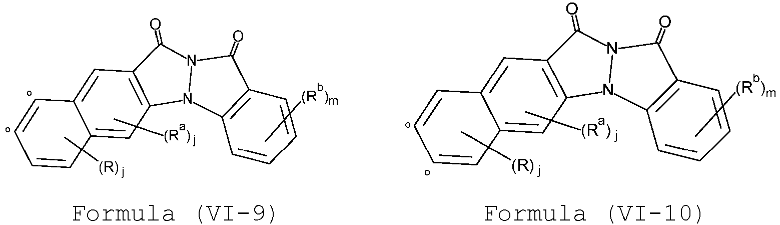 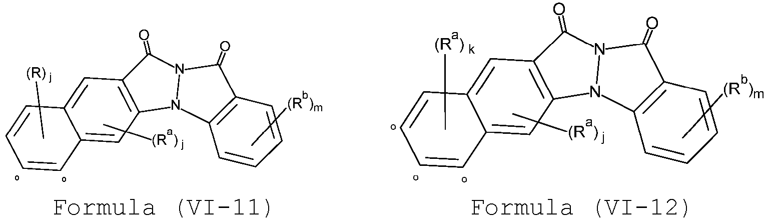 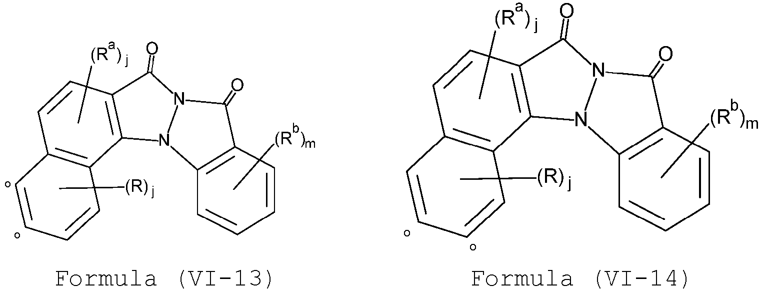 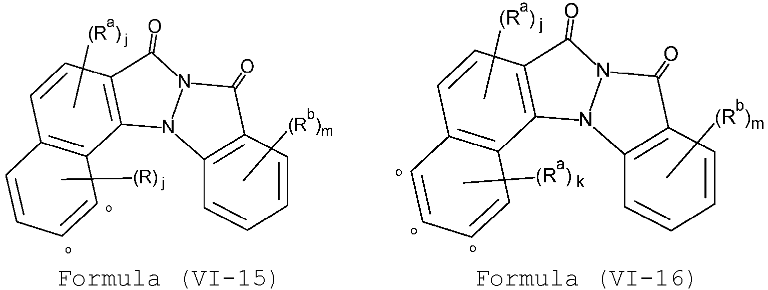 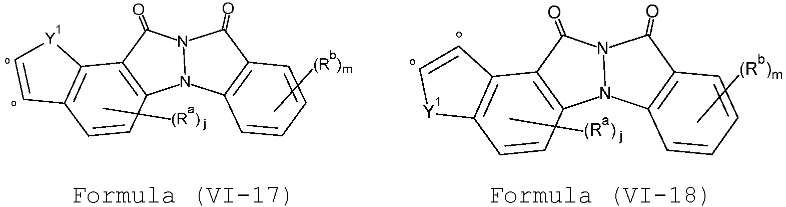 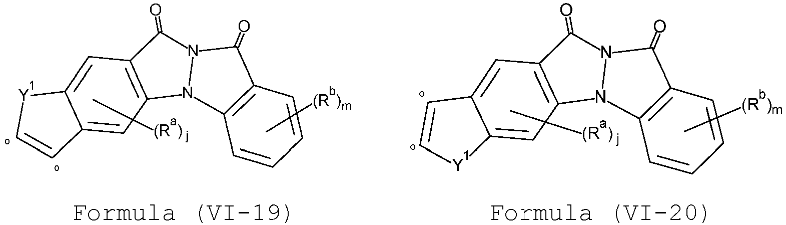 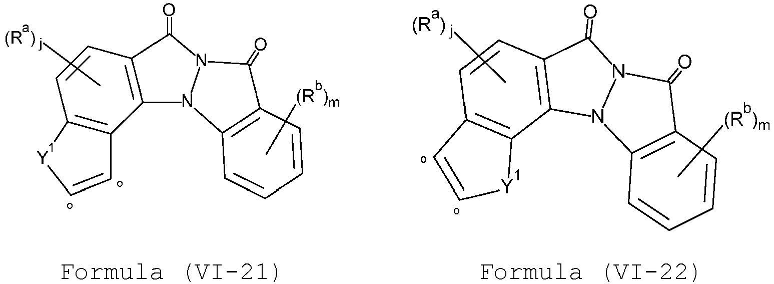 where the symbols R, R^{a}, R^{b}, Y¹ and Y² have the definitions given in Claim 1 or Claim 2, the symbol o represents the sites of attachment, and the further symbols have the following definition:
m is 0, 1, 2, 3 or 4;
n is 0, 1, 2 or 3;
j is 0, 1 or 2.

10. Compound according to one or more of Claims 1 to 9, **characterized in that** at least one substituent R, R^{a}, R^{b} is the same or different at each instance and is selected from the group consisting of H, D, a branched or cyclic alkyl, alkoxy or thioalkoxy group having 3 to 20 carbon atoms or an aromatic or heteroaromatic ring system selected from the groups of the following formulae Ar-1 to Ar-78: 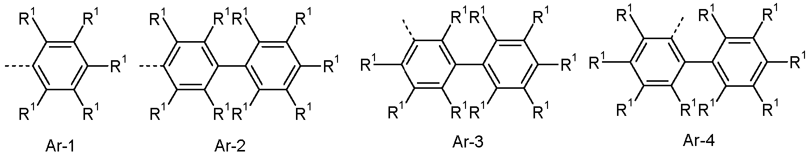 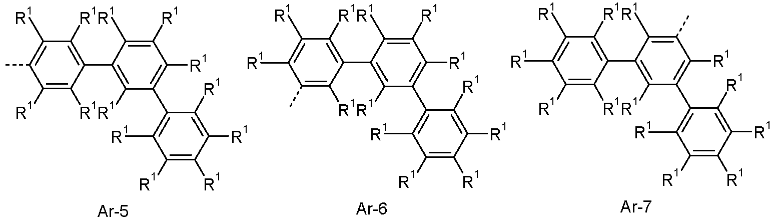 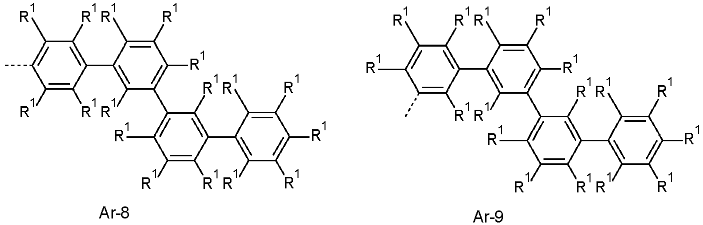 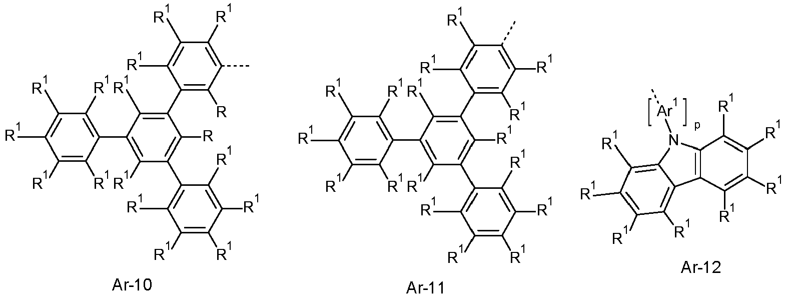 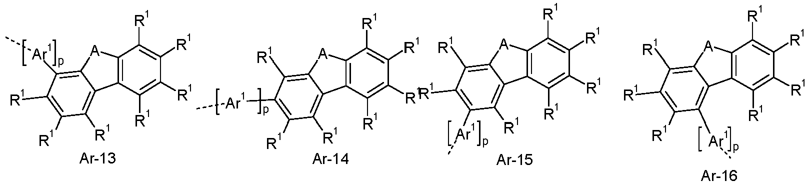 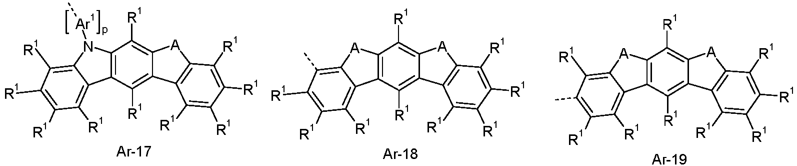 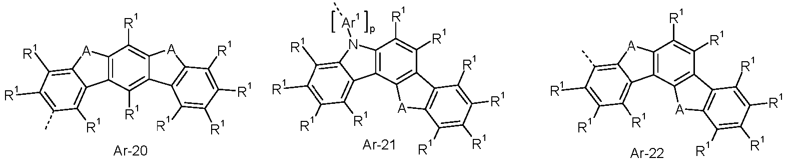 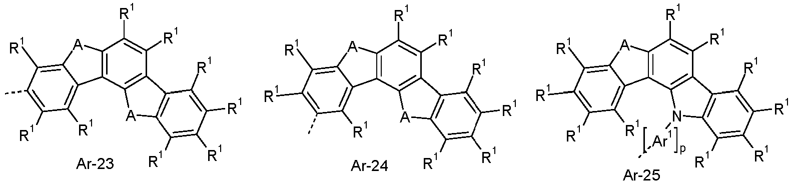 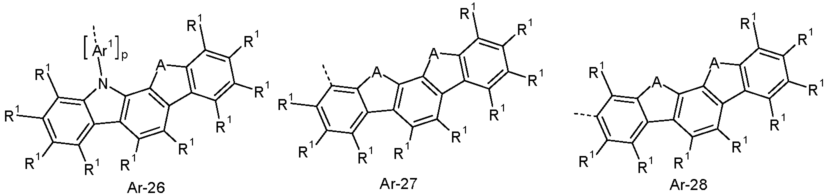 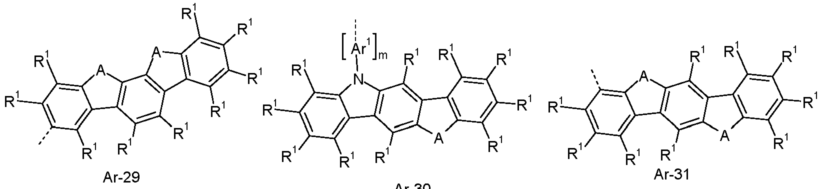 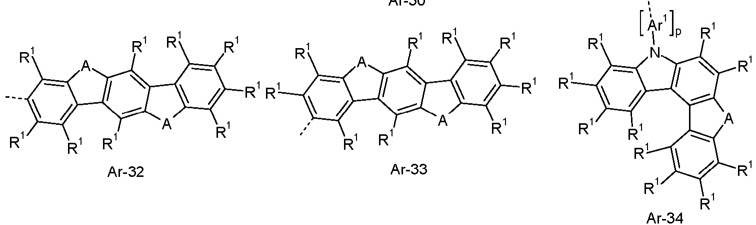 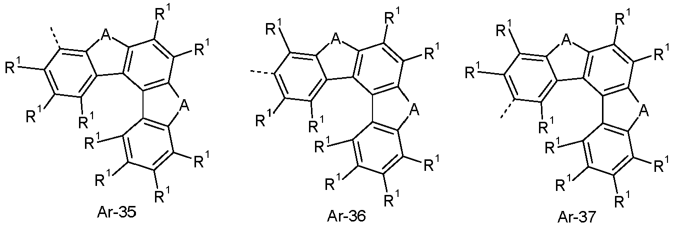 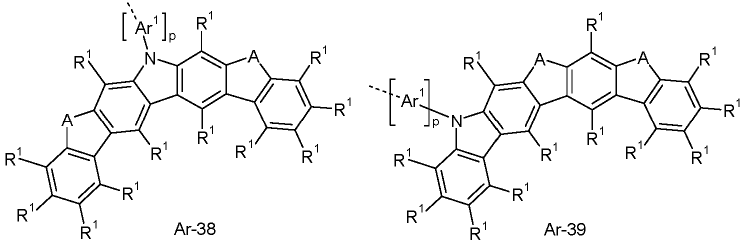 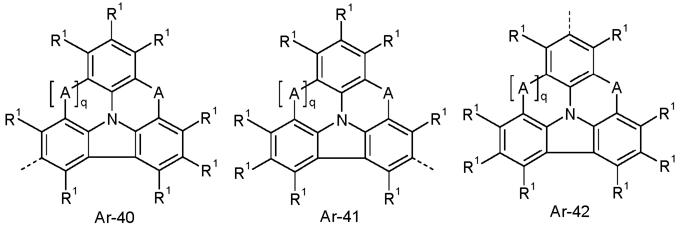 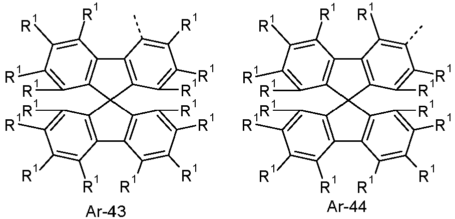 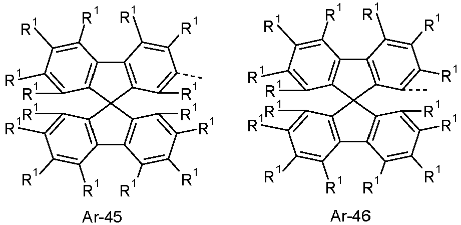 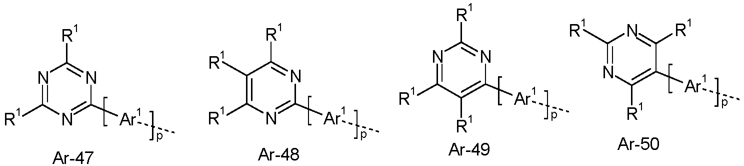 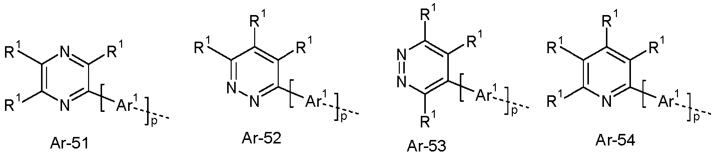 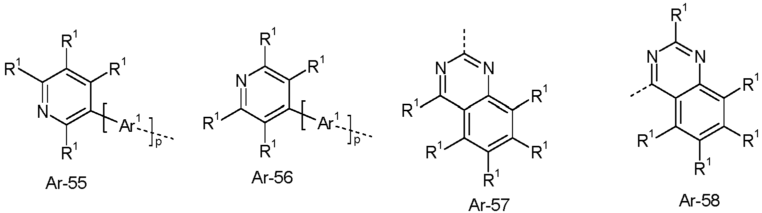 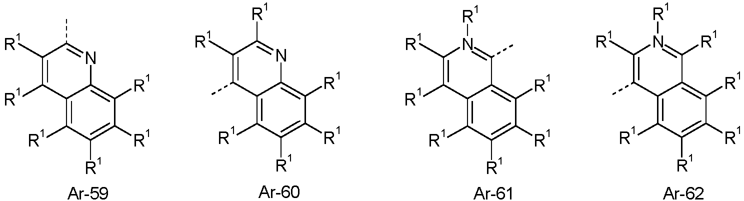 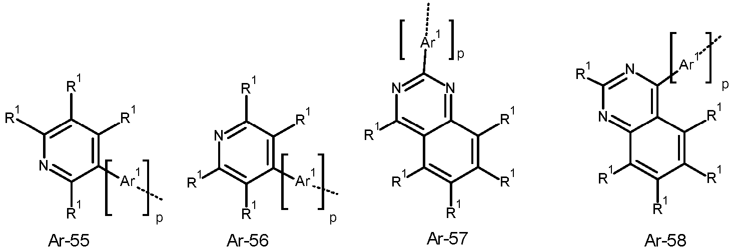 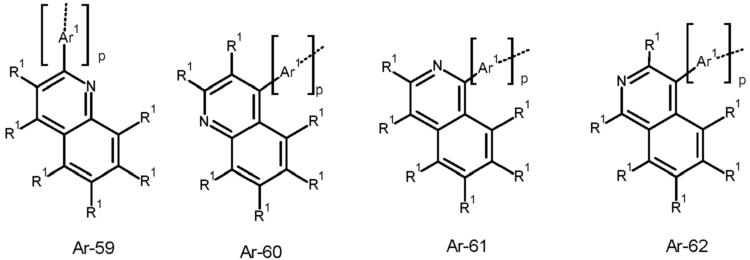 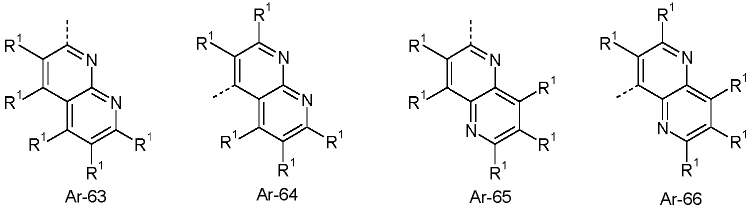 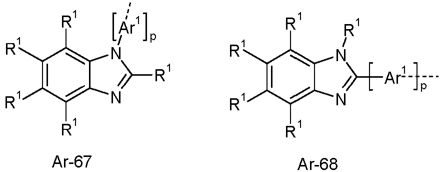 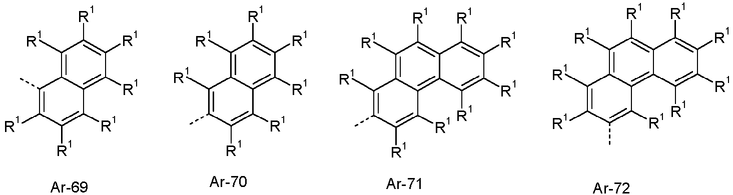 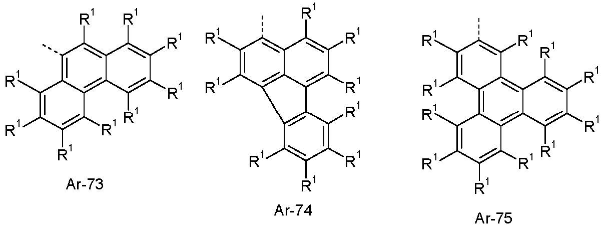 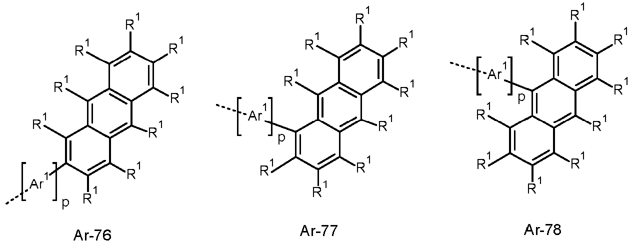 where R¹ has the definitions given above, the dotted bond represents the bond on the corresponding group and in addition:
Ar¹ is the same or different at each instance and is a bivalent aromatic or heteroaromatic ring system which has 6 to 18 aromatic ring atoms and may be substituted in each case by one or more R¹ radicals;
A is the same or different at each instance and is C(R¹)₂, NR¹, O or S;
p is 0 or 1, where p = 0 means that the Ar¹ group is absent and that the corresponding aromatic or heteroaromatic group is bonded directly to the corresponding radical;
q is 0 or 1, where q = 0 means that no A group is bonded at this position and R¹ radicals are bonded to the corresponding carbon atoms instead.

11. Compound according to at least one of the preceding Claims 5 to 10, **characterized in that** the substituent R^{c}, R^{d} is the same or different at each instance and represents an aromatic or heteroaromatic ring system which has 6 to 30 aromatic ring atoms and may be substituted by one or more R¹ radicals selected from the groups of the formulae (Ar-1) to (Ar-78) shown in Claim 10.

12. Compound according to at least one of the preceding Claims 1 to 11, **characterized in that** the compound includes exactly two or exactly three structures of formula (I), (II-1) to (II-42), (III-1) to (III-41), (IV-1) to (IV-41), (V-1) to (V-8) and/or (VI-1) to (VI-22).

13. Oligomer, polymer or dendrimer containing one or more compounds according to any of Claims 1 to 11, wherein, rather than a hydrogen atom or a substituent, there are one or more bonds of the compounds to the polymer, oligomer or dendrimer.

14. Formulation comprising at least one compound according to one or more of Claims 1 to 12 or an oligomer, polymer or dendrimer according to Claim 13 and at least one further compound, where the further compound is preferably selected from one or more solvents.

15. Composition comprising at least one compound of the formula (I) 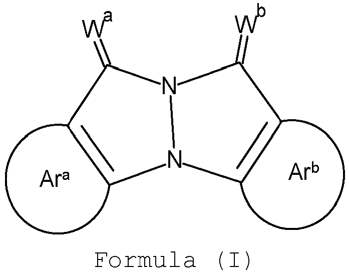 wherein the symbols used have the definitions given in Claim 1, or an oligomer, polymer or dendrimer as claimed in Claim 13 and at least one further compound selected from the group consisting of fluorescent emitters, phosphorescent emitters, emitters that exhibit TADF, host materials, electron transport materials, electron injection materials, hole conductor materials, hole injection materials, electron blocker materials and hole blocker materials.

16. Process for preparing a compound according to one or more of Claims 1 to 12, **characterized in that** a base skeleton having at least one of the W^{a} groups or a precursor of one of the W^{a} groups is synthesized, and an aromatic or heteroaromatic radical is introduced by means of a nucleophilic aromatic substitution reaction or a coupling reaction.

17. Use of a compound of formula (I) 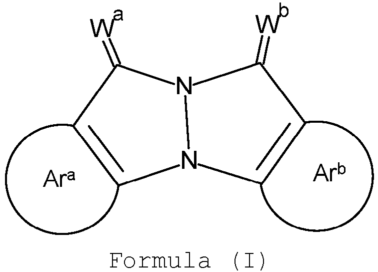 where the symbols used have the definitions given in Claim 1, or an oligomer, polymer or dendrimer according to Claim 13 in an electronic device.

18. Electronic device comprising at least one compound of formula (I) 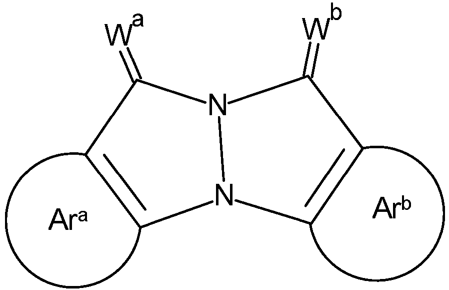 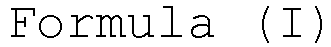 where the symbols used have the definitions given in Claim 1, or an oligomer, polymer or dendrimer according to Claim 13.

## Revendications

1. Composé comprenant au moins une structure de Formule (I) 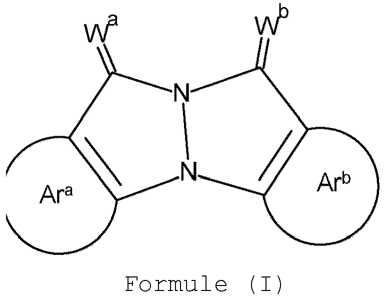
dans lequel le cycle Ar^{a}, à chaque occurrence est identique ou différent et représente un système cyclique aromatique ou hétéroaromatique ayant 5 à 60 atomes nucléaires aromatiques, qui peut être substitué par un ou plusieurs radicaux Ar ou R^{a}, le cycle Ar^{b}, à chaque occurrence étant identique ou différent et représentant un système cyclique aromatique ou hétéroaromatique ayant 5 à 60 atomes nucléaires aromatiques, qui peut être substitué par un ou plusieurs radicaux Ar ou R^{b} ;
et, pour les autres symboles et indices utilisés :
W^{a}, W^{b} est, à chaque occurrence, identique ou différent, et représente O ou S ;
Ar, à chaque occurrence est identique ou différent et représente un système cyclique aromatique ou hétéroaromatique ayant 5 à 60 atomes nucléaires aromatiques, qui peut être substitué par un ou plusieurs radicaux R, le groupe Ar pouvant alors former avec au moins un groupe Ar, R, R^{a}, R^{b} ou un autre groupe, un système cyclique ;
R, R^{a}, R^{b} sont, à chaque occurrence, identiques ou différents et représentent H, D, OH, F, Cl, Br, I, CN, NO₂, N(Ar')₂, N(R')₂, C(=O)N(Ar)₂, C(=O)N(R')₂, C(Ar')₃, C(R¹)₃, Si(Ar')₃, Si(R¹)₃, B(Ar')₂, B(R¹)₂, C(=O)Ar', C(=O)R¹, P (=O)(Ar')₂, P(=O)(R¹)₂, P(Ar)₂, P(R¹)₂, S(=O)Ar', S(=O)R¹, S(=O)₂Ar', S(=O)₂R¹, OSO₂Ar', OSO₂R¹, un groupe alkyle, alcoxy ou thioalcoxy à chaîne droite ayant 1 à 40 atomes de carbone ou un groupe alcényle ou alcynyle ayant 2 à 40 atomes de carbone ou un groupe alkyle, alcoxy ou thioalcoxy ramifié ou cyclique ayant 3 à 20 atomes de carbone, chaque groupe alkyle, alcoxy, thioalcoxy, alcényle ou alcynyle pouvant être substitué par un ou plusieurs radicaux R¹, un ou plusieurs groupes CH₂ non voisins pouvant être remplacés par R¹C=CR¹, C=C, Si(R¹)₂, C=O, C=S, C=Se, C=NR¹, -C(=O)O-, -C (=O)NR¹-, NR¹, P(=O)(R¹), -O-, -S-, SO ou SO₂, ou un système cyclique aromatique ou hétéroaromatique ayant 5 à 60 atomes nucléaires aromatiques, dont chacun peut être substitué par un ou plusieurs radicaux R¹, ou un groupe aryloxy ou hétéroaryloxy ayant 5 à 60 atomes nucléaires de carbone, qui peut être substitué par un ou plusieurs radicaux R¹, ou un groupe hétéroarylthio ayant 5 à 60 atomes nucléaires aromatiques, qui peut être remplacé par un ou plusieurs radicaux R¹, ou un groupe diarylamino, arylhétéroarylamino, dihétéroarylamino ayant 5 à 60 atomes nucléaires aromatiques, qui peut être substitué par un ou plusieurs radicaux R¹, ou un groupe arylalkyle ou hétéroarylalkyle ayant 5 à 60 atomes nucléaires aromatiques et 1 à 10 atomes de carbone dans le radical alkyle, qui peut être substitué par un ou plusieurs radicaux R¹, deux radicaux R, R^{a}, R^{b} pouvant alors former aussi l'un avec l'autre, ou avec un autre groupe, un système cyclique ;
Ar' est, à chaque occurrence, identique ou différent et représente un système cyclique aromatique ou hétéroaromatique ayant 5 à 60 atomes nucléaires aromatiques, qui peut être substitué par un ou plusieurs radicaux R¹, deux radicaux Ar' qui sont liés au même atome C, au même atome Si, au même atome N, au même atome P ou au même atome B étant également pontés l'un à l'autre par une liaison simple ou un pont choisi parmi B(R¹), C(R¹)₂, Si(R¹)₂, C=O, C=NR¹, C=C(R¹)₂, O, S, S=O, SO₂, N(R¹), P(R¹) et P(=O)R¹ ;
R¹ est, à chaque occurrence, identique ou différent et représente H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C (R²)₃ Si(Ar")₃, Si(R²)₃, un groupe alkyle, alcoxy ou thioalcoxy à chaîne droite ayant 1 à 40 atomes de carbone ou un groupe alkyle, alcoxy ou thioalcoxy ramifié ou cyclique ayant 3 à 40 atomes de carbone ou un groupe alcényle ayant 2 à 40 atomes de carbone, chacun pouvant être substitué par un ou plusieurs radicaux R², un ou plusieurs groupes CH₂ non voisins pouvant être remplacés par -R²C=CR²-,
-C=C-, Si(R²)₂, C=O, C=S, C=Se, C=NR², -C(=O)O-, -C(=O)NR²-, NR², P(=O)(R²), -O-, -S-, SO ou SO₂, et un ou plusieurs atomes H pouvant être remplacés par D, F, Cl, Br, I, CN ou NO₂, ou un système cyclique aromatique ou hétéroaromatique ayant 5 à 60 atomes nucléaires aromatiques, dont chacun peut être substitué par un ou plusieurs radicaux R², ou un groupe aryloxy ou hétéroaryloxy ayant 5 à 60 atomes nucléaires aromatiques, qui peut être substitué par un ou plusieurs radicaux R², ou un groupe aralkyle ou hétéroaralkyle ayant 5 à 60 atomes nucléaires aromatiques, qui peut être substitué par un ou plusieurs radicaux R², ou une combinaison de ces systèmes ; deux ou plusieurs radicaux R¹ pouvant former les uns avec les autres un système cyclique, un ou plusieurs radicaux R¹ pouvant alors, avec une autre partie du composé, former un système cyclique ;
Ar" est, à chaque occurrence, identique ou différent et représente un système cyclique aromatique ou hétéroaromatique ayant 5 à 30 atomes nucléaires aromatiques, qui peut être substitué par un ou plusieurs radicaux R², deux radicaux Ar" qui se lient au même atome C, au même atome Si, au même atome N, au même atome P ou au même atome B, pouvant aussi être pontés l'un à l'autre par une liaison simple ou un pont choisi parmi B(R²), C(R²)₂, Si(R²)₂, C=O, C=NR², C=C(R²)₂, O, S, S=O, SO₂, N(R²), P(R²) et P(=O)R² ;
R² est, à chaque occurrence, identique ou différent et est choisi parmi le groupe constitué de H, D, F, CN, d'un radical hydrocarboné aliphatique ayant 1 à 20 atomes de carbone ou d'un système cyclique aromatique ou hétéroaromatique ayant 5 à 30 atomes nucléaires aromatiques, dans lequel un ou plusieurs atomes H peuvent être remplacés par D, F, Cl, Br, I ou CN, et qui peut être substitué par un ou plusieurs groupes alkyle ayant chacun 1 à 4 atomes de carbone, deux ou plusieurs substituants R² pouvant alors former les uns avec les autres un système cyclique ;
les composés de formules (A), (B) et (C) 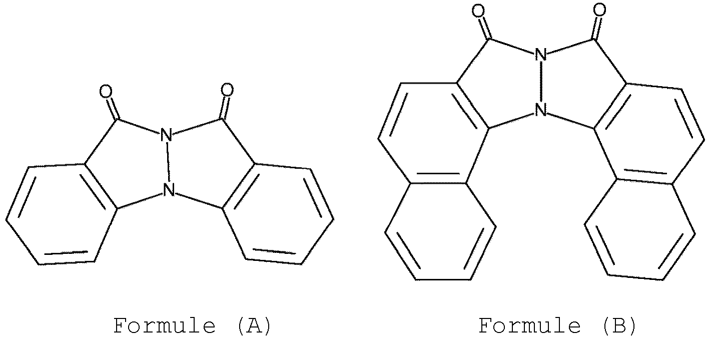 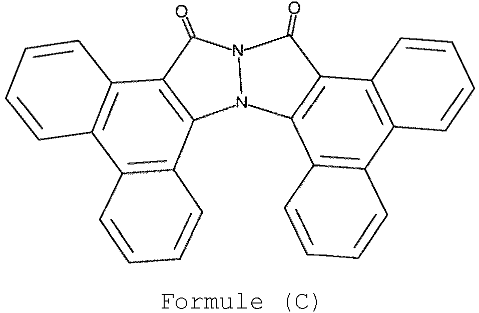
étant exclus de la protection.

2. Composé selon la revendication 1, comprenant au moins une structure de formules (II-1) à (II-42), 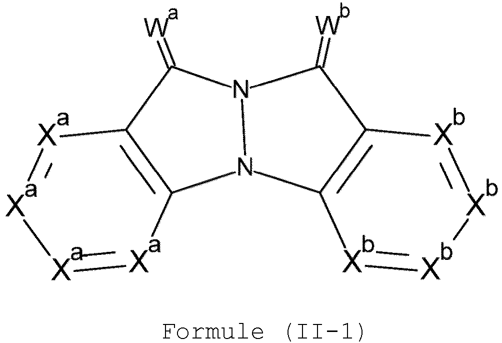 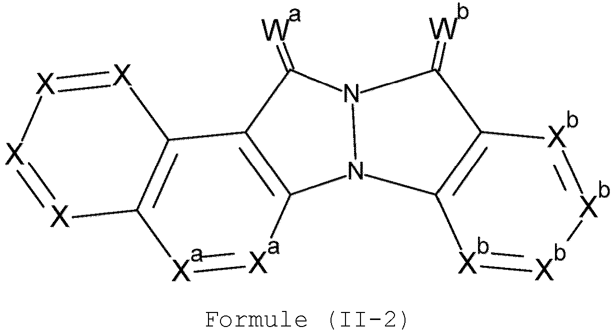 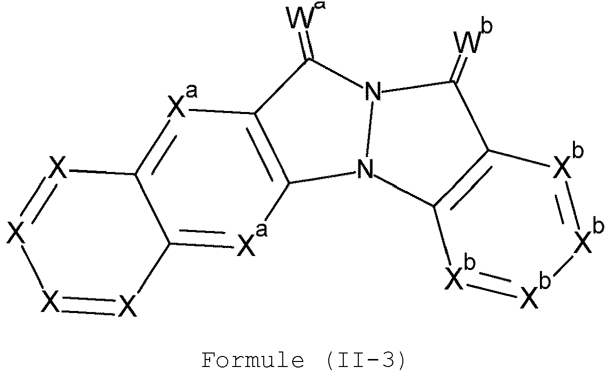 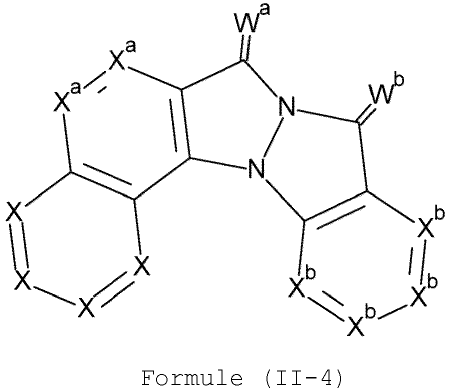 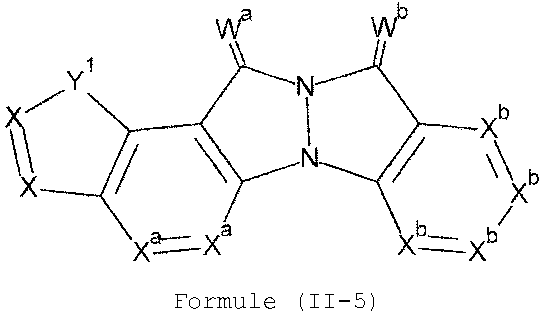 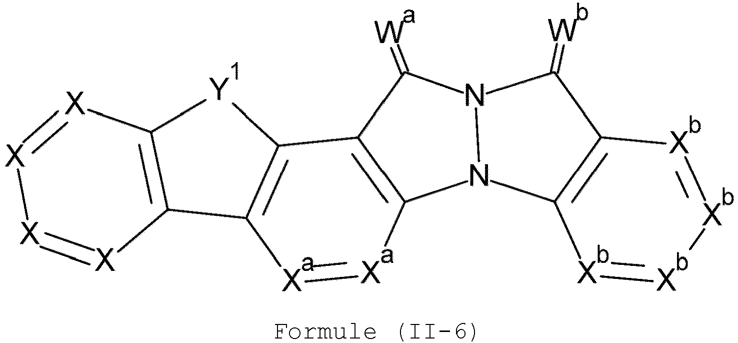 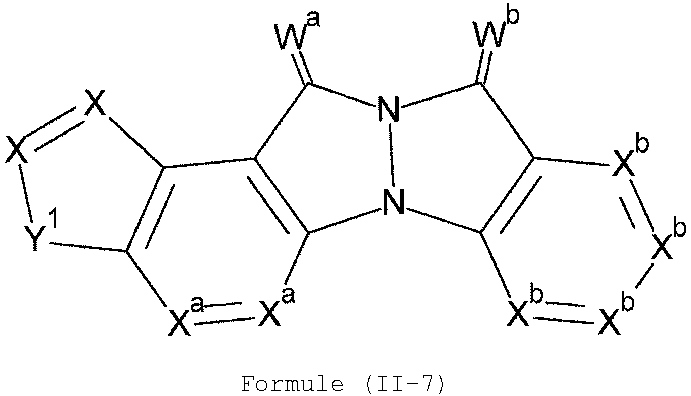 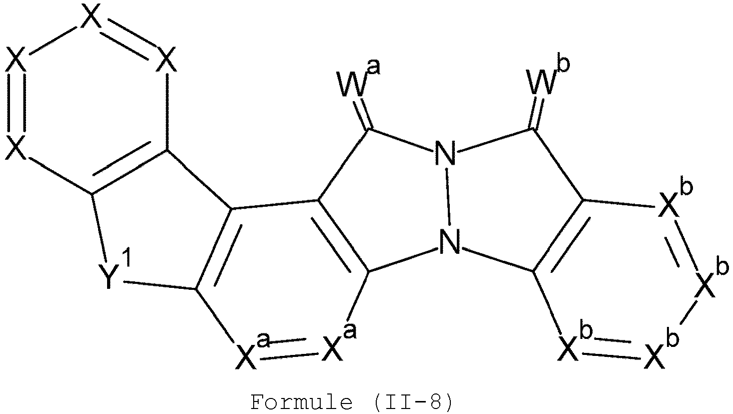 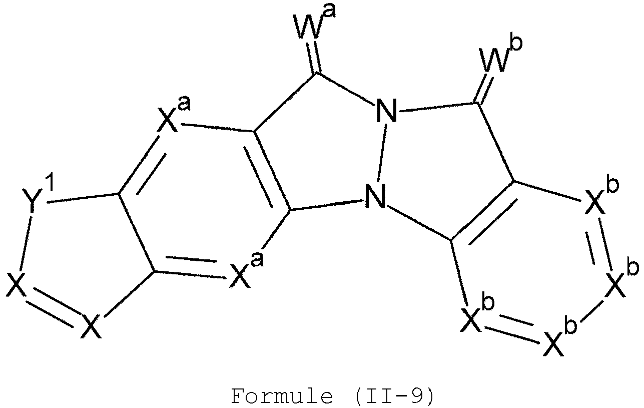 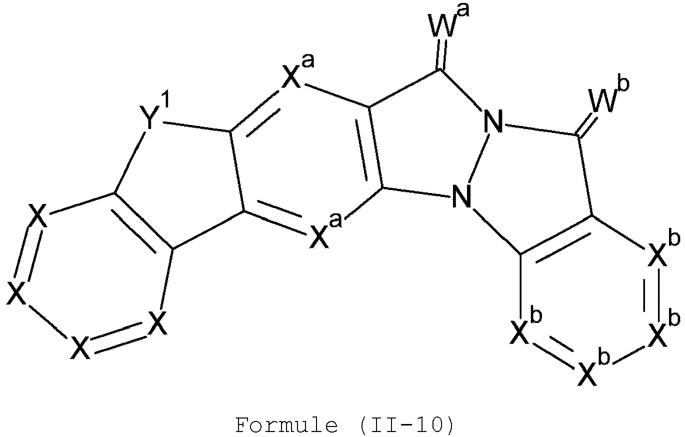 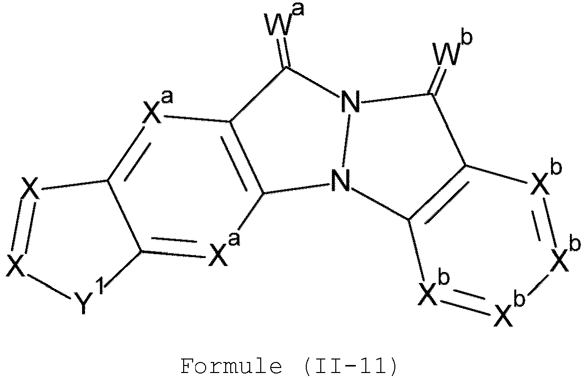 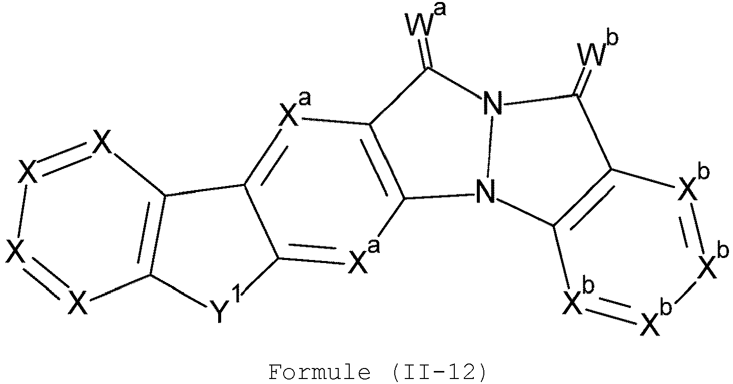 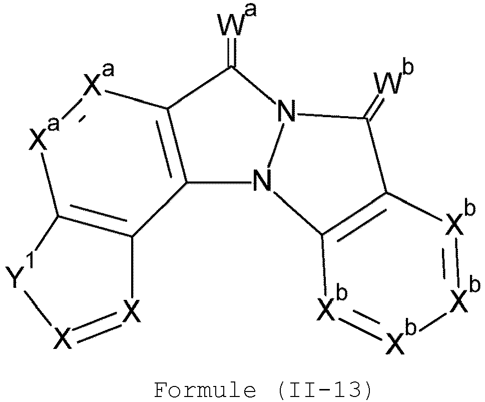 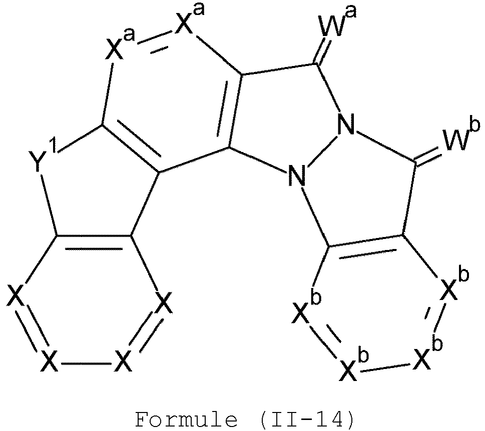 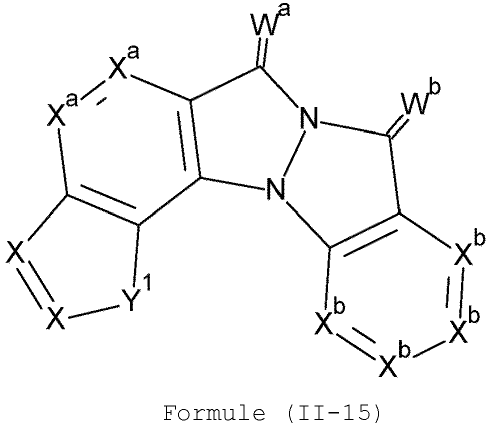 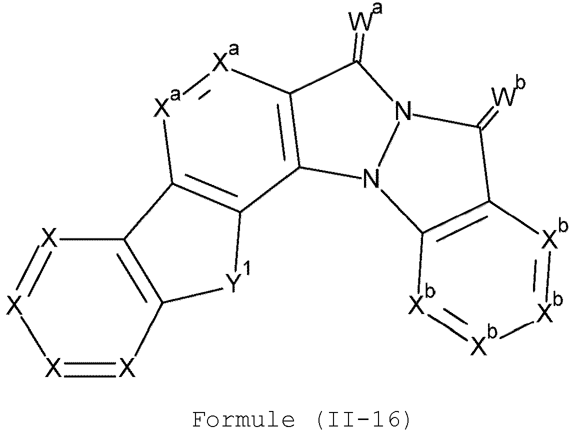 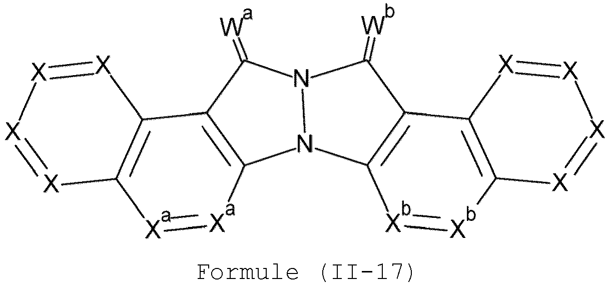 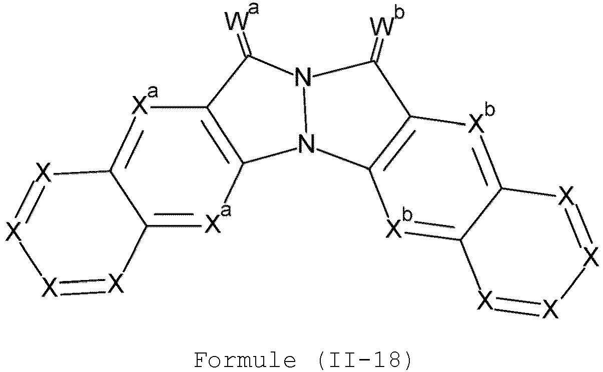 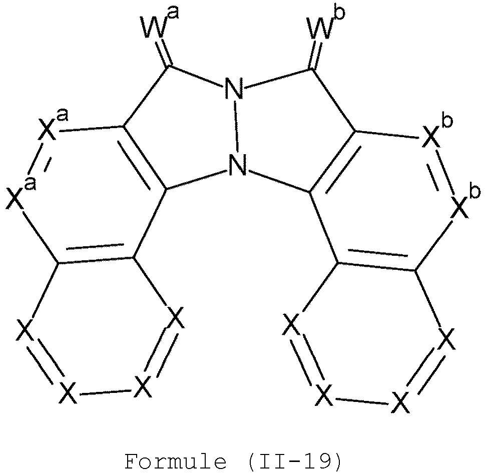 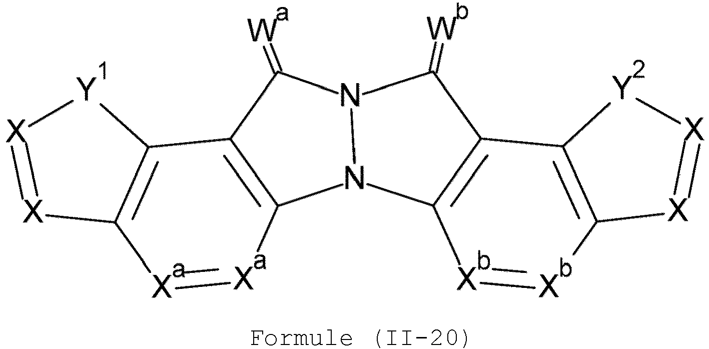 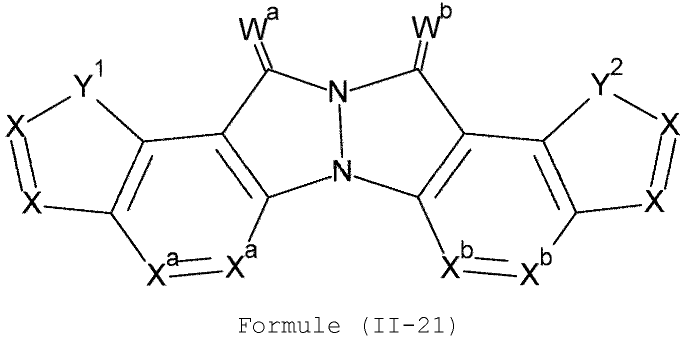 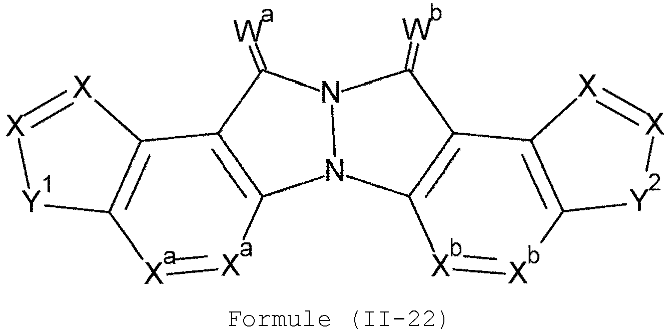 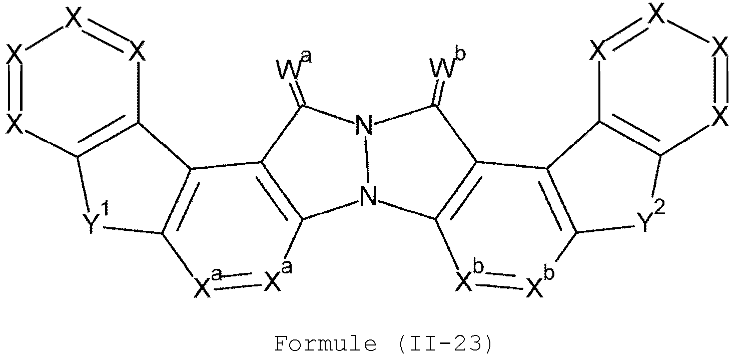 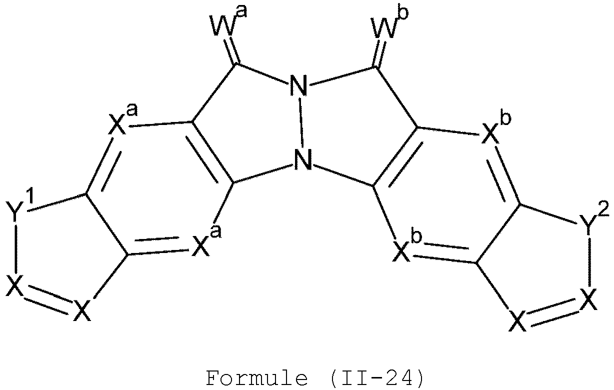 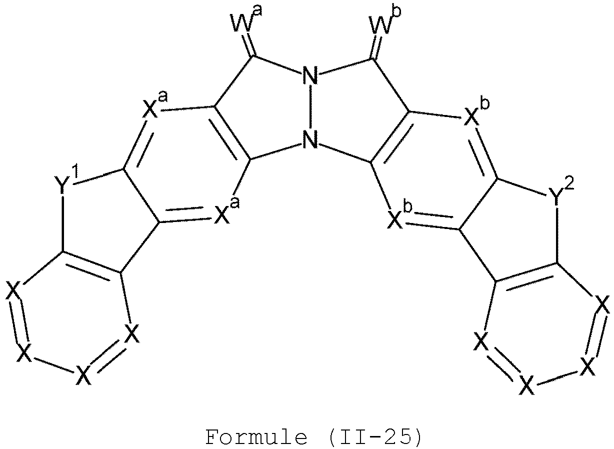 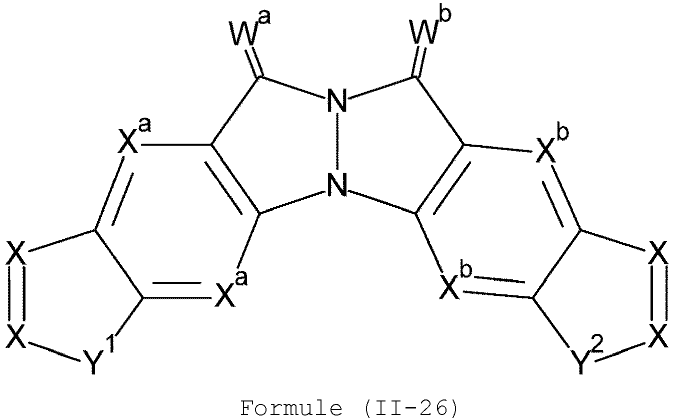 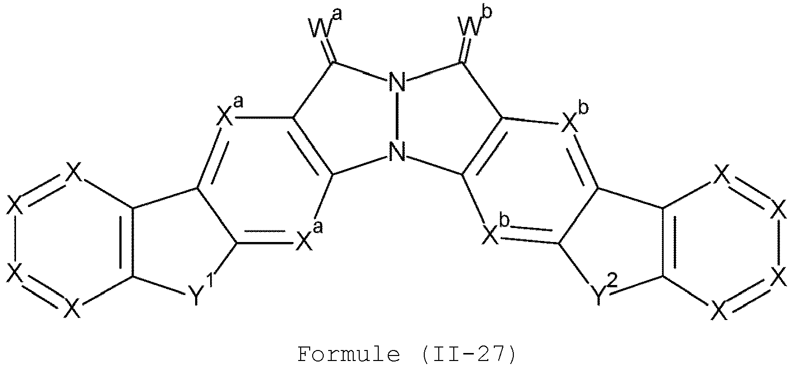 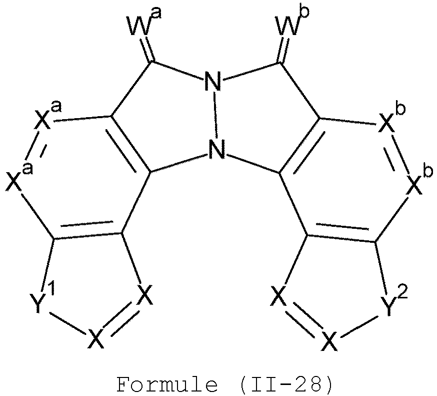 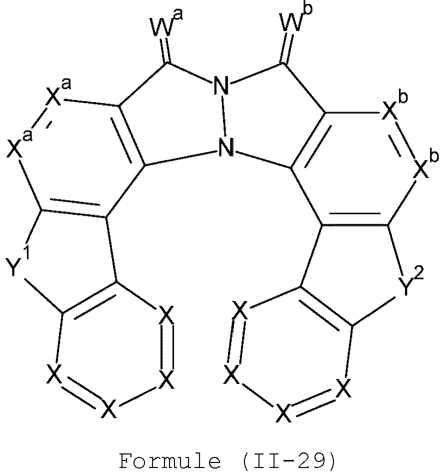 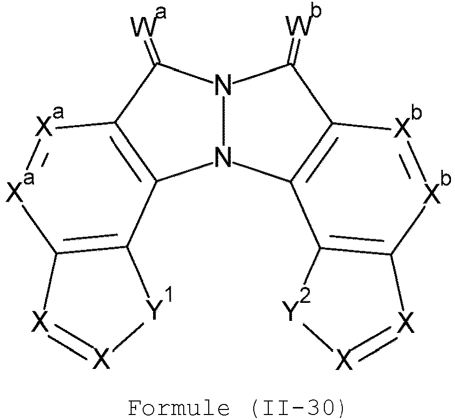 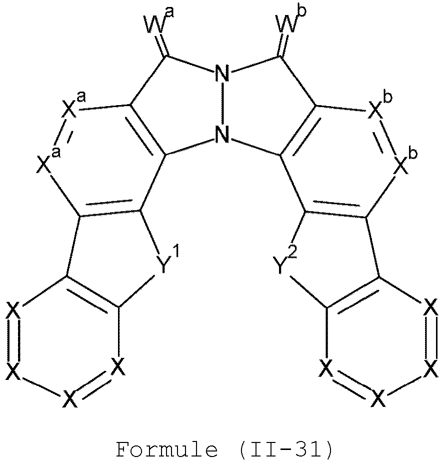 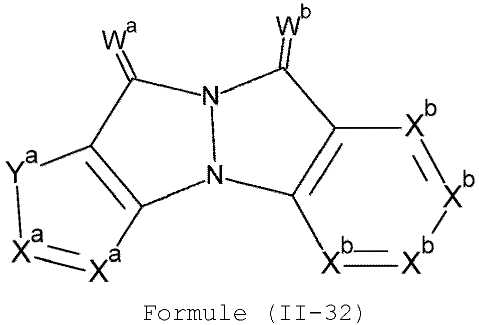 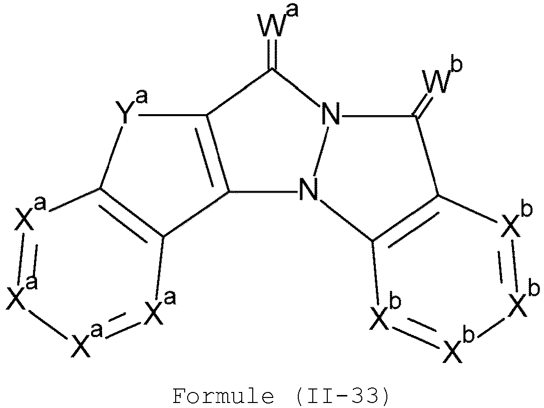 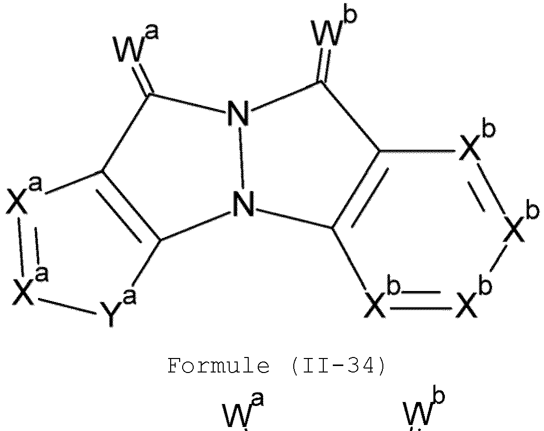 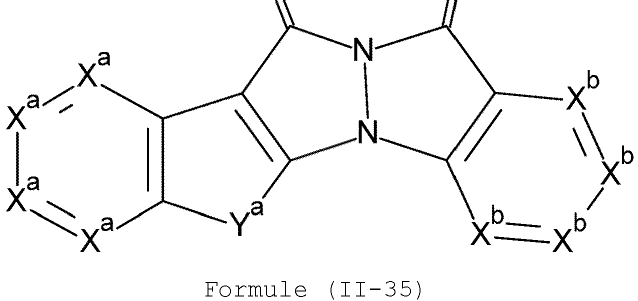 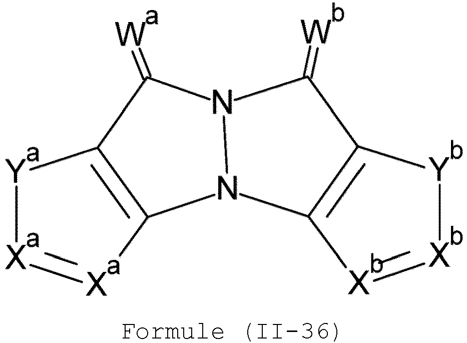 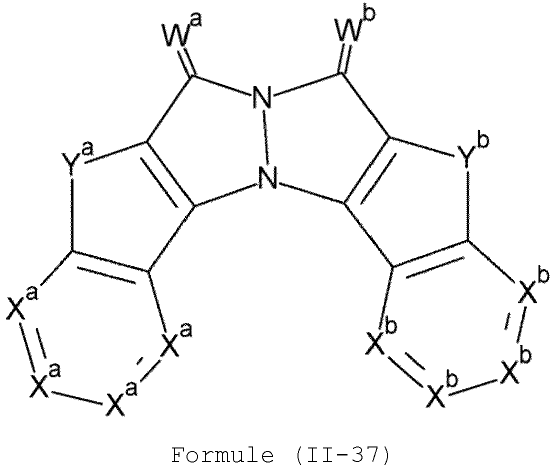 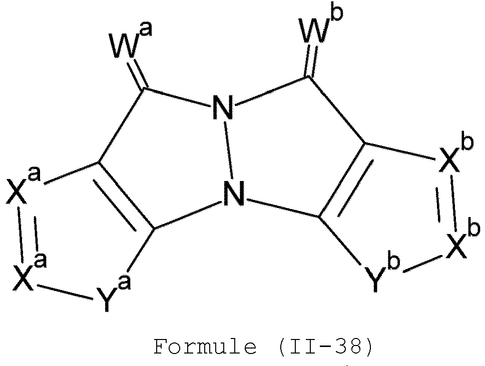 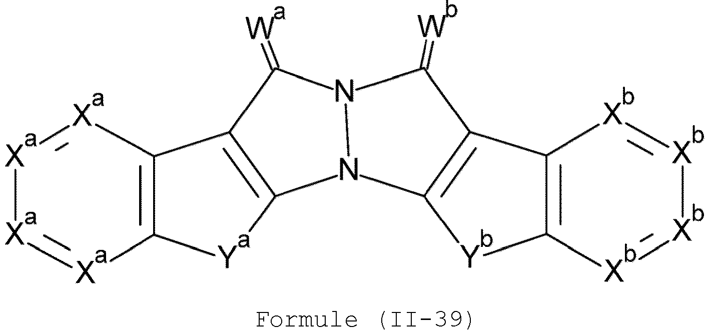 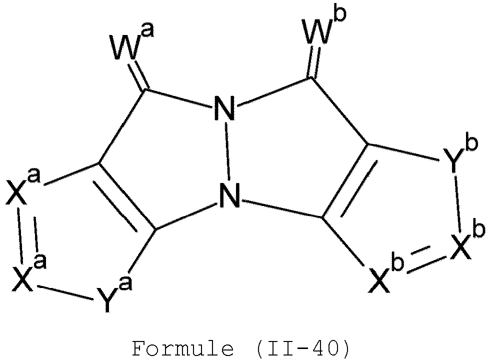 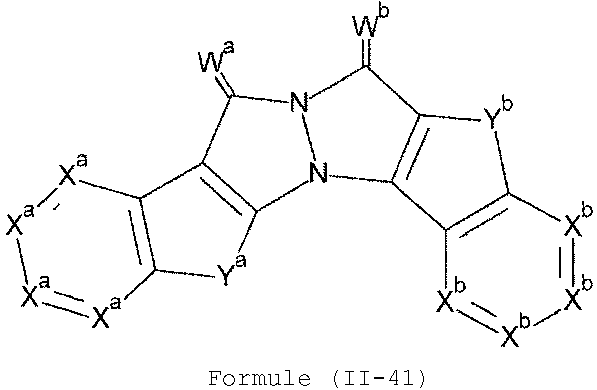 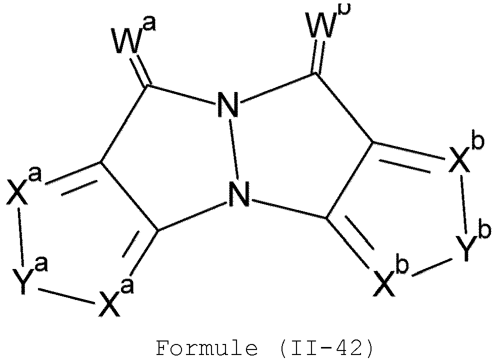 dans lequel les symboles W^{a} et W^{b} ont les significations données dans la revendication 1 et, pour les autres symboles :
Y^{a} est, à chaque occurrence, identique ou différent et représente N(Ar), N(R^{a}), P(Ar), P(R^{a}), P(=O)Ar, P(=O)R^{a}, P(=S)Ar, P(=S)R^{a}, B (Ar), B(R^{a}), Al(Ar), Al(R^{a}), Ga(Ar), Ga(R^{a}), C=O, C(R^{a})₂, Si(R^{a})₂, Ge(R^{a})₂, C=NR^{a}, C=NAr, C=C(R^{a})₂, C=C(R^{a})(Ar), O, S, Se, S=O ou SO₂, de préférence N(Ar), N(R^{a}), B(Ar), B(R^{a}), P(=O)R^{a}, P (=O) Ar, C=O, C(R^{a})₂, C=C(R^{a})₂, C=C(R^{a})(Ar), Si (R^{a})₂, O, S, Se, S=O ou SO₂, d'une manière particulièrement préférée N(Ar), C(R^{a})₂, O ou S, R^{a} ayant la signification donnée dans la revendication 1 ;
Y^{b} est, à chaque occurrence, identique ou différent et représente N(Ar), N(R^{b}), P(Ar), P(R^{b}), P(=O)Ar, P(=O)R^{b}, P(=S)Ar, P(=S)R^{b}, B(Ar), B(R^{b}), Al(Ar), Al(R^{b}), Ga(Ar), Ga(R^{b}), C=O, C(R^{b})₂, Si(R^{b})₂, Ge(R^{b})₂, C=NR^{b}, C=NAr, C=C(R^{b})₂, C=C(R^{b})(Ar), O, S, Se, S=O ou SO₂, de préférence N(Ar), N(R^{b}), B(Ar), B(R^{b}), P(=O)R^{b}, P(=O)Ar, C=O, C(R^{b})₂, C=C(R^{b})₂, C=C(R^{b})(Ar), Si(R^{b})₂, O, S, Se, S=O ou SO₂, d'une manière particulièrement préférée N(Ar), C(R^{b})₂, O ou S, R^{b} ayant la signification donnée dans la revendication 1 ;
Y¹, Y² sont, à chaque occurrence, identiques ou différents et représentent N(Ar), N(R), P(Ar), P(R), P(=O)Ar, P(=O)R, P(=S)Ar, P(=S)R, B (Ar), B (R), Al(Ar), Al(R), Ga(Ar), Ga(R), C=O, C(R)₂, Si(R)₂, Ge(R)₂, C=NR, C=NAr, C=C(R)₂, C=C(R)(Ar), O, S, Se, S=O ou SO₂, de préférence N(Ar), N(R), B(Ar), B (R), P(=O)R, P(=O)Ar, C=O, C(R)₂, C=C(R)₂, C=C(R) (Ar), Si(R)₂, O, S, Se, S=O ou SO₂, d'une manière particulièrement préférée N(Ar), C(R)₂, O ou S, R ayant la signification donnée dans la revendication 1 ;
X est, à chaque occurrence, identique ou différent et représente N ou CR, à la condition que pas plus de deux groupes X d'un cycle représentent N, R ayant la signification donnée dans la revendication 1 ;
X^{a} est, à chaque occurrence, identique ou différent et représente N ou CR^{a}, à la condition que pas plus de deux groupes X^{a} d'un cycle représentent N, R^{a} ayant la signification donnée dans la revendication 1 ;
X^{b} est, à chaque occurrence, identique ou différent et représente N ou CR^{b}, à la condition que pas plus de deux groupes X^{b} d'un cycle représentent N, R^{b} ayant la signification donnée dans la revendication 1.

3. Composé selon la revendication 1 ou 2, comprenant au moins une structure de formules (III-1) à (III-41), 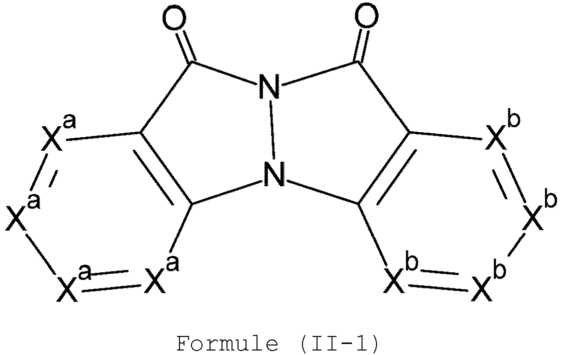 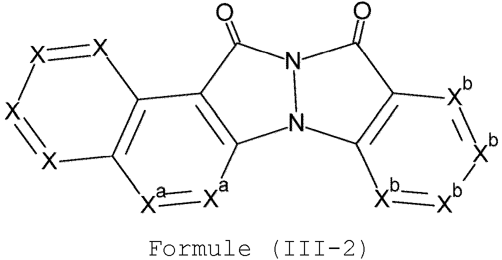 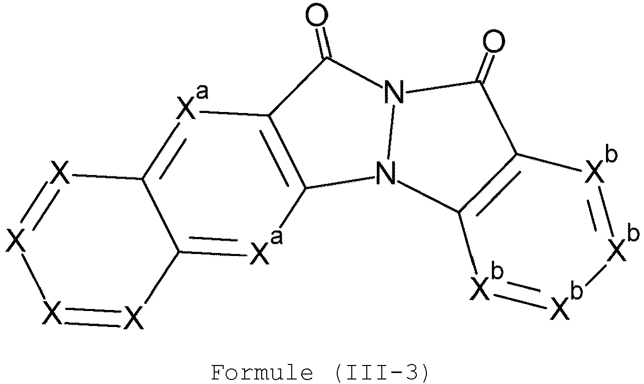 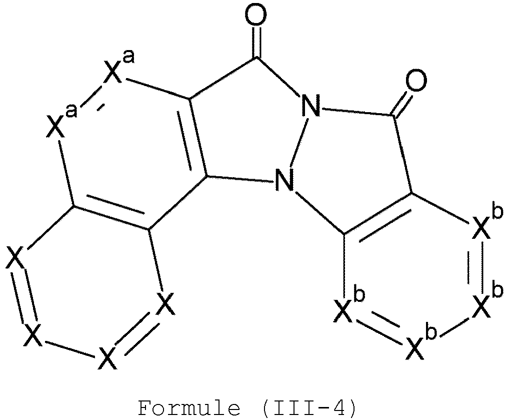 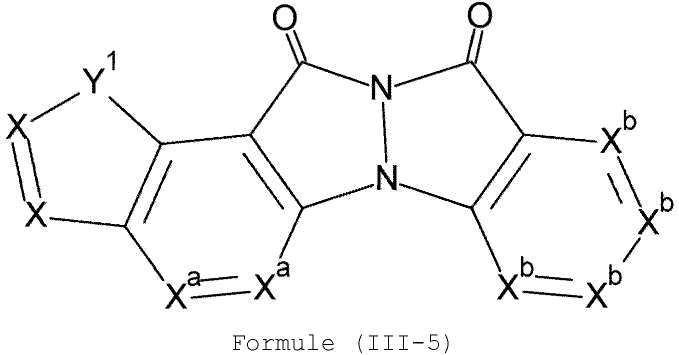 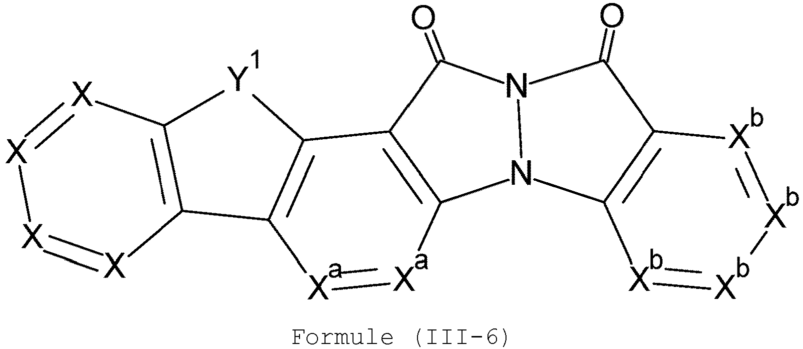 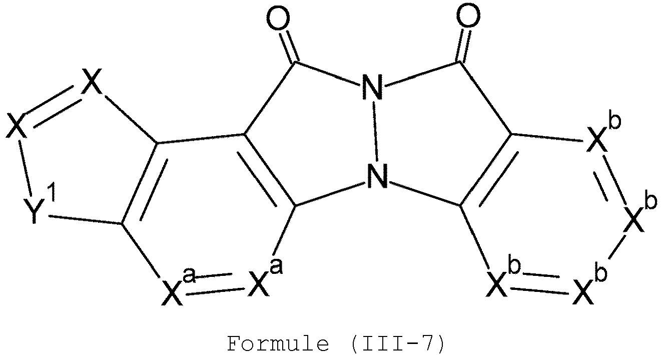 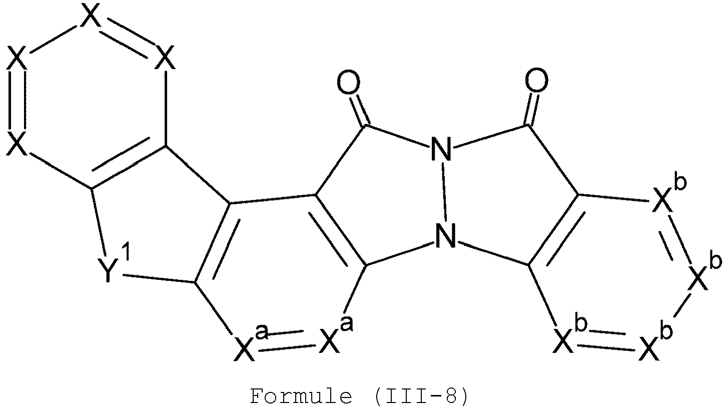 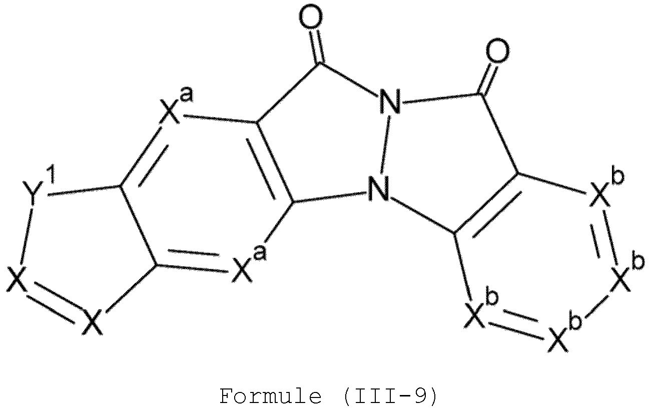 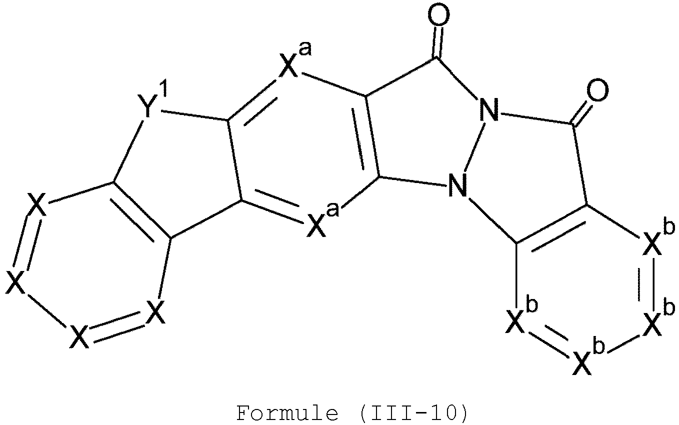 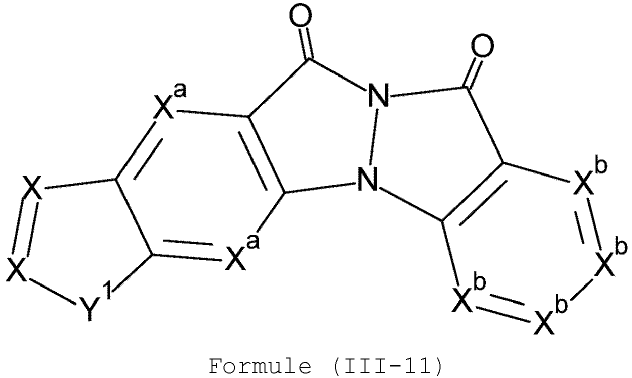 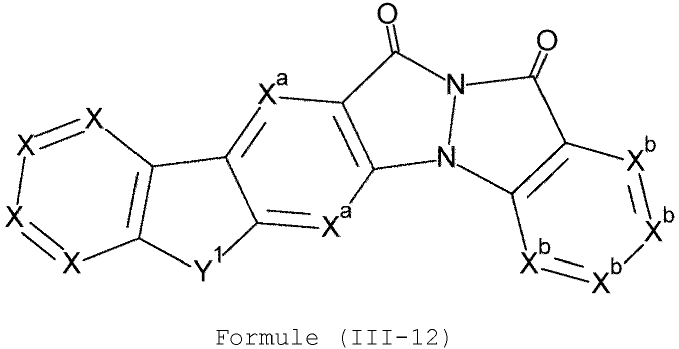 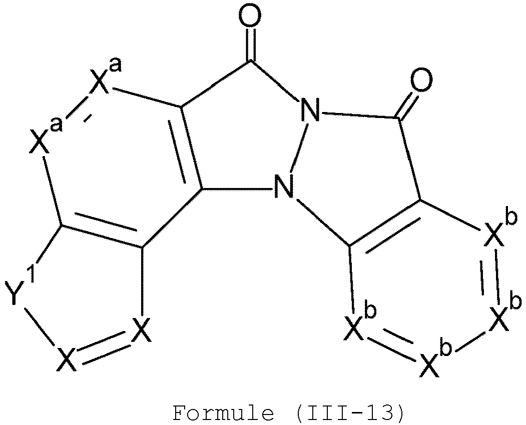 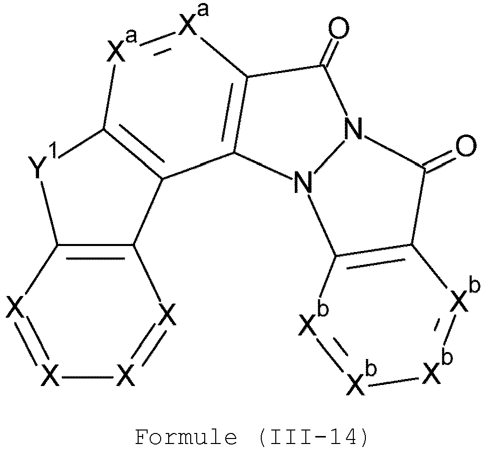 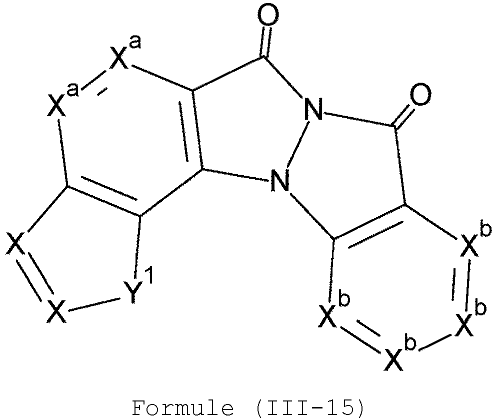 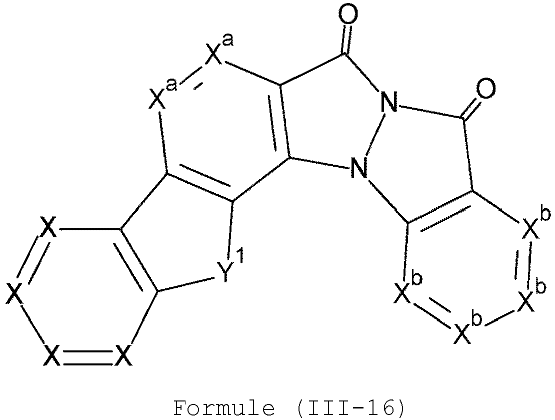 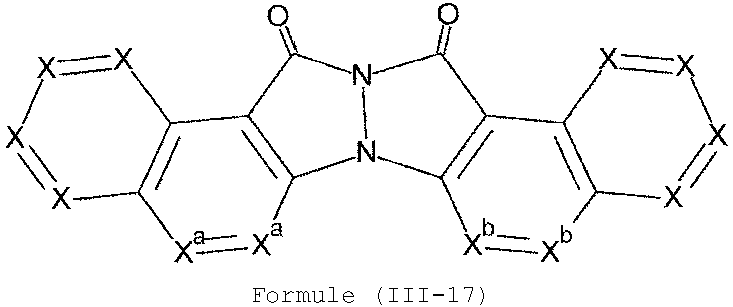 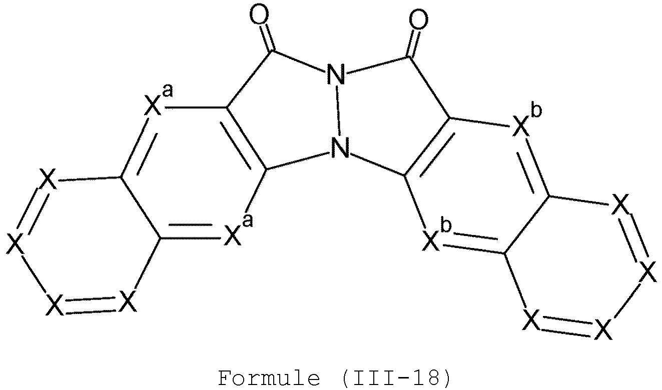 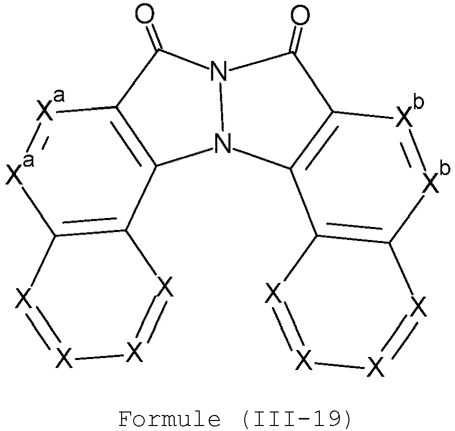 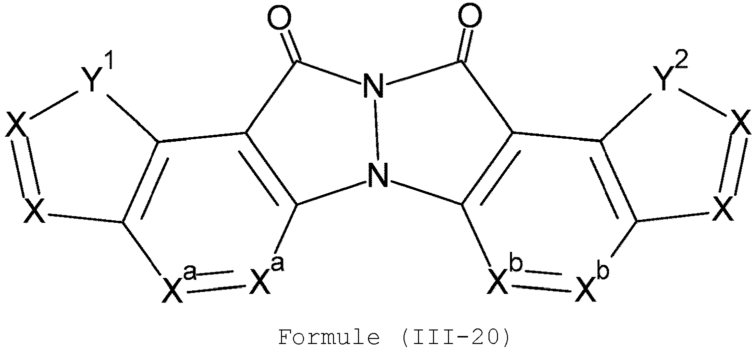 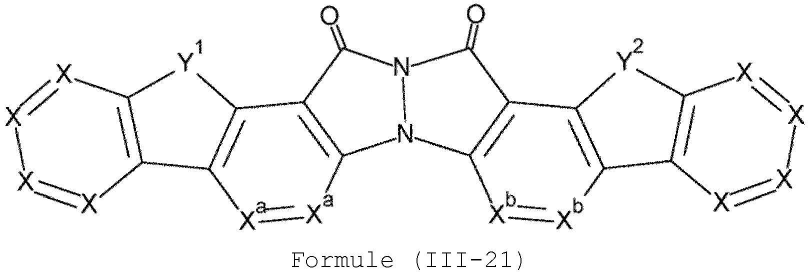 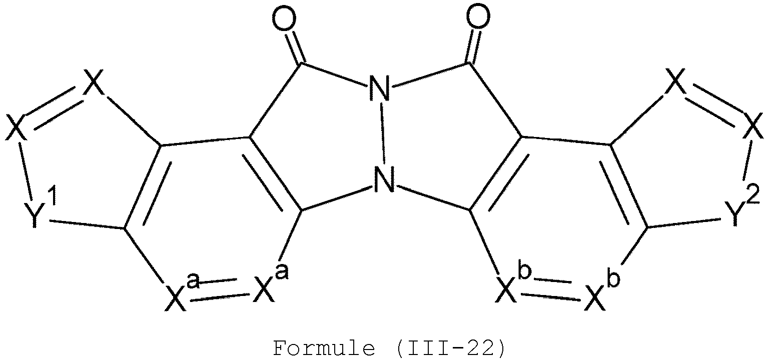 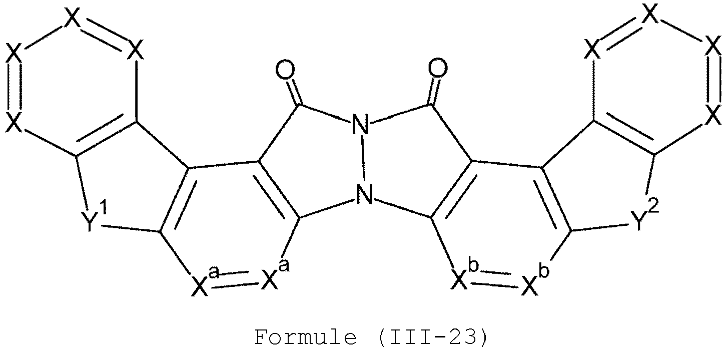 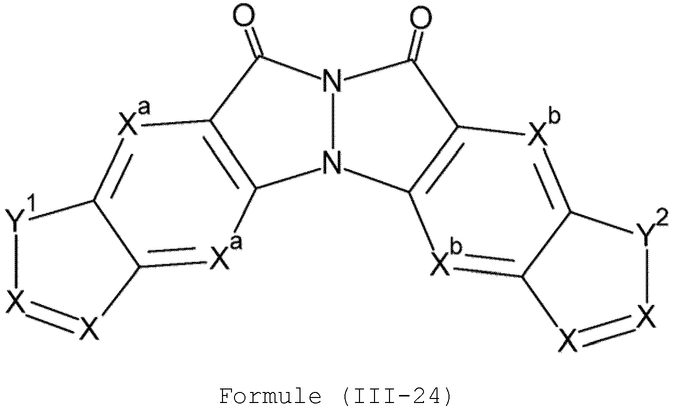 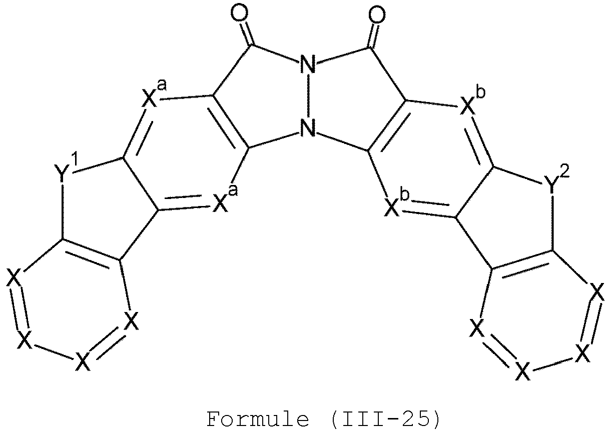 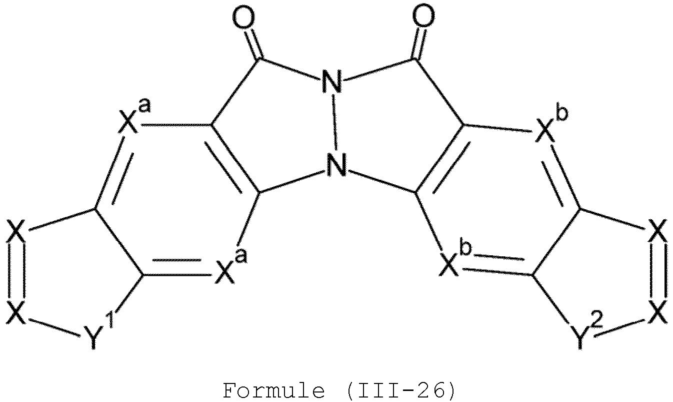 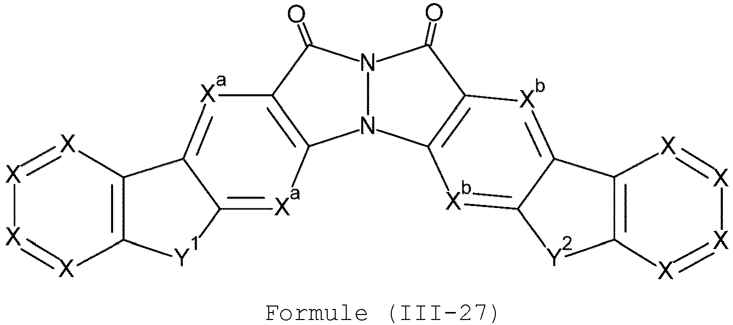 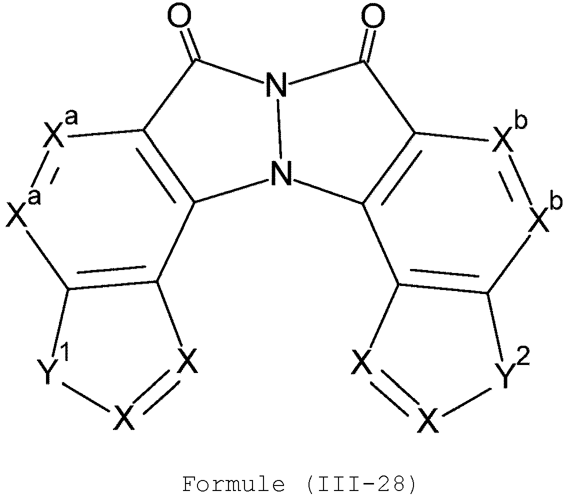 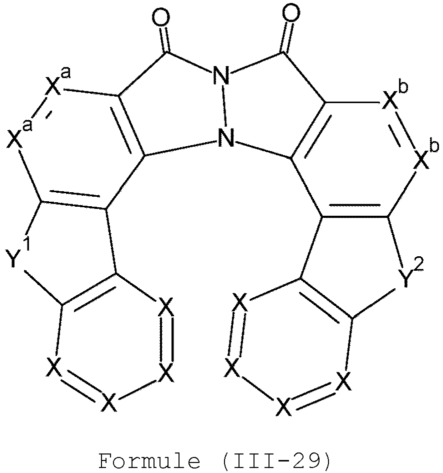 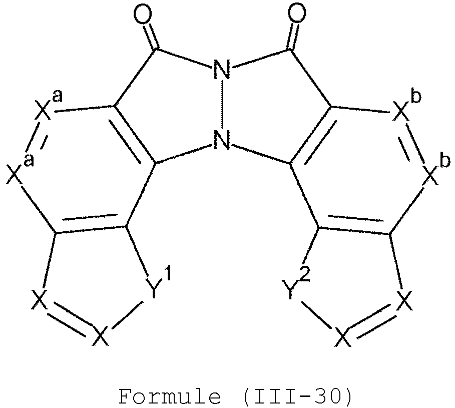 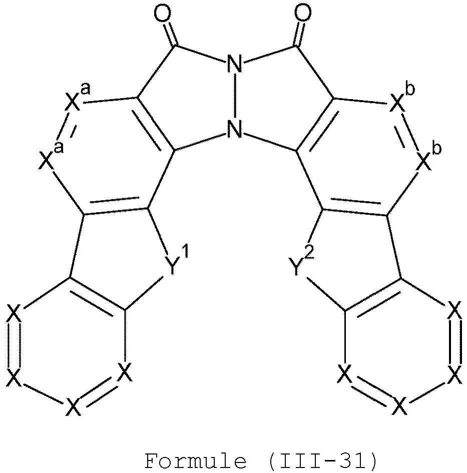 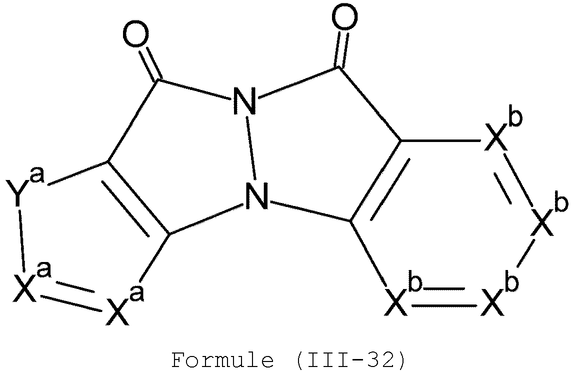 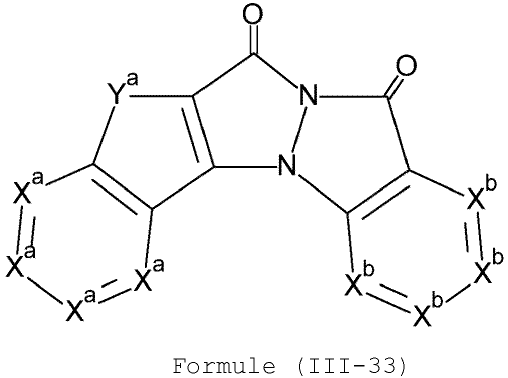 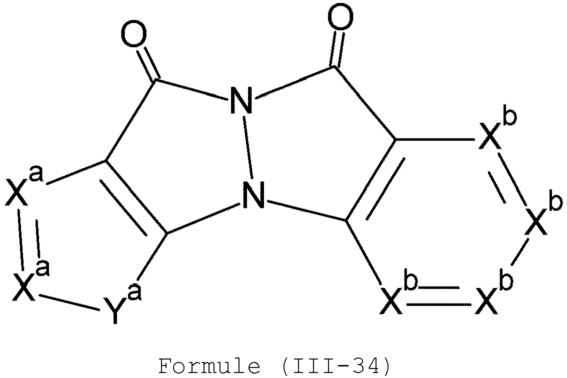 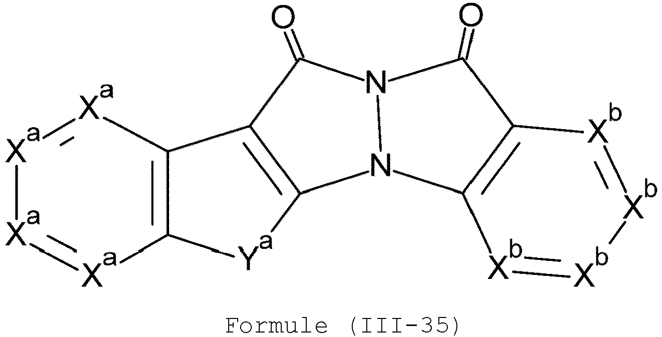 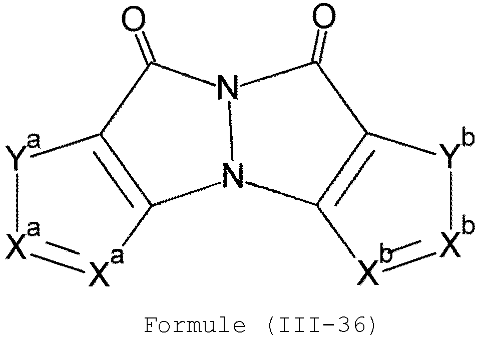 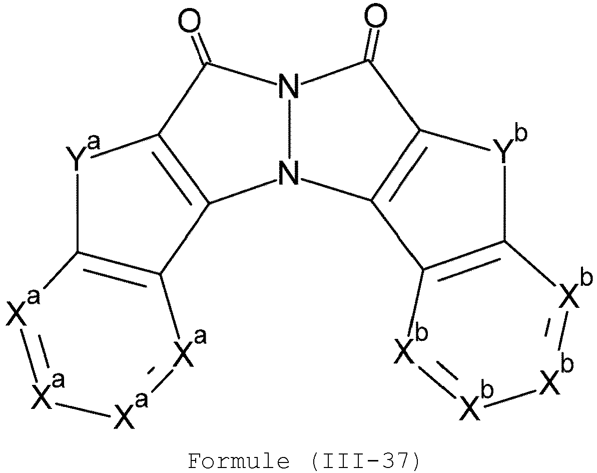 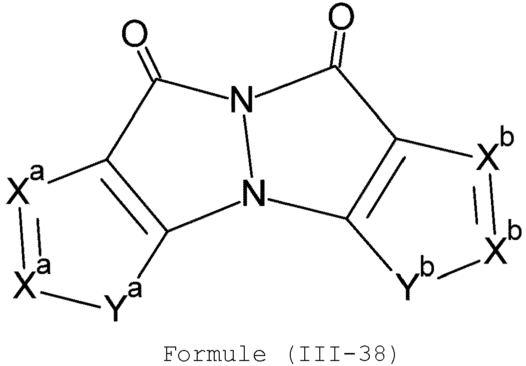 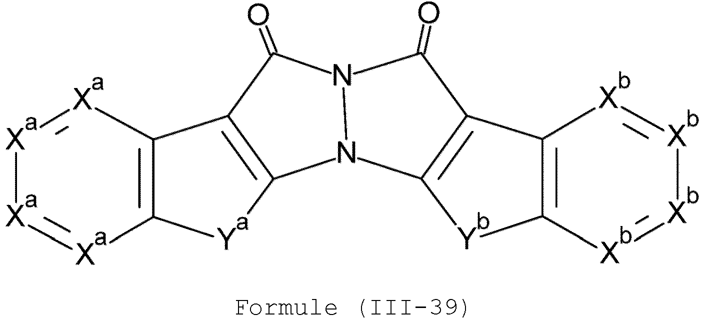 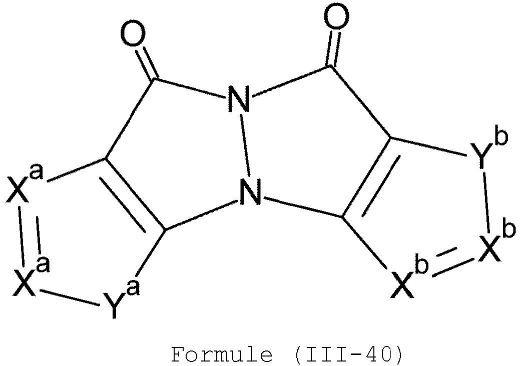 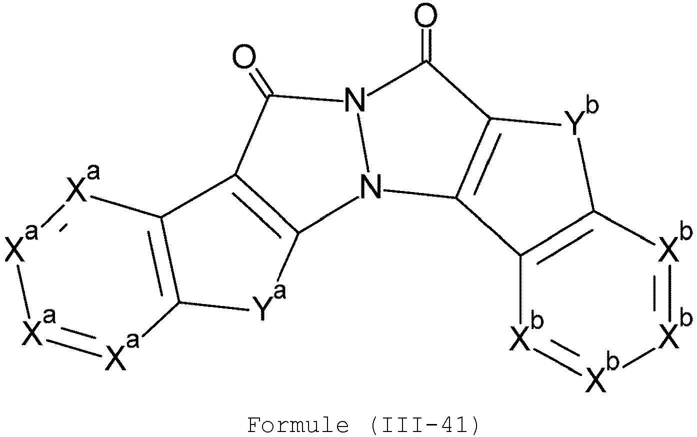 dans lequel les symboles Y¹, Y², Y^{a}, Y^{b}, X, X^{a} et X^{b} ont les significations données dans la revendication 2.

4. Composé selon l'une ou plusieurs des revendications 1 à 3, comprenant au moins une structure de formules (IV-1) à (IV-41), 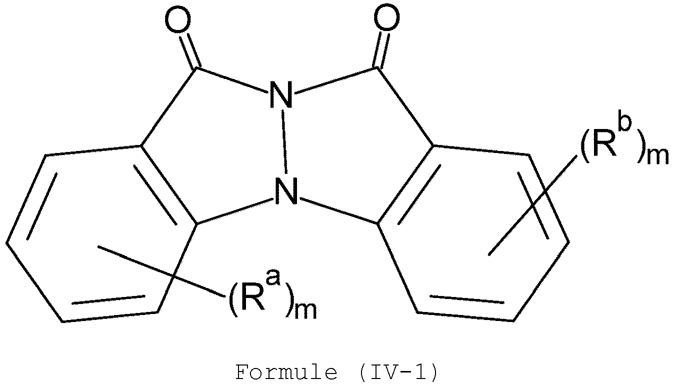 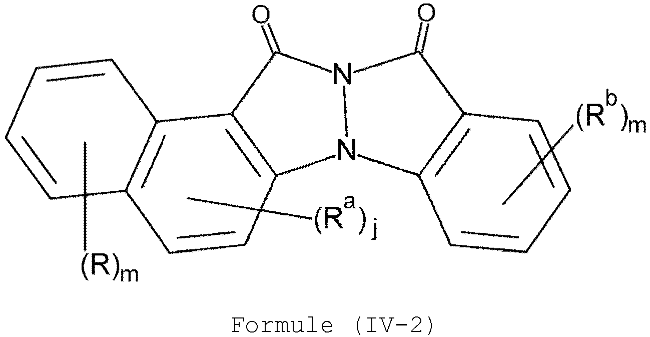 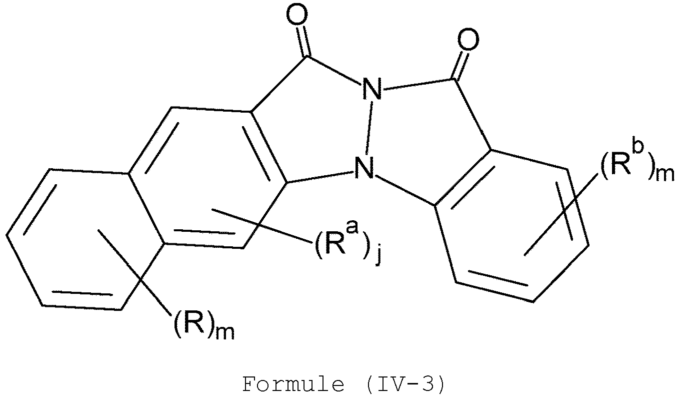 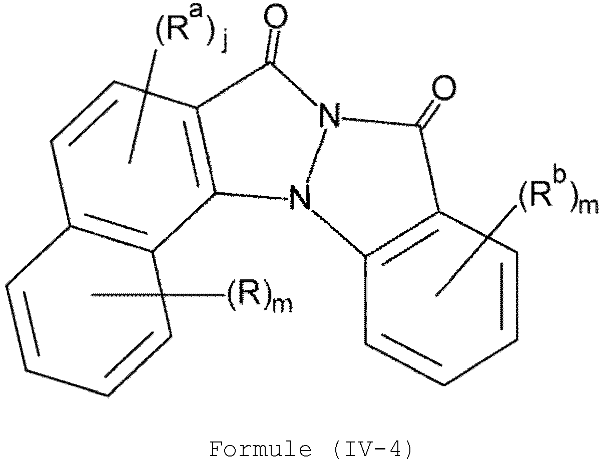 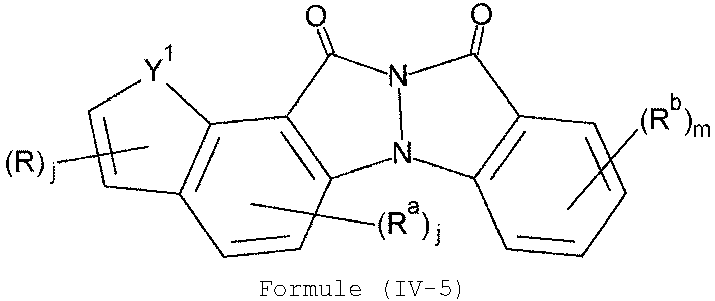 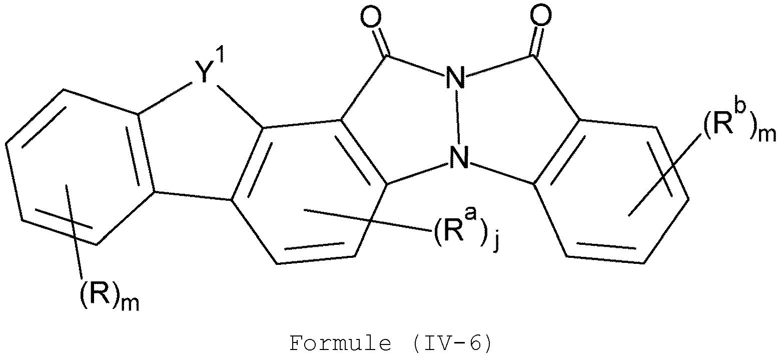 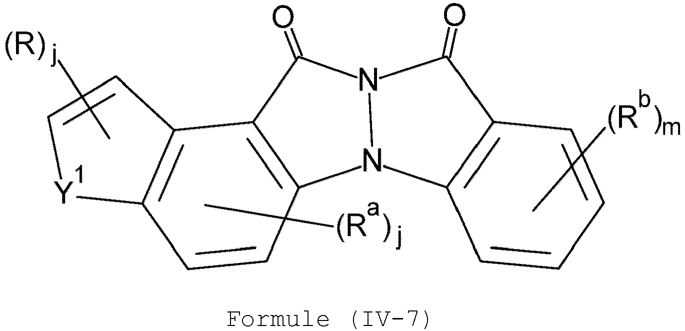 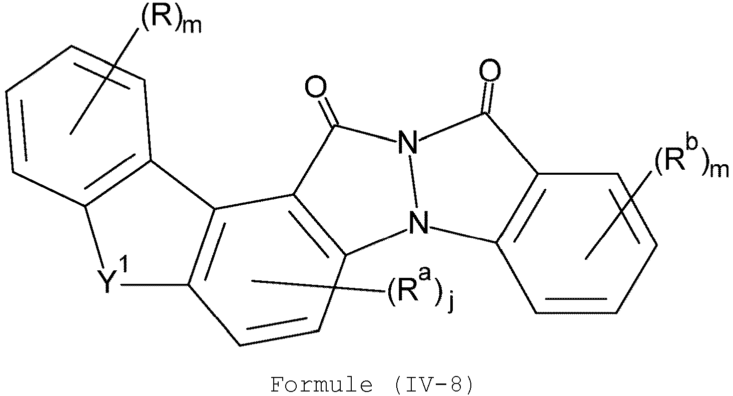 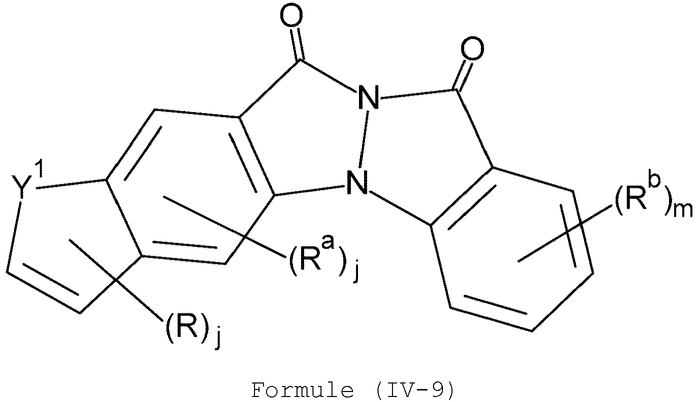 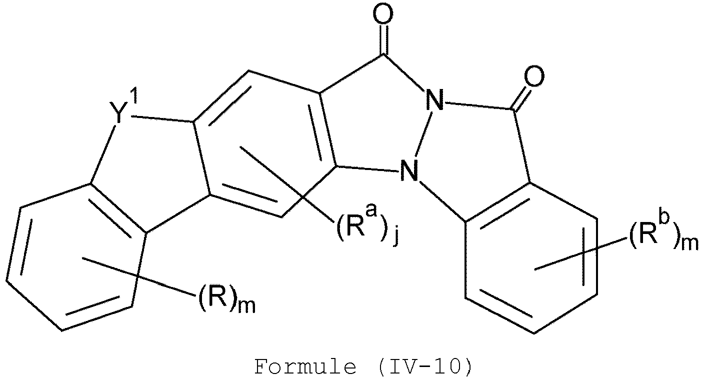 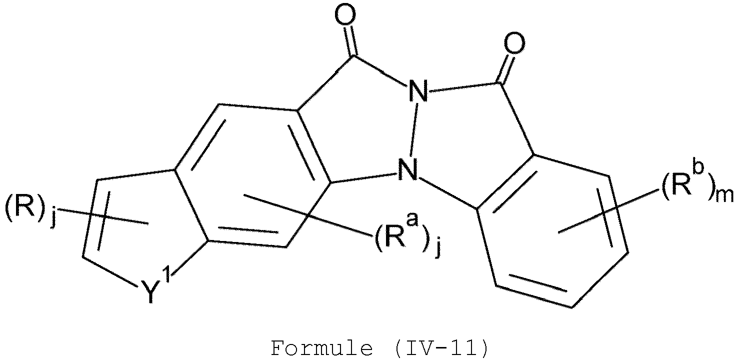 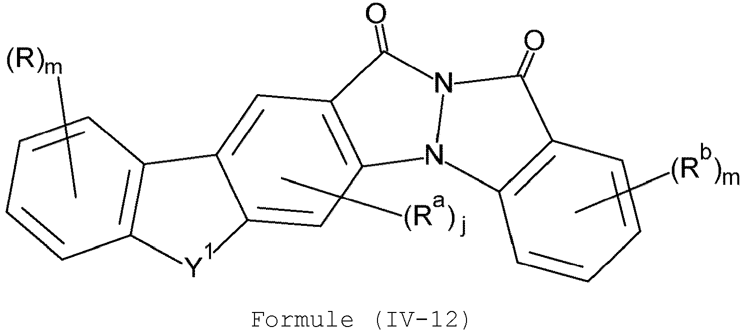 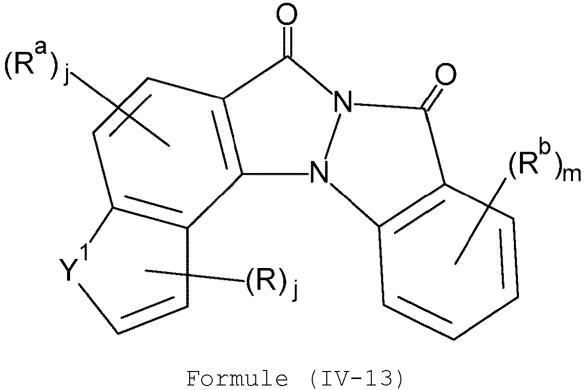 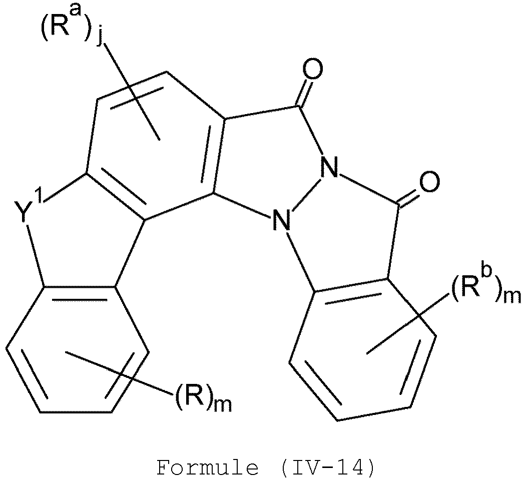 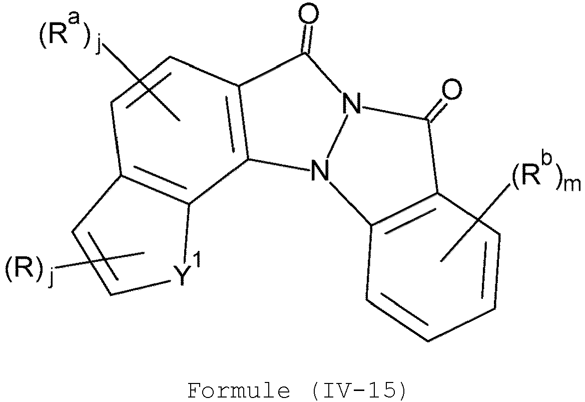 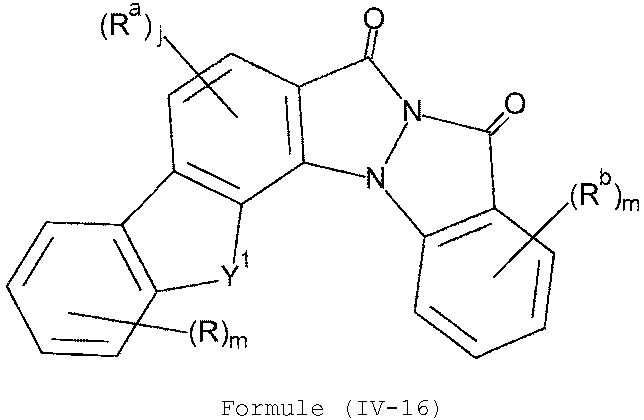 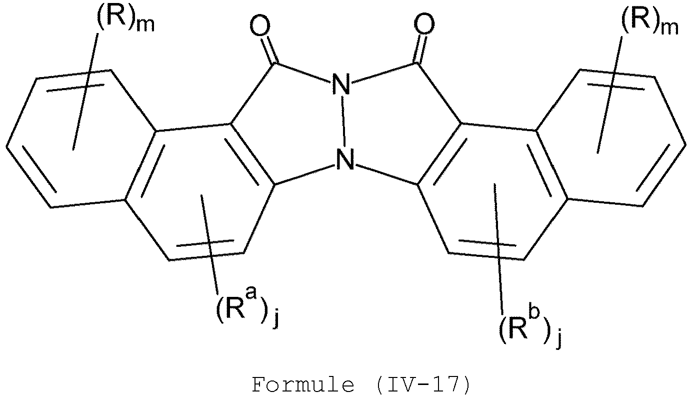 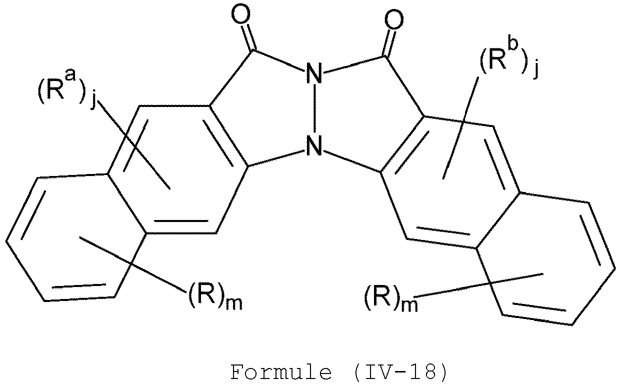 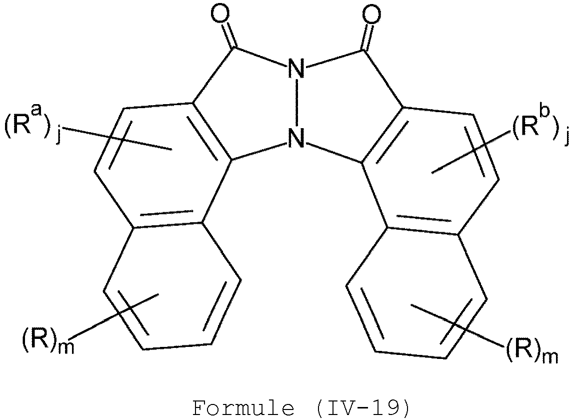 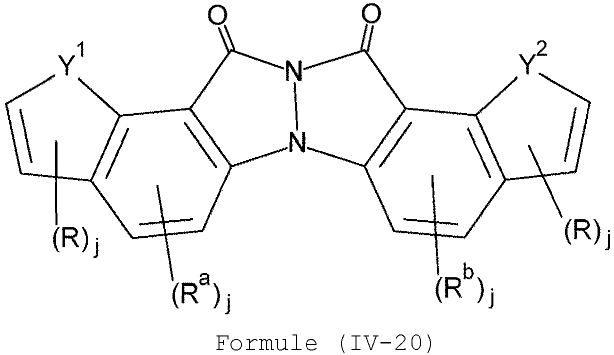 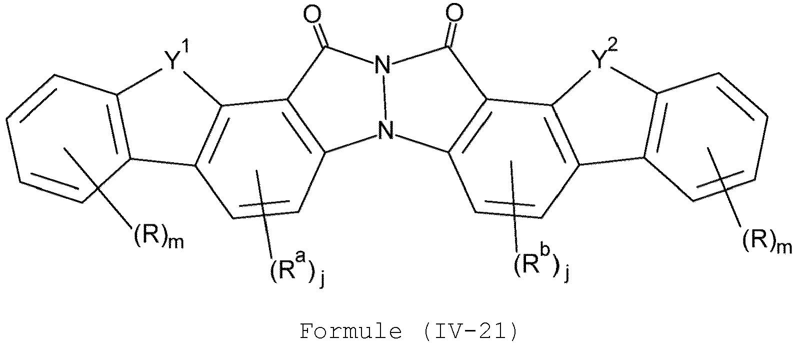 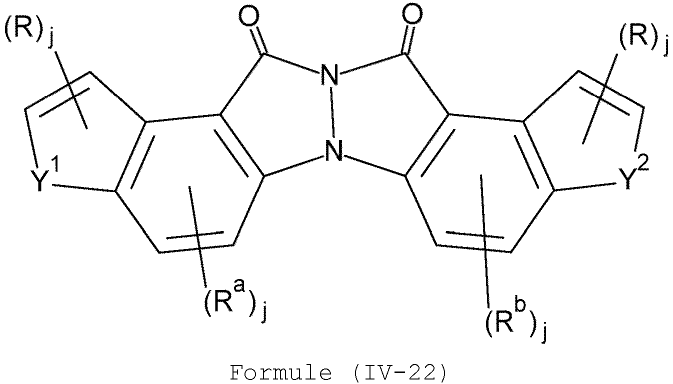 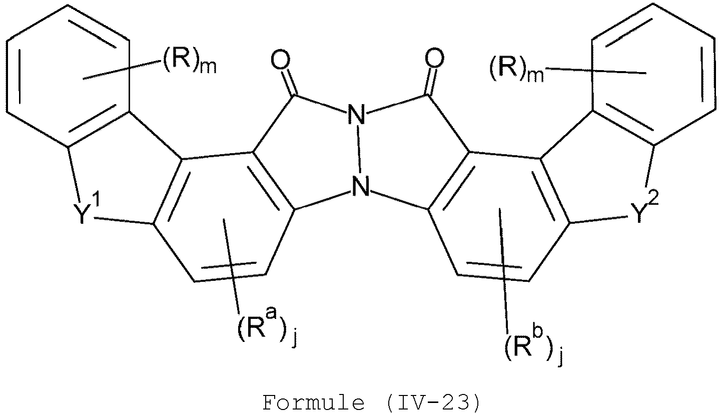 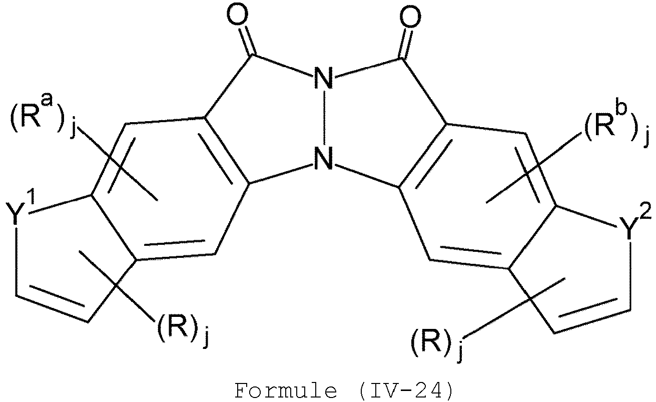 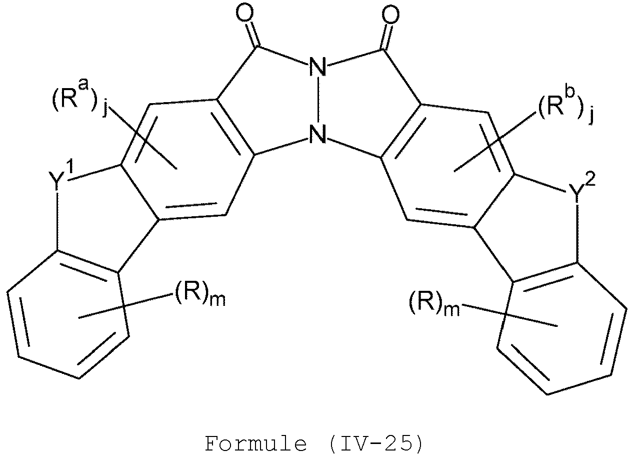 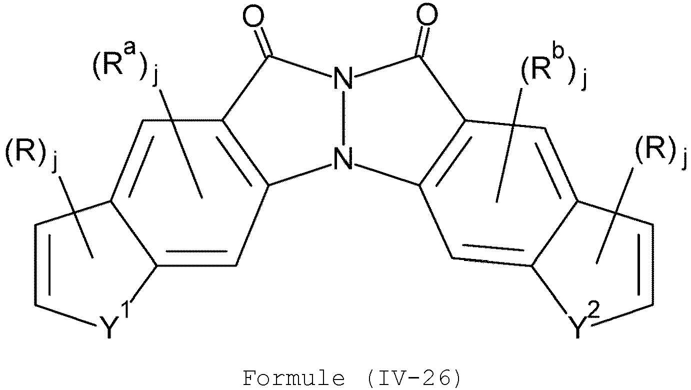 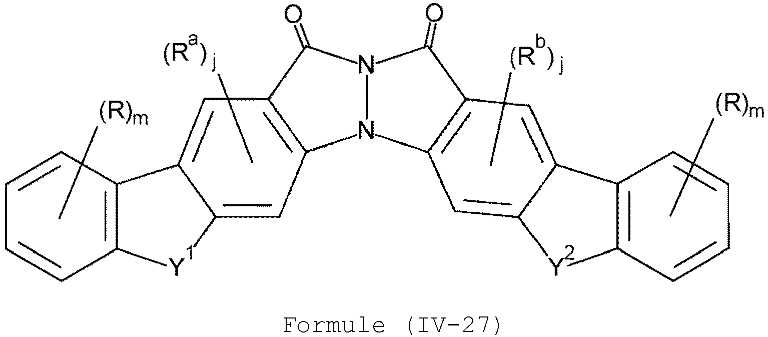 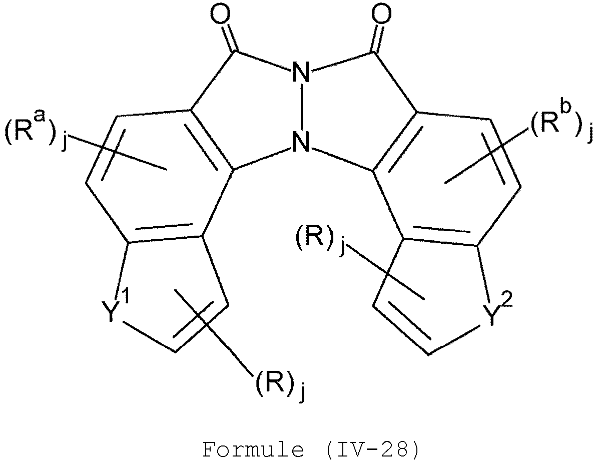 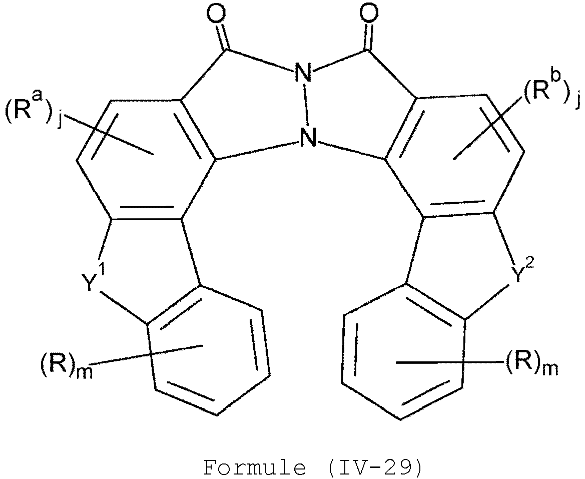 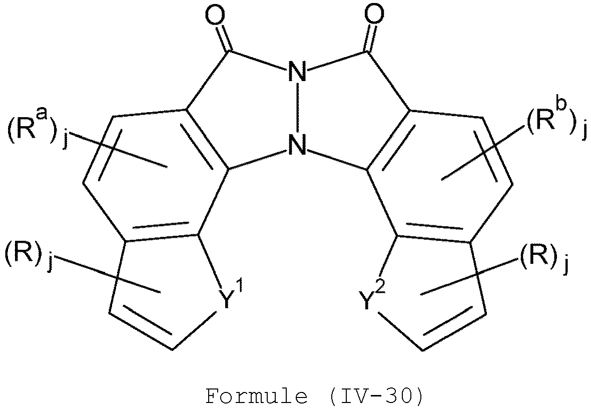 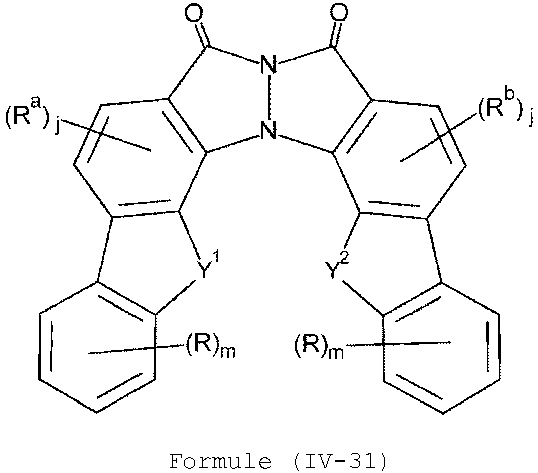 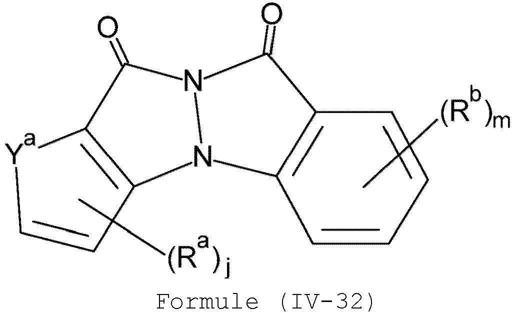 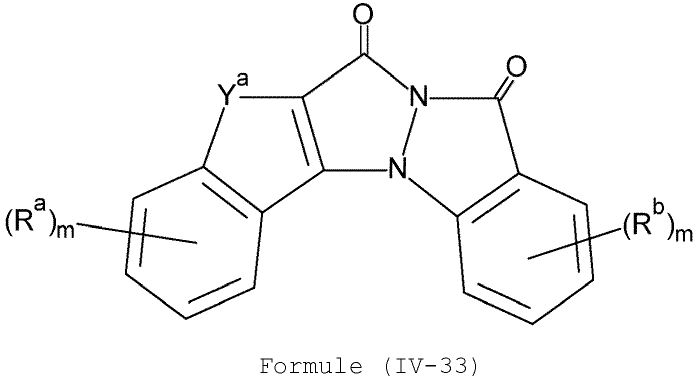 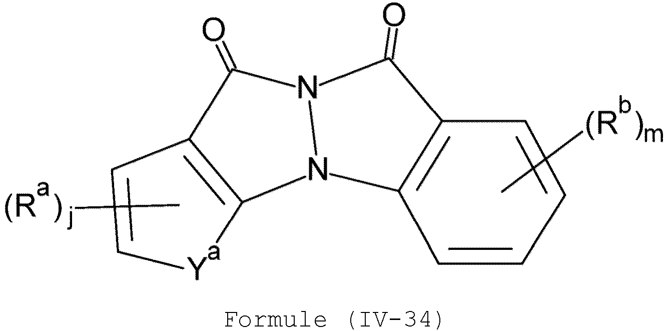 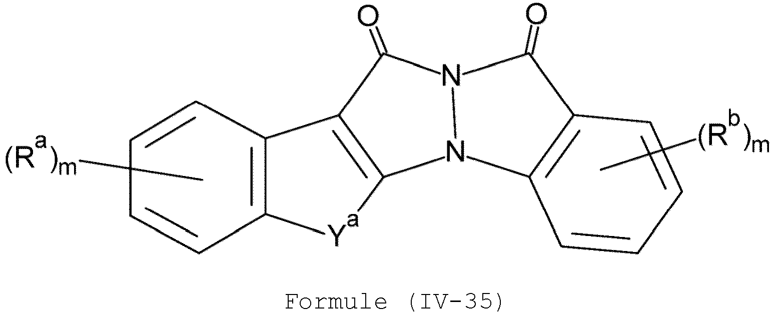 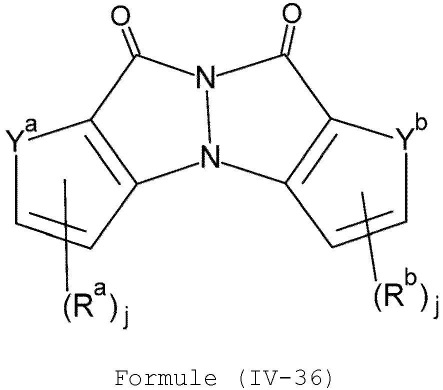 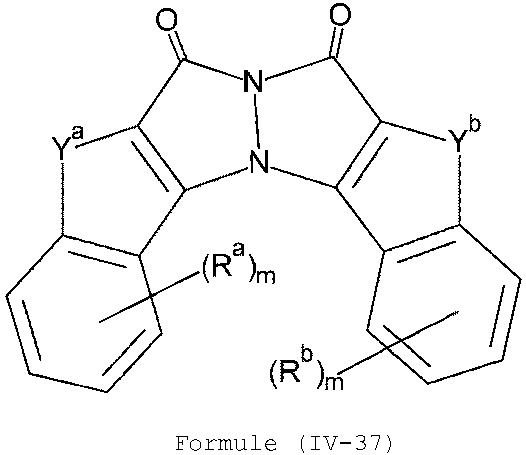 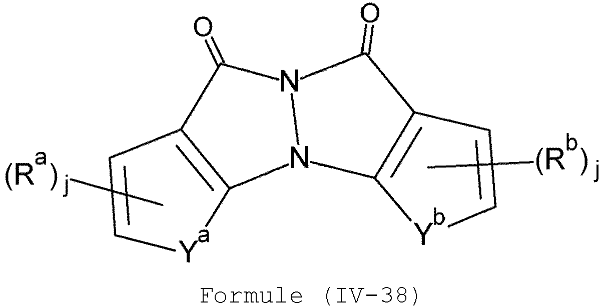 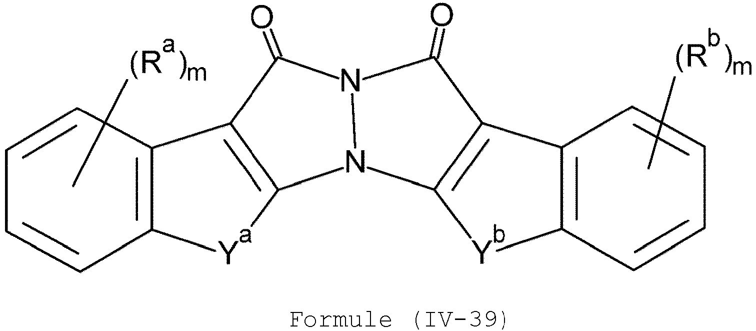 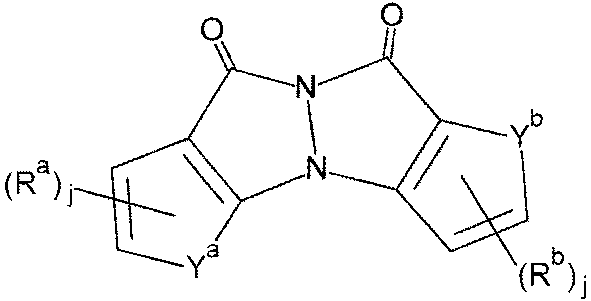 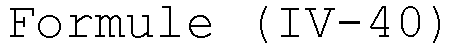 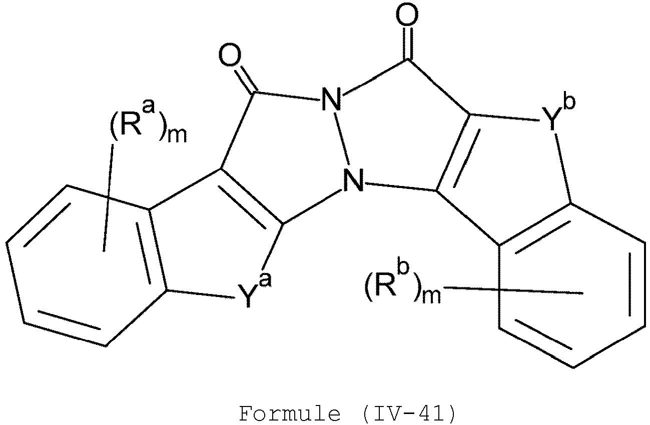 dans lequel les symboles R, R^{a} et R^{b} ont les significations données dans la revendication 1, les symboles Y¹, Y², Y^{a} et Y^{b} ont les significations données dans la revendication 2 et les autres symboles ont la signification suivante :
m représente 0, 1, 2, 3 ou 4 ;
j représente 0, 1 ou 2.

5. Composé selon l'une ou plusieurs des revendications 1 à 4, comprenant au moins une structure de Formules (V-1) à (V-8) 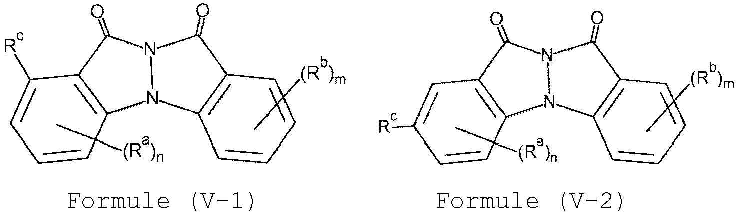 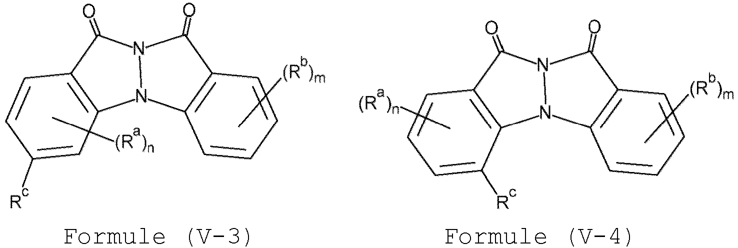 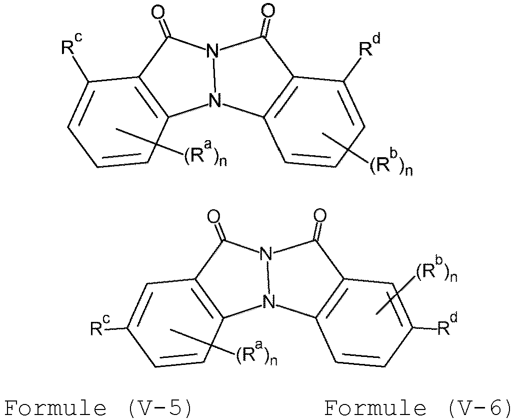 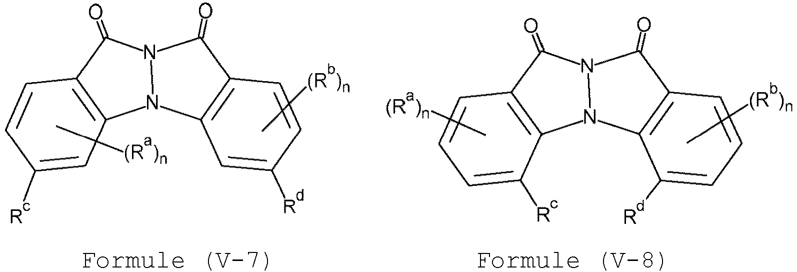 dans lequel les symboles R^{a} et R^{b} ont les significations données dans la revendication 1, et les autres symboles ont la signification suivante :
R^{c}, R^{d} sont, à chaque occurrence, identiques ou différents et représentent N(Ar')₂, N(R¹)₂, C(=O)N(Ar')₂, C(=O)N(R')₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, un groupe alkyle, alcoxy ou thioalcoxy à chaîne droite ayant 1 à 40 atomes de carbone ou un groupe alcényle ou alcynyle ayant 2 à 40 atomes de carbone ou un groupe alkyle, alcoxy ou thioalcoxy ramifié ou cyclique ayant 3 à 20 atomes de carbone, chaque groupe alkyle, alcoxy, thioalcoxy, alcényle ou alcynyle pouvant être substitué par un ou plusieurs radicaux R¹, un ou plusieurs groupes CH₂ non voisins pouvant être remplacés par R¹C=CR¹, C≡C, Si(R¹)₂, C=O, C=S, C=Se, C=NR¹, -C(=O)O-, -C(=O)NR¹-, NR¹, P(=O)(R¹), -O-, -S-, SO ou SO₂, ou un système cyclique aromatique ou hétéroaromatique ayant 5 à 60 atomes nucléaires aromatiques, dont chacun peut être substitué par un ou plusieurs radicaux R¹, ou un groupe aryloxy ou hétéroaryloxy ayant 5 à 60 atomes nucléaires aromatiques, qui peut être substitué par un ou plusieurs radicaux R¹ ; ou un groupe hétéroarylthio ayant 5 à 60 atomes nucléaires aromatiques, qui peut être substitué par un ou plusieurs radicaux R¹, ou un groupe diarylamino, arylhétéroarylamino, dihétéroarylamino ayant 5 à 60 atomes nucléaires aromatiques, qui peut être substitué par un ou plusieurs radicaux R¹, ou un groupe arylalkyle ou hétéroarylalkyle ayant 5 à 60 atomes nucléaires aromatiques et 1 à 10 atomes de carbone dans le radical alkyle, qui peut être substitué par un ou plusieurs radicaux R¹ ; un radical R^{c} formant avec un radical R, R^{a}, R^{b}, R^{d} ou un autre groupe un système cyclique ; un radical R^{d} formant avec un radical R, R^{a}, R^{b}, R^{c} ou un autre groupe un système cyclique ;
m représente 0, 1, 2, 3 ou 4 ;
n représente 0, 1, 2 ou 3.

6. Composé selon l'une ou plusieurs des revendications 1 à 5, **caractérisé en ce qu'**au moins un radical R, R^{a}, R^{b}, R^{c}, R^{d} représente un groupe alkyle, alcoxy ou thioalcoxy à chaîne droite ayant 1 à 40 atomes de carbone ou un groupe alcényle ou alcynyle ayant 2 à 40 atomes de carbone ou un groupe alkyle, alcoxy ou thioalcoxy ramifié ou cyclique ayant 3 à 20 atomes de carbone, chaque groupe alkyle, alcoxy, thioalcoxy, alcényle ou alcynyle pouvant être substitué par un ou plusieurs radicaux R¹, un ou plusieurs groupes CH₂ non voisins pouvant être remplacés par R¹C=CR¹, C=C, Si(R¹)₂, C=O, C=S, C=Se, C=NR¹, -C(=O)O-, -C(=O)NR¹-, NR¹, P(=O) (R¹), -O-, -S-, SO ou SO₂, ou un système cyclique aromatique ou hétéroaromatique ayant 5 à 60 atomes nucléaires aromatiques, dont chacun peut être substitué par un ou plusieurs radicaux R¹.

7. Composé selon l'une ou plusieurs des revendications 1 à 6, **caractérisé en ce qu'**au moins deux radicaux R, R^{a}, R^{b}, R^{c}, R^{d} forment avec les autres groupes auxquels sont liés les deux radicaux R, R^{a}, R^{b}, R^{c}, R^{d} un cycle condensé, les deux radicaux R, R^{a}, R^{b}, R^{c}, R^{d} formant au moins une structure de formules (RA-1) à (RA-12) 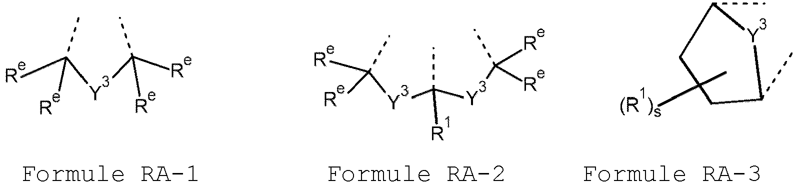 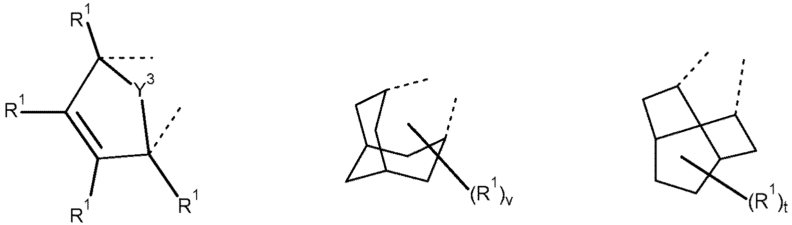 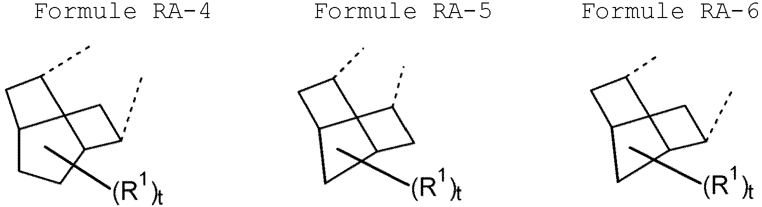 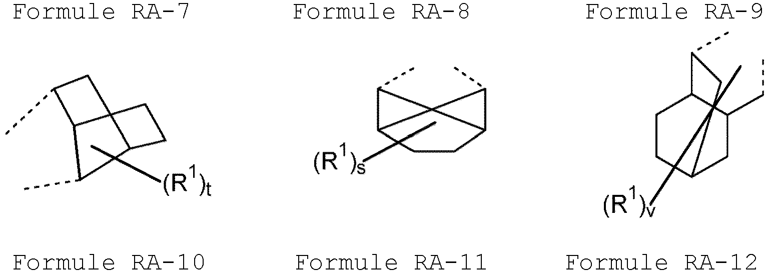 dans lequel R¹ a la signification donnée ci-dessus, les liaisons en tirets représentent les points de liaison par lesquels les deux radicaux R, R^{a}, R^{b}, R^{c}, R^{d} se lient aux autres groupes, et les autres symboles ont la signification suivante :
Y³ est à chaque occurrence identique ou différent et représente C(R¹)₂, (R¹)₂C-C(R¹)₂, (R¹)C=C(R¹), NR¹, NAr', O ou S ;
R^{e} est, à chaque occurrence, identique ou différent et représente F, un groupe alkyle, alcoxy ou thioalcoxy à chaîne droite ayant 1 à 40 atomes de carbone ou un groupe alcényle ou alcynyle ayant 2 à 40 atomes de carbone ou un groupe alkyle, alcoxy ou thioalcoxy ramifié ou cyclique ayant 3 à 20 atomes de carbone, chaque groupe alkyle, alcoxy, thioalcoxy, alcényle ou alcynyle pouvant être substitué par un ou plusieurs radicaux R², un ou plusieurs groupes CH₂ non voisins pouvant être remplacés par R²C=CR², C≡C, Si(R²)₂, C=O, C=S, C=Se, C=NR², -C(=O)O-, -C(=O)NR²-, NR², P(=O) (R¹), -O-, -S-, SO ou SO₂, ou un système cyclique aromatique ou hétéroaromatique ayant 5 à 60 atomes nucléaires aromatiques, dont chacun peut être substitué par un ou plusieurs radicaux R², ou un groupe aryloxy ou hétéroaryloxy ayant 5 à 60 atomes nucléaires aromatiques, qui peut être substitué par un ou plusieurs radicaux R² ; deux radicaux R^{e} pouvant alors former aussi l'un avec l'autre, ou un radical R^{e} peut former avec un radical R¹ ou avec un autre groupe, un système cyclique ;
s représente 0, 1, 2, 3, 4, 5 ou 6 ;
t représente 0, 1, 2, 3, 4, 5, 6, 7 ou 8 ;
v représente 0, 1, 2, 3, 4, 5, 6, 7, 8 ou 9.

8. Composé selon l'une ou plusieurs des revendications 1 à 7, **caractérisé en ce qu'**au moins deux radicaux R, R^{a}, R^{b}, R^{c}, R^{d} forment avec les autres groupes auxquels se lient les deux radicaux R, R^{a}, R^{b}, R^{c}, R^{d} un cycle condensé, les deux radicaux R, R^{a}, R^{b}, R^{c}, R^{d} formant des structures de formule (RB), 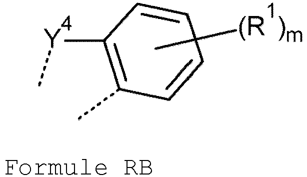 dans lequel R¹ a la signification donnée dans la revendication 1, les liaisons en tirets représentent les points de liaison par lesquels les deux radicaux R, R^{a}, R^{b}, R^{c}, R^{d} se lient aux autres groupes, l'indice m représente 0, 1, 2, 3 ou 4 et Y⁴ représente C(R¹)₂, NR¹, NAr', BR¹, BAr', O ou S.

9. Composé selon l'une ou plusieurs des revendications 1 à 8, comprenant au moins une structure de formules (VI-1) à (VI-22), les composés comportant au moins un cycle condensé, 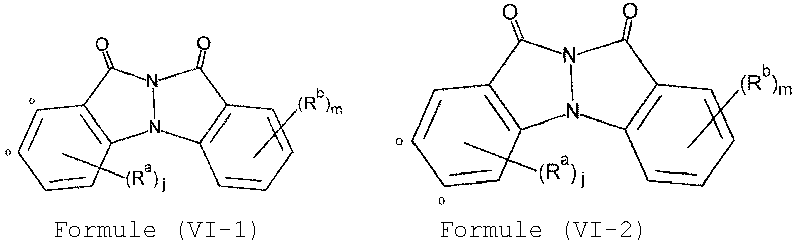 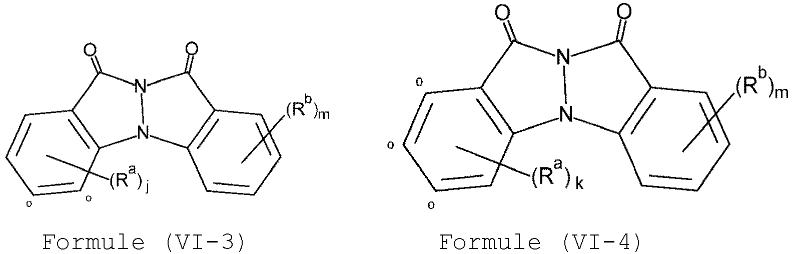 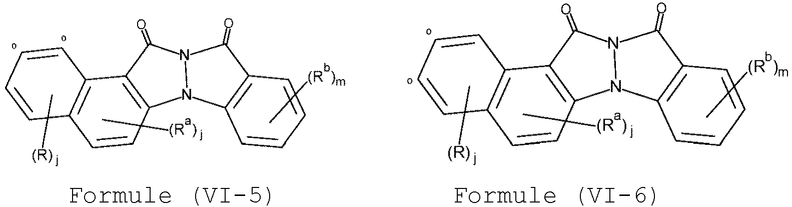 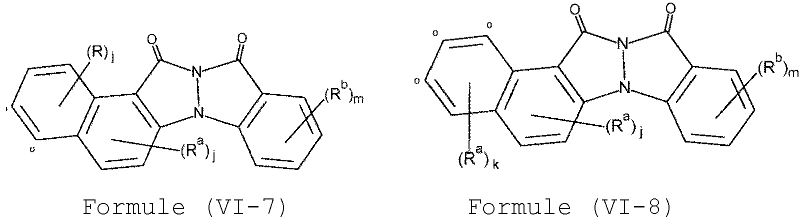 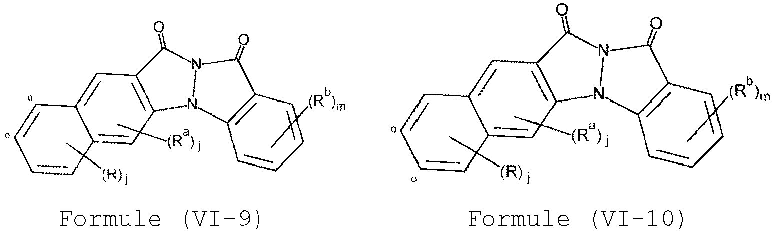 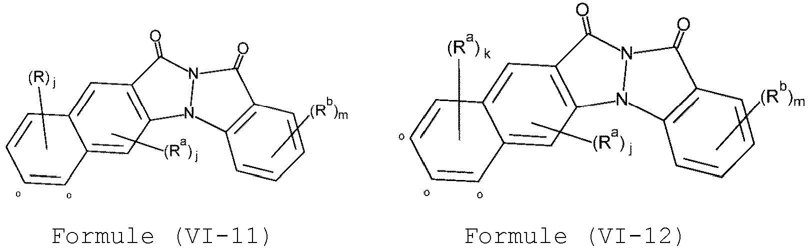 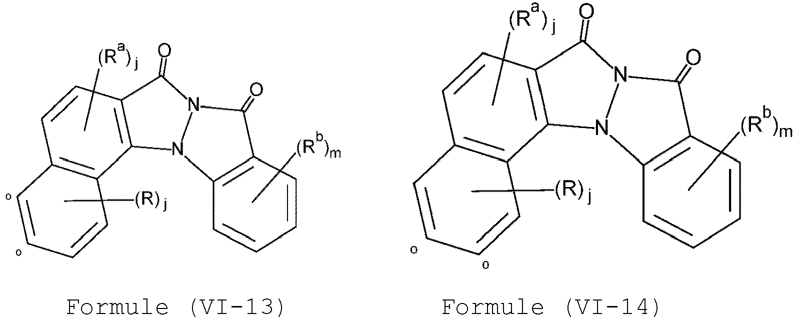 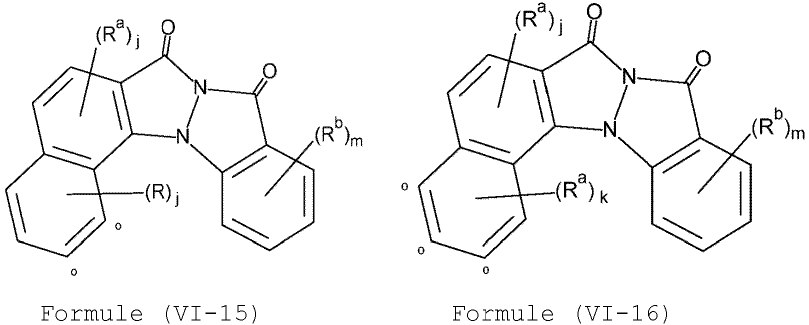 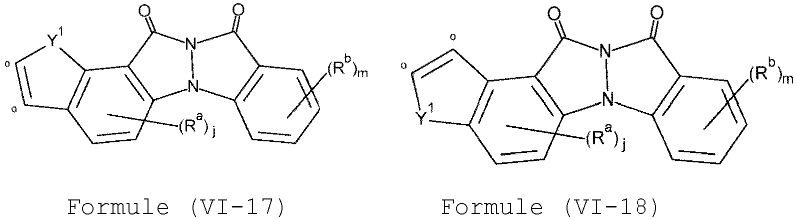 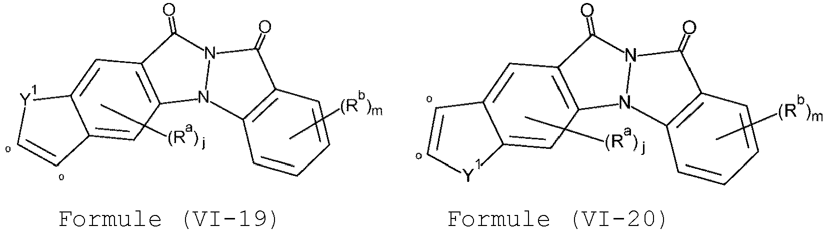 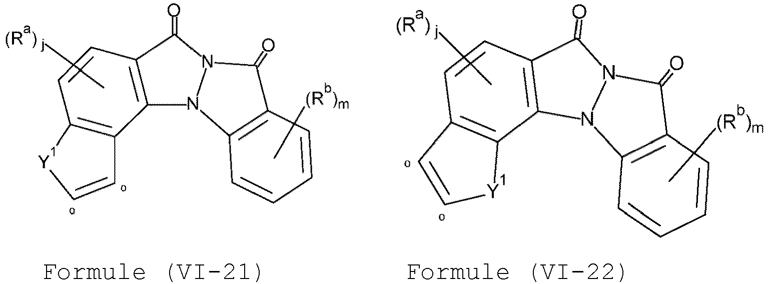 dans lequel les symboles R, R^{a}, R^{b}, Y¹ et Y² ont les significations données respectivement dans la revendication 1 et dans la revendication 2, le symbole o représente les points de liaison et les autres symboles ont la signification suivante :
m représente 0, 1, 2, 3 ou 4 ;
n représente 0, 1, 2 ou 3 ;
j représente 0, 1 ou 2.

10. Composé selon l'une ou plusieurs des revendications 1 à 9, **caractérisé en ce qu'**au moins un substituant R, R^{a}, R^{b} est à chaque occurrence identique ou différent et est choisi dans le groupe constitué de H, D, d'un groupe alkyle, alcoxy ou thioalcoxy ramifié ou cyclique ayant 3 à 20 atomes de carbone ou d'un système cyclique aromatique ou hétéroaromatique choisi dans les groupes de formules Ar-1 à Ar-78 suivants 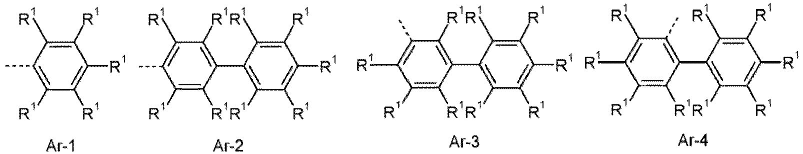 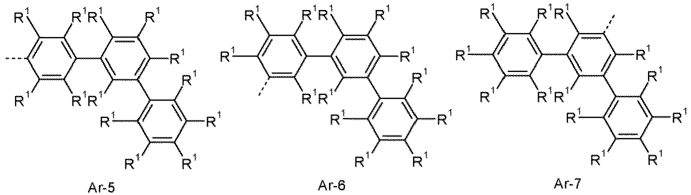 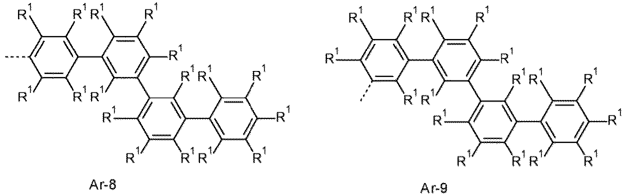 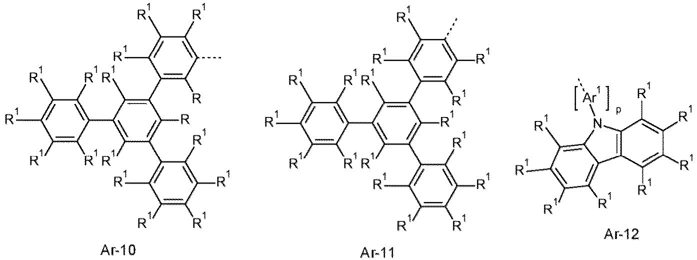 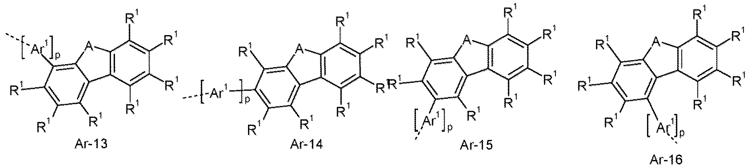 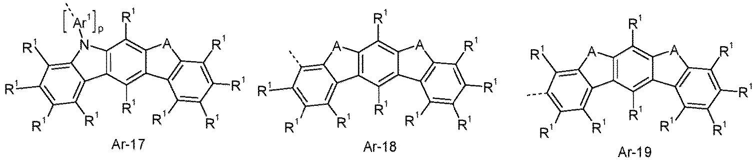 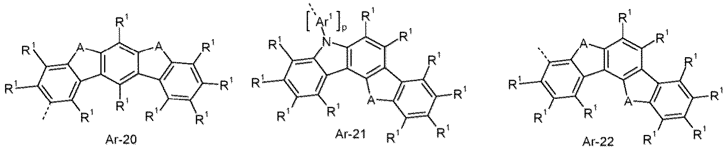 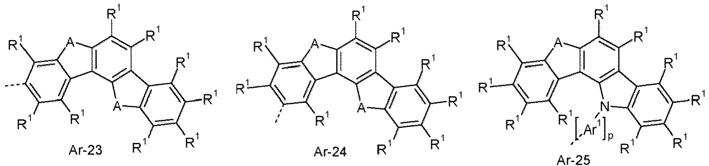 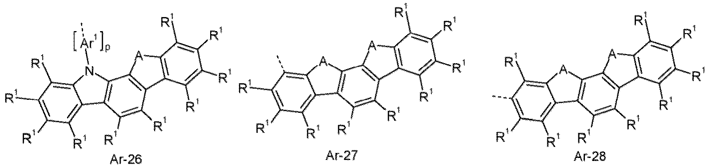 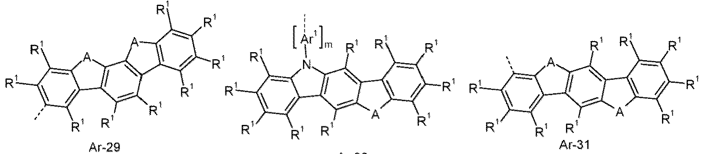 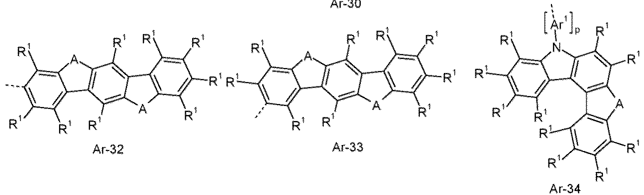 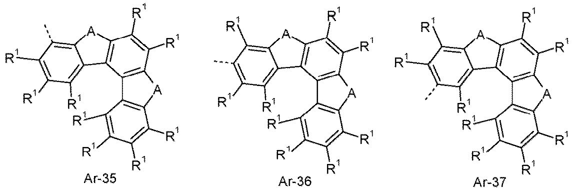 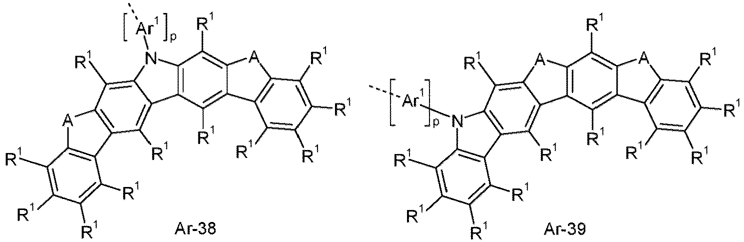 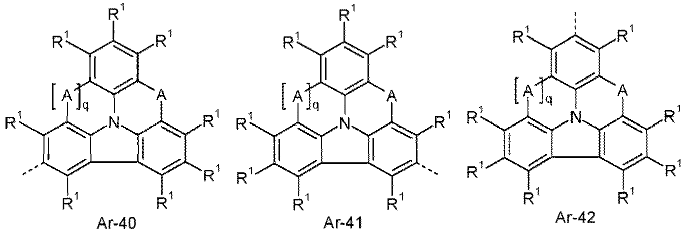 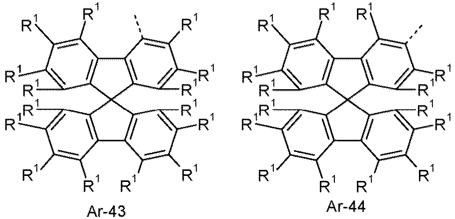 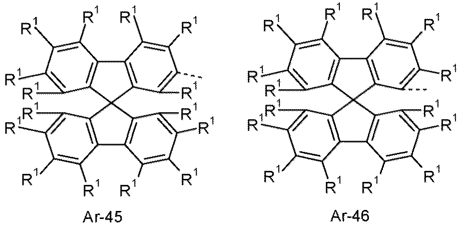 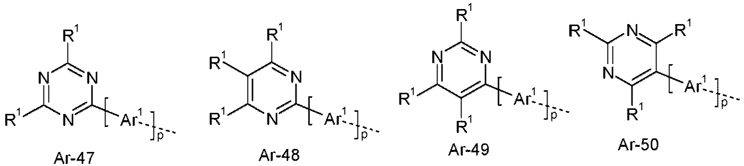 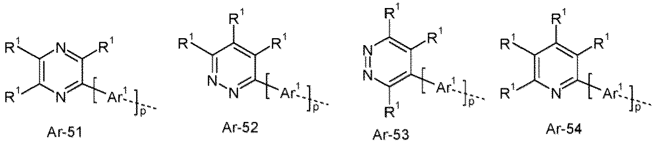 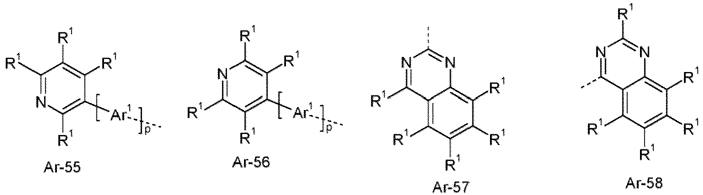 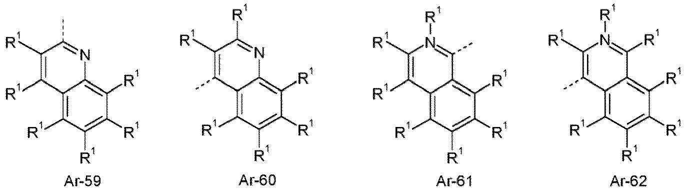 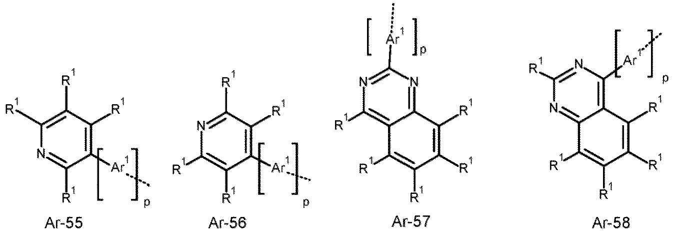 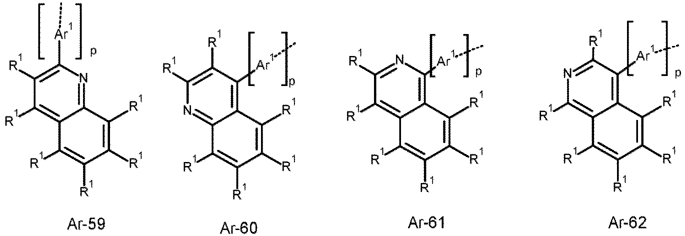 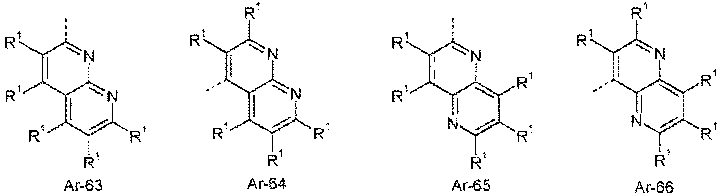 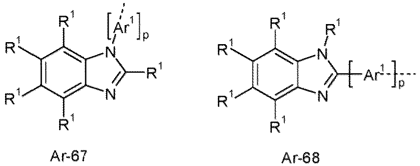 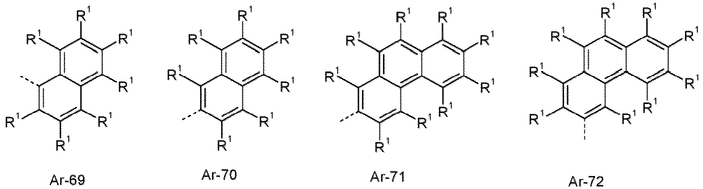 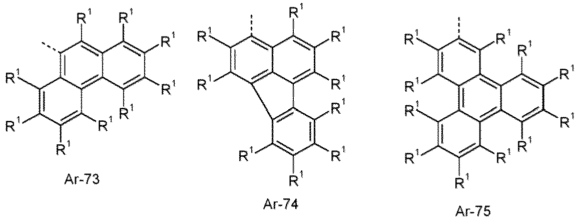 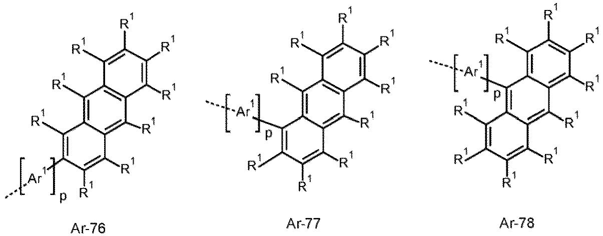 dans lequel R¹ a les significations données ci-dessus, la liaison en tirets représente la liaison au groupe correspondant, et en outre :
Ar¹ est, à chaque occurrence, identique ou différent et représente un système cyclique bivalent aromatique ou hétéroaromatique ayant 6 à 18 atomes nucléaires aromatiques, dont chacun peut être substitué par un ou plusieurs radicaux R¹ ;
A est, à chaque occurrence, identique ou différent et représente C(R¹)₂, NR¹, O ou S ;
p représente 0 ou 1, p = 0 signifiant que le groupe Ar¹ n'est pas présent et que le groupe aromatique ou hétéroaromatique correspondant est lié directement au radical correspondant ;
q représente 0 ou 1, q = 0 signifiant qu'aucun groupe A n'est lié à cette position, et que ce sont des radicaux R¹ qui, à la place, sont liés aux atomes de carbone correspondants.

11. Composé selon au moins l'une ou plusieurs des revendications 5 à 10, **caractérisé en ce que** le substituant R^{c}, R^{d} est, à chaque occurrence, identique ou différent et représente un système cyclique aromatique ou hétéroaromatique ayant 6 à 30 atomes nucléaires aromatiques, qui peut être substitué par un ou plusieurs radicaux R¹, qui est choisi parmi les groupes ayant les formules (Ar-1) à (Ar-78) présentées dans la revendication 10.

12. Composé selon au moins l'une des revendications 1 à 11, **caractérisé en ce que** le composé comprend exactement deux ou exactement trois structures de formules (I), (II-1) à (II-42), (III-1) à (III-41), (IV-1) à (IV-41), (V-1) à (V-8) et/ou (VI-1) à (VI-22).

13. Oligomère, polymère ou dendrimère contenant un ou plusieurs composés selon l'une des revendications 1 à 11, dans lequel, au lieu d'un atome d'hydrogène ou d'un substituant, une ou plusieurs liaisons des composés au polymère, à l'oligomère ou au dendrimère sont présentes.

14. Formulation contenant au moins un composé selon l'une ou plusieurs des revendications 1 à 12 ou un oligomère, un polymère ou un dendrimère selon la revendication 13 et au moins un autre composé, l'autre composé étant de préférence choisi parmi un ou plusieurs solvants.

15. Composition contenant au moins un composé de formule (I) 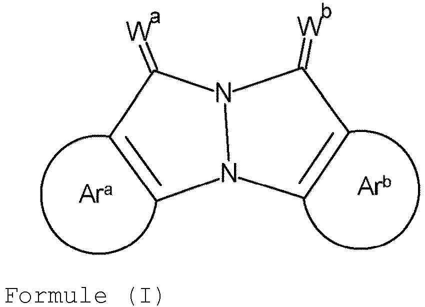 dans lequel les symboles utilisés ont les significations données dans la revendication 1, ou un oligomère, un polymère ou un dendrimère selon la revendication 13, et au moins un autre composé choisi dans le groupe consistant en les émetteurs fluorescents, les émetteurs phosphorescents, les émetteurs TADF, les matériaux hôtes, les matériaux de transport d'électrons, les matériaux d'injection d'électrons, les matériaux conducteurs de trous, les matériaux d'injection de trous, les matériaux bloqueurs d'électrons et les matériaux bloqueurs de trous.

16. Procédé de fabrication d'un composé selon l'une ou plusieurs des revendications 1 à 12, **caractérisé en ce qu'**on synthétise une charpente de base avec au moins l'un des groupes W^{a} ou un précurseur de l'un des groupes W^{a}, et on introduit un radical aromatique ou hétéroaromatique par une réaction de substitution aromatique nucléophile ou une réaction de couplage.

17. Utilisation d'un composé de formule (I) 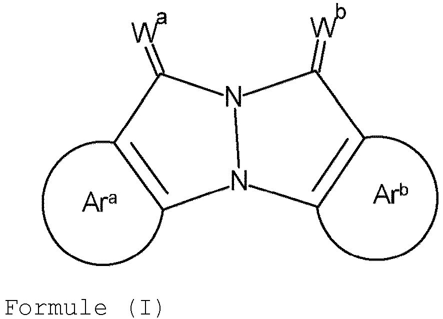 dans lequel les symboles utilisés ont les significations données dans la revendication 1, ou d'un oligomère, d'un polymère ou d'un dendrimère selon la revendication 13, dans un dispositif électronique.

18. Dispositif électronique contenant au moins un composé de formule (I) 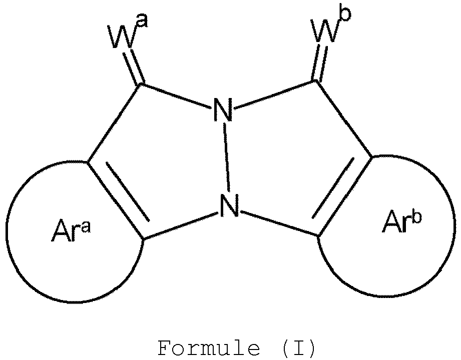 dans lequel les symboles utilisés ont les significations données dans la revendication 1, ou un oligomère, un polymère ou un dendrimère selon la revendication 13.
